# EUROPEAN PATENT SPECIFICATION

(11) **EP 4 212 521 B1**
(45) Date of publication and mention of the grant of the patent: **04.11.2026**
(21) Application number: 22211329.2
(22) Date of filing: 05.12.2022
(51) Int. Cl.: C07D 307/91, C07D 333/76, C07D 405/12, C07D 409/12, H10K 50/00, H10K 85/60, H10K 50/15, H10K 59/32, H10K 59/38

(54) **LIGHT EMITTING ELEMENT AND AMINE COMPOUND FOR THE SAME**
LICHTEMITTIERENDES ELEMENT UND AMINVERBINDUNG DAFÜR
ÉLÉMENT ÉLECTROLUMINESCENT ET COMPOSÉ AMINE POUR CELUI-CI

(30) Priority: 18.01.2022 KR 20220007282
(43) Date of publication of application: 19.07.2023
(73) Proprietor: Samsung Display Co., Ltd., Yongin-si, Gyeonggi-do 17113 (KR)
(72) Inventor: JIN, XiuLan, Yokohama (JP); SAKAMOTO, Naoya, Yokohama (JP)
(74) Representative: Russell, Tim

(56) References cited:
- EP-A1- 3 401 313
- EP-A1- 3 502 107
- EP-A1- 3 518 303
- EP-A1- 3 518 304
- WO-A1-2021/261946
- KR-B1- 102 022 391

## Description

### BACKGROUND

### 1. Field

The present disclosure herein relates to an amine compound and a light emitting element including the same, and for example, to a light emitting element including a novel amine compound in a hole transport region.

### 2. Description of the Related Art

Recently, the development of an organic electroluminescence display device as an image display device is being actively conducted. The organic electroluminescence display device includes a so-called self-luminescent light emitting element in which holes and electrons injected from a first electrode and a second electrode recombine in an emission layer so that a light emitting material in the emission layer emits light to achieve display (e.g., to display an image).
WO2021261946A1 provides a heterocyclic compound of [Formula 1] for use in an organic light-emitting device, and an organic light-emitting device in which the heterocyclic compound is contained in an organic material layer. 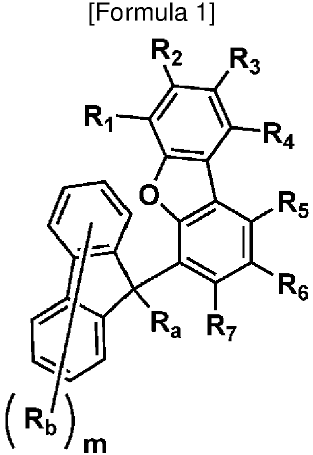 EP3502107A1 relates to an amine-based compound and an organic light-emitting device comprising the same are provided. The amine-based compound is represented by one of Formulae 1A and 1B: 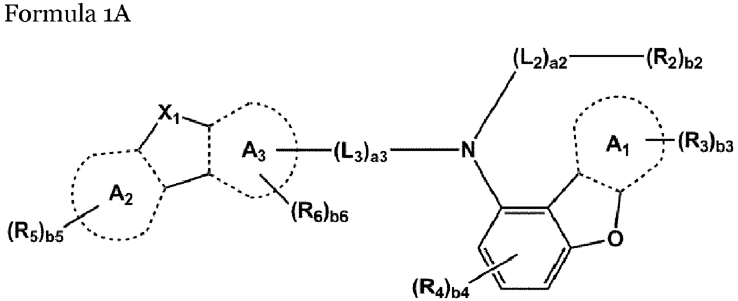 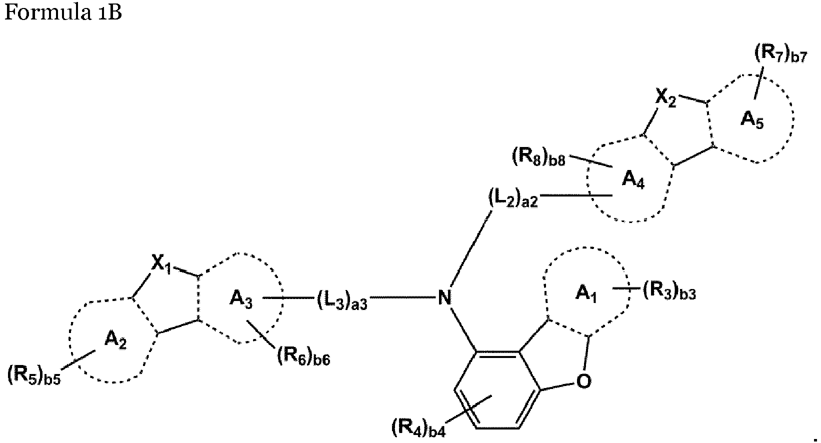 KR102022391B1 relates to an amine compound and an organic light emitting device including the same are disclosed. The amine compound is represented by one of Formulae 1A and 1B:

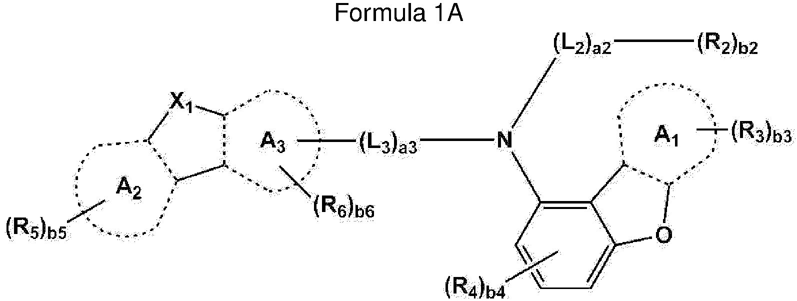

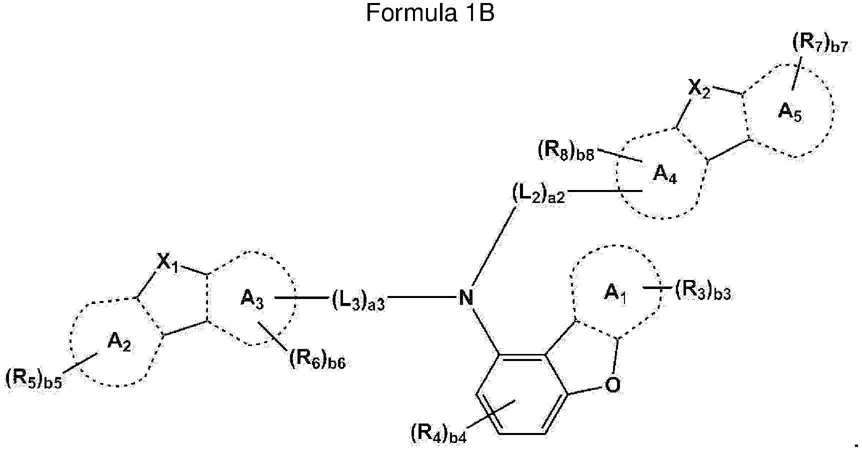

EP3401313A1 relates to an amine compound is represented by Formula 1:

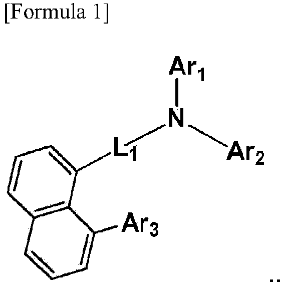

EP3518303A1 relates to an organic electroluminescence device and a monoamine compound represented by the following Formula 1 for an organic electroluminescence device:

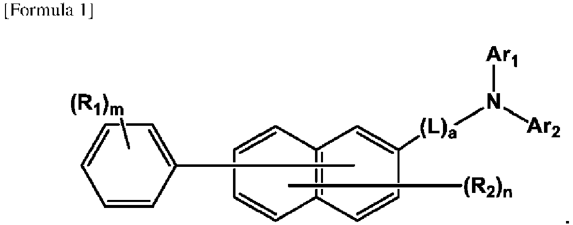

EP3518304A1 relates to an organic electroluminescence device and a monoamine compound by the following Formula 1 for an organic electroluminescence device:

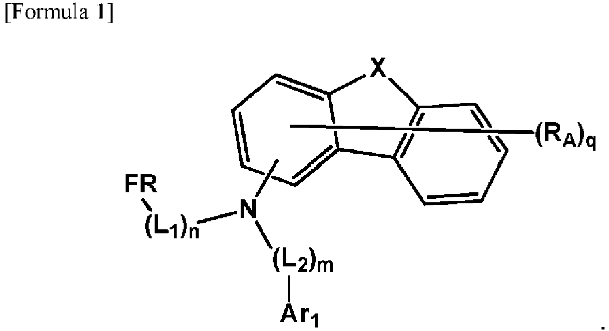

### SUMMARY

The invention is defined by the claims.

Aspects according to embodiments of the present disclosure are directed toward a light emitting element showing excellent or suitable emission efficiency and long-life characteristics.

Aspects according to embodiments of the present disclosure are directed toward an amine compound which is a material for a light emitting element having high efficiency and long-life characteristics.

Additional aspects will be set forth in part in the description which follows and, in part, will be apparent from the description, or may be learned by practice of the presented embodiments of the disclosure.

According to the present disclosure, an amine compound is represented by Formula 1.

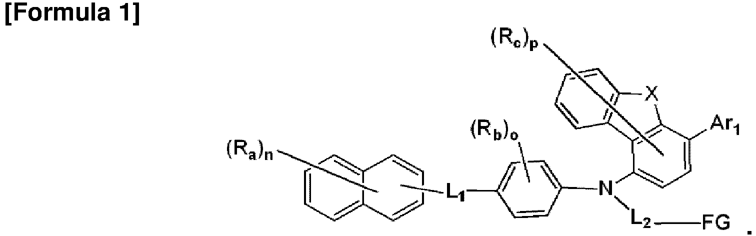

In Formula 1, X may be O or S, L₁ and L₂ may each independently be a direct linkage, a substituted or unsubstituted arylene group of 6 to 15 ring-forming carbon atoms, or a substituted or unsubstituted heteroarylene group of 1 to 12 ring-forming carbon atoms. "n" may be an integer of 0 to 7, "o" may be an integer of 0 to 4, and "p" may be an integer of 0 to 6. Rₐ, R_{b} and R_{c} may each independently be a deuterium atom, a halogen atom, a substituted or unsubstituted alkyl group of 1 to 10 carbon atoms, a substituted or unsubstituted aryl group of 6 to 30 ring-forming carbon atoms, or a substituted or unsubstituted heteroaryl group of 1 to 30 ring-forming carbon atoms, Ar₁ may be a substituted or unsubstituted aryl group of 6 to 40 ring-forming carbon atoms, or a substituted or unsubstituted heteroaryl group of 1 to 40 ring-forming carbon atoms, and FG may be represented by any one of Formula 2 to Formula 5. In the invention, Ar₁ is a substituted or unsubstituted phenyl group, a substituted or unsubstituted naphthyl group, a substituted or unsubstituted phenanthrenyl group, a substituted or unsubstituted dibenzothiophene group, or a substituted or unsubstituted dibenzofuran group.

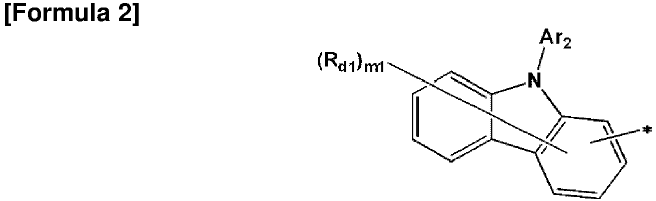

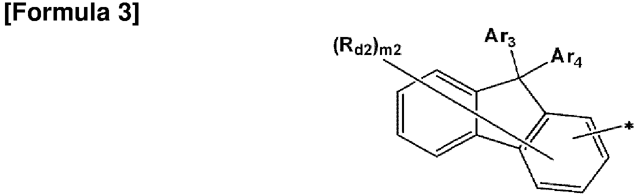

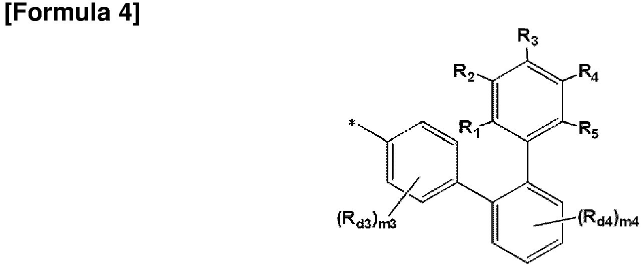

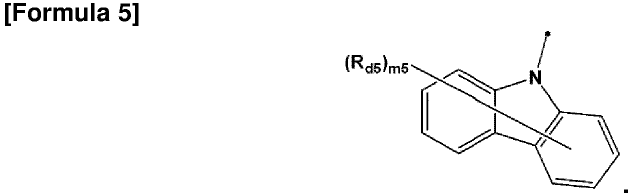

In Formula 2 and Formula 3, Ar₂ to Ar₄ may each independently be a substituted or unsubstituted aryl group of 6 to 40 ring-forming carbon atoms, or a substituted or unsubstituted heteroaryl group of 1 to 40 ring-forming carbon atoms. In Formula 2 to Formula 5, R_{d1} to R_{d5} may each independently be a deuterium atom, a halogen atom, a substituted or unsubstituted alkyl group of 1 to 20 carbon atoms, a substituted or unsubstituted alkenyl group of 2 to 20 carbon atoms, a substituted or unsubstituted oxy group, a substituted or unsubstituted thio group, a substituted or unsubstituted amine group, a substituted or unsubstituted aryl group of 6 to 30 ring-forming carbon atoms, a substituted or unsubstituted heteroaryl group of 1 to 30 ring-forming carbon atoms, or combined with an adjacent group to form a hydrocarbon ring or a heterocycle. m1 and m2 may each independently be an integer of 0 to 7, m3 and m4 may each independently be an integer of 0 to 4, and m5 is an integer of 0 to 8. In Formula 4, R₁ to R₅ may each independently be a hydrogen atom, a deuterium atom, a halogen atom, a substituted or unsubstituted alkyl group of 1 to 20 carbon atoms, a substituted or unsubstituted alkenyl group of 2 to 20 carbon atoms, a substituted or unsubstituted oxy group, a substituted or unsubstituted thio group, a substituted or unsubstituted amine group, a substituted or unsubstituted aryl group of 6 to 30 ring-forming carbon atoms, a substituted or unsubstituted heteroaryl group of 1 to 30 ring-forming carbon atoms, or combined with an adjacent group to form a hydrocarbon ring or a heterocycle.

In an embodiment, the amine compound may be represented by any one of Formula 1-1 to Formula 1-4.

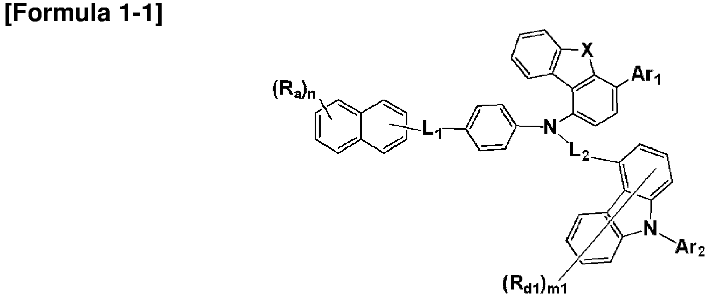

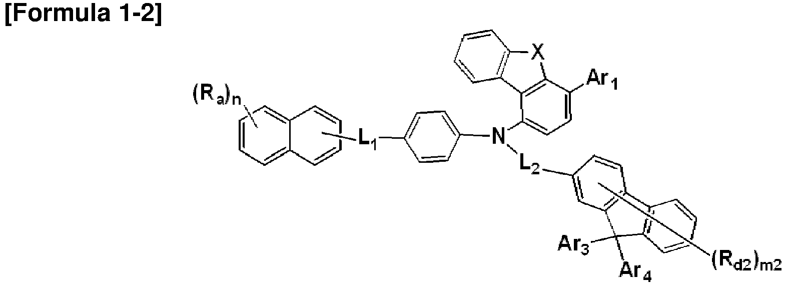

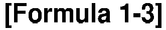

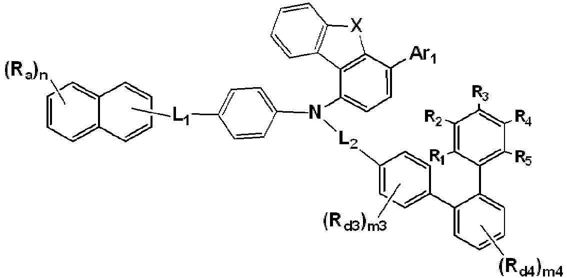

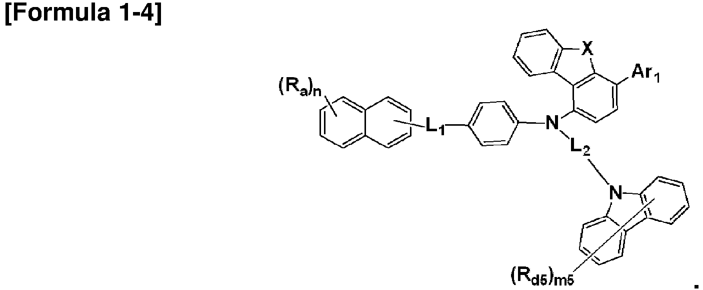

In Formula 1-1 to Formula 1-4, X, L₁, L₂, "n", Rₐ, and Ar₁ may each independently be the same as defined in Formula 1. In Formula 1-1 and Formula 1-2, Ar₂ to Ar₄ may each independently be the same as defined in Formula 2 and Formula 3. In Formula 1-3, R₁ to R₅ may each independently be the same as defined in Formula 4, and in Formula 1-1 to Formula 1-4, R_{d1} to R_{d5}, and m1 to m5 may each independently be the same as defined in Formula 2 to Formula 5.

In an embodiment, L₁ may be a direct linkage, an unsubstituted phenylene group, or an unsubstituted divalent biphenyl group.

In an embodiment, L₂ may be a direct linkage, an unsubstituted phenylene group, an unsubstituted naphthalene group, an unsubstituted divalent dibenzofuran group, or an unsubstituted divalent dibenzothiophene group.

In the invention, Ar₁ is a substituted or unsubstituted phenyl group, a substituted or unsubstituted naphthyl group, a substituted or unsubstituted phenanthrenyl group, a substituted or unsubstituted dibenzothiophene group, or a substituted or unsubstituted dibenzofuran group.

In an embodiment, Ar₁ may be represented by any one of Ar1-1 to Ar1-5.

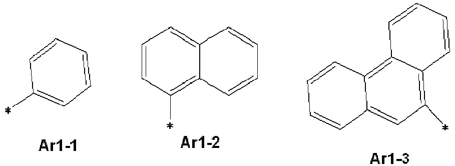

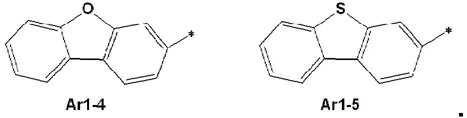

In an embodiment, in Formula 4, R₁ to R₅ may be combined with the benzene ring at which R₁ to R₅ are substituted, to form a dibenzofuran ring, a dibenzothiophene ring, or a carbazole ring.

In an embodiment, the amine compound may comprise a structure in which at least one hydrogen atom is substituted with a deuterium atom.

In an embodiment, the amine compound may be a monoamine compound.

In an embodiment, the amine compound may be any one of the compounds in Compound Group 1.

According to another embodiment of the present disclosure, a light emitting element comprises: a first electrode; a second electrode facing the first electrode; and at least one functional layer between the first electrode and the second electrode, and comprising the amine compound according to an embodiment.

In an embodiment, the at least one functional layer may comprise an emission layer, a hole transport region between the first electrode and the emission layer, and an electron transport region between the emission layer and the second electrode, and the hole transport region may comprise the amine compound according to an embodiment.

In an embodiment, the hole transport region may comprise at least one of a hole injection layer, a hole transport layer, and an electron blocking layer, and the at least one of the hole injection layer, the hole transport layer, and the electron blocking layer may comprise the amine compound of an embodiment.

In an embodiment, the emission layer may comprise a compound represented by Formula E-1.

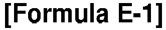

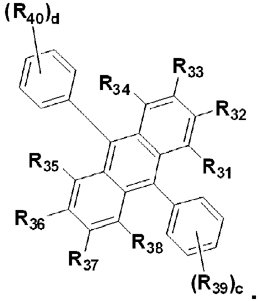

In Formula E-1, "c" and "d" may each independently be an integer of 0 to 5, and R₃₁ to R₄₀ may each independently be a hydrogen atom, a deuterium atom, a halogen atom, a substituted or unsubstituted silyl group, a substituted or unsubstituted thio group, a substituted or unsubstituted oxy group, a substituted or unsubstituted alkyl group of 1 to 10 carbon atoms, a substituted or unsubstituted alkenyl group of 2 to 10 carbon atoms, a substituted or unsubstituted aryl group of 6 to 30 ring-forming carbon atoms, a substituted or unsubstituted heteroaryl group of 2 to 30 ring-forming carbon atoms, or combined with an adjacent group to form a ring.

### BRIEF DESCRIPTION OF THE DRAWINGS

The accompanying drawings are included to provide a further understanding of the present disclosure and are incorporated in and constitute a part of this specification. The drawings illustrate embodiments of the present disclosure and, together with the description, serve to explain principles of the present disclosure. In the drawings:
FIG. 1 is a plan view showing a display apparatus according to an embodiment;
FIG. 2 is a cross-sectional view of a display apparatus according to an embodiment;
FIG. 3 is a cross-sectional view schematically showing a light emitting element of an embodiment;
FIG. 4 is a cross-sectional view schematically showing a light emitting element of an embodiment;
FIG. 5 is a cross-sectional view schematically showing a light emitting element of an embodiment;
FIG. 6 is a cross-sectional view schematically showing a light emitting element of an embodiment;
FIG. 7 is a cross-sectional view of a display apparatus according to an embodiment;
FIG. 8 is a cross-sectional view of a display apparatus according to an embodiment;
FIG. 9 is a cross-sectional view showing a display apparatus according to an embodiment; and
FIG. 10 is a cross-sectional view showing a display apparatus according to an embodiment.

### DETAILED DESCRIPTION

Like reference numerals refer to like elements throughout, and duplicative descriptions thereof may not be provided. In the drawings, the dimensions of structures may be exaggerated for clarity of illustration. It will be understood that, although the terms first, second, etc. may be used herein to describe various elements, these elements should not be limited by these terms. These terms are only used to distinguish one element from another element. Thus, a first element could be termed a second element without departing from the teachings of the present disclosure. Similarly, a second element could be termed a first element. As used herein, the singular forms are intended to include the plural forms as well, unless the context clearly indicates otherwise.

In the description, it will be further understood that the terms "comprises" and/or "comprising," when used in this specification, specify the presence of stated features, numerals, steps, operations, elements, parts, or the combination thereof, but do not preclude the presence or addition of one or more other features, numerals, steps, operations, elements, parts, or the combination thereof. As used herein, the singular forms "a", "an" and "the" are intended to include the plural forms as well, unless the context clearly indicates otherwise.

In the description, when a layer, a film, a region, a plate, etc. is referred to as being "on" or "above" another part, it can be "directly on" the other part, or one or more intervening layers may also be present. In contrast, when a layer, a film, a region, a plate, etc. is referred to as being "under" or "below" another part, it can be "directly under" the other part, or intervening layers may also be present. Also, when an element is referred to as being disposed "on" another element, it can be disposed on or under the other element.

In the description, *- represents a binding site to a neighboring atom.

In the description, the term "substituted or unsubstituted" corresponds to a functional group that is substituted or unsubstituted with at least one substituent selected from the group consisting of a deuterium atom, a halogen atom, a cyano group, a nitro group, an amino group, a silyl group, an oxy group, a thio group, a sulfinyl group, a sulfonyl group, a carbonyl group, a boron group, a phosphine oxide group, a phosphine sulfide group, an alkyl group, an alkenyl group, an alkynyl group, an alkoxy group, a hydrocarbon ring group, an aryl group, and a heterocyclic group. In addition, each of the exemplified substituents may be substituted or unsubstituted. For example, a biphenyl group may be interpreted as an aryl group or a phenyl group substituted with a phenyl group.

In the description, the term "combined with an adjacent group to form..." may refer to that a group is bonded to an adjacent group to form a substituted or unsubstituted hydrocarbon ring, or a substituted or unsubstituted heterocycle. The hydrocarbon ring includes an aliphatic hydrocarbon ring and an aromatic hydrocarbon ring. The heterocycle includes an aliphatic heterocycle and an aromatic heterocycle. The hydrocarbon ring and the heterocycle may be monocycles or polycycles. In addition, the ring formed via the combination with an adjacent group may be combined with another ring to form a spiro structure.

In the description, the term "adjacent group" may refer to a substituent substituted for an atom which is directly combined with an atom substituted with a corresponding substituent, another substituent substituted for an atom which is substituted with a corresponding substituent, or a substituent sterically positioned at the nearest position to a corresponding substituent. For example, in 1,2-dimethylbenzene, the two methyl groups may be interpreted as "adjacent groups" to each other, and in 1,1-diethylcyclopentene, the two ethyl groups may be interpreted as "adjacent groups" to each other. In addition, in 4,5-dimethylphenanthrene, the two methyl groups may be interpreted as "adjacent groups" to each other.

In the description, a halogen atom may be a fluorine atom, a chlorine atom, a bromine atom, or an iodine atom.

In the description, an alkyl group may be a linear or branched alkyl group. The carbon number of the alkyl group may be 1 to 50, 1 to 30, 1 to 20, 1 to 10, or 1 to 6. Non-limiting examples of the alkyl group may include a methyl group, an ethyl group, an n-propyl group, an isopropyl group, an n-butyl group, an s-butyl group, a t-butyl group, an i-butyl group, a 2-ethylbutyl group, a 3,3-dimethylbutyl group, an n-pentyl group, an i-pentyl group, a neopentyl group, a t-pentyl group, a 1-methylpentyl group, a 3-methylpentyl group, a 2-ethylpentyl group, a 4-methyl-2-pentyl group, an n-hexyl group, a 1-methylhexyl group, a 2-ethylhexyl group, a 2-butylhexyl group, an n-heptyl group, a 1-methylheptyl group, a 2,2-dimethylheptyl group, a 2-ethylheptyl group, a 2-butylheptyl group, an n-octyl group, a t-octyl group, a 2-ethyloctyl group, a 2-butyloctyl group, a 2-hexyloctyl group, a 3,7-dimethyloctyl group, an n-nonyl group, an n-decyl group, an adamantyl group, a 2-ethyldecyl group, a 2-butyldecyl group, a 2-hexyldecyl group, a 2-octyldecyl group, an n-undecyl group, an n-dodecyl group, a 2-ethyldodecyl group, a 2-butyldodecyl group, a 2-hexyldocecyl group, a 2-octyldodecyl group, an n-tridecyl group, an n-tetradecyl group, an n-pentadecyl group, an n-hexadecyl group, a 2-ethylhexadecyl group, a 2-butylhexadecyl group, a 2-hexylhexadecyl group, a 2-octylhexadecyl group, an n-heptadecyl group, an n-octadecyl group, an n-nonadecyl group, an n-eicosyl group, a 2-ethyleicosyl group, a 2-butyleicosyl group, a 2-hexyleicosyl group, a 2-octyleicosyl group, an n-henicosyl group, an n-docosyl group, an n-tricosyl group, an n-tetracosyl group, an n-pentacosyl group, an n-hexacosyl group, an n-heptacosyl group, an n-octacosyl group, an n-nonacosyl group, an n-triacontyl group, etc.

In the description, an alkenyl group refers to a hydrocarbon group including one or more carbon-carbon double bonds in the middle and/or at the terminal end of an alkyl group having 2 or more carbon atoms. The alkenyl group may be a linear chain or a branched chain. The carbon number is not specifically limited, but may be 2 to 30, 2 to 20, or 2 to 10. Examples of the alkenyl group may include a vinyl group, a 1-butenyl group, a 1-pentenyl group, a 1,3-butadienyl aryl group, a styrenyl group, a styrylvinyl group, etc., without limitation.

In the description, a hydrocarbon ring group refers to an optional functional group or substituent derived from an aliphatic hydrocarbon ring. The hydrocarbon ring group may be a saturated hydrocarbon ring group of 5 to 20 ring-forming carbon atoms.

In the description, an aryl group refers to an optional functional group or substituent derived from an aromatic hydrocarbon ring. The aryl group may be a monocyclic aryl group or a polycyclic aryl group. The number of ring-forming carbon atoms in the aryl group may be 6 to 30, 6 to 20, or 6 to 15. Non-limiting examples of the aryl group may include a phenyl group, a naphthyl group, a fluorenyl group, an anthracenyl group, a phenanthryl group, a biphenyl group, a terphenyl group, a quaterphenyl group, a quinquephenyl group, a sexiphenyl group, a triphenylenyl group, a pyrenyl group, a benzofluoranthenyl group, a chrysenyl, etc.

In the description, a fluorenyl group may be substituted, and two substituents may be combined with each other to form a spiro structure. Examples of a substituted fluorenyl group are as follows, but an embodiment of the present disclosure is not limited thereto.

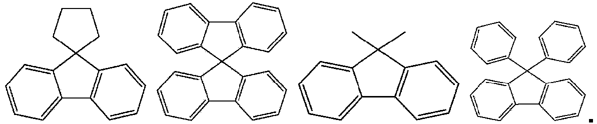

In the description, a heterocyclic group refers to an optional functional group or substituent derived from a ring including one or more from among B, O, N, P, Si, and S as ring-forming heteroatoms. The heterocyclic group includes an aliphatic heterocyclic group and an aromatic heterocyclic group. The aromatic heterocyclic group may be a heteroaryl group. The aliphatic heterocyclic group and the aromatic heterocyclic group may be a monocycle or a polycycle.

In the description, a heterocyclic group may include one or more from among B, O, N, P, Si and S as ring-forming heteroatoms. When the heterocyclic group includes two or more heteroatoms, the two or more heteroatoms may be the same or different. In the description, the heterocyclic group may be a monocyclic heterocyclic group or polycyclic heterocyclic group and may include a heteroaryl group. The number of ring-forming carbon atoms of the heterocyclic group may be 2 to 30, 2 to 20, or 2 to 10.

In the description, an aliphatic heterocyclic group may include one or more from among B, O, N, P, Si, and S as ring-forming heteroatoms. The number of ring-forming carbon atoms of the aliphatic heterocyclic group may be 2 to 30, 2 to 20, or 2 to 10. Non-limiting examples of the aliphatic heterocyclic group may include an oxirane group, a thiirane group, a pyrrolidine group, a piperidine group, a tetrahydrofuran group, a tetrahydrothiophene group, a thiane group, a tetrahydropyran group, a 1,4-dioxane group, etc.

In the description, a heteroaryl group may include one or more from among B, O, N, P, Si, and S as ring-forming heteroatoms. When the heteroaryl group includes two or more heteroatoms, the two or more heteroatoms may be the same or different. The heteroaryl group may be a monocyclic heterocyclic group or polycyclic heterocyclic group. The number of ring-forming carbon atoms of the heteroaryl group may be 2 to 30, 2 to 20, or 2 to 10. Non-limiting examples of the heteroaryl group may include a thiophene group, a furan group, a pyrrole group, an imidazole group, a pyridine group, a bipyridine group, a pyrimidine group, a triazine group, a triazole group, an acridyl group, a pyridazine group, a pyrazinyl group, a quinoline group, a quinazoline group, a quinoxaline group, a phenoxazine group, a phthalazine group, a pyrido pyrimidine group, a pyrido pyrazine group, a pyrazino pyrazine group, an isoquinoline group, an indole group, a carbazole group, an N-arylcarbazole group, an N-heteroarylcarbazole group, an N-alkylcarbazole group, a benzoxazole group, a benzimidazole group, a benzothiazole group, a benzocarbazole group, a benzothiophene group, a dibenzothiophene group, a thienothiophene group, a benzofuran group, a phenanthroline group, a thiazole group, an isooxazole group, an oxazole group, an oxadiazole group, a thiadiazole group, a phenothiazine group, a dibenzosilole group, a dibenzofuran group, etc.

In the description, the same explanation on the above-described aryl group may be applied to an arylene group except that the arylene group is a divalent group. The same explanation on the above-described heteroaryl group may be applied to a heteroarylene group except that the heteroarylene group is a divalent group.

In the description, a boryl group may refer to an alkyl boryl group and an aryl boryl group. Non-limiting examples of the boryl group may include a dimethylboryl group, a diethylboryl group, a t-butylboryl group, a diphenylboryl group, a phenylboryl group, and/or the like. For example, the alkyl group in the alkyl boryl group may be the same as the above-described alkyl group, and the aryl group in the aryl boryl group may be the same as the above-described aryl group.

In the description, a silyl group includes an alkyl silyl group and an aryl silyl group. Non-limiting examples of the silyl group may include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, etc.

In the description, the number of carbon atoms in a carbonyl group is not specifically limited, but may be 1 to 40, 1 to 30, or 1 to 20. For example, the carbonyl group may have the structures below, but the embodiment of the present disclosure is not limited thereto.

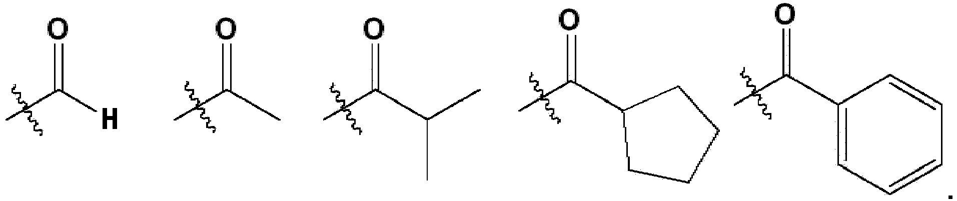

In the description, the number of carbon atoms in a sulfinyl group and a sulfonyl group is not specifically limited, but may be 1 to 30. The sulfinyl group may include an alkyl sulfinyl group and an aryl sulfinyl group. The sulfonyl group may include an alkyl sulfonyl group and an aryl sulfonyl group.

In the description, a thio group may include an alkyl thio group and an aryl thio group. The thio group may refer to the above-defined alkyl group or aryl group combined with a sulfur atom. Non-limiting examples of the thio group may include a methylthio group, an ethylthio group, a propylthio group, a pentylthio group, a hexylthio group, an octylthio group, a dodecylthio group, a cyclopentylthio group, a cyclohexylthio group, a phenylthio group, a naphthylthio group, etc.

In the description, an oxy group may refer to the above-defined alkyl group or aryl group which is combined with an oxygen atom. The oxy group may include an alkoxy group and an aryl oxy group. The alkoxy group may be a linear or branched chain. The number of carbon atoms in the alkoxy group is not specifically limited but may be, for example, 1 to 20 or 1 to 10. Examples of the oxy group may include a methoxy group, an ethoxy group, an n-propoxy group, an isopropoxy group, a butoxy group, a pentyloxy group, a hexyloxy group, an octyloxy group, a nonyloxy group, a decyloxy group, a benzyloxy, etc. However, an embodiment of the present disclosure is not limited thereto.

In the description, the number of carbon atoms in an amine group is not specifically limited, but may be 1 to 30. The amine group may include an alkyl amine group and an aryl amine group. Non-limiting examples of the amine group may include a methylamine group, a dimethylamine group, a phenylamine group, a diphenylamine group, a naphthylamine group, a 9-methyl-anthracenylamine group, a triphenylamine group, etc.

In the description, the alkyl group in the alkylthio group, the alkylsulfoxy group, the alkylaryl group, the alkylamino group, the alkylboron group, the alkyl silyl group, and the alkyl amine group may be the same as the examples of the above-described alkyl group.

In the description, the aryl group in the aryloxy group, the arylthio group, the arylsulfoxy group, the aryl amino group, the arylboron group, and the aryl silyl group may be the same as the examples of the above-described aryl group.

In the description, a direct linkage may refer to a single bond.

In the description, 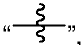 and "-* "refer to positions to be connected.

Hereinafter, the light emitting element of an embodiment will be explained by referring to the drawings.

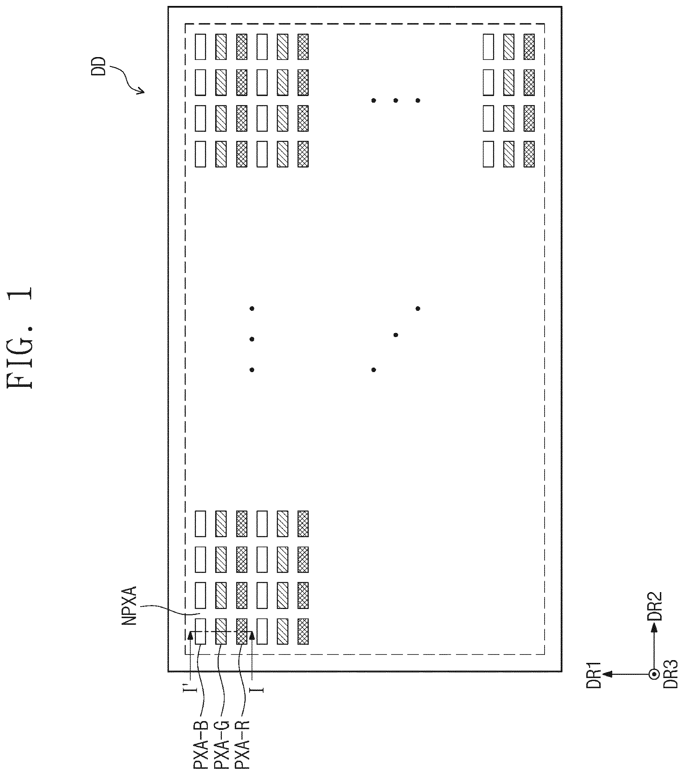

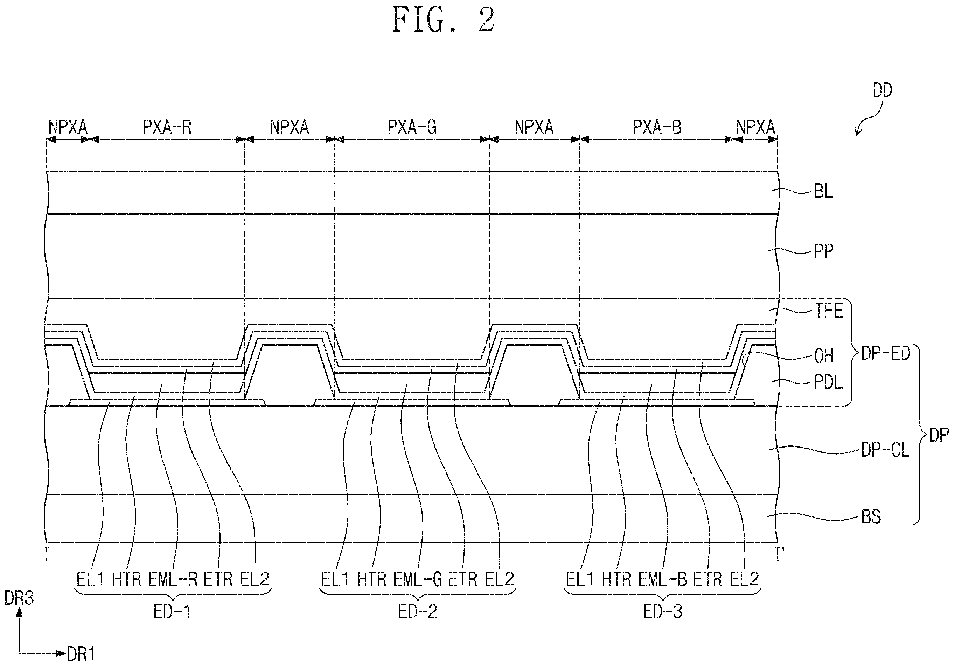

FIG. 1 is a plan view showing an embodiment of a display apparatus DD. FIG. 2 is a cross-sectional view of a display apparatus DD of an embodiment. FIG. 2 is a cross-sectional view showing a part corresponding to the line I-I' of FIG. 1.

The display apparatus DD may include a display panel DP and an optical layer PP disposed on the display panel DP. The display panel DP may include light emitting elements ED-1, ED-2 and ED-3. The display apparatus DD may include multiple light emitting elements ED-1, ED-2 and ED-3. The optical layer PP may be disposed on the display panel DP and control reflection of external light at the display panel DP. The optical layer PP may include, for example, a polarization layer and/or a color filter layer. In some embodiments, different from the drawings, the optical layer PP may not be provided in the display apparatus DD of an embodiment.

On the optical layer PP, a base substrate BL may be disposed. The base substrate BL may be a member providing a base surface on which the optical layer PP is disposed. The base substrate BL may be a glass substrate, a metal substrate, a plastic substrate, etc. However, an embodiment of the present disclosure is not limited thereto, and the base substrate BL may be an inorganic layer, an organic layer or a composite material layer. In some embodiments, different from the drawings, the base substrate BL may not be provided in an embodiment.

The display apparatus DD according to an embodiment may further include a plugging layer (e.g., filling layer). The plugging layer may be disposed between a display element layer DP-ED and the base substrate BL. The plugging layer may be an organic layer. The plugging layer may include at least one from among an acrylic resin, a silicon-based resin and an epoxy-based resin.

The display panel DP may include a base layer BS, a circuit layer DP-CL provided on the base layer BS and a display element layer DP-ED. The display element layer DP-ED may include a pixel definition layer PDL, light emitting elements ED-1, ED-2 and ED-3 disposed in the pixel definition layer PDL, and an encapsulating layer TFE disposed on the light emitting elements ED-1, ED-2 and ED-3.

The base layer BS may be a member providing a base surface where the display element layer DP-ED is disposed. The base layer BS may be a glass substrate, a metal substrate, a plastic substrate, etc. However, an embodiment of the present disclosure is not limited thereto, and the base layer BS may be an inorganic layer, an organic layer or a composite material layer.

In an embodiment, the circuit layer DP-CL is disposed on the base layer BS, and the circuit layer DP-CL may include multiple transistors. Each of the transistors may include a control electrode, an input electrode, and an output electrode. For example, the circuit layer DP-CL may include switching transistors and driving transistors for driving the light emitting elements ED-1, ED-2 and ED-3 of the display element layer DP-ED.

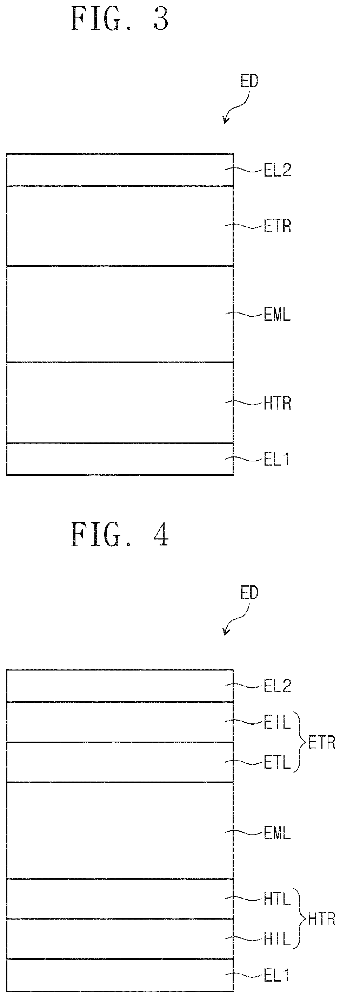

The light emitting elements ED-1, ED-2 and ED-3 may have the structures of the light emitting elements ED of embodiments according to FIG. 3 to FIG. 6, which will be explained in more detail later. The light emitting elements ED-1, ED-2 and ED-3 may each include a first electrode EL1, a hole transport region HTR, emission layers EML-R, EML-G and EML-B (e.g., a corresponding one of the emission layer EML-R, the emission layer EML-G, or the emission layer EML-B), an electron transport region ETR and a second electrode EL2.

FIG. 2 shows an embodiment where the emission layers EML-R, EML-G and EML-B of light emitting elements ED-1, ED-2 and ED-3 are disposed in opening portions OH defined in a pixel definition layer PDL, and a hole transport region HTR, an electron transport region ETR and a second electrode EL2 are provided as common layers in all light emitting elements ED-1, ED-2 and ED-3. However, an embodiment of the present disclosure is not limited thereto. Different from FIG. 2, in an embodiment, the hole transport region HTR and the electron transport region ETR may be patterned and provided in the opening portions OH defined in the pixel definition layer PDL. For example, in an embodiment, the hole transport region HTR, the emission layers EML-R, EML-G and EML-B, and the electron transport region ETR of the light emitting elements ED-1, ED-2 and ED-3 may be patterned and provided through an ink jet printing method.

An encapsulating layer TFE may cover the light emitting elements ED-1, ED-2 and ED-3. The encapsulating layer TFE may encapsulate the display element layer DP-ED. The encapsulating layer TFE may be a thin film encapsulating layer. The encapsulating layer TFE may be one layer or a stacked layer of multiple layers. The encapsulating layer TFE includes at least one insulating layer. The encapsulating layer TFE according to an embodiment may include at least one inorganic layer (hereinafter, encapsulating inorganic layer). In some embodiments, the encapsulating layer TFE according to an embodiment may include at least one organic layer (hereinafter, encapsulating organic layer) and at least one encapsulating inorganic layer.

The encapsulating inorganic layer protects the display element layer DP-ED from moisture/oxygen, and the encapsulating organic layer protects the display element layer DP-ED from foreign materials such as dust particles. The encapsulating inorganic layer may include silicon nitride, silicon oxy nitride, silicon oxide, titanium oxide, and/or aluminum oxide, but the embodiment of the present disclosure is not particularly limited thereto. The encapsulating organic layer may include an acrylic compound, an epoxy-based compound, etc. The encapsulating organic layer may include a photopolymerizable organic material, but the embodiment of the present disclosure is not particularly limited thereto.

The encapsulating layer TFE may be disposed on the second electrode EL2 and may be disposed to fill the opening portion OH.

Referring to FIG. 1 and FIG. 2, the display apparatus DD may include a non-luminous (e.g., non-light emitting) area NPXA and luminous (e.g., light emitting) areas PXA-R, PXA-G and PXA-B. The luminous areas PXA-R, PXA-G and PXA-B may be areas emitting light produced from the light emitting elements ED-1, ED-2 and ED-3, respectively. The luminous areas PXA-R, PXA-G and PXA-B may be separated from each other on a plane (e.g., in a plan view).

The luminous areas PXA-R, PXA-G and PXA-B may be areas separated by the pixel definition layer PDL. The non-luminous areas NPXA may be areas between neighboring luminous areas PXA-R, PXA-G and PXA-B and may be areas corresponding to the pixel definition layer PDL. In some embodiments, in the disclosure, each of the luminous areas PXA-R, PXA-G and PXA-B may correspond to a pixel. The pixel definition layer PDL may divide the light emitting elements ED-1, ED-2 and ED-3. The emission layers EML-R, EML-G and EML-B of the light emitting elements ED-1, ED-2 and ED-3 may be disposed and divided in the opening portions OH defined in the pixel definition layer PDL.

The luminous areas PXA-R, PXA-G and PXA-B may be divided into multiple groups according to the color of light produced from the light emitting elements ED-1, ED-2 and ED-3. In the display apparatus DD of an embodiment, shown in FIG. 1 and FIG. 2, three luminous areas PXA-R, PXA-G and PXA-B emitting red light, green light and blue light, respectively, are illustrated as an embodiment. For example, the display apparatus DD of an embodiment may include a red luminous area PXA-R, a green luminous area PXA-G and a blue luminous area PXA-B, which are separated from each other.

In the display apparatus DD according to an embodiment, multiple light emitting elements ED-1, ED-2 and ED-3 may be to emit light having different wavelength regions. For example, in an embodiment, the display apparatus DD may include a first light emitting element ED-1 emitting red light, a second light emitting element ED-2 emitting green light, and a third light emitting element ED-3 emitting blue light. For example, each of the red luminous area PXA-R, the green luminous area PXA-G, and the blue luminous area PXA-B of the display apparatus DD may correspond to the first light emitting element ED-1, the second light emitting element ED-2, and the third light emitting element ED-3, respectively.

However, an embodiment of the present disclosure is not limited thereto, and the first to third light emitting elements ED-1, ED-2 and ED-3 may be to emit light in substantially the same wavelength region, or at least one thereof may be to emit light in a different wavelength region. For example, the first to third light emitting elements ED-1, ED-2 and ED-3 may all emit blue light.

The luminous areas PXA-R, PXA-G and PXA-B in the display apparatus DD according to an embodiment may be arranged in a stripe shape. Referring to FIG. 1, multiple red luminous areas PXA-R may be arranged with each other along a second directional axis DR2, multiple green luminous areas PXA-G may be arranged with each other along the second directional axis DR2, and multiple blue luminous areas PXA-B may be arranged with each other along the second directional axis DR2. In some embodiments, the red luminous area PXA-R, the green luminous area PXA-G and the blue luminous area PXA-B may be alternately arranged in this stated order along a first directional axis DR1.

In FIG. 1 and FIG. 2, the areas of the luminous areas PXA-R, PXA-G and PXA-B are shown as similar in size, but an embodiment of the present disclosure is not limited thereto. The areas of the luminous areas PXA-R, PXA-G and PXA-B may be different from each other according to the wavelength region of light emitted. Meanwhile, the areas of the luminous areas PXA-R, PXA-G and PXA-B may refer to areas when viewed on a plane defined by the first directional axis DR1 and the second directional axis DR2 (e.g., in a plan view).

In some embodiments, the arrangement of the luminous areas PXA-R, PXA-G and PXA-B is not limited to the configuration shown in FIG. 1, and the arrangement order of the red luminous areas PXA-R, the green luminous areas PXA-G and the blue luminous areas PXA-B may be provided in one or more suitable combinations according to the properties of display quality required or desired for the display apparatus DD. For example, the luminous areas PXA-R, PXA-G and PXA-B may be arranged in a PENTILE^{®} arrangement form, or a Diamond Pixel^{™} arrangement form. PENTILE^{®} and Diamond Pixel^{™} are trademarks of Samsung Display Co., Ltd.

In some embodiments, the areas of the luminous areas PXA-R, PXA-G and PXA-B may be different from each other. For example, in an embodiment, the area of the green luminous area PXA-G may be smaller than the area of the blue luminous area PXA-B, but an embodiment of the present disclosure is not limited thereto.

Hereinafter, FIG. 3 to FIG. 6 are cross-sectional views schematically showing light emitting elements according to embodiments. The light emitting element ED according to an embodiment may include a first electrode EL1, a second electrode EL2 oppositely disposed to the first electrode EL1, and at least one functional layer disposed between the first electrode EL1 and the second electrode EL2. The light emitting element ED of an embodiment may include an amine compound of an embodiment, which will be explained in more detail later, in the at least one functional layer.

The light emitting element ED may include a hole transport region HTR, an emission layer EML, an electron transport region ETR, and/or the like, stacked in the stated order, as the at least one functional layer. Referring to FIG. 3, the light emitting element ED of an embodiment may include a first electrode EL1, a hole transport region HTR, an emission layer EML, an electron transport region ETR, and a second electrode EL2, stacked in the stated order.

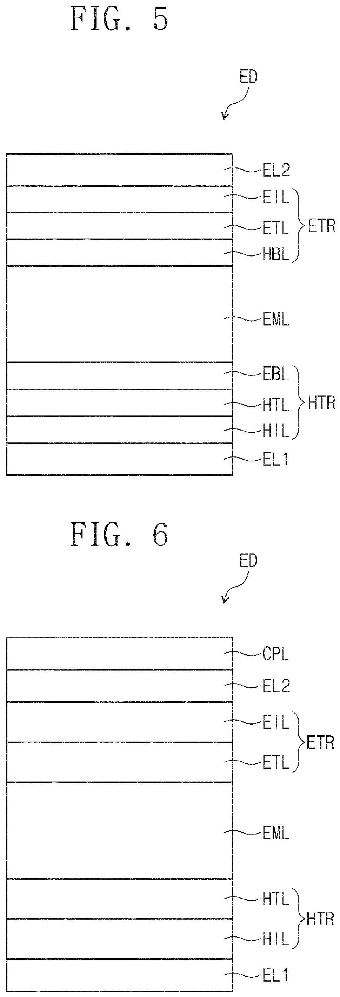

When compared with FIG. 3, FIG. 4 shows the cross-sectional view of a light emitting element ED of an embodiment, in which a hole transport region HTR includes a hole injection layer HIL and a hole transport layer HTL, and an electron transport region ETR includes an electron injection layer EIL and an electron transport layer ETL. In addition, when compared with FIG. 3, FIG. 5 shows the cross-sectional view of a light emitting element ED of an embodiment, in which a hole transport region HTR includes a hole injection layer HIL, a hole transport layer HTL, and an electron blocking layer EBL, and an electron transport region ETR includes an electron injection layer EIL, an electron transport layer ETL, and a hole blocking layer HBL. When compared with FIG. 4, FIG. 6 shows the cross-sectional view of a light emitting element ED of an embodiment, further including a capping layer CPL disposed on the second electrode EL2.

The light emitting element ED of an embodiment may include an amine compound of an embodiment, which will be explained in more detail later, in a hole transport region HTR. The light emitting element ED of an embodiment may include an amine compound of an embodiment in at least one from among the hole injection layer HIL, hole transport layer HTL and electron blocking layer EBL of the hole transport region HTR.

In the light emitting element ED according to an embodiment, the first electrode EL1 has conductivity (e.g., is a conductor). The first electrode EL1 may be formed utilizing a metal material, a metal alloy or a conductive compound. The first electrode EL1 may be an anode or a cathode. However, an embodiment of the present disclosure is not limited thereto. In some embodiments, the first electrode EL1 may be a pixel electrode. The first electrode EL1 may be a transmissive electrode, a transflective electrode, or a reflective electrode. The first electrode EL1 may include at least one selected from among Ag, Mg, Cu, Al, Pt, Pd, Au, Ni, Nd, Ir, Cr, Li, Ca, LiF, Mo, Ti, W, In, Sn, Zn, compounds of two or more selected therefrom, mixtures of two or more selected therefrom, and/or oxides thereof.

When the first electrode EL1 is the transmissive electrode, the first electrode EL1 may include a transparent metal oxide such as indium tin oxide (ITO), indium zinc oxide (IZO), zinc oxide (ZnO), and/or indium tin zinc oxide (ITZO). When the first electrode EL1 is the transflective electrode or the reflective electrode, the first electrode EL1 may include Ag, Mg, Cu, Al, Pt, Pd, Au, Ni, Nd, Ir, Cr, Li, Ca, LiF/Ca (a stacked structure of LiF and Ca), LiF/Al (a stacked structure of LiF and Al), Mo, Ti, W, compounds thereof, or mixtures thereof (for example, a mixture of Ag and Mg). Also, the first electrode EL1 may have a structure including multiple layers including a reflective layer or a transflective layer formed utilizing the above materials, and a transmissive conductive layer formed utilizing ITO, IZO, ZnO, or ITZO. For example, the first electrode EL1 may have a three-layer structure of ITO/Ag/ITO. However, an embodiment of the present disclosure is not limited thereto. The first electrode EL1 may include one or more of the above-described metal materials, combinations of two or more metal materials selected from the above-described metal materials, and/or one or more of oxides of the above-described metal materials. The thickness of the first electrode EL1 may be from about 700 Å to about 10,000 Å. For example, the thickness of the first electrode EL1 may be from about 1,000 Å to about 3,000 Å.

The hole transport region HTR is provided on the first electrode EL1. The hole transport region HTR may have a single layer formed utilizing a single material, a single layer formed utilizing multiple different materials, or a multilayer structure including multiple layers formed utilizing multiple different materials.

The hole transport region HTR may include at least one of a hole injection layer HIL, a hole transport layer HTL, or an electron blocking layer EBL. In some embodiments, though not shown, the hole transport region HTR may include multiple hole transport layers stacked over one another.

In some embodiments, the hole transport region HTR may have the structure of a single layer of a hole injection layer HIL or a hole transport layer HTL, or may have a structure of a single layer formed utilizing a hole injection material and a hole transport material. In an embodiment, the hole transport region HTR may have a structure of a single layer formed utilizing multiple different materials, or a structure of hole injection layer HIL/hole transport layer HTL, hole injection layer HIL/hole transport layer HTL/buffer layer, hole injection layer HIL/buffer layer, or hole transport layer HTL/buffer layer, stacked in the respective stated order from the first electrode EL1, but the embodiment of the present disclosure is not limited thereto.

The thickness of the hole transport region HTR may be, for example, about 50 Å to about 15,000 Å. The hole transport region HTR may be formed utilizing one or more suitable methods such as a vacuum deposition method, a spin coating method, a cast method, a Langmuir-Blodgett (LB) method, an inkjet printing method, a laser printing method, and/or a laser induced thermal imaging (LITI) method.

The light emitting element ED of an embodiment may include the amine compound of an embodiment in a hole transport region HTR. The amine compound of an embodiment may be represented by Formula 1.

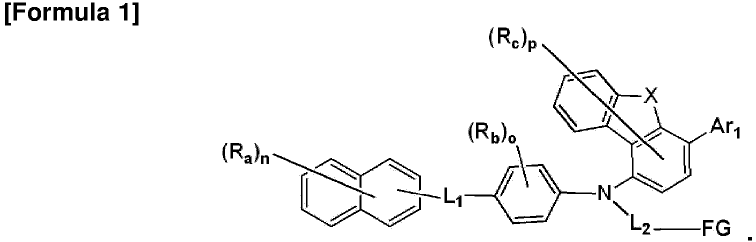

In Formula 1, FG is represented by any one from among Formula 2 to Formula 5.

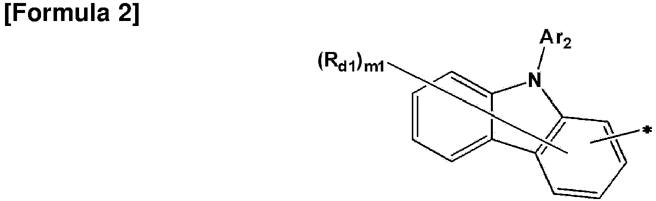

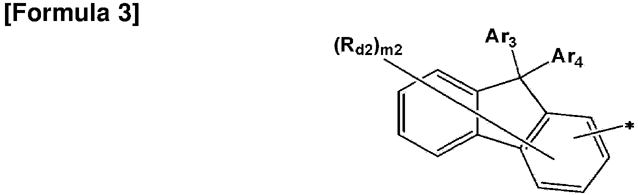

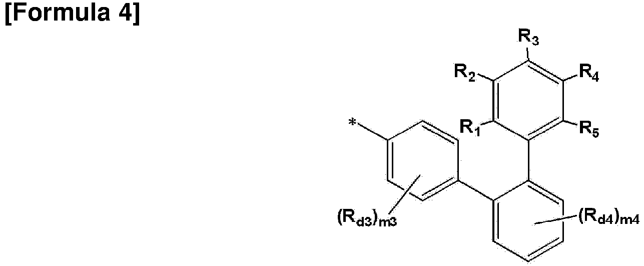

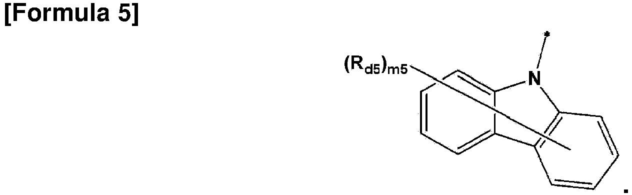

The amine compound of an embodiment may include a substituted dibenzoheterole group directly linked to a nitrogen atom, a naphthyl group bonded to the nitrogen atom via a linker, and one substituent selected from a carbazole group, a fluorene group or a terphenyl derivatives. In addition, in the amine compound of an embodiment, the dibenzoheterole group bonded to the nitrogen atom may be substituted with a substituent selected from an aryl group and a heteroaryl group, and the bonding position of the substituent corresponds to the para position with respect to the nitrogen atom.

In Formula 1, X may be O or S. That is, in the amine compound of an embodiment, the dibenzoheterole group directly bonded to the nitrogen atom may be a dibenzofuran group or a dibenzothiophene group.

In Formula 1 of the disclosure, Ar₁ may be a substituted or unsubstituted aryl group of 6 to 40 ring-forming carbon atoms, or a substituted or unsubstituted heteroaryl group of 1 to 40 ring-forming carbon atoms. That is, in the amine compound of an embodiment, the dibenzoheterole group directly bonded to the nitrogen atom may be substituted with an aryl group or a heteroaryl group.

For example, in a dibenzoheterole group represented below, position 1 may be bonded to the nitrogen atom, and the substituent of Ar₁ may be substituted at position 4.

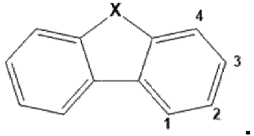

In Formula 1 of the invention, Ar₁ is a substituted or unsubstituted phenyl group, a substituted or unsubstituted naphthyl group, a substituted or unsubstituted phenanthrenyl group, a substituted or unsubstituted dibenzothiophene group, or a substituted or unsubstituted dibenzofuran group. For example, Ar₁ may be represented by any one from among Ar1-1 to Ar1-5.

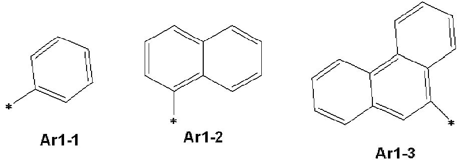

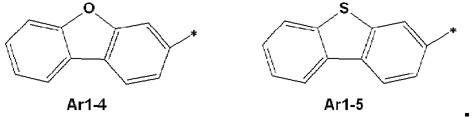

In Formula 1, L₁ and L₂ may each independently be a direct linkage, a substituted or unsubstituted arylene group of 6 to 15 ring-forming carbon atoms, or a substituted or unsubstituted heteroarylene group of 1 to 12 ring-forming carbon atoms.

For example, in the amine compound of an embodiment, represented by Formula 1, L₁ may be a direct linkage, an unsubstituted phenylene group, or an unsubstituted divalent biphenyl group. In some embodiments, L₂ may be a direct linkage, an unsubstituted phenylene group, an unsubstituted naphthalene group, an unsubstituted divalent dibenzofuran group, or an unsubstituted divalent dibenzothiophene group. However, an embodiment of the present disclosure is not limited thereto.

In Formula 1, "n" may be an integer of 0 to 7. In Formula 1, when "n" is 0, a naphthyl group may be unsubstituted. In some embodiments, when "n" is an integer of 2 or more, multiple Rₐ may all be the same, or at least one may be different from the remainder thereof.

Rₐ may be a deuterium atom, a halogen atom, a substituted or unsubstituted alkyl group of 1 to 10 carbon atoms, a substituted or unsubstituted aryl group of 6 to 30 ring-forming carbon atoms, or a substituted or unsubstituted heteroaryl group of 1 to 30 ring-forming carbon atoms.

In Formula 1, "o" may be an integer of 0 to 4. R_{b} may be a deuterium atom, a halogen atom, a substituted or unsubstituted alkyl group of 1 to 10 carbon atoms, a substituted or unsubstituted aryl group of 6 to 30 ring-forming carbon atoms, or a substituted or unsubstituted heteroaryl group of 1 to 30 ring-forming carbon atoms. In some embodiments, when "o" is 2 or more, multiple R_{b} may all be the same, or at least one may be different from the remainder thereof. For example, in an embodiment, "o" may be 0.

In Formula 1, "p" may be an integer of 0 to 6. R_{c} may be a deuterium atom, a halogen atom, a substituted or unsubstituted alkyl group of 1 to 10 carbon atoms, a substituted or unsubstituted aryl group of 6 to 30 ring-forming carbon atoms, or a substituted or unsubstituted heteroaryl group of 1 to 30 ring-forming carbon atoms. In some embodiments, when "p" is 2 or more, multiple R_{c} may all be the same, or at least one may be different from the remainder thereof. For example, in an embodiment, "p" may be 0.

In the amine compound of an embodiment, represented by Formula 1, FG may be a carbazole derivative as represented by Formula 2 or Formula 5, a fluorene derivative as represented by Formula 3, or a terphenyl derivative as represented by Formula 4. In some embodiments, the terphenyl derivative represented by Formula 4 may be in a state where two rings are bonded at ortho positions to each other based on the central phenylene group. In the description, the shape of the terphenyl derivative represented by Formula 4 is referred to as a substituent including a Z-type or kind aryl group. In the terphenyl derivative represented by Formula 4, one from among the benzene rings may be combined with one or more substituents to form one or more saturated or unsaturated rings.

In Formula 2, Ar₂ may be a substituted or unsubstituted aryl group of 6 to 40 ring-forming carbon atoms, or a substituted or unsubstituted heteroaryl group of 1 to 40 ring-forming carbon atoms. For example, Ar₂ may be an unsubstituted phenyl group, an unsubstituted naphthyl group, a deuterium-substituted phenyl group, or a deuterium-substituted naphthyl group. However, an embodiment of the present disclosure is not limited thereto.

In Formula 2 to Formula 5, R_{d1} to R_{d5} are each independently a deuterium atom, a halogen atom, a substituted or unsubstituted alkyl group of 1 to 20 carbon atoms, a substituted or unsubstituted alkenyl group of 2 to 20 carbon atoms, a substituted or unsubstituted oxy group, a substituted or unsubstituted thio group, a substituted or unsubstituted amine group, a substituted or unsubstituted aryl group of 6 to 30 ring-forming carbon atoms, a substituted or unsubstituted heteroaryl group of 1 to 30 ring-forming carbon atoms, or combined with an adjacent group to form a hydrocarbon ring or a heterocycle.

In some embodiments, in Formula 2 to Formula 5, R_{d1} to R_{d5} are each independently a deuterium atom, a halogen atom, a substituted or unsubstituted alkyl group of 1 to 20 carbon atoms, a substituted or unsubstituted alkenyl group of 2 to 20 carbon atoms, a substituted or unsubstituted oxy group, a substituted or unsubstituted thio group, a substituted or unsubstituted amine group, a substituted or unsubstituted aryl group of 6 to 30 ring-forming carbon atoms, or a substituted or unsubstituted heteroaryl group of 1 to 30 ring-forming carbon atoms; optionally wherein when one or more of R_{d1} to R_{d5} is a substituted or unsubstituted alkyl group of 1 to 20 carbon atoms, a substituted or unsubstituted alkenyl group of 2 to 20 carbon atoms, a substituted or unsubstituted oxy group, a substituted or unsubstituted thio group, a substituted or unsubstituted amine group, a substituted or unsubstituted aryl group of 6 to 30 ring-forming carbon atoms, or a substituted or unsubstituted heteroaryl group of 1 to 30 ring-forming carbon atoms, said R_{d1} to R_{d5} may be individually combined with an adjacent group to form a hydrocarbon ring or a heterocycle.

In some embodiments, in Formula 2, m1 may be an integer of 0 to 7, and R_{d1} may be a deuterium atom, a halogen atom, a substituted or unsubstituted alkyl group of 1 to 20 carbon atoms, a substituted or unsubstituted alkenyl group of 2 to 20 carbon atoms, a substituted or unsubstituted oxy group, a substituted or unsubstituted thio group, a substituted or unsubstituted amine group, a substituted or unsubstituted aryl group of 6 to 30 ring-forming carbon atoms, a substituted or unsubstituted heteroaryl group of 1 to 30 ring-forming carbon atoms, or combined with an adjacent group to form a hydrocarbon ring or a heterocycle. When m1 is 2 or more, multiple R_{d1} may all be the same, or at least one may be different from the remainder thereof. For example, m1 may be 0. However, an embodiment of the present disclosure is not limited thereto.

In Formula 3, Ar₃ and Ar₄ may each independently be a substituted or unsubstituted aryl group of 6 to 40 ring-forming carbon atoms, or a substituted or unsubstituted heteroaryl group of 1 to 40 ring-forming carbon atoms. In an embodiment, Ar₃ and Ar₄ may be the same. However, an embodiment of the present disclosure is not limited thereto.

For example, Ar₃ and Ar₄ may each independently be an unsubstituted phenyl group, or a deuterium-substituted phenyl group. However, an embodiment of the present disclosure is not limited thereto.

In some embodiments, in Formula 3, m2 may be an integer of 0 to 7, and R_{d2} may be a deuterium atom, a halogen atom, a substituted or unsubstituted alkyl group of 1 to 20 carbon atoms, a substituted or unsubstituted alkenyl group of 2 to 20 carbon atoms, a substituted or unsubstituted oxy group, a substituted or unsubstituted thio group, a substituted or unsubstituted amine group, a substituted or unsubstituted aryl group of 6 to 30 ring-forming carbon atoms, a substituted or unsubstituted heteroaryl group of 1 to 30 ring-forming carbon atoms, or combined with an adjacent group to form a hydrocarbon ring or a heterocycle. When m2 is 2 or more, multiple R_{d2} may all be the same, or at least one may be different from the remainder thereof. For example, m2 may be 0. However, an embodiment of the present disclosure is not limited thereto.

In Formula 4, R₁ to R₅ may each independently be a hydrogen atom, a deuterium atom, a halogen atom, a substituted or unsubstituted alkyl group of 1 to 20 carbon atoms, a substituted or unsubstituted alkenyl group of 2 to 20 carbon atoms, a substituted or unsubstituted oxy group, a substituted or unsubstituted thio group, a substituted or unsubstituted amine group, a substituted or unsubstituted aryl group of 6 to 30 ring-forming carbon atoms, a substituted or unsubstituted heteroaryl group of 1 to 30 ring-forming carbon atoms, or combined with an adjacent group to form a hydrocarbon ring or a heterocycle.

In some embodiments, in Formula 4, R₁ to R₅ may each independently be a hydrogen atom, a deuterium atom, a halogen atom, a substituted or unsubstituted alkyl group of 1 to 20 carbon atoms, a substituted or unsubstituted alkenyl group of 2 to 20 carbon atoms, a substituted or unsubstituted oxy group, a substituted or unsubstituted thio group, a substituted or unsubstituted amine group, a substituted or unsubstituted aryl group of 6 to 30 ring-forming carbon atoms, or a substituted or unsubstituted heteroaryl group of 1 to 30 ring-forming carbon atoms; optionally wherein when one or more of R₁ to R₅ is a substituted or unsubstituted alkyl group of 1 to 20 carbon atoms, a substituted or unsubstituted alkenyl group of 2 to 20 carbon atoms, a substituted or unsubstituted oxy group, a substituted or unsubstituted thio group, a substituted or unsubstituted amine group, a substituted or unsubstituted aryl group of 6 to 30 ring-forming carbon atoms, or a substituted or unsubstituted heteroaryl group of 1 to 30 ring-forming carbon atoms, said R₁ to R₅ may be individually combined with an adjacent group to form a hydrocarbon ring or a heterocycle.

For example, R₁ to R₅ may each independently be a hydrogen atom, a substituted or unsubstituted phenyl group, or a substituted or unsubstituted naphthyl group. In some embodiments, one or two selected from R₁ to R₅ may be substituted or unsubstituted phenyl groups, and any remainder thereof may be hydrogen atoms.

In some embodiments, in Formula 4, when R₁ to R₅ are combined with adjacent groups to form one or more rings, R₁ to R₅ may be combined with the benzene ring at which R₁ to R₅ are substituted to form a dibenzofuran ring, a dibenzothiophene ring, or a carbazole ring. However, an embodiment of the present disclosure is not limited thereto.

In some embodiments, in Formula 4, m3 and m4 may each independently be an integer of 0 to 4, and R_{d3} and R_{d4} may each independently be a deuterium atom, a halogen atom, a substituted or unsubstituted alkyl group of 1 to 20 carbon atoms, a substituted or unsubstituted alkenyl group of 2 to 20 carbon atoms, a substituted or unsubstituted oxy group, a substituted or unsubstituted thio group, a substituted or unsubstituted amine group, a substituted or unsubstituted aryl group of 6 to 30 ring-forming carbon atoms, a substituted or unsubstituted heteroaryl group of 1 to 30 ring-forming carbon atoms, or combined with an adjacent group to form a hydrocarbon ring or a heterocycle. When m3 is 2 or more, multiple R_{d3} may all be the same, or at least one may be different from the remainder thereof. For example, m3 may be 0. When m4 is 2 or more, multiple R_{d4} may all be the same, or at least one may be different from the remainder thereof.

In Formula 5, m5 may be an integer of 0 to 8, R_{d5} and may be a deuterium atom, a halogen atom, a substituted or unsubstituted alkyl group of 1 to 20 carbon atoms, a substituted or unsubstituted alkenyl group of 2 to 20 carbon atoms, a substituted or unsubstituted oxy group, a substituted or unsubstituted thio group, a substituted or unsubstituted amine group, a substituted or unsubstituted aryl group of 6 to 30 ring-forming carbon atoms, a substituted or unsubstituted heteroaryl group of 1 to 30 ring-forming carbon atoms, or combined with an adjacent group to form a hydrocarbon ring or a heterocycle. When m5 is 2 or more, multiple R_{d5} may all be the same, or at least one may be different from the remainder thereof. For example, m5 may be 0.

In the amine compound of an embodiment, represented by Formula 1, at least one from among Rₐ to R_{c}, Ar₁, and FG may include a deuterium atom or a deuterium atom-containing substituent. For example, the amine compound of an embodiment may include at least one deuterium atom as a substituent.

In some embodiments, the amine compound of an embodiment may be a monoamine compound. The amine compound of an embodiment may not include (e.g., may exclude) an amine group as a substituent.

In an embodiment, Formula 1 may be represented by any one from among Formula 1-1 to Formula 1-4. In Formula 1-1 to Formula 1-4, the same explanation on X, L₁, L₂, "n", Rₐ, and Ar₁ referring to Formula 1 may be applied.

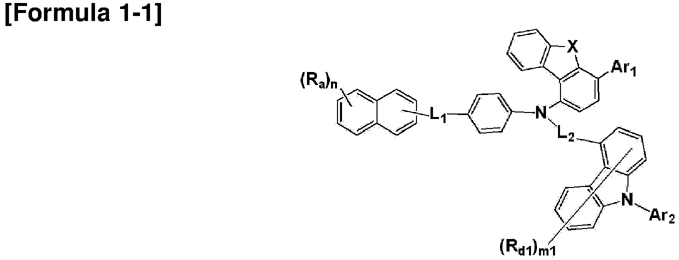

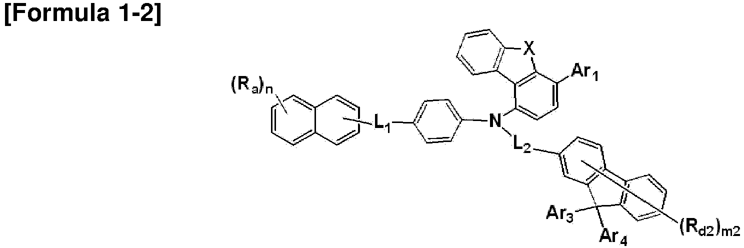

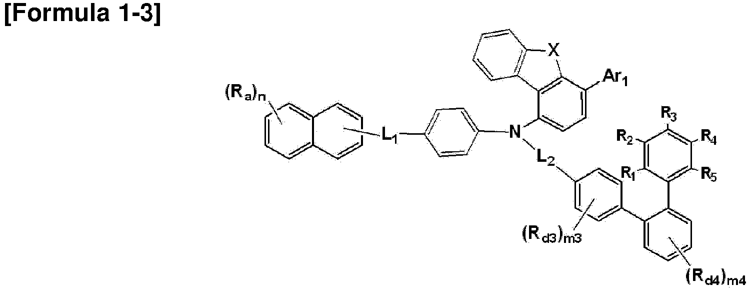

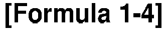

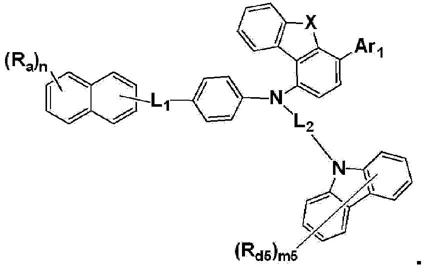

In some embodiments, in Formula 1-1 and Formula 1-2, the same explanation on Ar₂ to Ar₄ referring to Formula 2 and Formula 3 may be applied, in Formula 1-3, the same explanation on R₁ to R₅ referring to Formula 4 may be applied, and in Formula 1-1 to Formula 1-4, the same explanation on R_{d1} to R_{d5}, and m1 to m5 referring to Formula 2 to Formula 5 may be applied.

The amine compound of an embodiment, represented by Formula 1 may be any one of the compounds in Compound Group 1. The hole transport region HTR of the light emitting element ED of an embodiment may include at least one from among the amine compounds shown in Compound Group 1. In Compound Group 1, D is a deuterium atom.

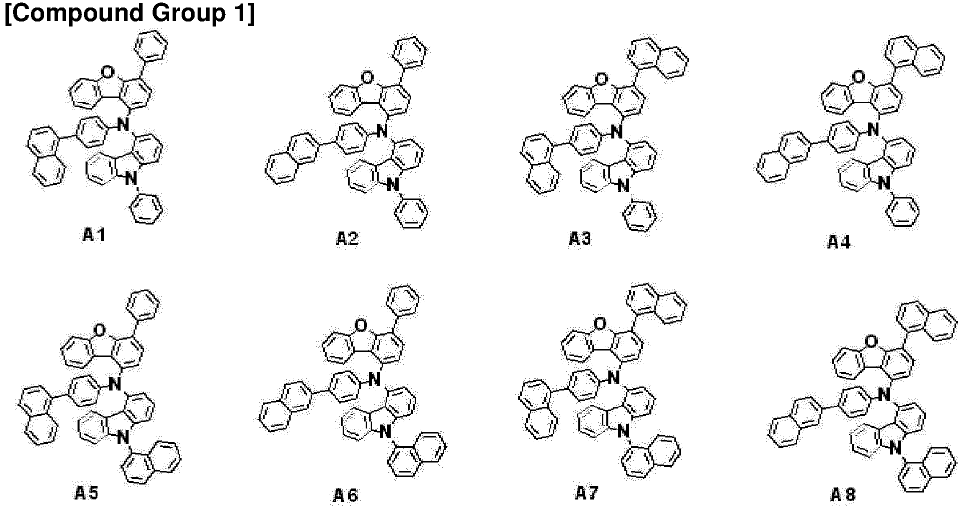

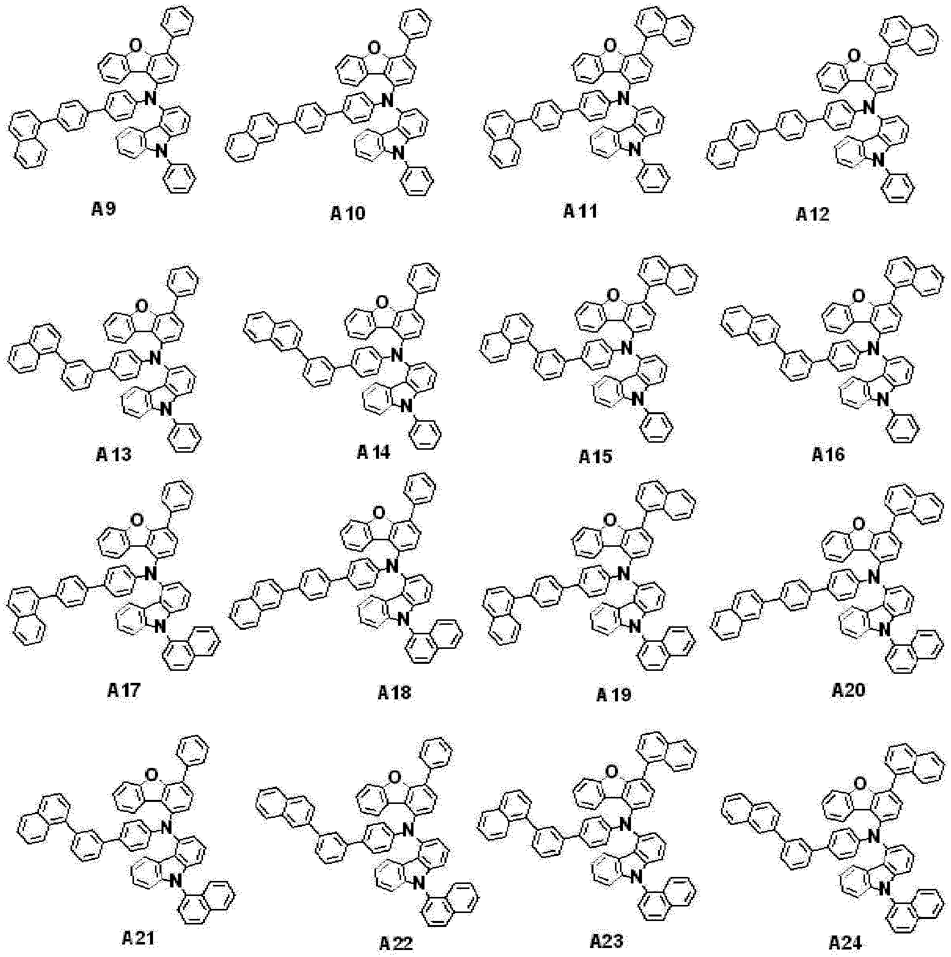

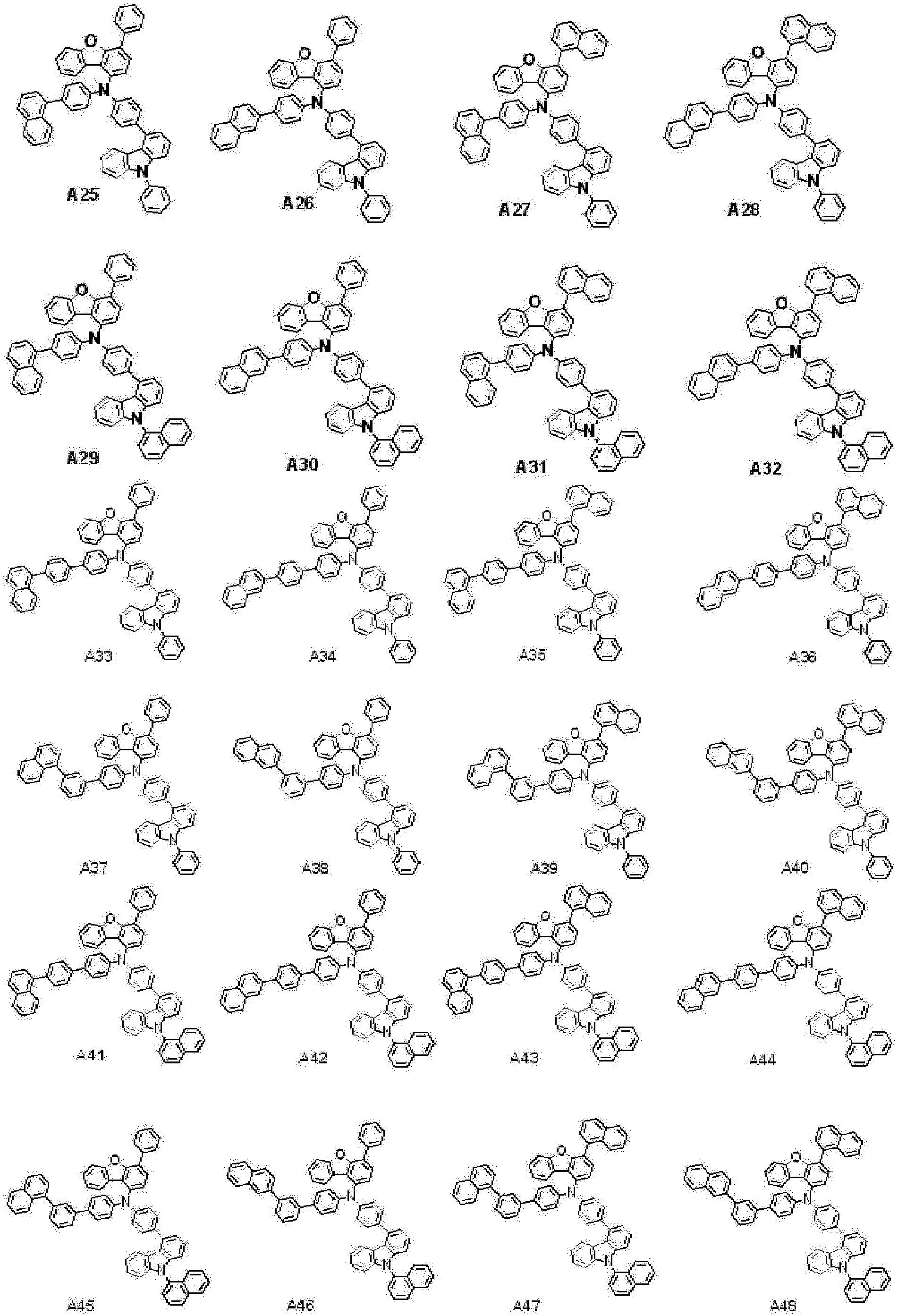

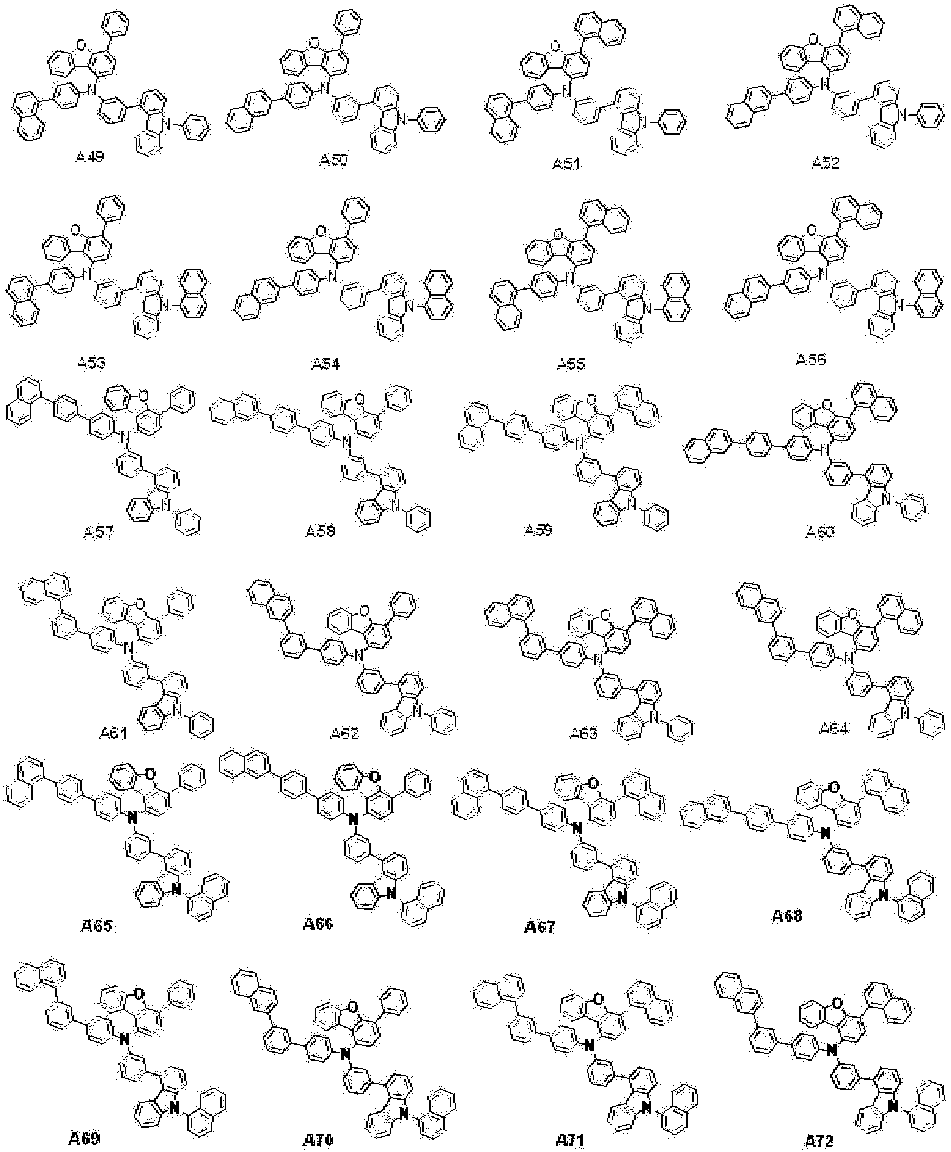

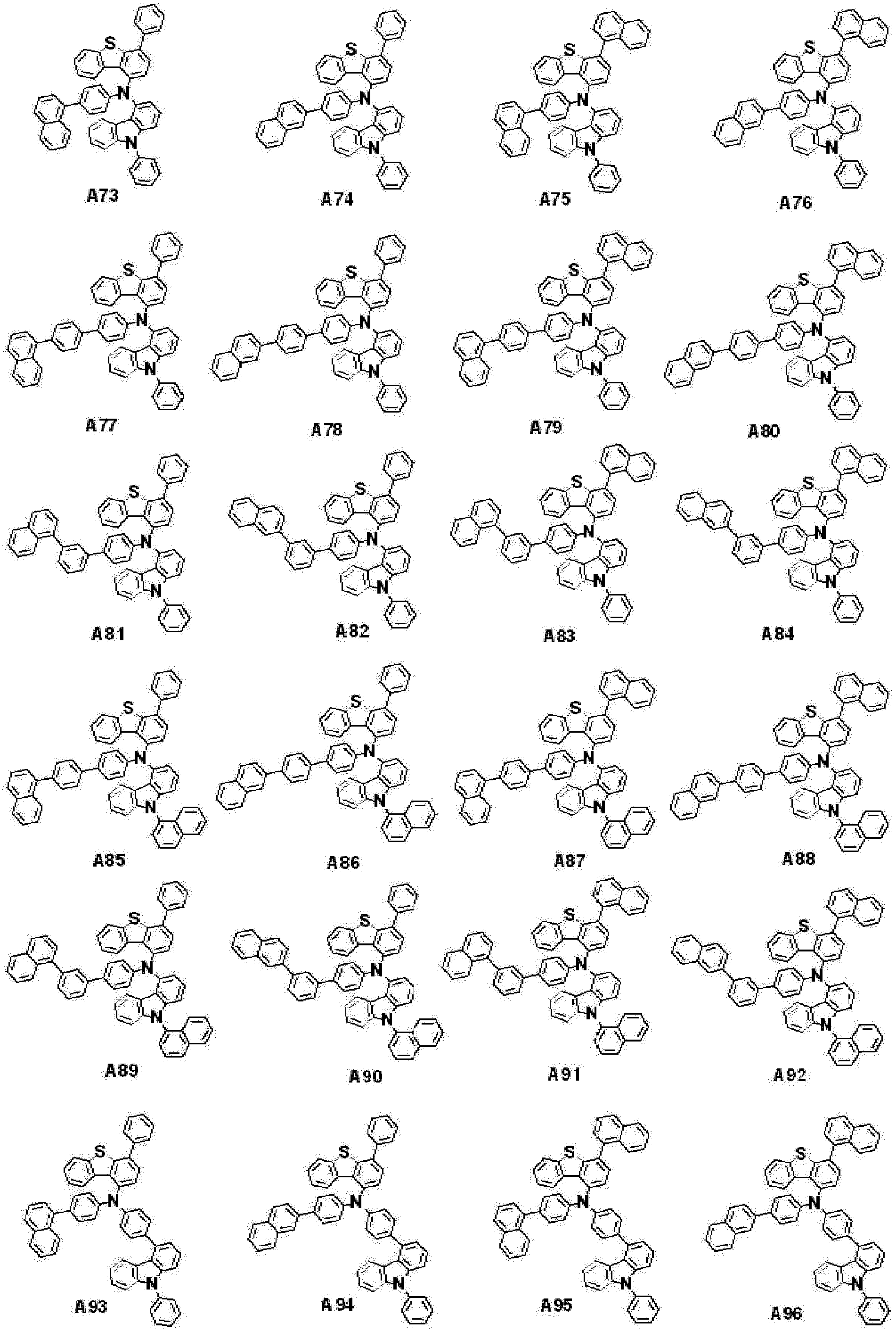

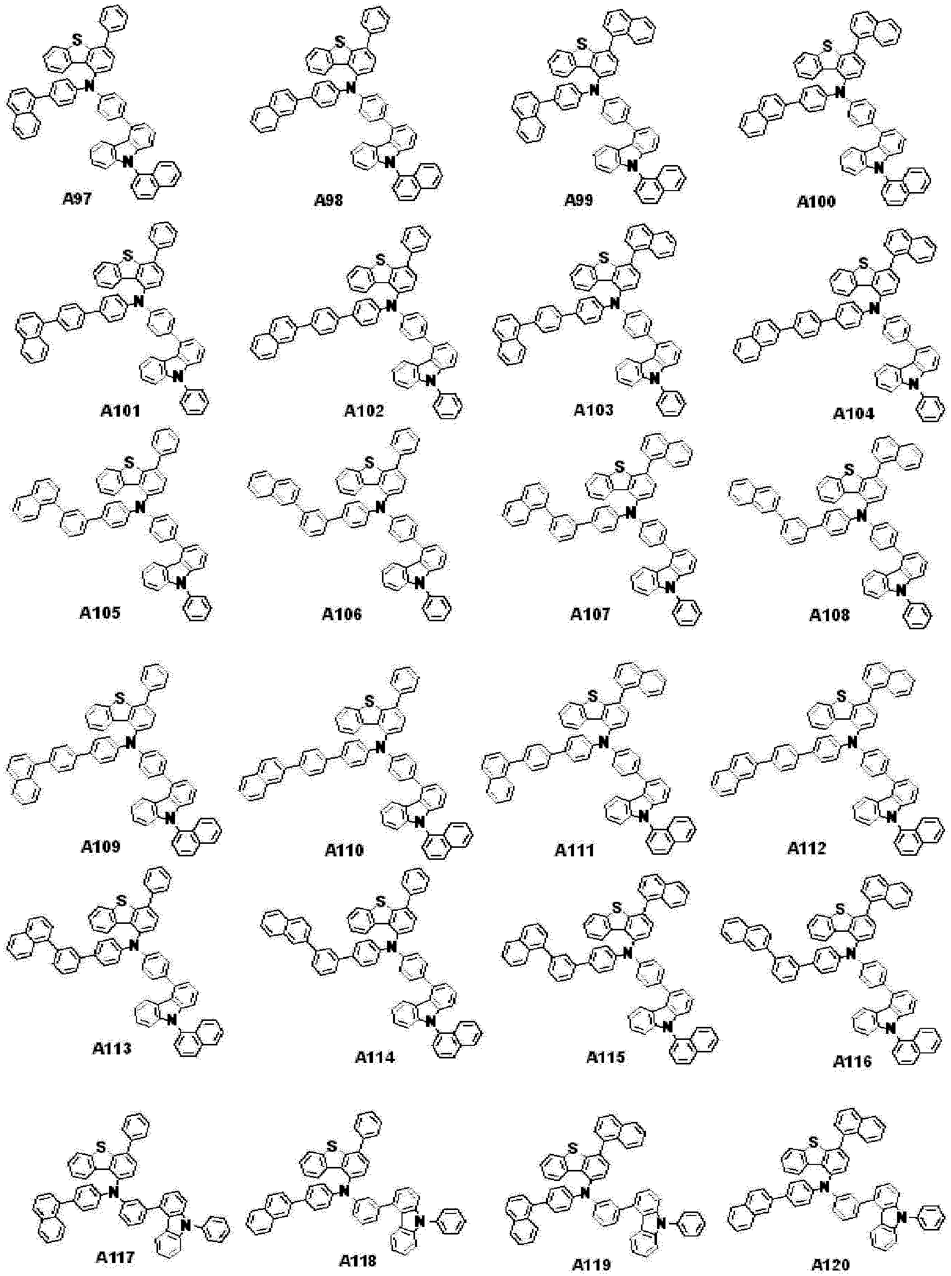

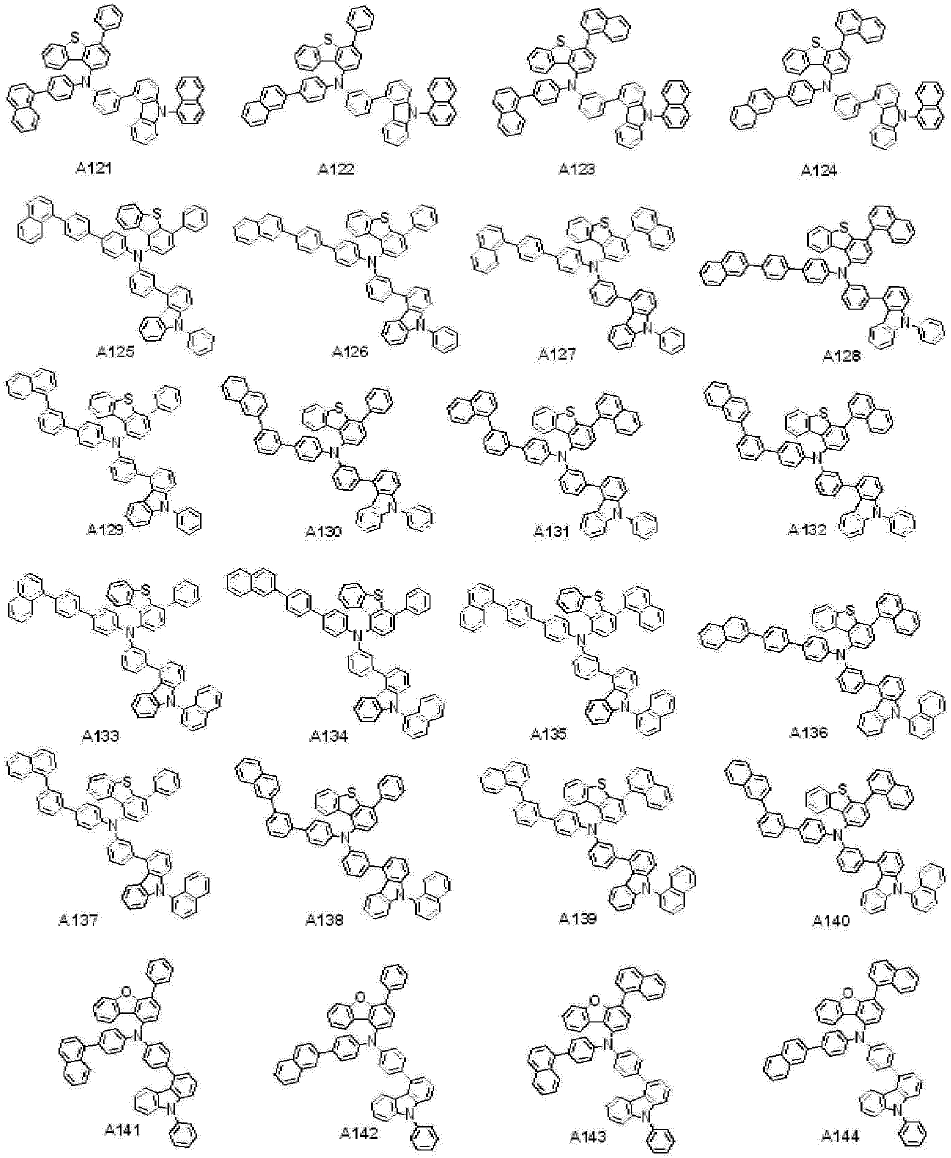

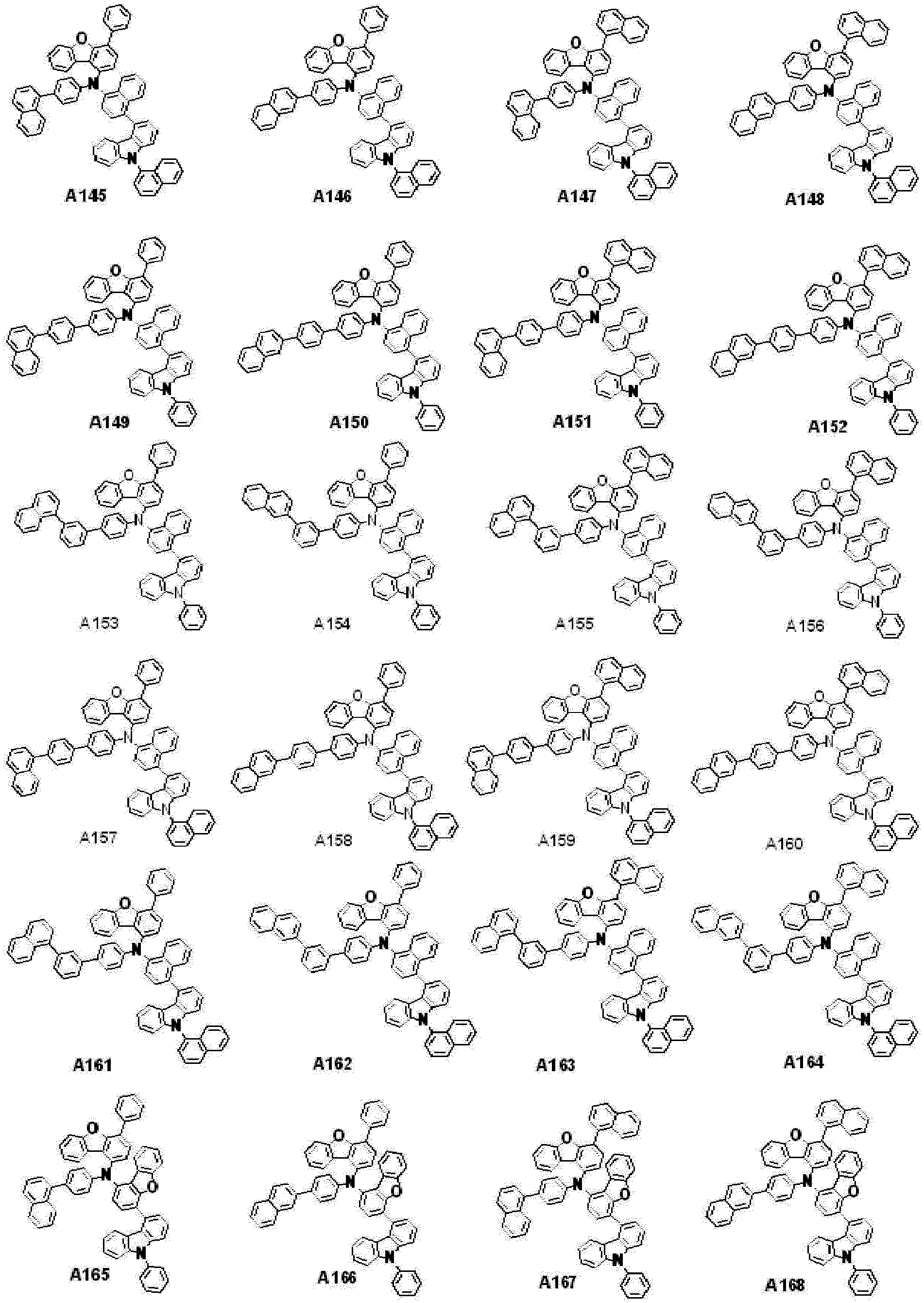

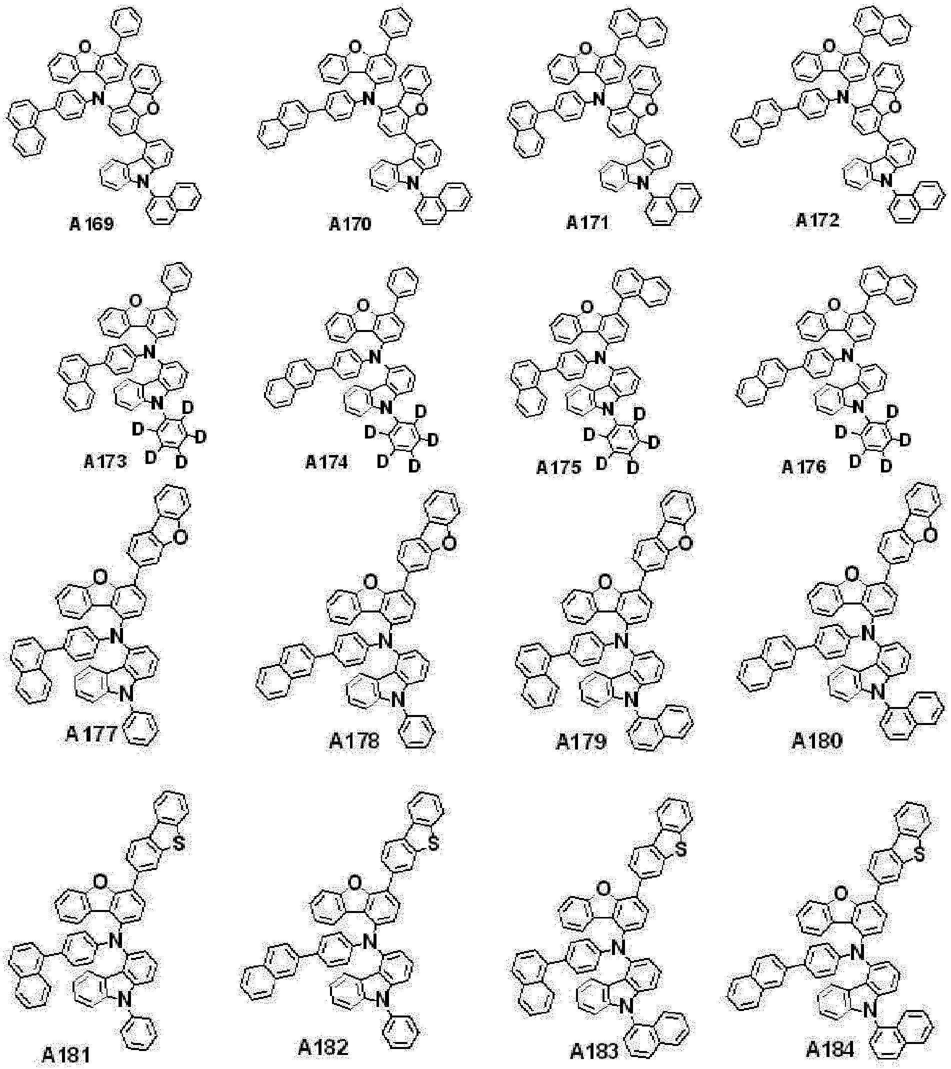

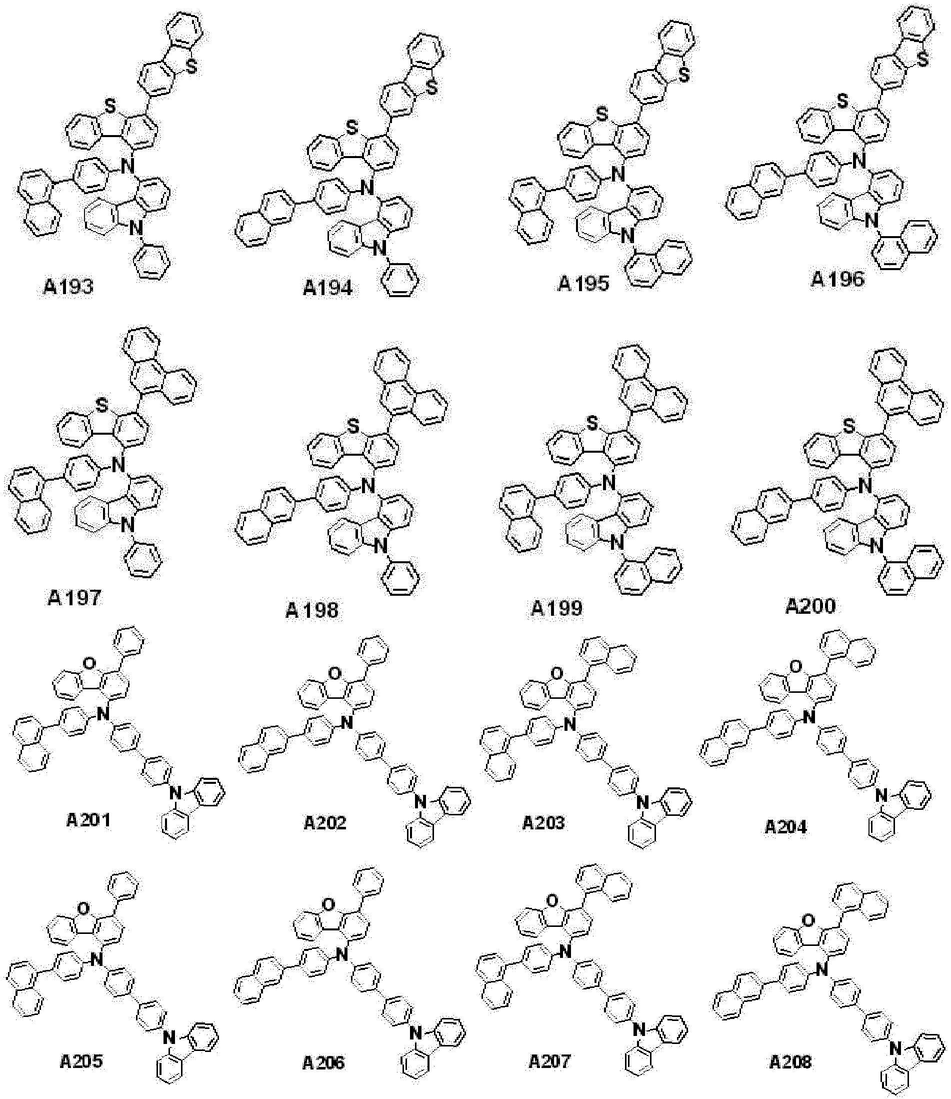

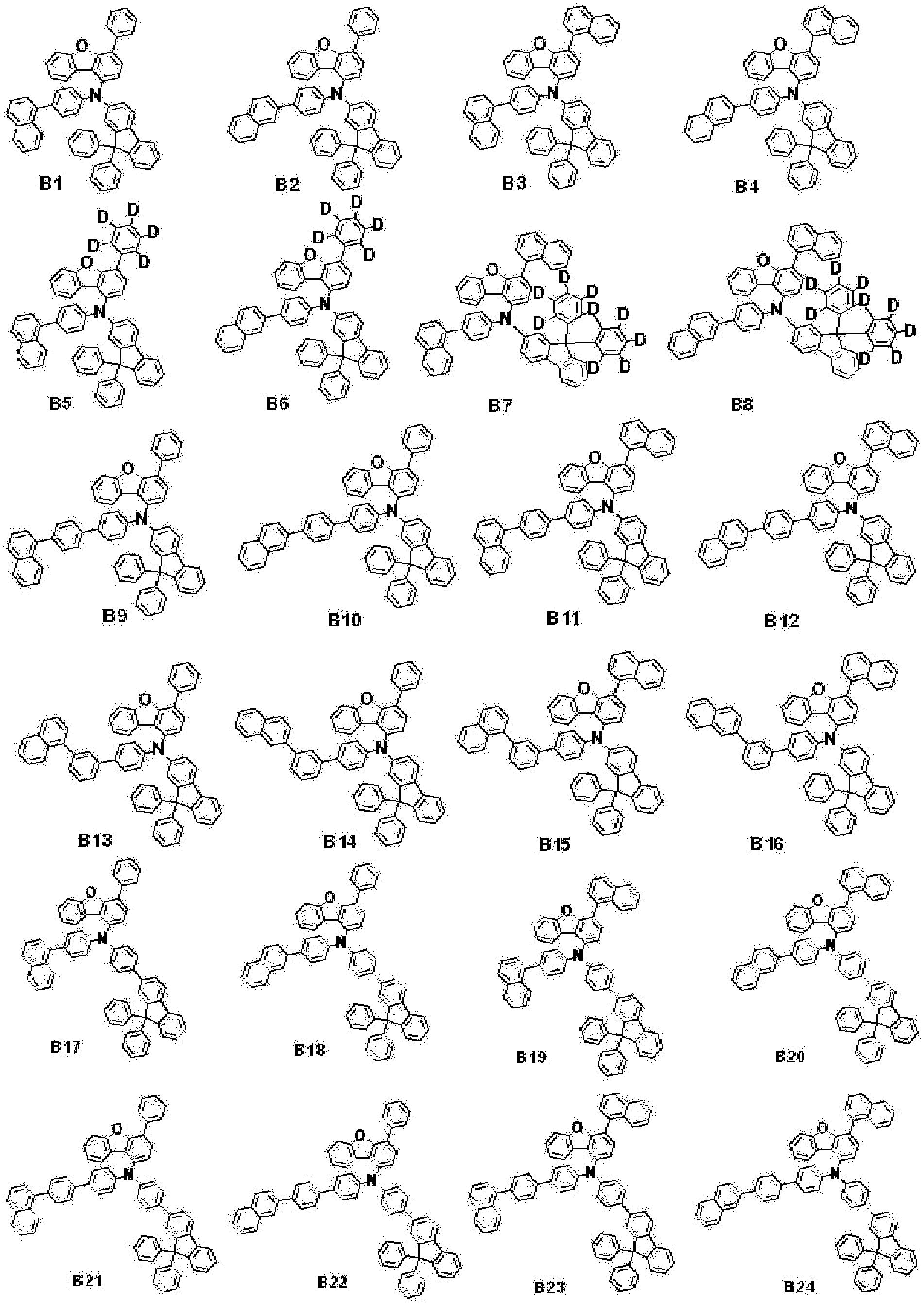

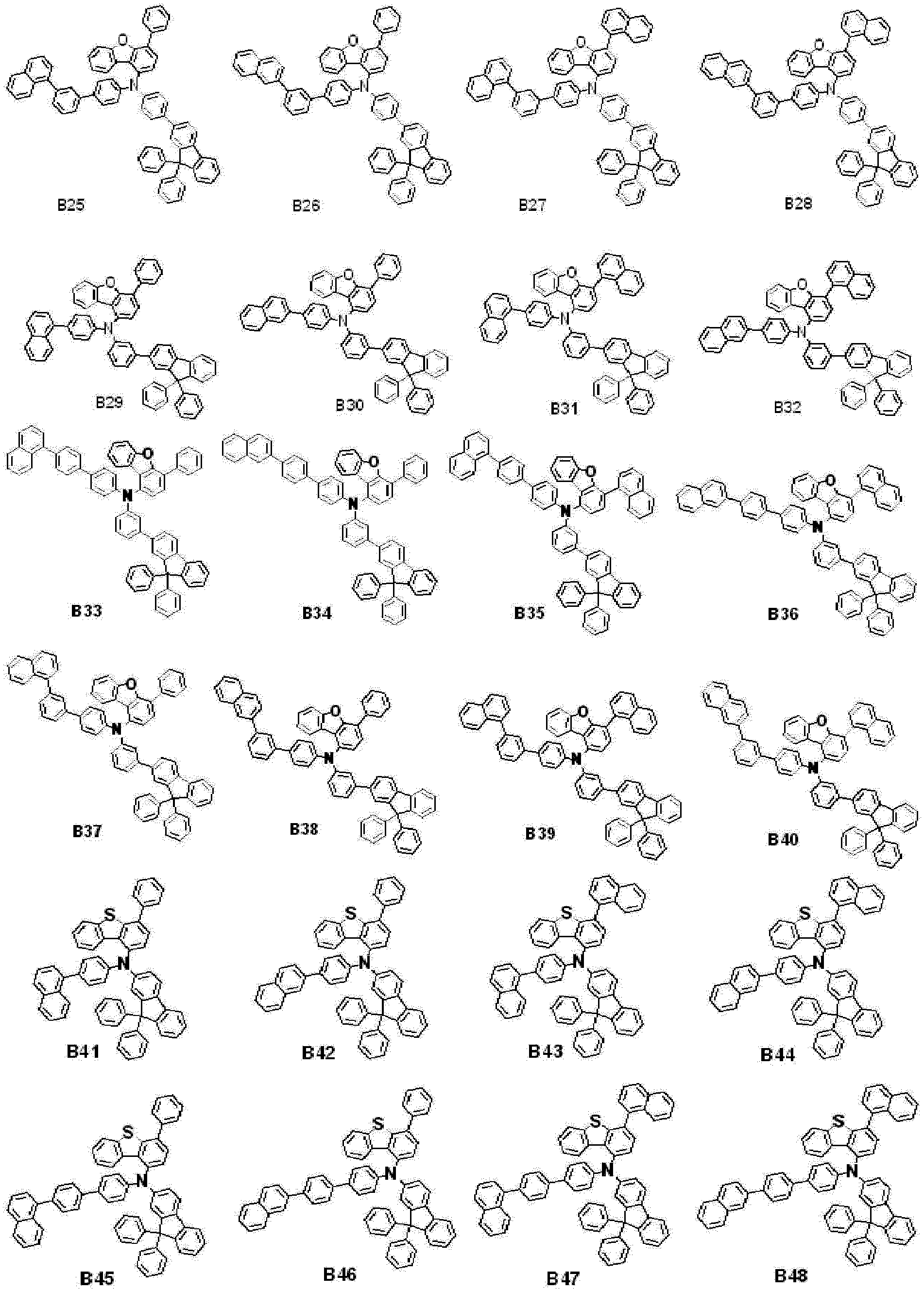

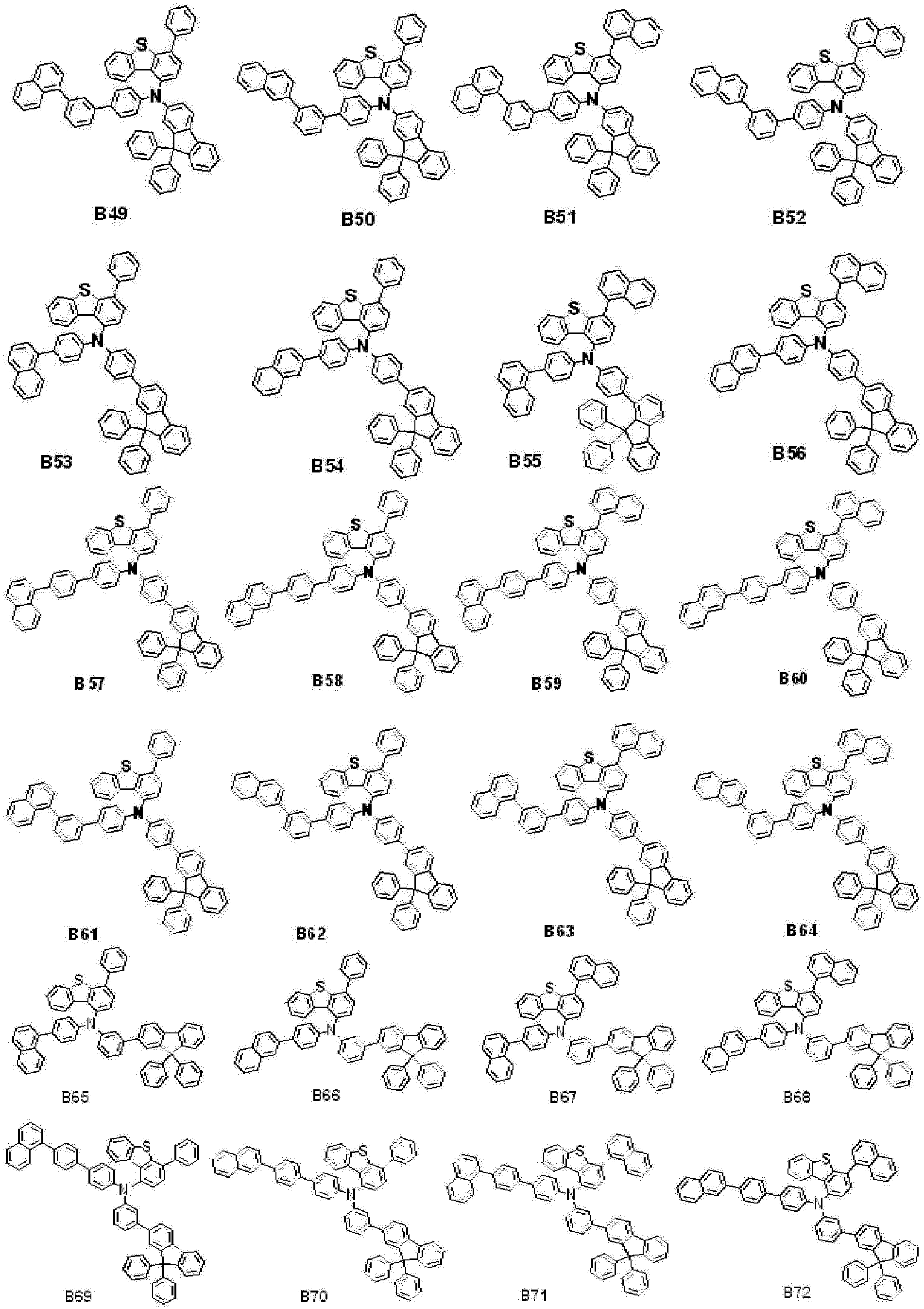

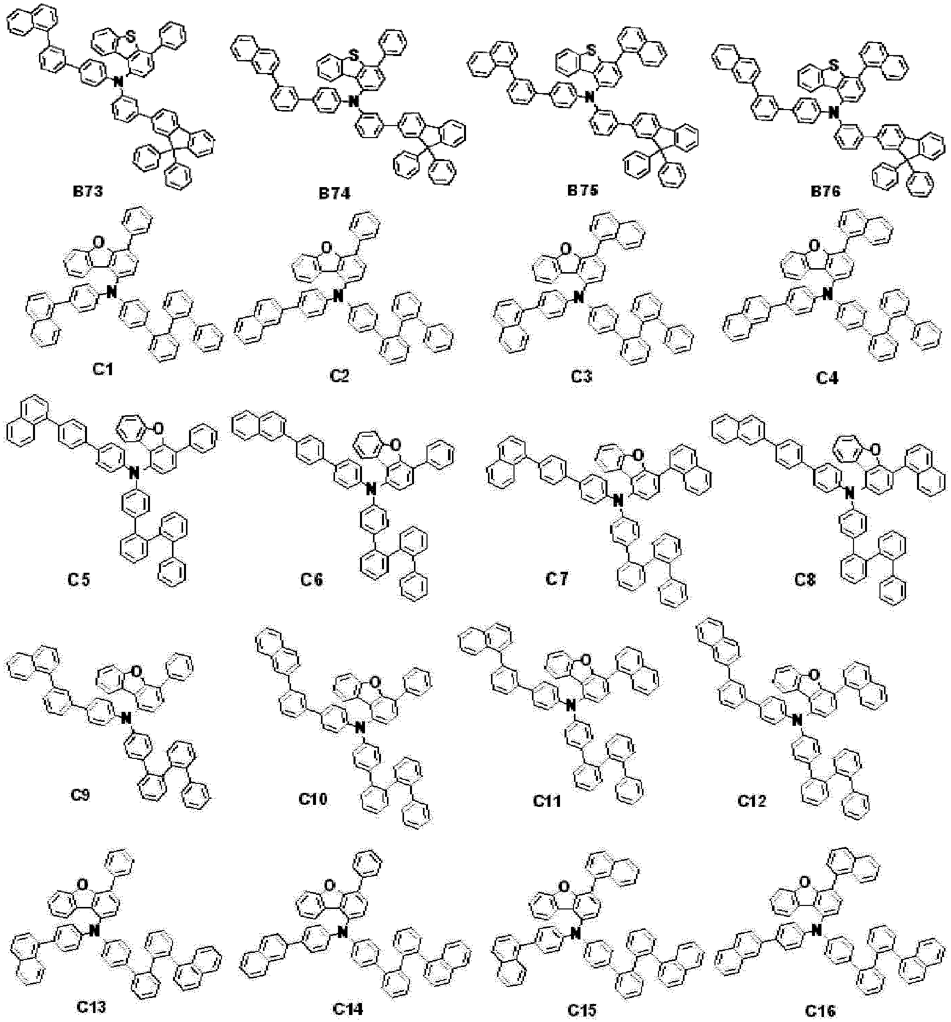

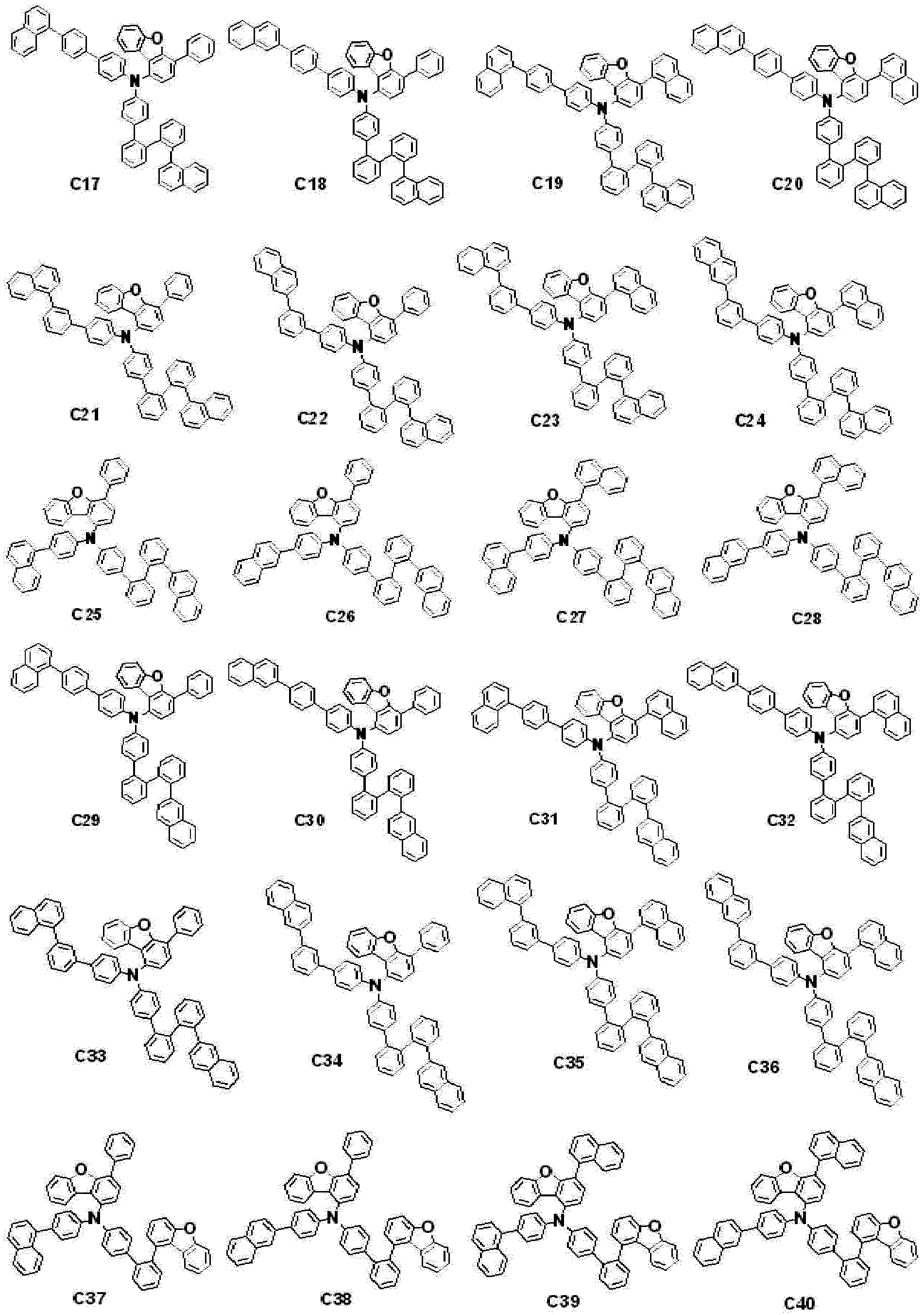

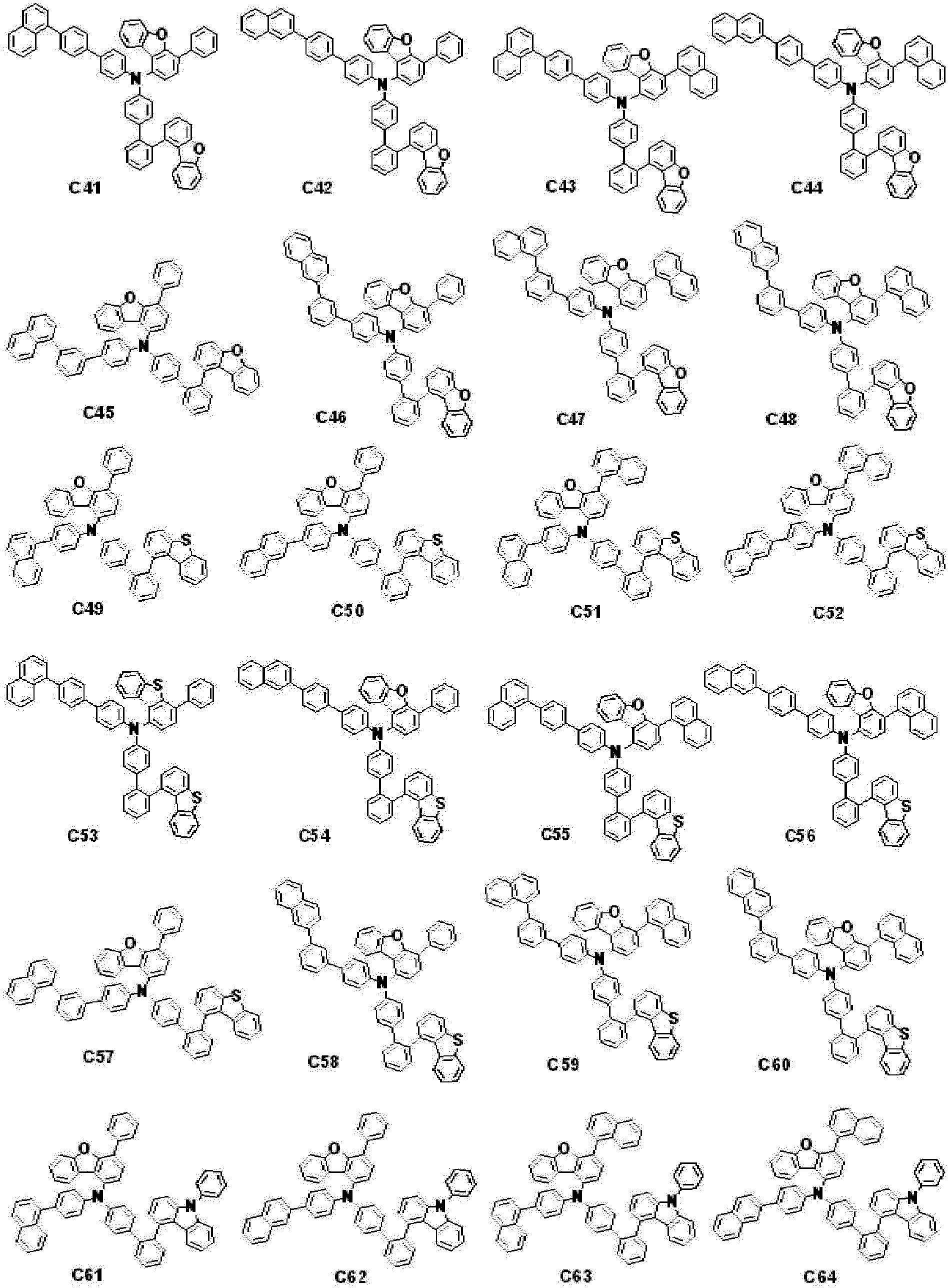

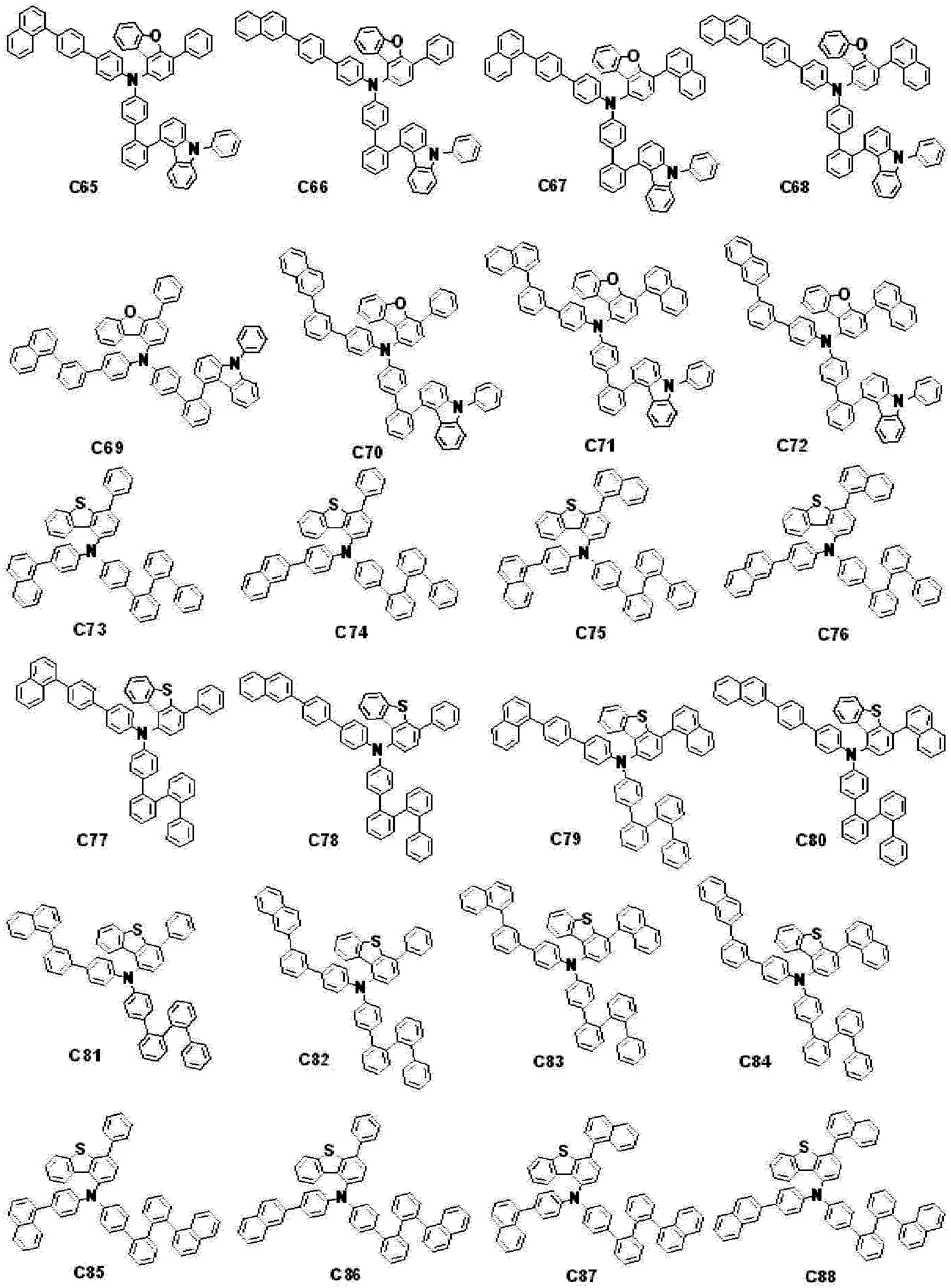

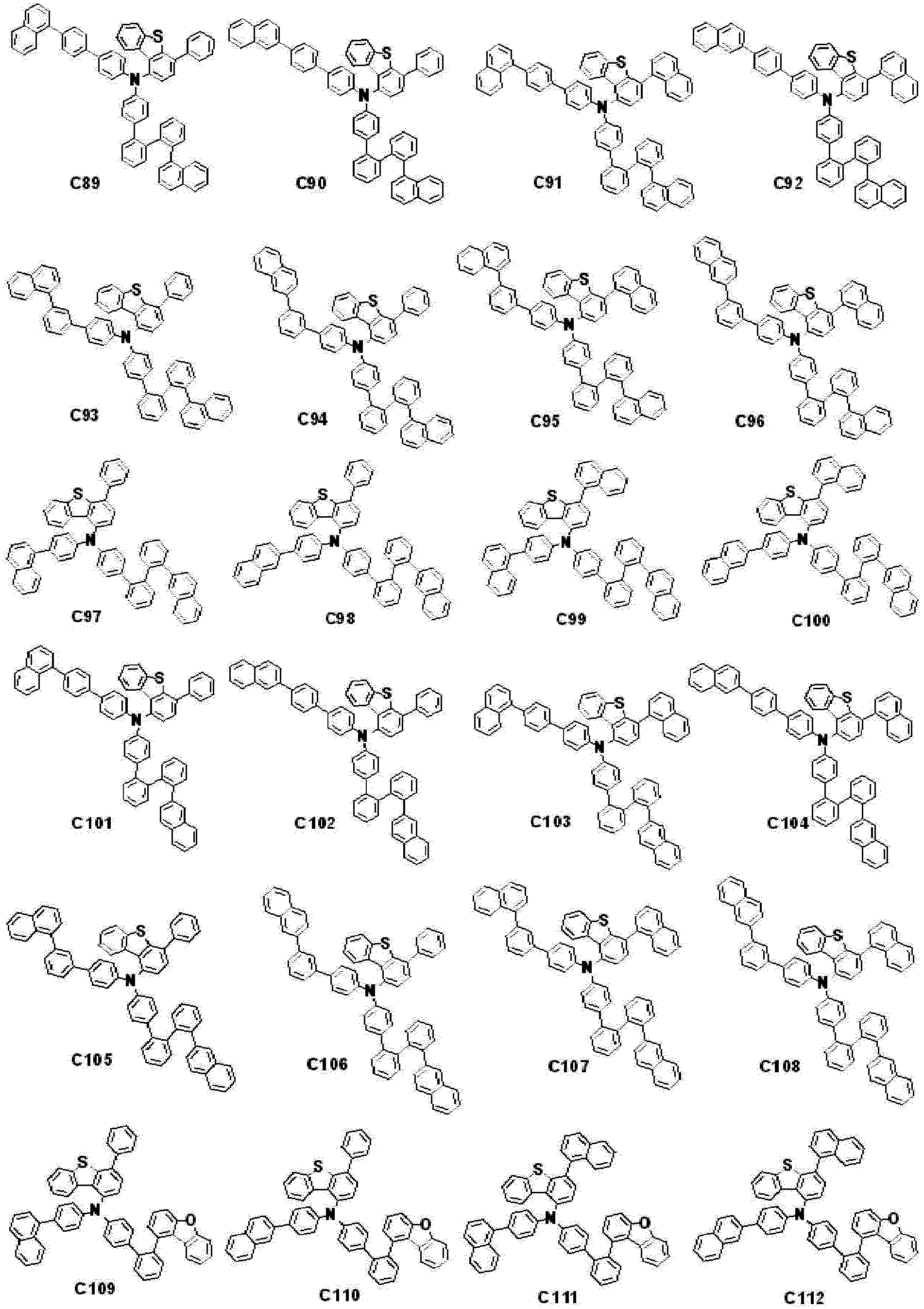

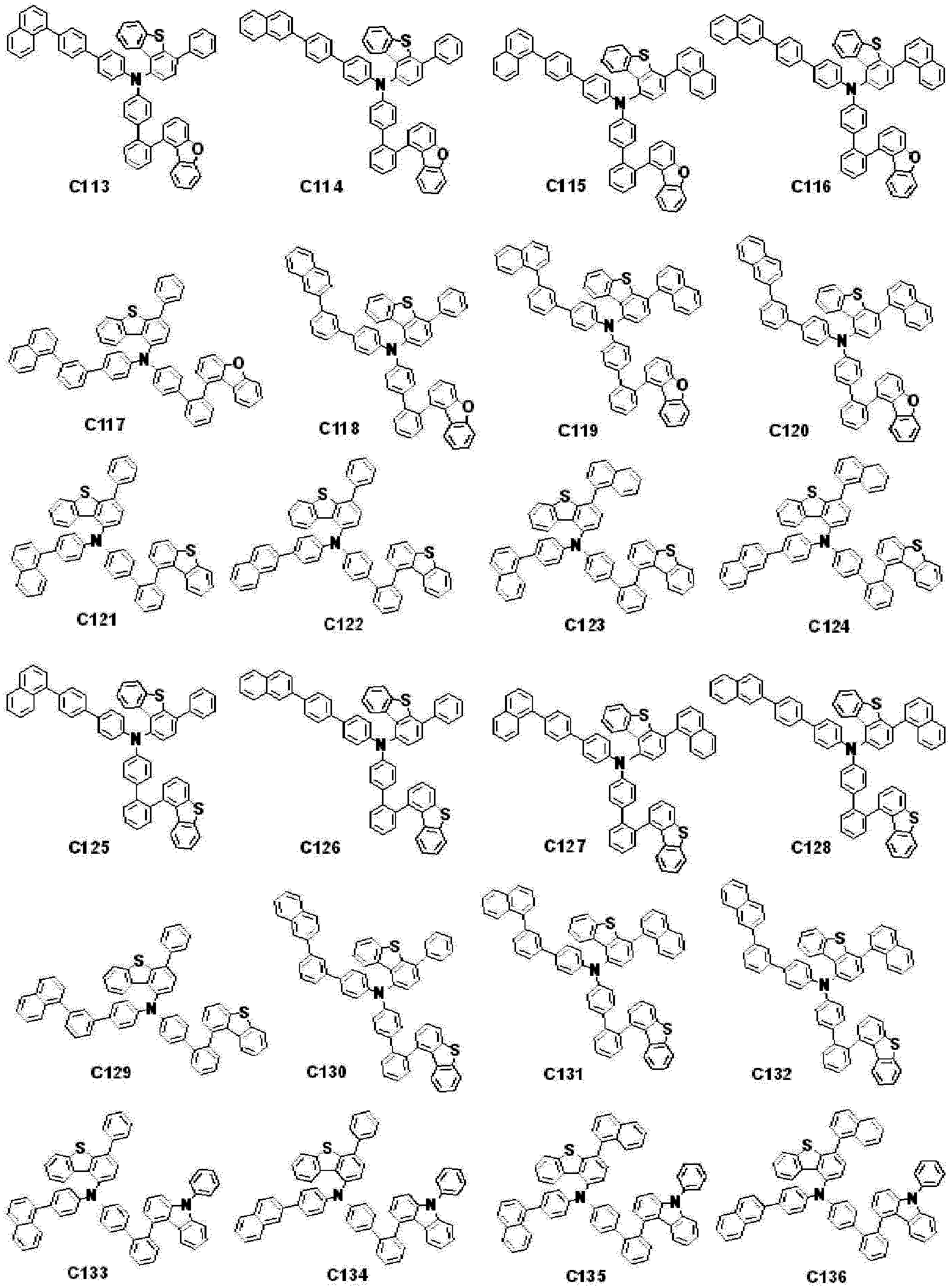

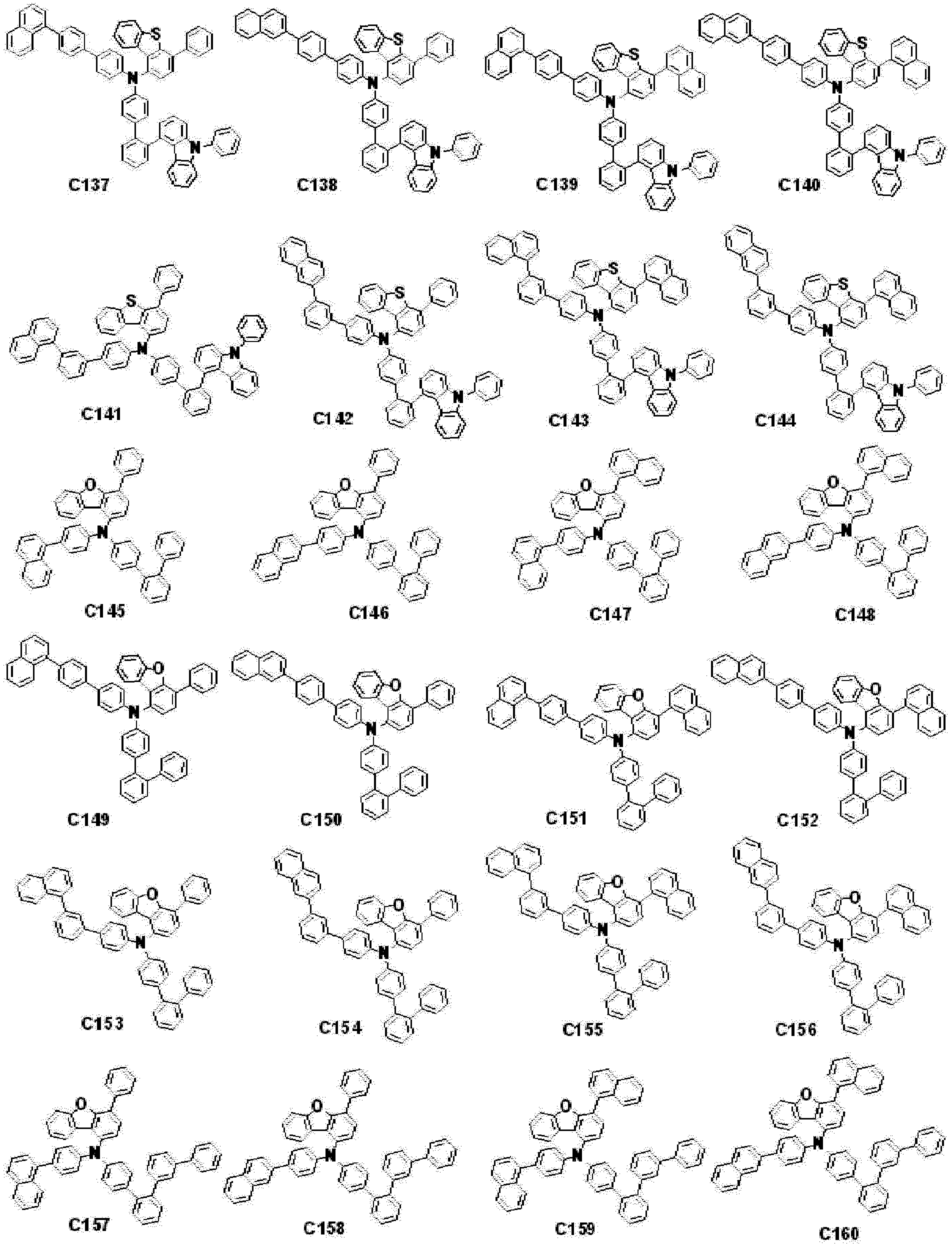

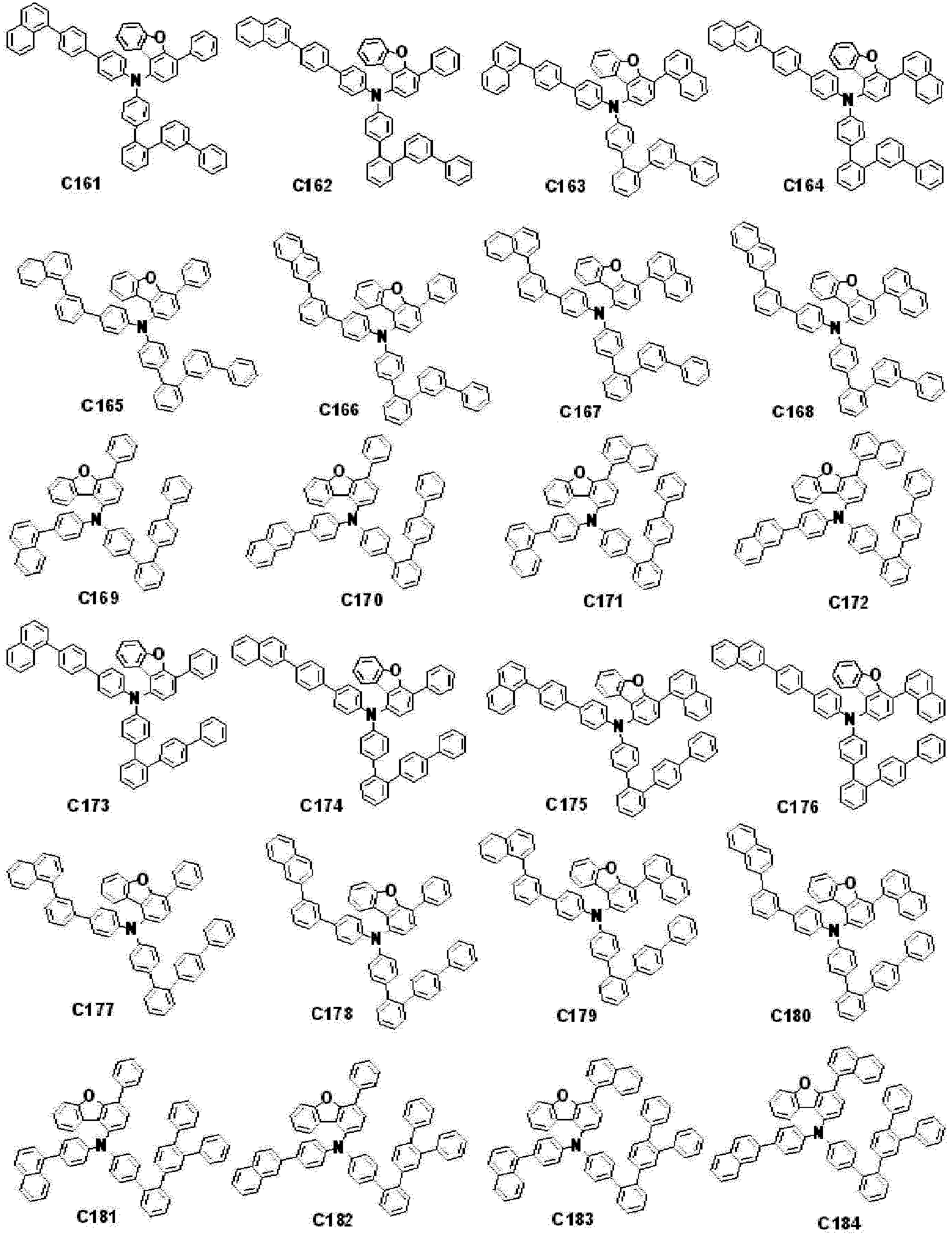

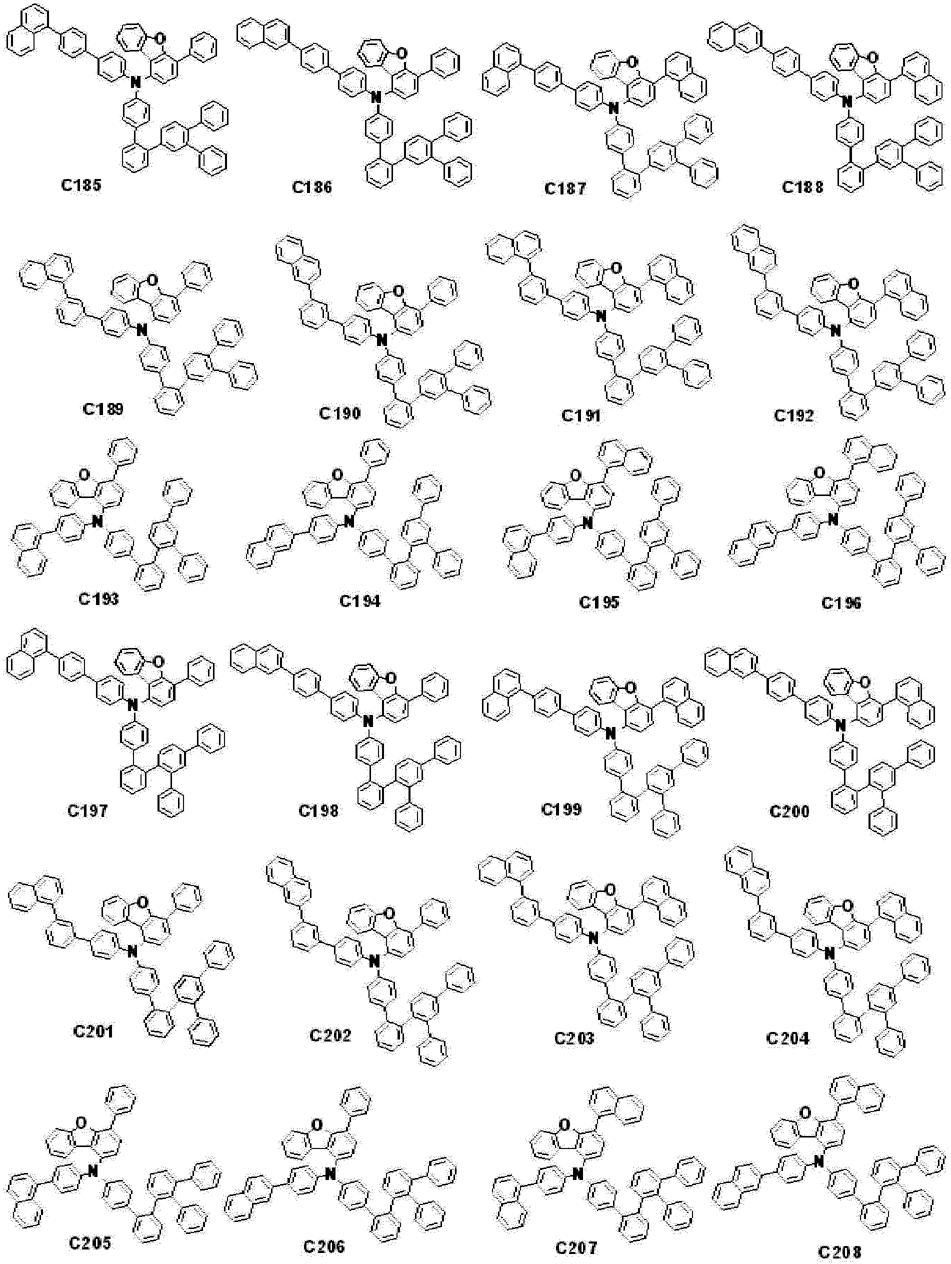

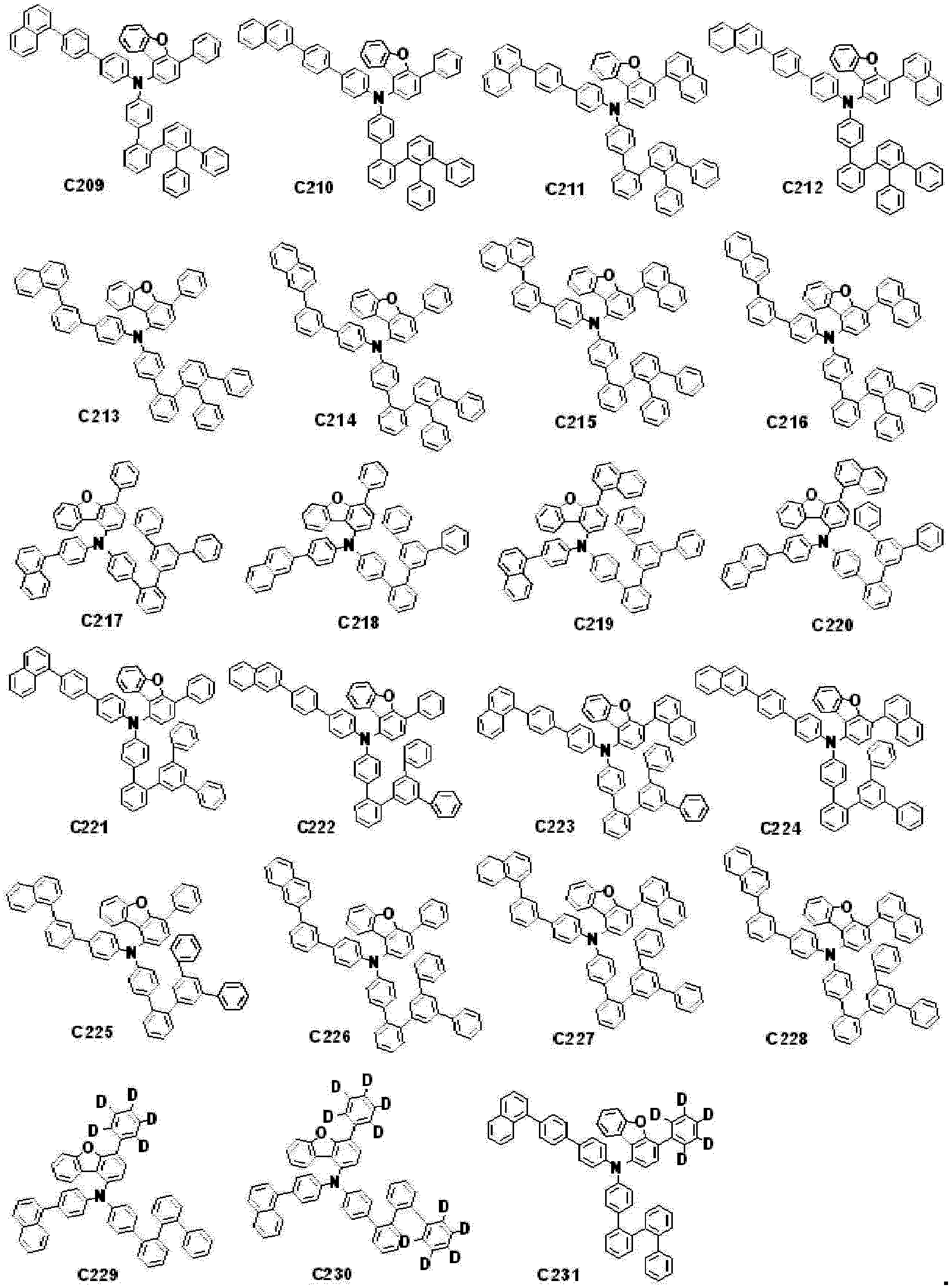

The amine compound of an embodiment, represented by Formula 1 includes a dibenzoheterole group substituted with an aryl group or a heteroaryl group, and may additionally include a fluorene group, a carbazole group, and/or a Z-type or kind aryl derivative as a substituent. The amine compound of an embodiment may have excellent or suitable electrical stability and high charge transport capacity due to the inclusion of these substituents and/or at the specific substitution position. Accordingly, the lifetime of the amine compound of an embodiment may be improved. In some embodiments, the emission efficiency and lifetime of the light emitting element of an embodiment, including the amine compound of an embodiment may be improved.

In the light emitting element ED of an embodiment, the hole transport region HTR may further include a compound represented by Formula H-1.

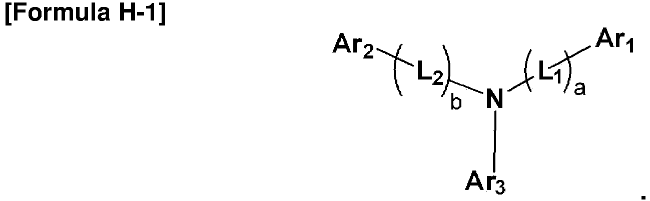

In Formula H-1 above, L₁ and L₂ may each independently be a direct linkage, a substituted or unsubstituted arylene group of 6 to 30 ring-forming carbon atoms, or a substituted or unsubstituted heteroarylene group of 2 to 30 ring-forming carbon atoms. "a" and "b" may each independently be an integer of 0 to 10. In some embodiments, when "a" or "b" is an integer of 2 or more, multiple L₁ and L₂ may each independently be a substituted or unsubstituted arylene group of 6 to 30 ring-forming carbon atoms, or a substituted or unsubstituted heteroarylene group of 2 to 30 ring-forming carbon atoms.

In Formula H-1, Ar₁ and Ar₂ may each independently be a substituted or unsubstituted aryl group of 6 to 30 ring-forming carbon atoms, or a substituted or unsubstituted heteroaryl group of 2 to 30 ring-forming carbon atoms. In some embodiments, in Formula H-1, Ar₃ may be a substituted or unsubstituted aryl group of 6 to 30 ring-forming carbon atoms, or a substituted or unsubstituted heteroaryl group of 2 to 30 ring-forming carbon atoms.

The compound represented by Formula H-1 may be a monoamine compound (e.g., a compound including a single amine group). In some embodiments, the compound represented by Formula H-1 may be a diamine compound in which at least one from among Ar₁ to Ar₃ includes an amine group as a substituent. In some embodiments, the compound represented by Formula H-1 may be a carbazole-based compound in which Ar₁ and/or Ar₂ includes a substituted or unsubstituted carbazole group, or a fluorene-based compound in which Ar₁ and/or Ar₂ includes a substituted or unsubstituted fluorene group.

The compound represented by Formula H-1 may be any one of the compounds in Compound Group H. However, the compounds listed in Compound Group H are only examples, and the compound represented by Formula H-1 is not limited to the compounds represented in Compound Group H.

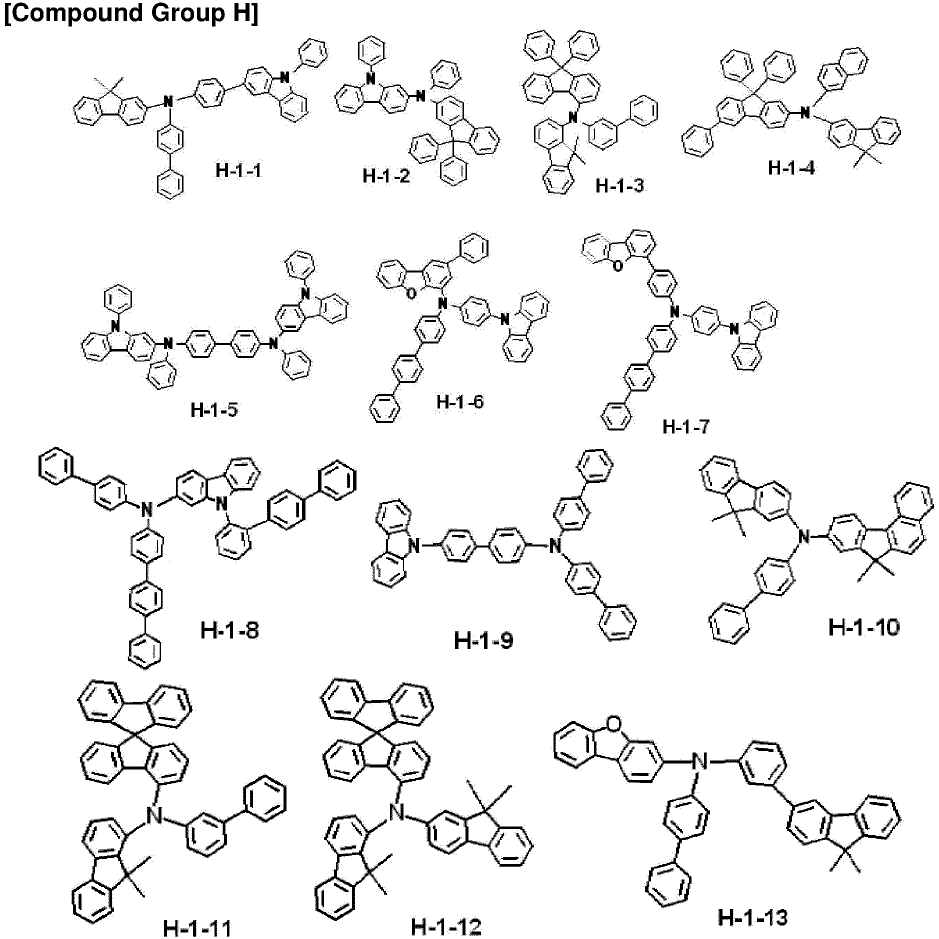

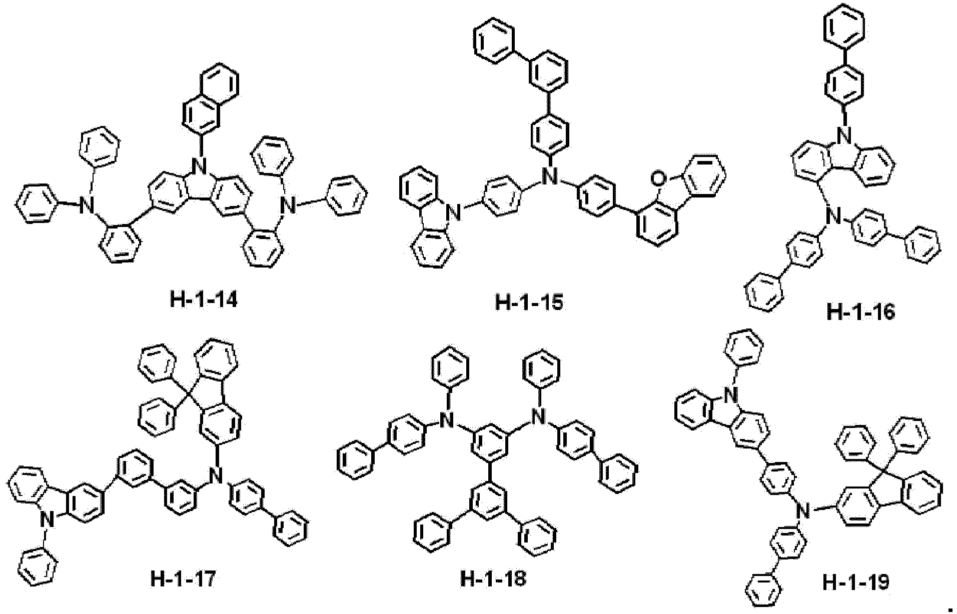

The hole transport region HTR may include a phthalocyanine compound such as copper phthalocyanine, N¹,N¹'-([1,1'-biphenyl]-4,4'-diyl)bis(N¹-phenyl-N⁴,N⁴-dim-tolylbenzene-1,4-diamine) (DNTPD), 4,4',4"-[tris(3-methylphenyl)phenylamino] triphenylamine (m-MTDATA), 4,4',4"-tris(N,N-diphenylamino)triphenylamine (TDATA), 4,4',4"-tris[N(2-naphthyl)-N-phenylamino]-triphenylamine (2-TNATA), poly(3,4-ethylenedioxythiophene)/poly(4-styrenesulfonate) (PEDOT/PSS), polyaniline/dodecylbenzenesulfonic acid (PANI/DBSA), polyaniline/camphor sulfonic acid (PANI/CSA), polyaniline/poly(4-styrenesulfonate) (PANI/PSS), N,N'-di(1-naphthalene-1-yl)-N,N'-diphenyl-benzidine (NPB or NPD), triphenylamine-containing polyetherketone (TPAPEK), 4-isopropyl-4'-methyldiphenyliodonium [tetrakis(pentafluorophenyl)borate], and/or dipyrazino[2,3-f:2',3'-h] quinoxaline-2,3,6,7,10,11-hexacarbonitrile (HAT-CN).

The hole transport region HTR may include carbazole derivatives such as N-phenyl carbazole and polyvinyl carbazole, fluorene-based derivatives, N,N'-bis(3-methylphenyl)-N,N'-diphenyl-[1,1'-biphenyl]-4,4'-diamine (TPD), triphenylamine-based derivatives such as 4,4',4"-tris(N-carbazolyl)triphenylamine (TCTA), N,N'-di(1-naphthalene-1-yl)-N,N'-diphenyl-benzidine (NPB), 4,4'-cyclohexylidene bis[N,N-bis(4-methylphenyl)benzeneamine] (TAPC), 4,4'-bis[N,N'-(3-tolyl)amino]-3,3'-dimethylbiphenyl (HMTPD), 1,3-bis(N-carbazolyl)benzene (mCP), etc.

In some embodiments, the hole transport region HTR may include 9-(4-tert-butylphenyl)-3,6-bis(triphenylsilyl)-9H-carbazole (CzSi), 9-phenyl-9H-3,9'-bicarbazole (CCP), 1,3-bis(1,8-dimethyl-9H-carbazol-9-yl)benzene (mDCP), etc.

The hole transport region HTR may include the above-described compounds of the hole transport region HTR in at least one from among the hole injection layer HIL, the hole transport layer HTL, and the electron blocking layer EBL.

The thickness of the hole transport region HTR may be from about 100 Å to about 10,000 Å, for example, from about 100 Å to about 5,000 Å. When the hole transport region HTR includes a hole injection layer HIL, the thickness of the hole injection region HIL may be, for example, from about 30 Å to about 1,000 Å. When the hole transport region HTR includes a hole transport layer HTL, the thickness of the hole transport layer HTL may be from about 30 Å to about 1,000 Å. For example, when the hole transport region HTR includes an electron blocking layer EBL, the thickness of the electron blocking layer EBL may be from about 10 Å to about 1,000 Å. When the thicknesses of the hole transport region HTR, the hole injection layer HIL, the hole transport layer HTL and the electron blocking layer EBL satisfy the above-described ranges, satisfactory hole transport properties may be achieved without a substantial increase of a driving voltage.

The hole transport region HTR may further include a charge generating material to increase conductivity in addition to the above-described materials. The charge generating material may be dispersed uniformly or non-uniformly in the hole transport region HTR. The charge generating material may be, for example, a p-dopant. The p-dopant may include one or more of metal halide compounds, quinone derivatives, metal oxides, and cyano group-containing compounds, but the embodiment of the present disclosure is not limited thereto. For example, the p-dopant may include one or more metal halide compounds such as Cul and/or Rbl, quinone derivatives such as tetracyanoquinodimethane (TCNQ) and/or 2,3,5,6-tetrafluoro-7,7',8,8-tetracyanoquinodimethane (F4-TCNQ), metal oxides such as tungsten oxide and/or molybdenum oxide, cyano group-containing compounds such as dipyrazino[2,3-f: 2',3'-h] quinoxaline-2,3,6,7,10,11-hexacarbonitrile (HATCN) and/or 4-[[2,3-bis[cyano-(4-cyano-2,3,5,6-tetrafluorophenyl)methylidene]cyclopropylidene]-cyanomethyl]-2,3,5,6-tetrafluorobenzonitrile (NDP9), etc., but the embodiment of the present disclosure is not limited thereto.

As described above, the hole transport region HTR may further include a buffer layer and/or an electron blocking layer EBL in addition to the hole injection layer HIL and the hole transport layer HTL. The buffer layer may compensate for a resonance distance according to the wavelength of light emitted from an emission layer EML and may thus increase emission efficiency. As materials included in the buffer layer, materials which may be included in the hole transport region HTR may be utilized. The electron blocking layer EBL is a layer playing the role of blocking the injection of electrons from the electron transport region ETR to the hole transport region HTR.

The emission layer EML is provided on the hole transport region HTR. The emission layer EML may have a thickness of, for example, about 100 Å to about 1,000 Å or about 100 Å to about 300 Å. The emission layer EML may have a single layer formed utilizing a single material, a single layer formed utilizing multiple different materials, or a multilayer structure having multiple layers formed utilizing multiple different materials.

In the light emitting element ED of an embodiment, the emission layer EML may be to emit blue light. The light emitting element ED of an embodiment may include the amine compound of an embodiment in a hole transport region HTR and may show suitable or high efficiency and long-life characteristics in a blue emission region. However, an embodiment of the present disclosure is not limited thereto.

In the light emitting element ED of an embodiment, the emission layer EML may include one or more anthracene derivatives, pyrene derivatives, fluoranthene derivatives, chrysene derivatives, dihydrobenzanthracene derivatives, and/or triphenylene derivatives. For example, the emission layer EML may include one or more anthracene derivatives and/or pyrene derivatives.

In the light emitting elements ED of embodiments, shown in FIG. 3 to FIG. 6, the emission layer EML may include a host and a dopant, and the emission layer EML may include a compound represented by Formula E-1. The compound represented by Formula E-1 may be utilized as a fluorescence host material.

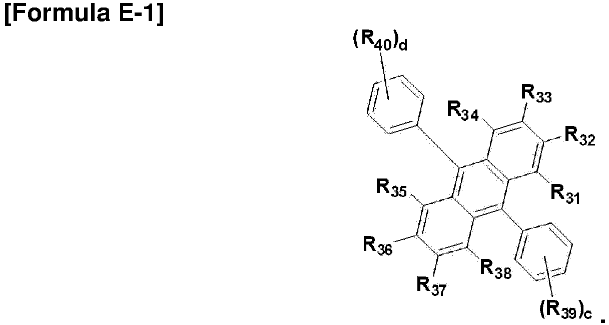

In Formula E-1, R₃₁ to R₄₀ may each independently be a hydrogen atom, a deuterium atom, a halogen atom, a substituted or unsubstituted silyl group, a substituted or unsubstituted thio group, a substituted or unsubstituted oxy group, a substituted or unsubstituted alkyl group of 1 to 10 carbon atoms, a substituted or unsubstituted alkenyl group of 2 to 10 carbon atoms, a substituted or unsubstituted aryl group of 6 to 30 ring-forming carbon atoms, a substituted or unsubstituted heteroaryl group of 2 to 30 ring-forming carbon atoms, or combined with an adjacent group to form a ring. In some embodiments, R₃₁ to R₄₀ may be combined with an adjacent group to form a saturated hydrocarbon ring, an unsaturated hydrocarbon ring, a saturated heterocycle, or an unsaturated heterocycle.

In Formula E-1, R₃₁ to R₄₀ may each independently be a hydrogen atom, a deuterium atom, a halogen atom, a substituted or unsubstituted silyl group, a substituted or unsubstituted thio group, a substituted or unsubstituted oxy group, a substituted or unsubstituted alkyl group of 1 to 10 carbon atoms, a substituted or unsubstituted alkenyl group of 2 to 10 carbon atoms, a substituted or unsubstituted aryl group of 6 to 30 ring-forming carbon atoms, or a substituted or unsubstituted heteroaryl group of 2 to 30 ring-forming carbon atoms; optionally wherein when one or more of R₃₁ to R₄₀ is a substituted or unsubstituted silyl group, a substituted or unsubstituted thio group, a substituted or unsubstituted oxy group, a substituted or unsubstituted alkyl group of 1 to 10 carbon atoms, a substituted or unsubstituted alkenyl group of 2 to 10 carbon atoms, a substituted or unsubstituted aryl group of 6 to 30 ring-forming carbon atoms, or a substituted or unsubstituted heteroaryl group of 2 to 30 ring-forming carbon atoms, said R₃₁ to R₄₀ may be individually combined with an adjacent group to form a ring. In some embodiments, R₃₁ to R₄₀ may be combined with an adjacent group to form a saturated hydrocarbon ring, an unsaturated hydrocarbon ring, a saturated heterocycle, or an unsaturated heterocycle.

In Formula E-1, "c" and "d" may each independently be an integer of 0 to 5.

The compound represented by Formula E-1 may be any one of Compound E1 to Compound E19.

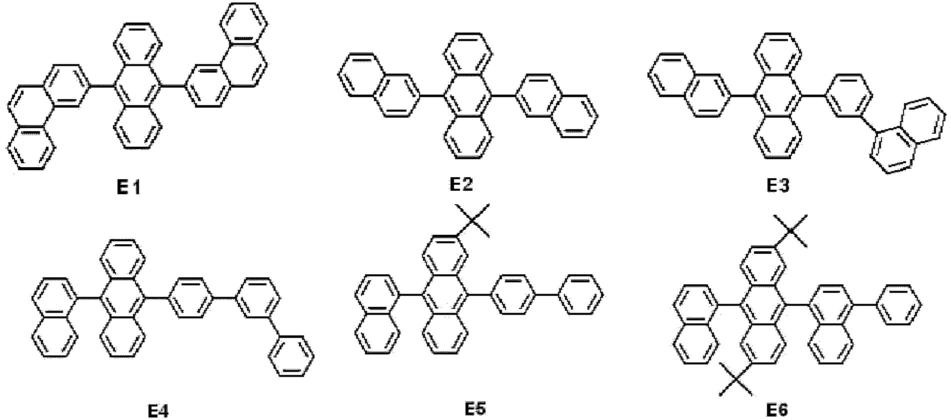

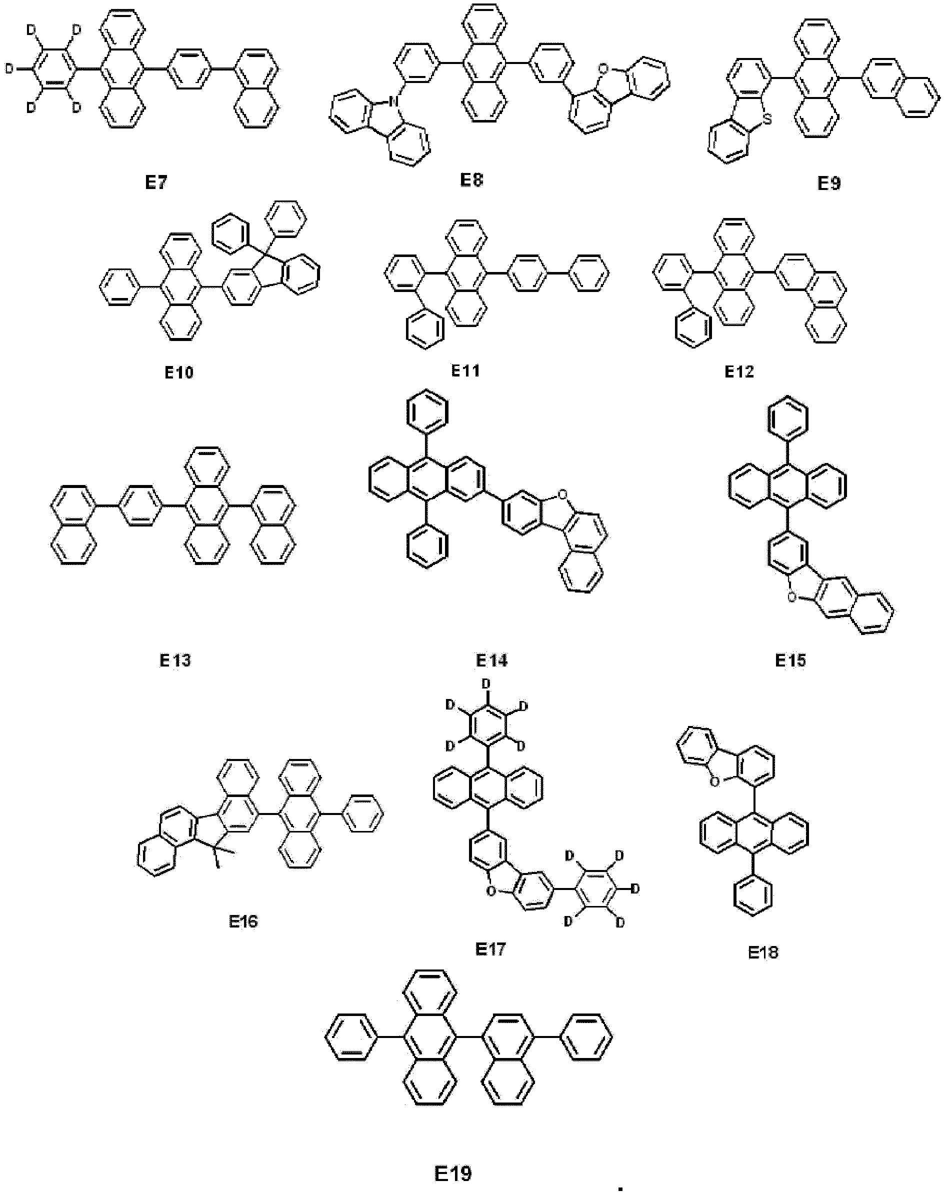

In an embodiment, the emission layer EML may include a compound represented by Formula E-2a or Formula E-2b. The compound represented by Formula E-2a or Formula E-2b may be utilized as a phosphorescence host material.

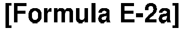

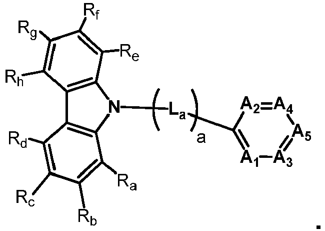

In Formula E-2a, "a" may be an integer of 0 to 10, and Lₐ may be a direct linkage, a substituted or unsubstituted arylene group of 6 to 30 ring-forming carbon atoms, or a substituted or unsubstituted heteroarylene group of 2 to 30 ring-forming carbon atoms. In some embodiments, when "a" is an integer of 2 or more, multiple Lₐ may each independently be a substituted or unsubstituted arylene group of 6 to 30 ring-forming carbon atoms, or a substituted or unsubstituted heteroarylene group of 2 to 30 ring-forming carbon atoms.

In some embodiments, in Formula E-2a, A₁ to A₅ may each independently be N or CRi. Rₐ to Rᵢ may each independently be a hydrogen atom, a deuterium atom, a substituted or unsubstituted amine group, a substituted or unsubstituted thio group, a substituted or unsubstituted oxy group, a substituted or unsubstituted alkyl group of 1 to 20 carbon atoms, a substituted or unsubstituted alkenyl group of 2 to 20 carbon atoms, a substituted or unsubstituted aryl group of 6 to 30 ring-forming carbon atoms, a substituted or unsubstituted heteroaryl group of 2 to 30 ring-forming carbon atoms, or combined with an adjacent group to form a ring. In an embodiment, Rₐ to Rᵢ may be combined with an adjacent group to form a hydrocarbon ring or a heterocycle including N, O, S, etc. as a ring-forming atom.

In some embodiments, in Formula E-2a, two or three selected from A₁ to A₅ may be N, and any remainder thereof may be CRᵢ.

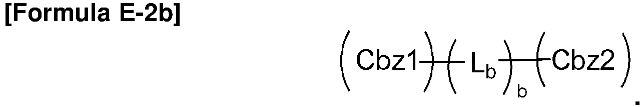

In Formula E-2b, Cbz1 and Cbz2 may each independently be an unsubstituted carbazole group, or a carbazole group substituted with an aryl group of 6 to 30 ring-forming carbon atoms. L_{b} may be a direct linkage, a substituted or unsubstituted arylene group of 6 to 30 ring-forming carbon atoms, or a substituted or unsubstituted heteroarylene group of 2 to 30 ring-forming carbon atoms. "b" is an integer of 0 to 10, and when "b" is an integer of 2 or more, multiple L_{b} may each independently be a substituted or unsubstituted arylene group of 6 to 30 ring-forming carbon atoms, or a substituted or unsubstituted heteroarylene group of 2 to 30 ring-forming carbon atoms.

The compound represented by Formula E-2a or Formula E-2b may be any one of the compounds in Compound Group E-2. However, the compounds listed in Compound Group E-2 are only examples, and the compound represented by Formula E-2a or Formula E-2b is not limited to the compounds represented in Compound Group E-2.

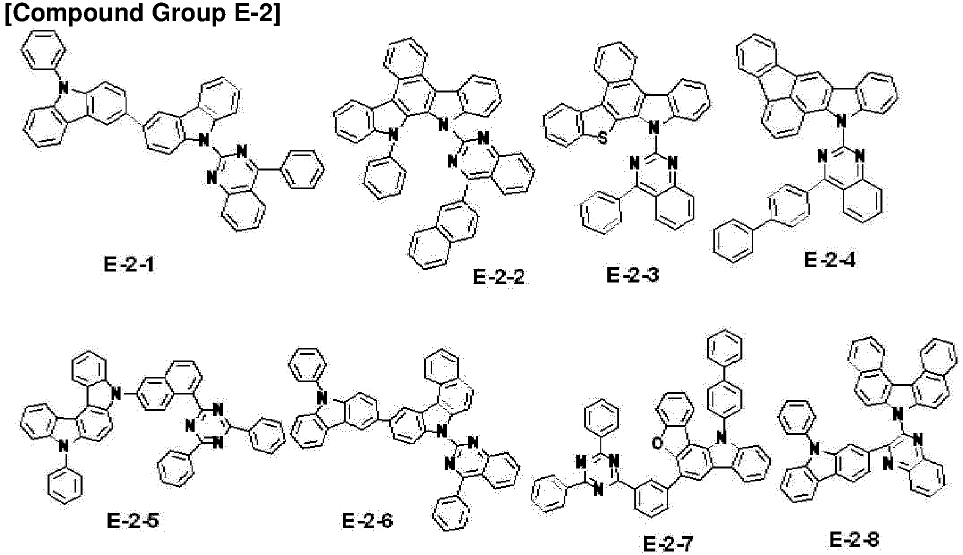

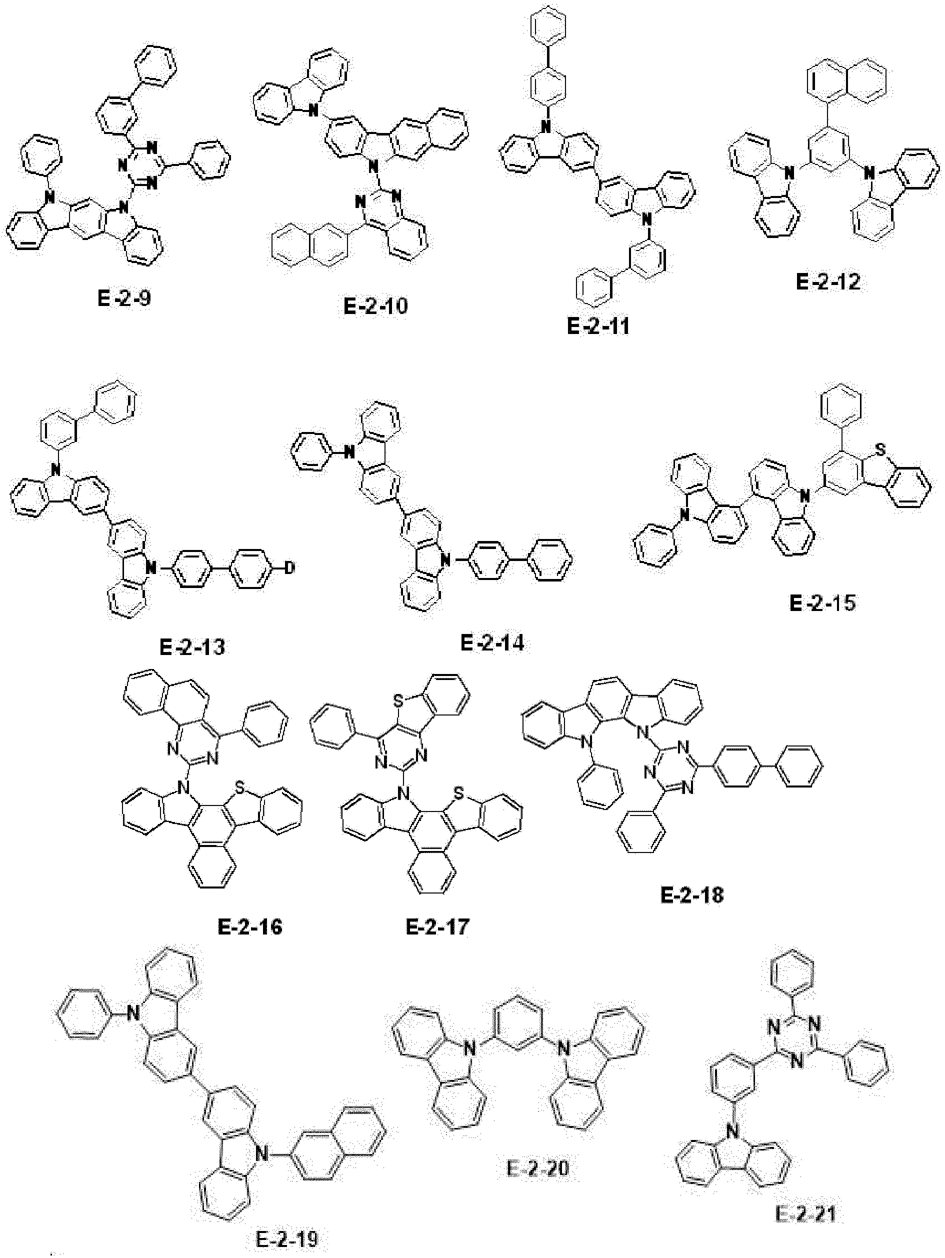

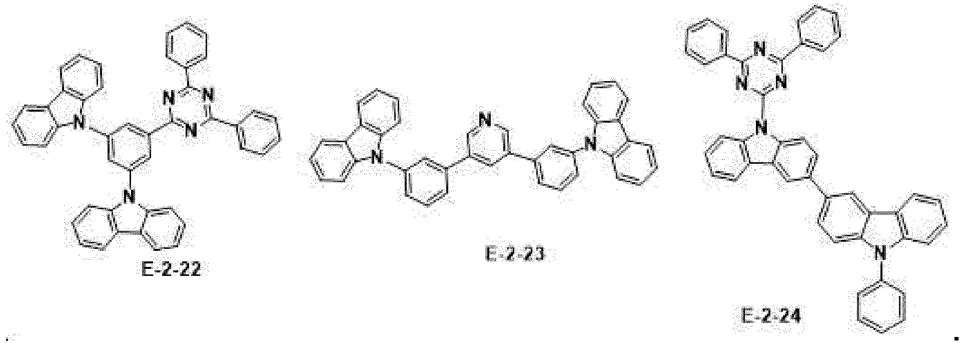

The emission layer EML may further include a suitable material in the art as a host material. For example, the emission layer EML may include as a host material, at least one of bis (4-(9H-carbazol-9-yl) phenyl) diphenylsilane (BCPDS), (4-(1-(4-(diphenylamino) phenyl) cyclohexyl) phenyl) diphenyl-phosphine oxide (POPCPA), bis[2-(diphenylphosphino)phenyl] ether oxide (DPEPO), 4,4'-bis(carbazol-9-yl)biphenyl (CBP), 1,3-bis(carbazol-9-yl)benzene (mCP), 2,8-bis(diphenylphosphoryl)dibenzo[b,d]furan (PPF), 4,4',4"-tris(carbazol-9-yl)-triphenylamine (TCTA), or 1,3,5-tris(1-phenyl-1H-benzo[d]imidazole-2-yl)benzene (TPBi). However, an embodiment of the present disclosure is not limited thereto. For example, tris(8-hydroxyquinolino)aluminum (Alq₃), 9,10-di(naphthalene-2-yl)anthracene (ADN), 2-tert-butyl-9,10-di(naphth-2-yl)anthracene (TBADN), distyrylarylene (DSA), 4,4'-bis(9-carbazolyl)-2,2'-dimethyl-biphenyl (CDBP), 2-methyl-9,10-bis(naphthalen-2-yl)anthracene (MADN), hexaphenyl cyclotriphosphazene (CP1), 1,4-bis(triphenylsilyl)benzene (UGH2), hexaphenylcyclotrisiloxane (DPSiO₃), octaphenylcyclotetra siloxane (DPSiO₄), etc. may be utilized as the host material.

The emission layer EML may include a compound represented by Formula M-a or Formula M-b. The compound represented by Formula M-a or Formula M-b may be utilized as a phosphorescence dopant material. In some embodiments, the compound represented by Formula M-a or Formula M-b may be utilized as an auxiliary dopant material.

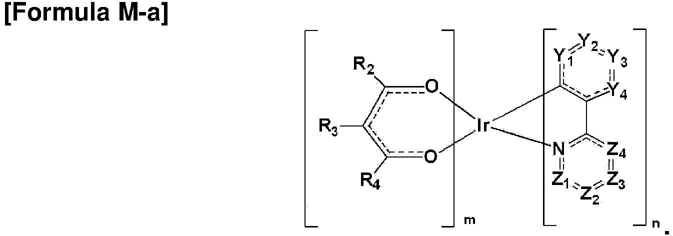

In Formula M-a, Y₁ to Y₄, and Z₁ to Z₄ may each independently be CR₁ or N, and R₁ to R₄ may each independently be a hydrogen atom, a deuterium atom, a substituted or unsubstituted amine group, a substituted or unsubstituted thio group, a substituted or unsubstituted oxy group, a substituted or unsubstituted alkyl group of 1 to 20 carbon atoms, a substituted or unsubstituted alkenyl group of 2 to 20 carbon atoms, a substituted or unsubstituted aryl group of 6 to 30 ring-forming carbon atoms, a substituted or unsubstituted heteroaryl group of 2 to 30 ring-forming carbon atoms, or combined with an adjacent group to form a ring. In Formula M-a, "m" may be 0 or 1, and "n" may be 2 or 3. In Formula M-a, when "m" is 0, "n" is 3, and when "m" is 1, "n" is 2.

The compound represented by Formula M-a may be utilized as a phosphorescence dopant.

The compound represented by Formula M-a may be any one of Compounds M-a1 to M-a25. However, Compounds M-a1 to M-a25 are examples, and the compound represented by Formula M-a is not limited to the compounds represented by Compounds M-a1 to M-a25.

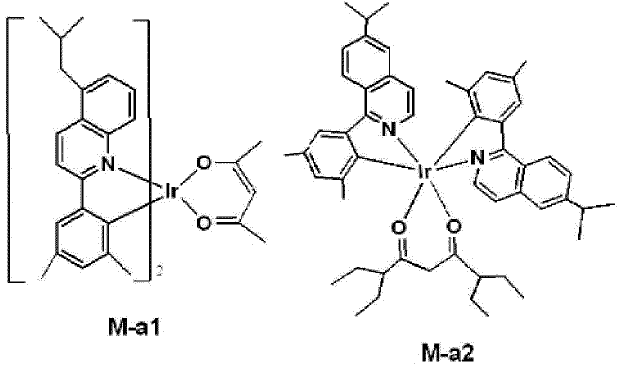

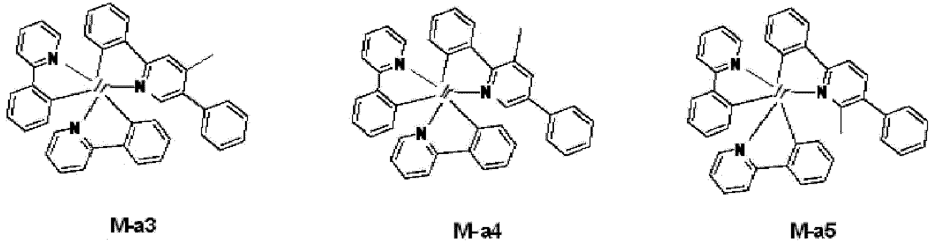

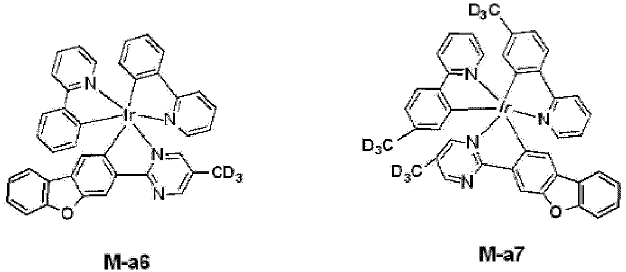

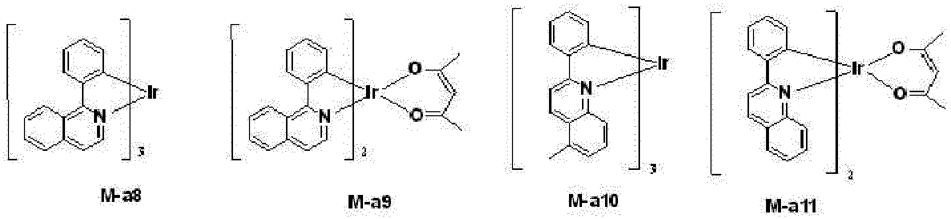

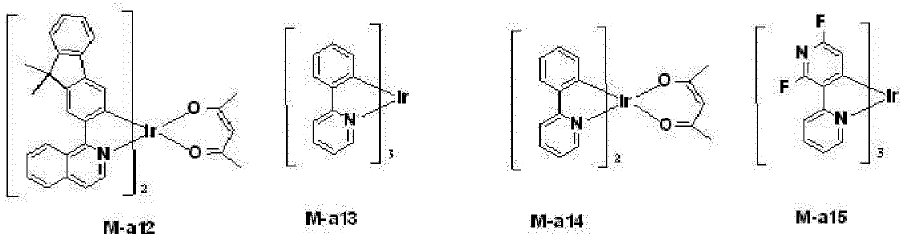

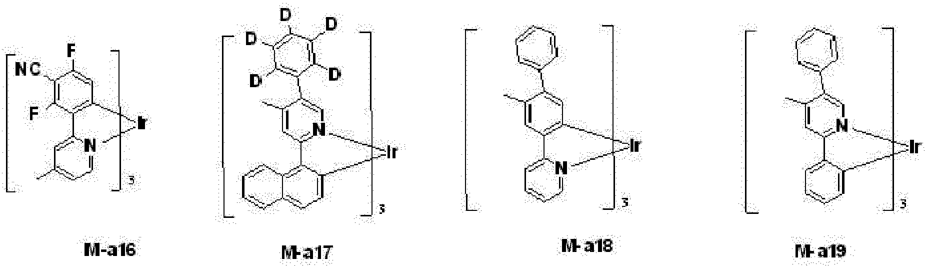

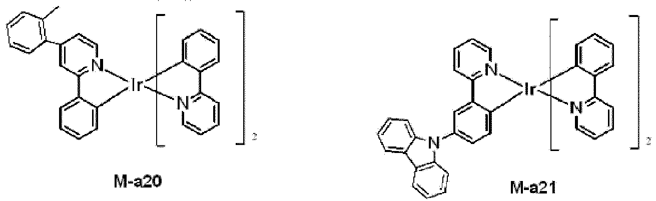

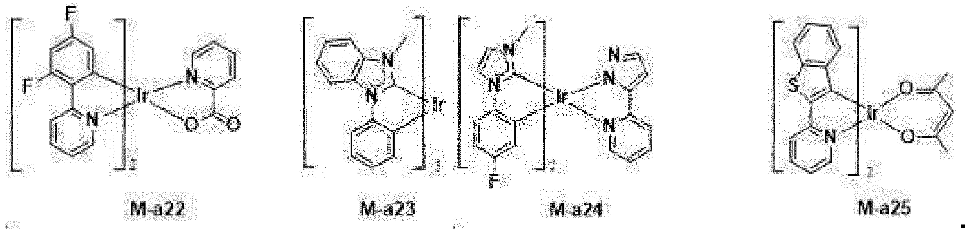

Compound M-a1 and Compound M-a2 may be utilized as red dopant materials, and Compound M-a3 to Compound M-a7 may be utilized as green dopant materials.

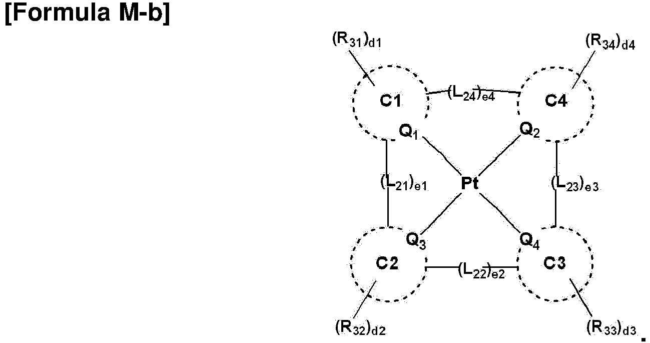

In Formula M-b, Q₁ to Q₄ may each independently be C or N, C1 to C4 may each independently be a substituted or unsubstituted hydrocarbon ring of 5 to 30 ring-forming carbon atoms, or a substituted or unsubstituted heterocycle of 2 to 30 ring-forming carbon atoms. L₂₁ to L₂₄ may each independently be a direct linkage, *-O-*, *-S-*, 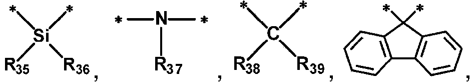 a substituted or unsubstituted divalent alkyl group of 1 to 20 carbon atoms, a substituted or unsubstituted arylene group of 6 to 30 ring-forming carbon atoms, or a substituted or unsubstituted heteroarylene group of 2 to 30 ring-forming carbon atoms, and e1 to e4 may each independently be 0 or 1. R₃₁ to R₃₉ may each independently be a hydrogen atom, a deuterium atom, a halogen atom, a cyano group, a substituted or unsubstituted amine group, a substituted or unsubstituted alkyl group of 1 to 20 carbon atoms, a substituted or unsubstituted aryl group of 6 to 30 ring-forming carbon atoms, a substituted or unsubstituted heteroaryl group of 2 to 30 ring-forming carbon atoms, or combined with an adjacent group to form a ring, and d1 to d4 may each independently be an integer of 0 to 4.

The compound represented by Formula M-b may be utilized as a blue phosphorescence dopant or a green phosphorescence dopant. In some embodiments, the compound represented by Formula M-b may be an auxiliary dopant in an embodiment and may be further included in the emission layer EML.

The compound represented by Formula M-b may be represented by any one of the compounds below. However, the compounds below are examples, and the compound represented by Formula M-b is not limited to the compounds represented below.

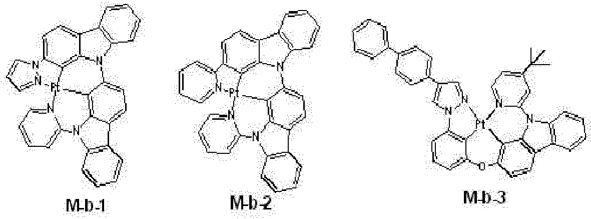

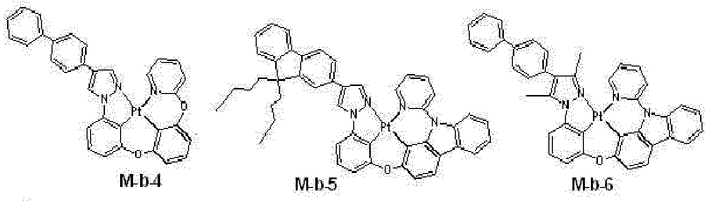

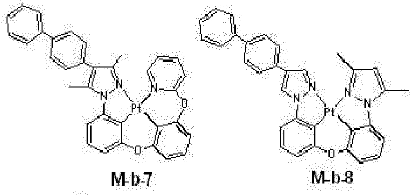

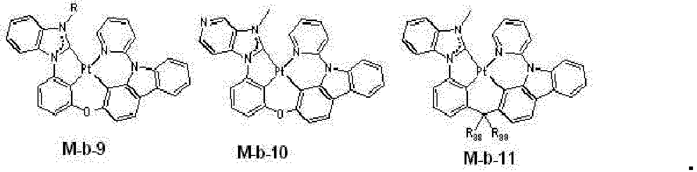

In the compounds above, R, R₃₈, and R₃₉ may each independently be a hydrogen atom, a deuterium atom, a halogen atom, a cyano group, a substituted or unsubstituted amine group, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms.

The emission layer EML may include a compound represented by any one from among Formula F-a to Formula F-c. The compounds represented by Formula F-a to Formula F-c may be utilized as fluorescence dopant materials.

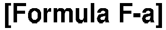

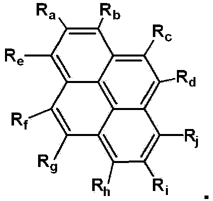

In Formula F-a, two groups selected from Rₐ to Rⱼ may each independently be substituted with *-NAr₁Ar₂. Any remainder not substituted with *-NAr₁Ar₂ from among Rₐ to Rⱼ may each independently be a hydrogen atom, a deuterium atom, a halogen atom, a cyano group, a substituted or unsubstituted amine group, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms.

In *-NAr₁Ar₂, Ar₁ and Ar₂ may each independently be a substituted or unsubstituted aryl group of 6 to 30 ring-forming carbon atoms, or a substituted or unsubstituted heteroaryl group of 2 to 30 ring-forming carbon atoms. For example, Ar₁ and/or Ar₂ may be a heteroaryl group including O or S as a ring-forming atom.

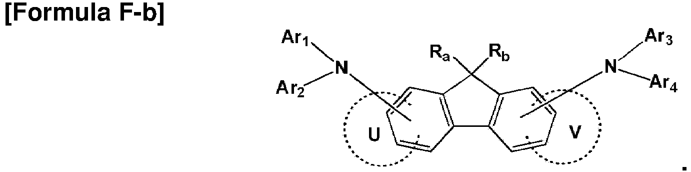

In Formula F-b, Rₐ and R_{b} may each independently be a hydrogen atom, a deuterium atom, a substituted or unsubstituted alkyl group of 1 to 20 carbon atoms, a substituted or unsubstituted alkenyl group of 2 to 20 carbon atoms, a substituted or unsubstituted aryl group of 6 to 30 ring-forming carbon atoms, a substituted or unsubstituted heteroaryl group of 2 to 30 ring-forming carbon atoms, or combined with an adjacent group to form a ring. Ar₁ to Ar₄ may each independently be a substituted or unsubstituted aryl group of 6 to 30 ring-forming carbon atoms, or a substituted or unsubstituted heteroaryl group of 2 to 30 ring-forming carbon atoms.

In Formula F-b, U and V may each independently be a substituted or unsubstituted hydrocarbon ring of 5 to 30 ring-forming carbon atoms, or a substituted or unsubstituted heterocycle of 2 to 30 ring-forming carbon atoms.

In Formula F-b, the number of rings represented by U and V may each independently be 0 or 1. For example, in Formula F-b, when the number of U or V is 1, one ring indicated by U or V forms a fused ring at the designated part (e.g., a portion indicated by U or V), and when the number of U or V is 0, a ring indicated by U or V does not exist. For example, when the number of U is 0, and the number of V is 1, or when the number of U is 1, and the number of V is 0, a fused ring having the fluorene core of Formula F-b may be a ring compound with four rings. In some embodiments, when the number of U and the number of V are both 0, the fused ring of Formula F-b may be a ring compound with three rings. In some embodiments, when the number of U and the number of V are both 1, a fused ring having the fluorene core of Formula F-b may be a ring compound with five rings.

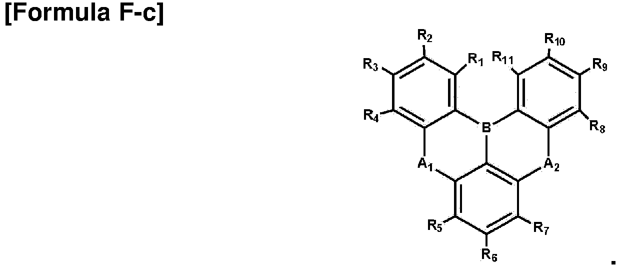

In Formula F-c, A₁ and A₂ may each independently be O, S, Se, or NRₘ, and Rₘ may be a hydrogen atom, a deuterium atom, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms. R₁ to R₁₁ may each independently be a hydrogen atom, a deuterium atom, a halogen atom, a cyano group, a substituted or unsubstituted amine group, a substituted or unsubstituted boryl group, a substituted or unsubstituted oxy group, a substituted or unsubstituted thio group, a substituted or unsubstituted alkyl group of 1 to 20 carbon atoms, a substituted or unsubstituted aryl group of 6 to 30 ring-forming carbon atoms, a substituted or unsubstituted heteroaryl group of 2 to 30 ring-forming carbon atoms, or combined with an adjacent group to form a ring.

In Formula F-c, A₁ and A₂ may each independently be combined with the substituents of an adjacent ring to form a fused ring. For example, when A₁ and A₂ may each independently be NRₘ, A₁ may be combined with R₄ or R₅ to form a ring. In some embodiments, A₂ may be combined with R₇ or R₈ to form a ring.

In an embodiment, the emission layer EML may include as a suitable dopant material, one or more styryl derivatives (for example, 1,4-bis[2-(3-N-ethylcarbazoryl)vinyl]benzene (BCzVB), 4-(di-p-tolylamino)-4'-[(di-p-tolylamino)styryl]stilbene (DPAVB), N-(4-((E)-2-(6-((E)-4-(diphenylamino)styryl)naphthalen-2-yl)vinyl)phenyl)-N-phenylbenzenamine (N-BDAVBi), and/or 4,4'-bis[2-(4-(N,N-diphenylamino)phenyl)vinyl]biphenyl (DPAVBi)), perylene and the derivatives thereof (for example, 2,5,8,11-tetra-t-butylperylene (TBP)), pyrene and the derivatives thereof (for example, 1,1-dipyrene, 1,4-dipyrenylbenzene, and/or 1,4-bis(N,N-diphenylamino)pyrene), etc.

In an embodiment, when multiple emission layers EML are included, at least one emission layer EML may include a suitable phosphorescence dopant material. For example, the phosphorescence dopant may utilize a metal complex including iridium (Ir), platinum (Pt), osmium (Os), gold (Au), titanium (Ti), zirconium (Zr), hafnium (Hf), europium (Eu), terbium (Tb) and/or thulium (Tm). For example, iridium(III) bis(4,6-difluorophenylpyridinato-N,C2')picolinate (Flrpic), bis(2,4-difluorophenylpyridinato)-tetrakis(1-pyrazolyl)borate iridium(III) (Fir₆), and/or platinum octaethyl porphyrin (PtOEP) may be utilized as the phosphorescence dopant. However, an embodiment of the present disclosure is not limited thereto.

In some embodiments, the emission layer EML may include a hole transport host and an electron transport host. In some embodiments, the emission layer EML may include an auxiliary dopant and a light emitting dopant. In some embodiments, the auxiliary dopant may include a phosphorescence dopant material and/or a thermally activated delayed fluorescence dopant. For example, in an embodiment, the emission layer EML may include a hole transport host, an electron transport host, an auxiliary dopant, and a light emitting dopant.

In some embodiments, an exciplex may be formed by the hole transport host and the electron transport host in the emission layer EML. In this case, the triplet energy of the exciplex formed by the hole transport host and the electron transport host may correspond to T1, which is a gap between the lowest unoccupied molecular orbital (LUMO) energy level of the electron transport host and the highest occupied molecular orbital (HOMO) energy level of the hole transport host.

In an embodiment, the triplet energy (T1) of the exciplex formed by the hole transport host and the electron transport host may be about 2.4 eV to about 3.0 eV. In some embodiments, the triplet energy of the exciplex may be a value smaller than the energy gap of each host material. Accordingly, the exciplex may have a triplet energy of about 3.0 eV or less, which is the energy gap between the hole transport host and the electron transport host.

In some embodiments, at least one emission layer EML may include a quantum dot material. The core of the quantum dot may be selected from one or more II-VI group compounds, III-VI group compounds, I-III-VI group compounds, III-V group compounds, III-II-V group compounds, IV-VI group compounds, IV group elements, IV group compounds, and combinations thereof.

The II-VI group compound may be selected from the group consisting of: a binary compound selected from the group consisting of CdSe, CdTe, CdS, ZnS, ZnSe, ZnTe, ZnO, HgS, HgSe, HgTe, MgSe, MgS, and mixtures thereof; a ternary compound selected from the group consisting of CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, MgZnS, and mixtures thereof; and a quaternary compound selected from the group consisting of HgZnTeS, CdZnSeS, CdZnSeTe, CdZnSTe, CdHgSeS, CdHgSeTe, CdHgSTe, HgZnSeS, HgZnSeTe, HgZnSTe, and mixtures thereof.

The III-VI group compound may include a binary compound such as In₂S₃, and/or In₂Se₃, a ternary compound such as InGaS₃, and/or InGaSe₃, or any combinations thereof.

The I-III-VI group compound may be selected from a ternary compound selected from the group consisting of AgInS, AgInS₂, CulnS, CuInS₂, AgGaS₂, CuGaS₂, CuGaO₂, AgGaO₂, AgAlO₂ and mixtures thereof, and a quaternary compound such as AgInGaS₂, and/or CuInGaS₂.

The III-V group compound may be selected from the group consisting of a binary compound selected from the group consisting of GaN, GaP, GaAs, GaSb, AlN, AlP, AlAs, AlSb, InN, InP, InAs, InSb, and mixtures thereof, a ternary compound selected from the group consisting of GaNP, GaNAs, GaNSb, GaPAs, GaPSb, AINP, AlNAs, AlNSb, AlPAs, AlPSb, InGaP, InAlP, InNP, InNAs, InNSb, InPAs, InPSb, and mixtures thereof, and a quaternary compound selected from the group consisting of GaAlNP, GaAINAs, GaAINSb, GaAlPAs, GaAlPSb, GalnNP, GalnNAs, GalnNSb, GalnPAs, GalnPSb, InAlNP, InAINAs, InAlNSb, InAIPAs, InAIPSb, and mixtures thereof. In some embodiments, the III-V group compound may further include a II group metal. For example, InZnP, etc. may be selected as a III-II-V group compound.

The IV-VI group compound may be selected from the group consisting of a binary compound selected from the group consisting of SnS, SnSe, SnTe, PbS, PbSe, PbTe, and mixtures thereof, a ternary compound selected from the group consisting of SnSeS, SnSeTe, SnSTe, PbSeS, PbSeTe, PbSTe, SnPbS, SnPbSe, SnPbTe, and mixtures thereof, and a quaternary compound selected from the group consisting of SnPbSSe, SnPbSeTe, SnPbSTe, and mixtures thereof. The IV group element may be selected from the group consisting of Si, Ge, and a mixture thereof. The IV group compound may be a binary compound selected from the group consisting of SiC, SiGe, and a mixture thereof.

In this case, the binary compound, the ternary compound and/or the quaternary compound may be present at a substantially uniform concentration in a particle or may be present at a partially different concentration distribution state in the same particle. In some embodiments, the quantum dot may have a core/shell structure in which one quantum dot is around (e.g., wraps or encapsulates) another quantum dot. The interface between the core and the shell may have a concentration gradient in which the concentration of an element present in the shell is decreased toward the center of the core.

In some embodiments, the quantum dot may have the above-described core-shell structure including a core including a nanocrystal and a shell around (e.g., wrapping) the core. The shell of the quantum dot may play the role of a protection layer for preventing or reducing the chemical deformation of the core to maintain semiconductor properties and/or a charging layer for imparting the quantum dot with electrophoretic properties. The shell may have a single layer or a multilayer. Examples of the shell of the quantum dot may include a metal or non-metal oxide, a semiconductor compound, or any combinations thereof.

For example, the metal or non-metal oxide may include a binary compound such as SiO₂, Al₂O₃, TiO₂, ZnO, MnO, Mn₂O₃, Mn₃O₄, CuO, FeO, Fe₂O₃, Fe₃O₄, CoO, Co₃O₄ and/or NiO, or a ternary compound such as MgAl₂O₄, CoFe₂O₄, NiFe₂O₄ and/or CoMn₂O₄, but an embodiment of the present disclosure is not limited thereto.

Also, the semiconductor compound may include CdS, CdSe, CdTe, ZnS, ZnSe, ZnTe, ZnSeS, ZnTeS, GaAs, GaP, GaSb, HgS, HgSe, HgTe, InAs, InP, InGaP, InSb, AlAs, AlP, AlSb, etc., but an embodiment of the present disclosure is not limited thereto.

The quantum dot may have a full width of half maximum (FWHM) of emission wavelength spectrum of about 45 nm or less, about 40 nm or less, or, about 30 nm or less. Within these ranges, color purity and/or color reproducibility may be improved. In some embodiments, light emitted via such quantum dot is emitted in all directions, and light view angle properties may be improved (e.g., a wider viewing angle may be obtained).

In some embodiments, the shape of the quantum dot may be any generally utilized shapes in the art, without specific limitation. For example, the quantum dot may have the shape of spherical, pyramidal, multi-arm, or cubic nanoparticle, nanotube, nanowire, nanofiber, nanoplate particle, etc.

The quantum dot may control the color of light emitted according to the particle size, and accordingly, the quantum dot may have one or more suitable emission colors such as blue, red and/or green.

In the light emitting elements ED of embodiments, as shown in FIG. 3 to FIG. 6, the electron transport region ETR is provided on the emission layer EML. The electron transport region ETR may include at least one of a hole blocking layer HBL, an electron transport layer ETL, or an electron injection layer EIL. However, an embodiment of the present disclosure is not limited thereto.

The electron transport region ETR may have a single layer formed utilizing a single material, a single layer formed utilizing multiple different materials, or a multilayer structure having multiple layers formed utilizing multiple different materials.

For example, the electron transport region ETR may have a single layer structure of an electron injection layer EIL or an electron transport layer ETL, or a single layer structure formed utilizing an electron injection material and an electron transport material. Further, the electron transport region ETR may have a single layer structure formed utilizing multiple different materials, or a structure in which electron transport layer ETL/electron injection layer EIL, hole blocking layer HBL/electron transport layer ETL/electron injection layer EIL, electron transport layer ETL/buffer layer /electron injection layer EIL, and/or the like, are stacked from the emission layer EML in the respective stated order, but the embodiment of the present disclosure is not limited thereto. The thickness of the electron transport region ETR may be, for example, from about 1,000 Å to about 1,500 Å.

The electron transport region ETR may be formed utilizing one or more suitable methods such as a vacuum deposition method, a spin coating method, a cast method, a Langmuir-Blodgett (LB) method, an inkjet printing method, a laser printing method, and/or a laser induced thermal imaging (LITI) method.
The electron transport region ETR may include a compound represented by Formula ET-1.

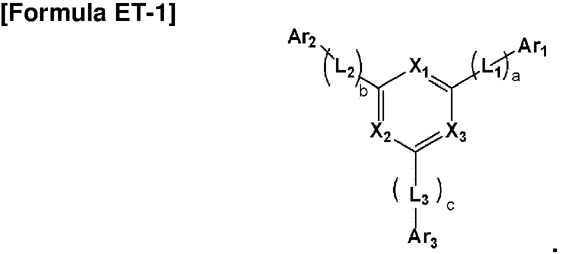

In Formula ET-1, at least one from among X₁ to X₃ may be N, and any remainder thereof may be CRₐ. Rₐ may be a hydrogen atom, a deuterium atom, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms. Ar₁ to Ar₃ may each independently be a hydrogen atom, a deuterium atom, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms.

In Formula ET-1, "a" to "c" may each independently be an integer of 0 to 10. In Formula ET-1, L₁ to L₃ may each independently be a direct linkage, a substituted or unsubstituted arylene group of 6 to 30 ring-forming carbon atoms, or a substituted or unsubstituted heteroarylene group of 2 to 30 ring-forming carbon atoms. In some embodiments, when "a" to "c" are each an integer of 2 or more, L₁ to L₃ may each independently be a substituted or unsubstituted arylene group of 6 to 30 ring-forming carbon atoms, or a substituted or unsubstituted heteroarylene group of 2 to 30 ring-forming carbon atoms.

The electron transport region ETR may include an anthracene-based compound. However, an embodiment of the present disclosure is not limited thereto, and the electron transport region ETR may include, for example, tris(8-hydroxyquinolinato)aluminum (Alq₃), 1,3,5-tri[(3-pyridyl)-phen-3-yl]benzene, 2,4,6-tris(3'-(pyridin-3-yl)biphenyl-3-yl)-1,3,5-triazine, 2-(4-(N-phenylbenzoimidazolyl-1-ylphenyl)-9,10-dinaphthylanthracene, 1,3,5-tri(1-phenyl-1H-benzo[d]imidazol-2-yl)benzene (TPBi), 2,9-dimethyl-4,7-diphenyl-1,10-phenanthroline (BCP), 4,7-diphenyl-1,10-phenanthroline (Bphen), 3-(4-biphenylyl)-4-phenyl-5-tert-butylphenyl-1,2,4-triazole (TAZ), 4-(naphthalen-1-yl)-3,5-diphenyl-4H-1,2,4-triazole (NTAZ), 2-(4-biphenylyl)-5-(4-tert-butylphenyl)-1,3,4-oxadiazole (tBu-PBD), bis(2-methyl-8-quinolinolato-N1,O8)-(1,1'-biphenyl-4-olato)aluminum (BAlq), berylliumbis(benzoquinolin-10-olate (Bebq₂), 9,10-di(naphthalene-2-yl)anthracene (ADN), 1,3-bis[3,5-di(pyridin-3-yl)phenyl]benzene (BmPyPhB), diphenyl(4-(triphenylsilyl)phenyl)phosphine oxide (TSPO1), and mixtures thereof, but an embodiment of the present disclosure is not limited thereto.

The electron transport region ETR may include at least one from among Compounds ET1 to ET36.

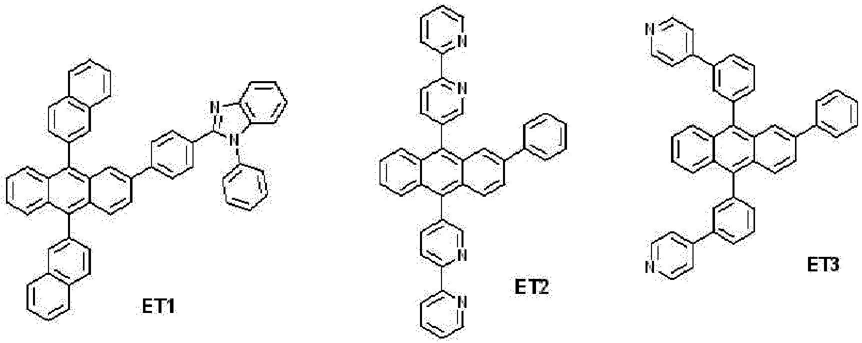

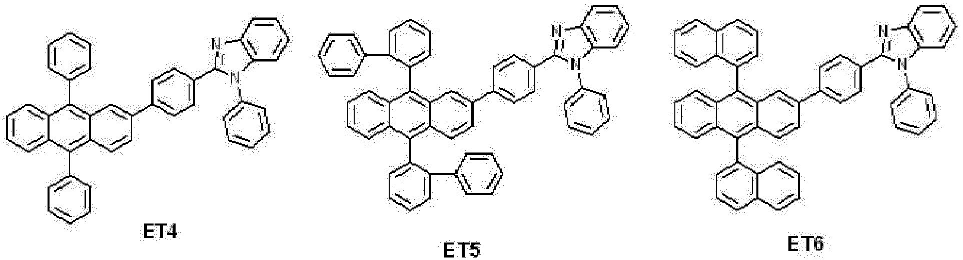

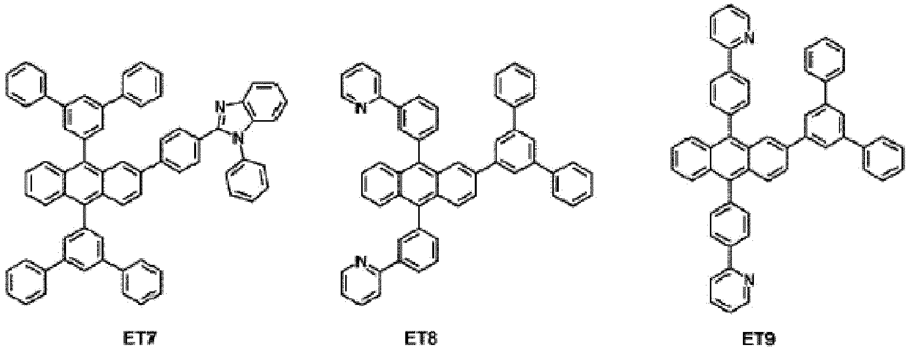

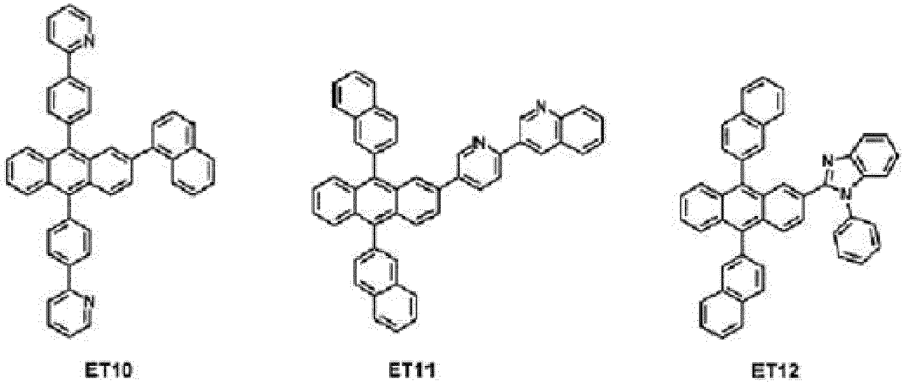

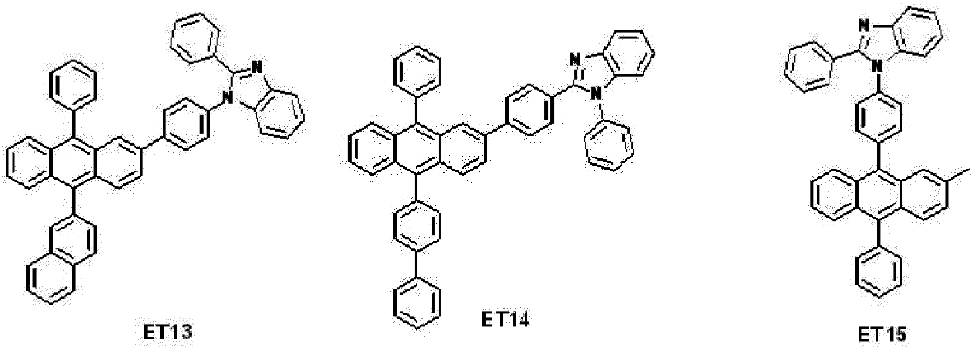

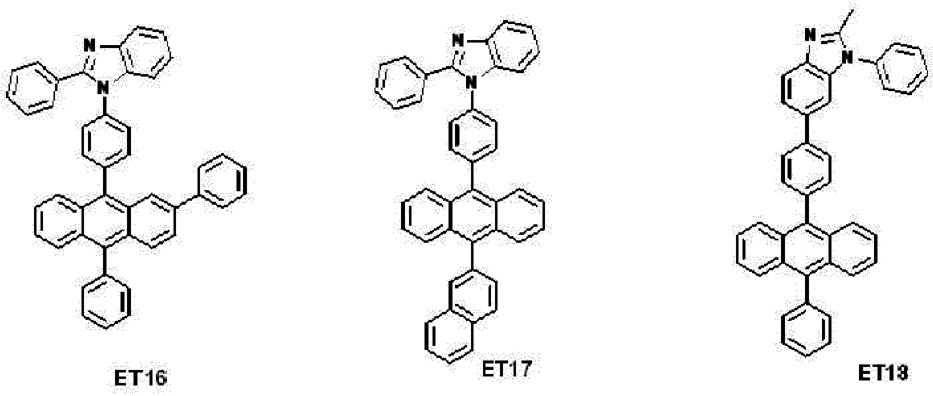

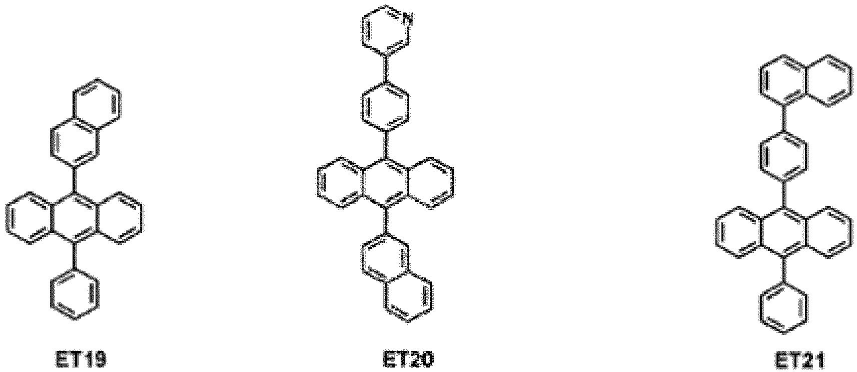

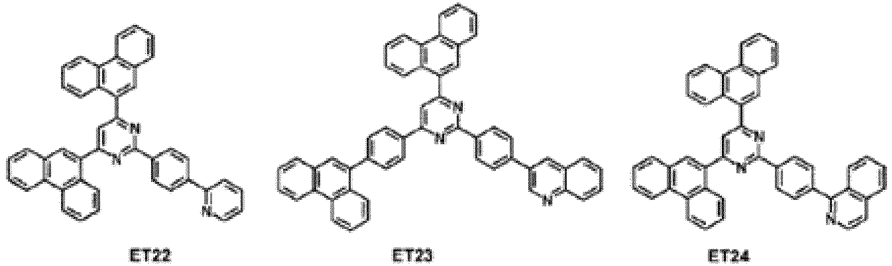

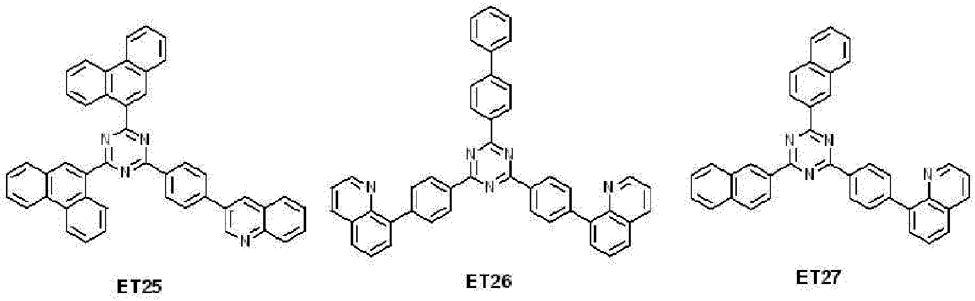

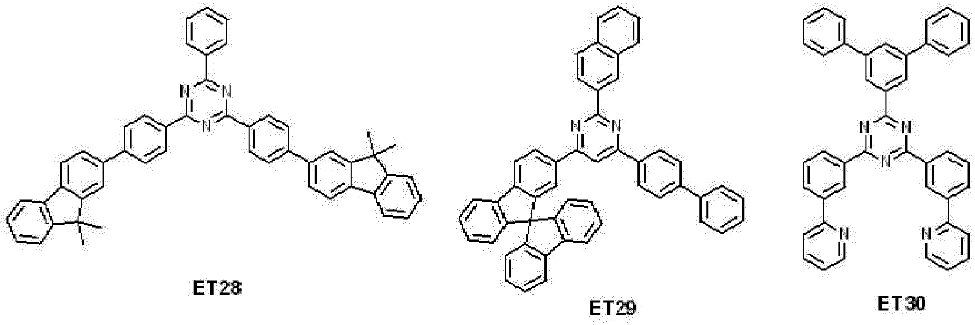

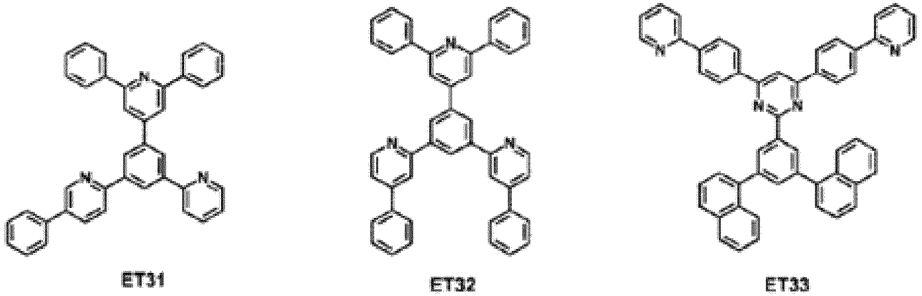

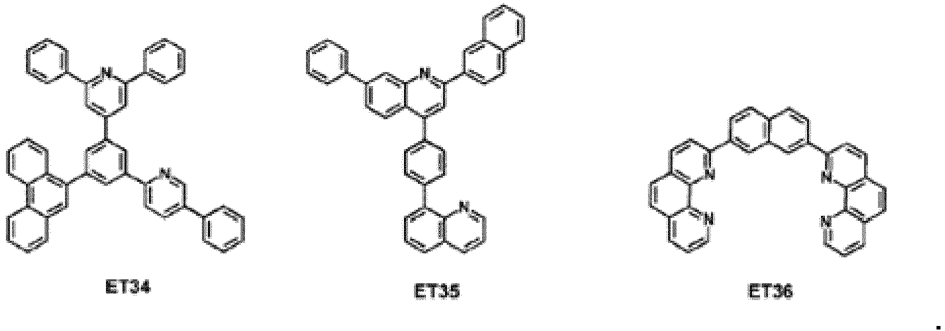

In some embodiments, the electron transport region ETR may include a metal halide such as LiF, NaCl, CsF, RbCl, Rbl, Cul and/or KI, a lanthanide metal such as Yb, and/or a co-deposited material of the metal halide and the lanthanide metal. For example, the electron transport region ETR may include KI:Yb, Rbl:Yb, LiF:Yb, etc., as the co-deposited material. In some embodiments, the electron transport region ETR may utilize a metal oxide such as Li₂O and/or BaO, and/or 8-hydroxy-lithium quinolate (Liq). However, an embodiment of the present disclosure is not limited thereto. The electron transport region ETR also may be formed utilizing a mixture material of an electron transport material and an insulating organo metal (e.g., organometallic) salt. The organo metal salt may be a material having an energy band gap of about 4 eV or more. For example, the organo metal salt may include, for example, one or more metal acetates, metal benzoates, metal acetoacetates, metal acetylacetonates, and/or metal stearates.

The electron transport region ETR may include at least one of 2,9-dimethyl-4,7-diphenyl-1,10-phenanthroline (BCP), diphenyl(4-(triphenylsilyl)phenyl)phosphine oxide (TSPO1) or 4,7-diphenyl-1,10-phenanthroline (Bphen) in addition to the aforementioned materials. However, an embodiment of the present disclosure is not limited thereto.

The electron transport region ETR may include the above-described compounds of the electron transport region ETR in at least one from among an electron injection layer EIL, an electron transport layer ETL, and a hole blocking layer HBL.

When the electron transport region ETR includes the electron transport layer ETL, the thickness of the electron transport layer ETL may be from about 100 Å to about 1,000 Å, for example, from about 150 Å to about 500 Å. When the thickness of the electron transport layer ETL satisfies the above-described ranges, satisfactory electron transport properties may be obtained without substantial increase of a driving voltage. When the electron transport region ETR includes the electron injection layer EIL, the thickness of the electron injection layer EIL may be from about 1 Å to about 100 Å, or from about 3 Å to about 90 Å. When the thickness of the electron injection layer EIL satisfies the above described range, satisfactory electron injection properties may be obtained without inducing substantial increase of a driving voltage.

The second electrode EL2 is provided on the electron transport region ETR. The second electrode EL2 may be a common electrode. The second electrode EL2 may be a cathode or an anode, but an embodiment of the present disclosure is not limited thereto. For example, when the first electrode EL1 is an anode, the second electrode EL2 may be a cathode, and when the first electrode EL1 is a cathode, the second electrode EL2 may be an anode. The second electrode EL2 may include at least one selected from among Ag, Mg, Cu, Al, Pt, Pd, Au, Ni, Nd, Ir, Cr, Li, Ca, LiF, Mo, Ti, W, In, Sn, Zn, compounds of two or more selected therefrom, mixtures of two or more selected therefrom, and/or oxides thereof.

The second electrode EL2 may be a transmissive electrode, a transflective electrode or a reflective electrode. When the second electrode EL2 is the transmissive electrode, the second electrode EL2 may include a transparent metal oxide, for example, ITO, IZO, ZnO, ITZO, etc.

When the second electrode EL2 is the transflective electrode or the reflective electrode, the second electrode EL2 may include Ag, Mg, Cu, Al, Pt, Pd, Au, Ni, Nd, Ir, Cr, Li, Ca, LiF/Ca (stacked structure of LiF and Ca), LiF/Al (stacked structure of LiF and Al), Mo, Ti, Yb, W, compounds thereof, or mixtures thereof (for example, AgMg, AgYb, or MgAg). In some embodiments, the second electrode EL2 may have a multilayered structure including a reflective layer or a transflective layer formed utilizing the above-described materials and a transparent conductive layer formed utilizing ITO, IZO, ZnO, ITZO, etc. For example, the second electrode EL2 may include the aforementioned metal materials, combinations of two or more metal materials selected from the aforementioned metal materials, and/or oxides of the aforementioned metal materials.

In some embodiments, the second electrode EL2 may be connected with an auxiliary electrode. When the second electrode EL2 is connected with the auxiliary electrode, the resistance of the second electrode EL2 may decrease.

In some embodiments, on the second electrode EL2 in the light emitting element ED of an embodiment, a capping layer CPL may be further disposed. The capping layer CPL may include a multilayer or a single layer.

In an embodiment, the capping layer CPL may be an organic layer or an inorganic layer. For example, when the capping layer CPL includes an inorganic material, the inorganic material may include an alkali metal compound such as LiF, an alkaline earth metal compound such as SiON, SiNx, SiOy, etc.

For example, when the capping layer CPL includes an organic material, the organic material may include α-NPD, NPB, TPD, m-MTDATA, Alq₃, CuPc, N4,N4,N4',N4'-tetra(biphenyl-4-yl) biphenyl-4,4'-diamine (TPD15), 4,4',4"-tris(carbazol sol-9-yl) triphenylamine (TCTA), etc., and/or may include an epoxy resin, or acrylate such as methacrylate. In some embodiments, a capping layer CPL may include at least one from Compounds P1 to P5, but an embodiment of the present disclosure is not limited thereto.

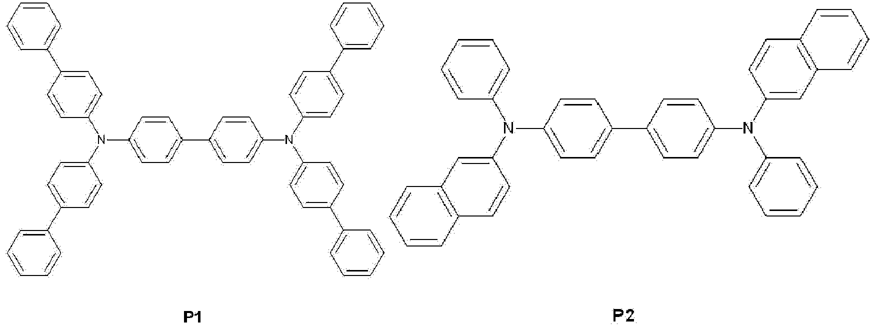

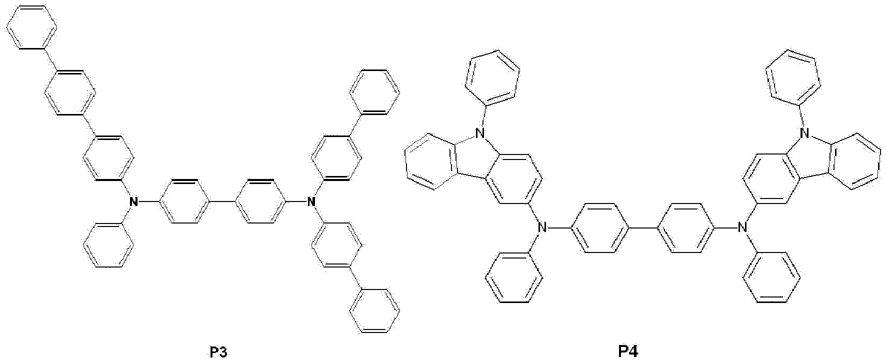

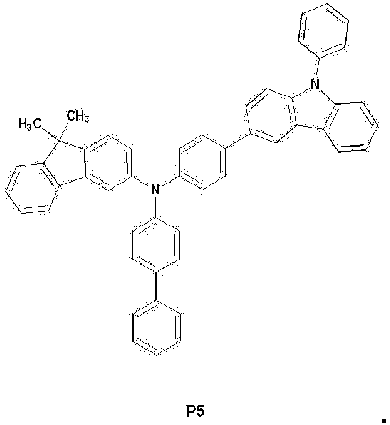

In some embodiments, the refractive index of the capping layer CPL may be about 1.6 or more. For example, the refractive index of the capping layer CPL with respect to light in a wavelength range of about 550 nm to about 660 nm may be about 1.6 or more.

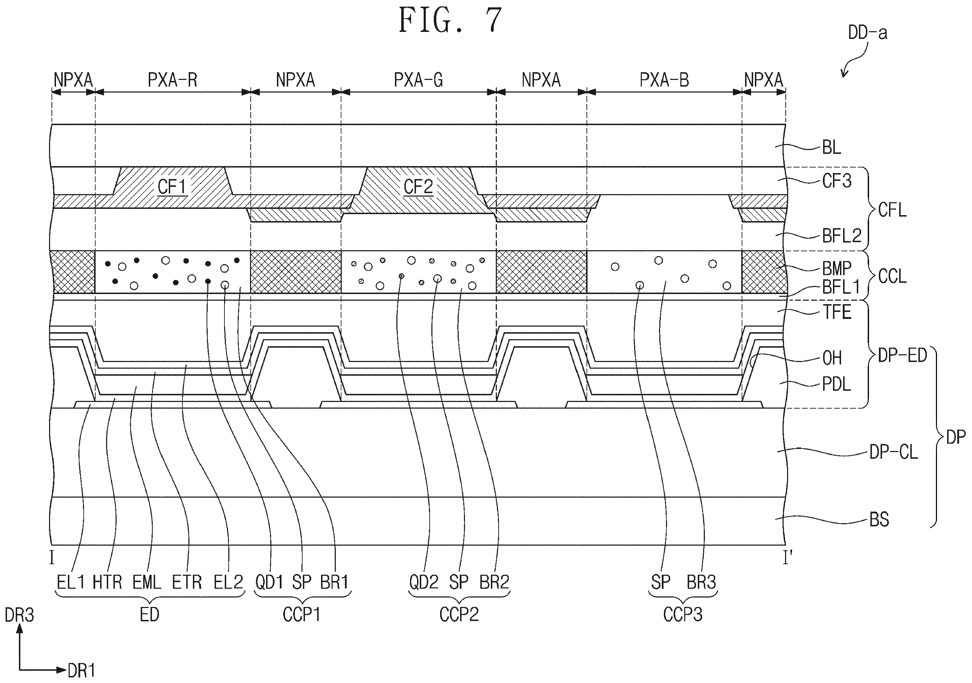

FIG. 7 to FIG. 10 are cross-sectional views on display apparatuses according to embodiments. In the explanation on the display apparatuses of embodiments, referring to FIG. 7 to FIG. 10, the overlapping parts with the explanation on FIG. 1 to FIG. 6 will not be explained again, and the different features will be mainly explained.

Referring to FIG. 7, a display apparatus DD-a according to an embodiment may include a display panel DP including a display element layer DP-ED, a light controlling layer CCL disposed on the display panel DP, and a color filter layer CFL.

In an embodiment shown in FIG. 7, the display panel DP includes a base layer BS, a circuit layer DP-CL provided on the base layer BS and a display element layer DP-ED, and the display element layer DP-ED may include a light emitting element ED.

The light emitting element ED may include a first electrode EL1, a hole transport region HTR disposed on the first electrode EL1, an emission layer EML disposed on the hole transport region HTR, an electron transport region ETR disposed on the emission layer EML, and a second electrode EL2 disposed on the electron transport region ETR. In some embodiments, the same structures of the light emitting elements of FIG. 3 to FIG. 6 may be applied to the structure of the light emitting element ED shown in FIG. 7.

The hole transport region HTR of the light emitting element ED included in the display apparatus DD-a according to an embodiment may include the amine compound of an embodiment, described above.

Referring to FIG. 7, the emission layer EML may be disposed in an opening part OH defined in a pixel definition layer PDL. For example, the emission layer EML divided by the pixel definition layer PDL and correspondingly provided to each of luminous areas PXA-R, PXA-G and PXA-B may be to emit light in substantially the same wavelength region. In the display apparatus DD-a of an embodiment, the emission layer EML may be to emit blue light. In some embodiments, different from the drawings, in an embodiment, the emission layer EML may be provided as a common layer for all luminous areas PXA-R, PXA-G and PXA-B.

The light controlling layer CCL may be disposed on the display panel DP. The light controlling layer CCL may include a light converter. The light converter may be a quantum dot or a phosphor. The light converter may transform the wavelength of light provided and then emit. For example, the light controlling layer CCL may be a layer including a quantum dot or a layer including a phosphor.

The light controlling layer CCL may include multiple light controlling parts CCP1, CCP2 and CCP3. The light controlling parts CCP1, CCP2 and CCP3 may be separated from one another.

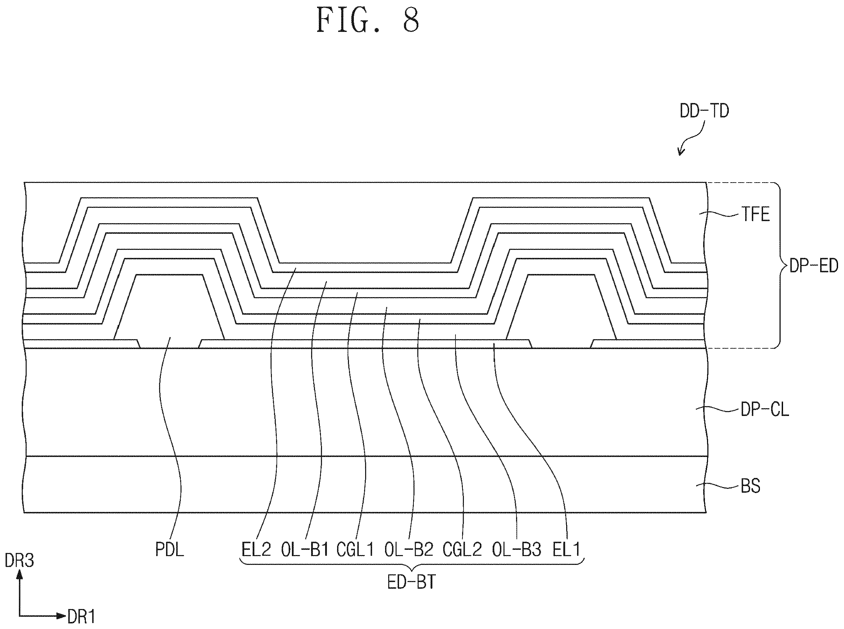

Referring to FIG. 7, a partition pattern BMP may be disposed between the separated light controlling parts CCP1, CCP2 and CCP3, but an embodiment of the present disclosure is not limited thereto. In FIG. 8, the partition pattern BMP is shown to not overlap with the light controlling parts CCP1, CCP2 and CCP3, but in some embodiments, at least a portion of the edge of the light controlling parts CCP1, CCP2 and CCP3 may overlap with the partition pattern BMP.

The light controlling layer CCL may include a first light controlling part CCP1 including a first quantum dot QD1 converting a first color light provided from the light emitting element ED into a second color light, a second light controlling part CCP2 including a second quantum dot QD2 converting the first color light into a third color light, and a third light controlling part CCP3 transmitting the first color light.

In an embodiment, the first light controlling part CCP1 may provide red light which is the second color light, and the second light controlling part CCP2 may provide green light which is the third color light. The third color controlling part CCP3 may be to transmit and provide blue light which is the first color light provided from the light emitting element ED. For example, the first quantum dot QD1 may be a red quantum dot, and the second quantum dot QD2 may be a green quantum dot. The quantum dots QD1 and QD2 may be the same as those described above.

In some embodiments, the light controlling layer CCL may further include a scatterer SP. The first light controlling part CCP1 may include the first quantum dot QD1 and the scatterer SP, the second light controlling part CCP2 may include the second quantum dot QD2 and the scatterer SP, and the third light controlling part CCP3 may not include (e.g., may exclude) any quantum dot but may include the scatterer SP.

The scatterer SP may be an inorganic particle. For example, the scatterer SP may include at least one from among TiO₂, ZnO, Al₂O₃, SiO₂, and hollow silica. The scatterer SP may include at least one from among TiO₂, ZnO, Al₂O₃, SiO₂, and hollow silica, or may be a mixture of two or more materials selected from among TiO₂, ZnO, Al₂O₃, SiO₂, and hollow silica.

Each of the first light controlling part CCP1, the second light controlling part CCP2, and the third light controlling part CCP3 may include a corresponding one of the base resins BR1, BR2 and BR3 for dispersing the quantum dots QD1 and QD2 and the scatterer SP. In an embodiment, the first light controlling part CCP1 may include the first quantum dot QD1 and the scatterer SP dispersed in the first base resin BR1, the second light controlling part CCP2 may include the second quantum dot QD2 and the scatterer SP dispersed in the second base resin BR2, and the third light controlling part CCP3 may include the scatterer particle SP dispersed in the third base resin BR3. The base resins BR1, BR2 and BR3 are mediums in which the quantum dots QD1 and QD2 and the scatterer SP are dispersed, and may be composed of one or more suitable resin compositions which may be generally referred to as a binder. For example, the base resins BR1, BR2 and BR3 may each independently be one or more of acrylic resins, urethane-based resins, silicone-based resins, epoxy-based resins, etc. The base resins BR1, BR2 and BR3 may each be transparent resins. In an embodiment, the first base resin BR1, the second base resin BR2 and the third base resin BR3 may be the same or different from each other.

The light controlling layer CCL may include a barrier layer BFL1. The barrier layer BFL1 may play the role of blocking the penetration of moisture and/or oxygen (hereinafter, will be referred to as "humidity/oxygen"). The barrier layer BFL1 may be disposed on the light controlling parts CCP1, CCP2 and CCP3 to block or reduce the exposure of the light controlling parts CCP1, CCP2 and CCP3 to humidity/oxygen. In some embodiments, the barrier layer BFL1 may cover the light controlling parts CCP1, CCP2 and CCP3. In some embodiments, the barrier layer BFL2 may be provided between the light controlling parts CCP1, CCP2 and CCP3 and a color filter layer CFL (e.g., along the thickness direction).

The barrier layers BFL1 and BFL2 may include at least one inorganic layer. For example, the barrier layers BFL1 and BFL2 may be formed by including an inorganic material. For example, the barrier layers BFL1 and BFL2 may be formed by including silicon nitride, aluminum nitride, zirconium nitride, titanium nitride, hafnium nitride, tantalum nitride, silicon oxide, aluminum oxide, titanium oxide, tin oxide, cerium oxide and/or silicon oxynitride and/or a metal thin film for securing light transmittance. In some embodiments, the barrier layers BFL1 and BFL2 may further include an organic layer. The barrier layers BFL1 and BFL2 may be composed of a single layer or multiple layers.

In the display apparatus DD-a of an embodiment, the color filter layer CFL may be disposed on the light controlling layer CCL. For example, the color filter layer CFL may be disposed directly on the light controlling layer CCL. In this case, the barrier layer BFL2 may not be provided.

The color filter layer CFL may include filters CF1, CF2 and CF3. The color filter layer CFL may include a first filter CF1 for transmitting the second color light, a second filter CF2 for transmitting the third color light, and a third filter CF3 for transmitting the first color light. For example, the first filter CF1 may be a red filter, the second filter CF2 may be a green filter, and the third filter CF3 may be a blue filter. Each of the filters CF1, CF2 and CF3 may include a polymer photosensitive resin and a pigment and/or dye. The first filter CF1 may include a red pigment and/or dye, the second filter CF2 may include a green pigment and/or dye, and the third filter CF3 may include a blue pigment and/or dye. In some embodiments, an embodiment of the present disclosure is not limited thereto, and the third filter CF3 may not include (e.g., may exclude) any pigment or dye. The third filter CF3 may include a polymer photosensitive resin and not include any pigment or dye. The third filter CF3 may be transparent. The third filter CF3 may be formed utilizing a transparent photosensitive resin.

In some embodiments, the first filter CF1 and the second filter CF2 may be yellow filters. The first filter CF1 and the second filter CF2 may be provided in one body without distinction (e.g., without being separated). In some embodiments, each of the first to third filters CF1, CF2 and CF3 may be disposed corresponding to each of a red luminous area PXA-R, green luminous area PXA-G, and blue luminous area PXA-B, respectively.

In some embodiments, though not shown, the color filter layer CFL may include a light blocking part. The color filter layer CFL may include the light blocking part disposed so as to overlap with the boundaries of the neighboring filters CF1, CF2 and CF3. The light blocking part may be a black matrix. The light blocking part may be formed by including an organic light blocking material or an inorganic light blocking material, including a black pigment and/or black dye. The light blocking part may divide the boundaries among adjacent filters CF1, CF2 and CF3. In some embodiments, the light blocking part may be formed as a blue filter.

On the color filter layer CFL, a base substrate BL may be disposed. The base substrate BL may be a member for providing a base surface on which the color filter layer CFL, the light controlling layer CCL, etc. are disposed. The base substrate BL may be a glass substrate, a metal substrate, a plastic substrate, etc. However, an embodiment of the present disclosure is not limited thereto, and the base substrate BL may be an inorganic layer, an organic layer or a composite material layer. In some embodiments, different from the drawing, the base substrate BL may not be provided.

FIG. 8 is a cross-sectional view showing a portion of the display apparatus according to an embodiment. In FIG. 8, the cross-sectional view of a portion corresponding to the display panel DP in FIG. 7 is shown. In a display apparatus DD-TD of an embodiment, the light emitting element ED-BT may include multiple light emitting structures OL-B1, OL-B2 and OL-B3. The light emitting element ED-BT may include oppositely disposed first electrode EL1 and second electrode EL2, and the multiple light emitting structures OL-B1, OL-B2 and OL-B3 stacked in the stated order in a thickness direction between the first electrode EL1 and the second electrode EL2. Each of the light emitting structures OL-B1, OL-B2 and OL-B3 may include an emission layer EML (FIG. 7), and a hole transport region HTR and an electron transport region ETR disposed with the emission layer EML (FIG. 7) therebetween.

For example, the light emitting element ED-BT included in the display apparatus DD-TD of an embodiment may be a light emitting element having a tandem structure including multiple emission layers.

In an embodiment shown in FIG. 8, light emitted from the light emitting structures OL-B1, OL-B2 and OL-B3 may all be blue light. However, an embodiment of the present disclosure is not limited thereto, and the wavelength regions of light emitted from the light emitting structures OL-B1, OL-B2 and OL-B3 may be different from each other. For example, the light emitting element ED-BT including the multiple light emitting structures OL-B1, OL-B2 and OL-B3 emitting light in different wavelength regions may be to emit white light.

Between neighboring light emitting structures OL-B1, OL-B2 and OL-B3, charge generating layers CGL1 and CGL2 may be disposed. The charge generating layers CGL1 and CGL2 may include a p-type or kind charge generating layer and/or an n-type or kind charge generating layer.

In at least one from among the light emitting structures OL-B1, OL-B2 and OL-B3, included in the display apparatus DD-TD of an embodiment, the amine compound of an embodiment may be included.

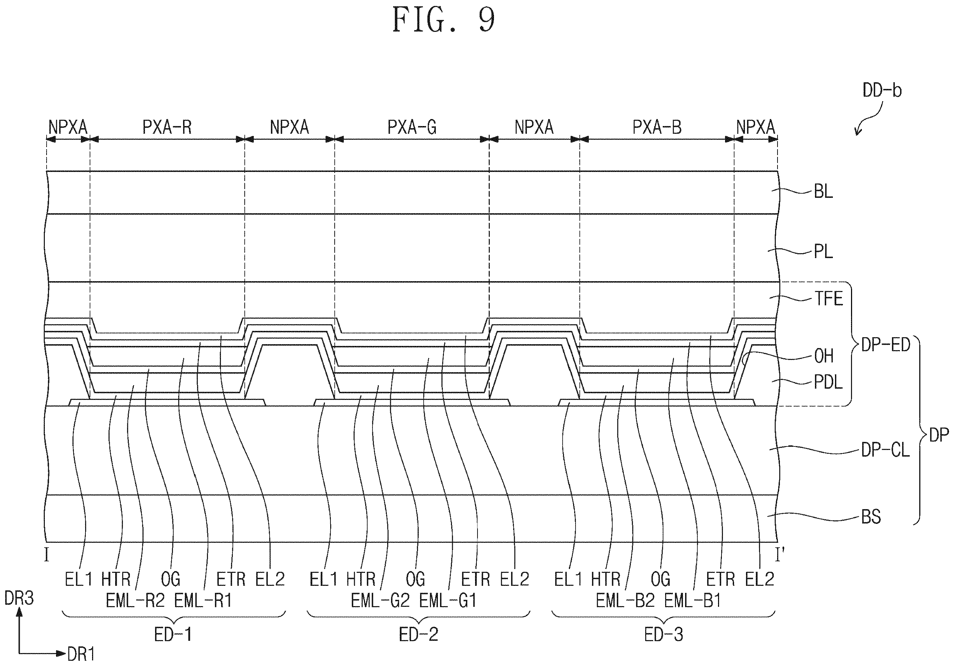

Referring to FIG. 9, a display apparatus DD-b according to an embodiment may include light emitting elements ED-1, ED-2 and ED-3, each formed by stacking two emission layers over each other. Compared to the display apparatus DD of an embodiment, shown in FIG. 2, an embodiment shown in FIG. 9 is different in that the first to third light emitting elements ED-1, ED-2 and ED-3 each include two emission layers stacked in a thickness direction. In the first to third light emitting elements ED-1, ED-2 and ED-3, the two emission layers may be to (e.g., may each) emit light in substantially the same wavelength region.

The first light emitting element ED-1 may include a first red emission layer EML-R1 and a second red emission layer EML-R2. The second light emitting element ED-2 may include a first green emission layer EML-G1 and a second green emission layer EML-G2. In addition, the third light emitting element ED-3 may include a first blue emission layer EML-B1 and a second blue emission layer EML-B2. Between the first red emission layer EML-R1 and the second red emission layer EML-R2, between the first green emission layer EML-G1 and the second green emission layer EML-G2, and between the first blue emission layer EML-B1 and the second blue emission layer EML-B2, an emission auxiliary part OG may be disposed.

The emission auxiliary part OG may include a single layer or a multilayer. The emission auxiliary part OG may include a charge generating layer. For example, the emission auxiliary part OG may include an electron transport region, a charge generating layer, and a hole transport region stacked in the stated order. The emission auxiliary part OG may be provided as a common layer in all of the first to third light emitting elements ED-1, ED-2 and ED-3. However, an embodiment of the present disclosure is not limited thereto, and the emission auxiliary part OG may be patterned and provided in an opening part OH defined in the pixel definition layer PDL.

The first red emission layer EML-R1, the first green emission layer EML-G1 and the first blue emission layer EML-B1 may be disposed between the electron transport region ETR and the emission auxiliary part OG. The second red emission layer EML-R2, the second green emission layer EML-G2 and the second blue emission layer EML-B2 may be disposed between the emission auxiliary part OG and the hole transport region HTR.

For example, the first light emitting element ED-1 may include a first electrode EL1, a hole transport region HTR, a second red emission layer EML-R2, an emission auxiliary part OG, a first red emission layer EML-R1, an electron transport region ETR, and a second electrode EL2, stacked in the stated order. The second light emitting element ED-2 may include a first electrode EL1, a hole transport region HTR, a second green emission layer EML-G2, an emission auxiliary part OG, a first green emission layer EML-G1, an electron transport region ETR, and a second electrode EL2, stacked in the stated order. The third light emitting element ED-3 may include a first electrode EL1, a hole transport region HTR, a second blue emission layer EML-B2, an emission auxiliary part OG, a first blue emission layer EML-B1, an electron transport region ETR, and a second electrode EL2, stacked in the stated order.

In some embodiments, an optical auxiliary layer PL may be disposed on the display element layer DP-ED. The optical auxiliary layer PL may include a polarization layer. The optical auxiliary layer PL may be disposed on a display panel DP and may control reflected light at the display panel DP by external light. Different from the drawings, the optical auxiliary layer PL may not be provided from the display apparatus according to an embodiment.

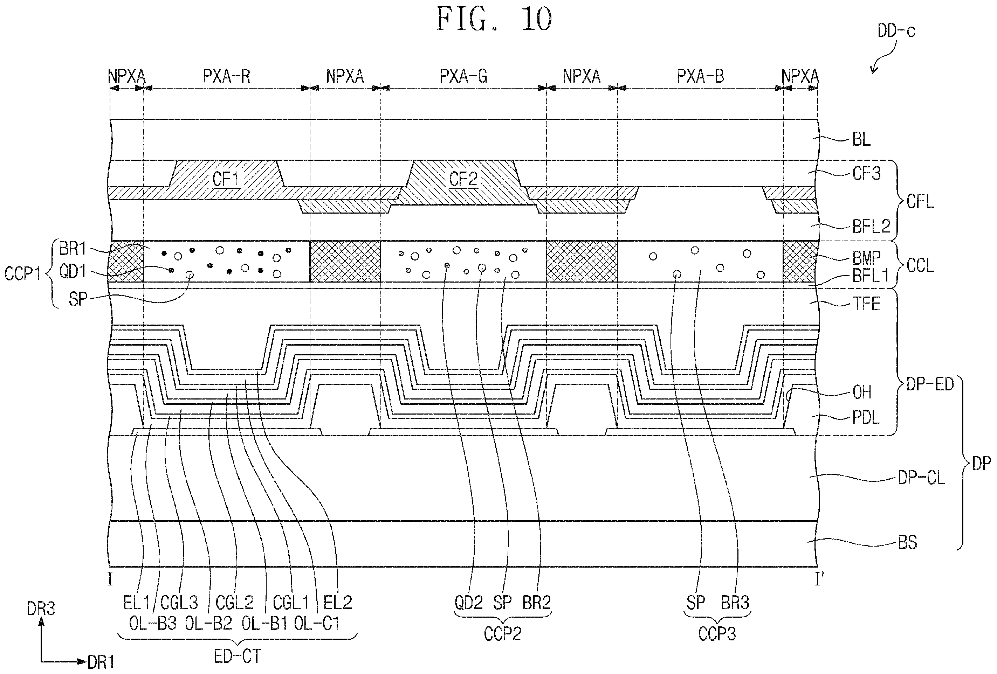

Different from FIG. 8 and FIG. 9, a display apparatus DD-c in FIG. 10 is shown to include four light emitting structures OL-B1, OL-B2, OL-B3 and OL-C1. A light emitting element ED-CT may include oppositely disposed first electrode EL1 and second electrode EL2, and first to fourth light emitting structures OL-B1, OL-B2, OL-B3 and OL-C1 stacked in the stated order in a thickness direction between the first electrode EL1 and the second electrode EL2. Between the first to fourth light emitting structures OL-B1, OL-B2, OL-B3 and OL-C1, charge generating layers CGL1, CGL2 and CGL3 may be disposed. From among the four light emitting structures, the first to third light emitting structures OL-B1, OL-B2 and OL-B3 may be to emit blue light, and the fourth light emitting structure OL-C1 may be to emit green light. However, an embodiment of the present disclosure is not limited thereto, and the first to fourth light emitting structures OL-B1, OL-B2, OL-B3 and OL-C1 may be to emit light of different wavelengths.

Charge generating layers CGL1, CGL2 and CGL3 disposed among neighboring light emitting structures OL-B1, OL-B2, OL-B3 and OL-C1 may include a p-type or kind charge generating layer and/or an n-type or kind charge generating layer.

In at least one from among the light emitting structures OL-B1, OL-B2, OL-B3 and OL-C1, included in the display apparatus DD-c of an embodiment, the amine compound of an embodiment may be included.

The light emitting element ED according to an embodiment of the present disclosure may include the amine compound of an embodiment in at least one functional layer disposed between the first electrode EL1 and the second electrode EL2 to show (e.g., realize) improved emission efficiency and improved life characteristics. The light emitting element ED according to an embodiment may include the amine compound of an embodiment in at least one from among a hole transport region HTR, an emission layer EML, and an electron transport region ETR, disposed between the first electrode EL1 and the second electrode EL2, or in a capping layer CPL.

For example, the amine compound according to an embodiment may be included in the hole transport region HTR of the light emitting element ED of an embodiment, and the light emitting element of an embodiment may show (e.g., realize) excellent or suitable emission efficiency and long-life characteristics.

The amine compound of an embodiment includes a dibenzoheterole group substituted with an aryl group or a heteroaryl group, and a substituent selected from one or more of carbazole derivatives, fluorene derivatives, and/or terphenyl derivatives, and may show (e.g., realize) a low driving voltage, high efficiency and increased life-characteristics.

Hereinafter, referring to embodiments (Examples) and comparative embodiments (Comparative Examples), the amine compound according to an embodiment and the light emitting element according to an embodiment of the present disclosure will be explained in more detail. In addition, the embodiments (examples) are illustrations to assist the understanding of the present disclosure, but the scope of the present disclosure is not limited thereto.

### [Examples]

### 1. Synthesis of amine compounds

First, the synthetic methods of (e.g., methods for forming or synthesizing) the amine compounds according to embodiments will be explained in more detail by illustrating the synthetic methods of Compound A2, Compound A13, Compound A29, Compound A73, Compound B1, and Compound C3. In addition, the synthetic methods of the amine compounds explained hereinafter are embodiments, and the synthetic method of the amine compound according to an embodiment of the present disclosure is not limited to the embodiments below.

### (1) Synthesis of Compound A2

Amine Compound A2 according to an embodiment may be synthesized, for example, by the steps (acts) of the reactions below.

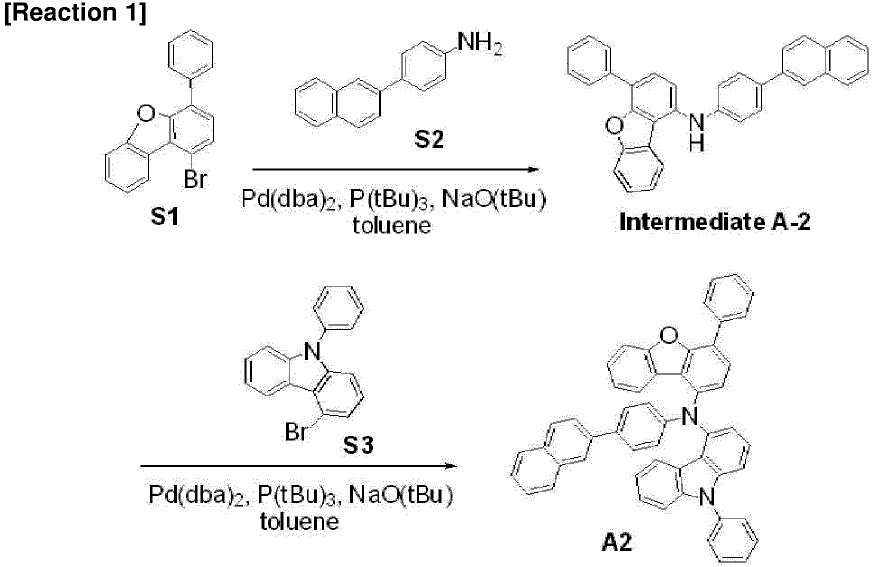

### Synthesis of Intermediate A-2

Under an argon (Ar) atmosphere, to a 1000 mL, three-neck flask, Compound S1 (10.18 g, 46.41 mmol), Pd(dba)₂ (1.33 g, 0.05 equiv, 2.32 mmol), NaO*^{t}*Bu (4.46 g, 1 equiv, 46.41 mmol), toluene (464 mL), Compound S2 (15.48 g, 1.0 equiv, 46.41 mmol) and P(tBu)s (1.88 g, 0.2 equiv, 9.28 mmol) were added in the stated order, followed by heating, refluxing and stirring for about 6 hours. After cooling to room temperature, water was added to the reaction solution, and an organic layer was separately taken. Then, toluene was added to an aqueous layer, and an organic layer was extracted further. The organic layers were collected, washed with a saline solution, and dried over MgSO₄. MgSO₄ was filtered, an organic layer was concentrated, and the crude product thus obtained was separated by silica gel column chromatography to obtain Intermediate A-2 (18.6 g, yield 87%) as a white solid compound.

By measuring FAB-MS, mass number of m/z = 461 was observed as a molecular ion peak, and Intermediate A-2 was confirmed.

### Synthesis of Compound A2

Under an argon atmosphere, to a 500 mL, three-neck flask, Intermediate A-2 (10.0 g, 21.67 mmol), Pd(dba)₂ (0.62 g, 0.05 equiv, 1.08 mmol), NaO*^{t}*Bu (2.08 g, 1 equiv, 21.67 mmol), toluene (216 mL), Compound S3 (6.98 g, 1.0 equiv, 21.67 mmol) and P(tBu)s (0.88 g, 0.2 equiv, 4.33 mmol) were added in the stated order, followed by heating, refluxing and stirring for about 6 hours. After cooling to room temperature, water was added to the reaction solution, and an organic layer was separately taken. Then, toluene was added to an aqueous layer, and an organic layer was extracted further. The organic layers were collected, washed with a saline solution, and dried over MgSO₄. MgSO₄ was filtered, an organic layer was concentrated, and the crude product thus obtained was separated by silica gel column chromatography to obtain Compound A2 (10.7 g, yield 70%) as a white solid compound.

By measuring FAB-MS, mass number of m/z = 702 was observed as a molecular ion peak, and Compound A2 was confirmed.

### (2) Synthesis of Compound A13

Amine Compound A13 according to an embodiment may be synthesized, for example, by the steps (acts) of the reactions below.

### Synthesis of Intermediate M1

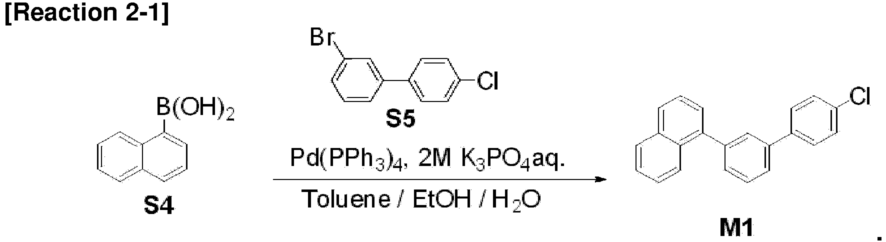

Under an argon atmosphere, to a 1000 mL, three-neck flask, Compound S4 (20.0 g, 116.29 mmol), Compound S5 (31.1 g, 1.0 equiv, 116.29 mmol), Pd(PPh₃)₄ (4.03 g, 0.03 equiv, 3.49 mmol), 2 M of K₃PO₄ (aq, 96.90 g), toluene (387 mL), and ethanol (194 mL) were added in the stated order, followed by heating, refluxing and stirring for about 6 hours. After cooling to room temperature, water was added to the reaction solution, and an organic layer was separately taken. Then, toluene was added to an aqueous layer, and an organic layer was extracted further. The organic layers were collected, washed with a saline solution, and dried over MgSO₄. MgSO₄ was filtered, an organic layer was concentrated, and the crude product thus obtained was separated by silica gel column chromatography to obtain Intermediate M1 (27.0 g, yield 73%) as a white solid compound.

By measuring FAB-MS, mass number of m/z = 314 was observed as a molecular ion peak, and Intermediate M1 was confirmed.

### Synthesis of Compound A13

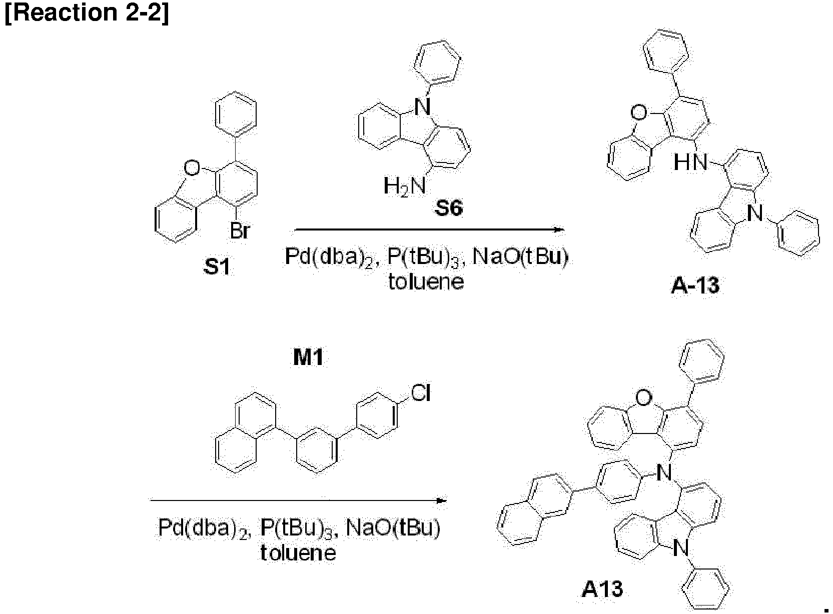

Intermediate A-13 was synthesized by substantially the same method as the synthetic method of Intermediate A-2 except for utilizing Compound S6 instead of Compound S2. By measuring FAB-MS, mass number of m/z = 500 was observed as a molecular ion peak, and Intermediate A-13 was confirmed.

Compound A13 was synthesized by substantially the same method as the synthetic method of Compound A2 except for adding Intermediate M1 to Intermediate A-13. By measuring FAB-MS, mass number of m/z = 702 was observed as a molecular ion peak, and Compound A13 was confirmed.

### (3) Synthesis of Compound A29

Amine Compound A29 according to an embodiment may be synthesized, for example, by the steps (acts) of the reactions below.

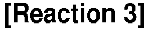

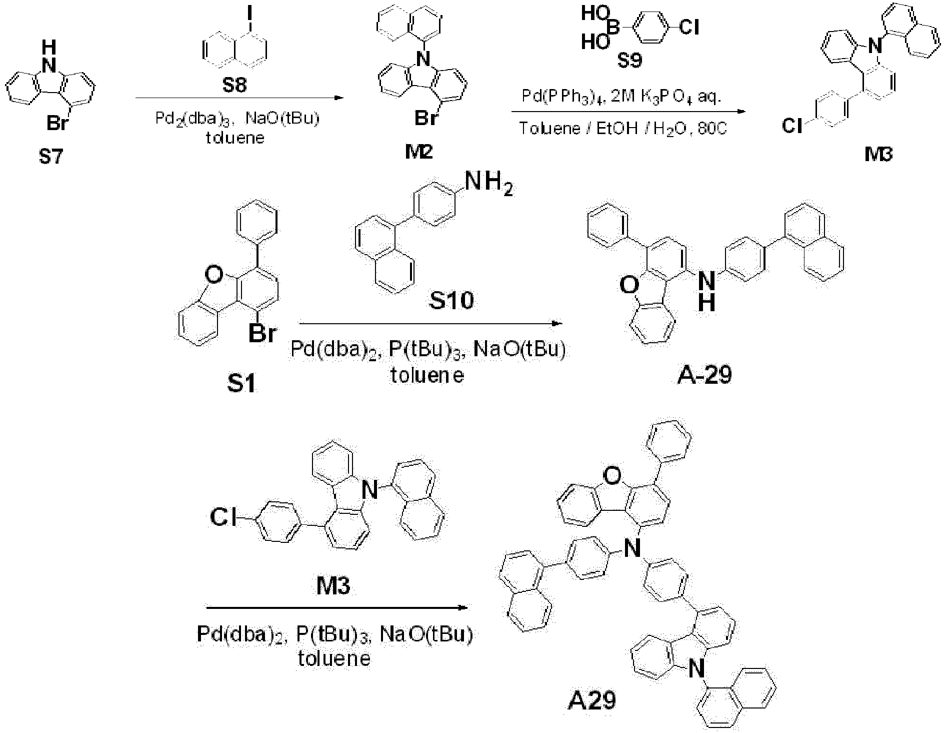

### Synthesis of Intermediate M2

Under an argon atmosphere, to a 1000 mL, three-neck flask, Compound S7 (15.0 g, 60.95 mmol), Compound S8 (15.49 g, 1.0 equiv, 60.95 mmol), Pd₂(dba)₃ (3.15 g, 0.05 equiv, 3.05 mmol), NaO*^{t}*Bu (5.86 g, 1 equiv, 60.95 mmol), and toluene (610 mL) were added in the stated order, followed by heating, refluxing and stirring for about 6 hours. After cooling to room temperature, water was added to the reaction solution, and an organic layer was separately taken. Then, toluene was added to an aqueous layer, and an organic layer was extracted further. The organic layers were collected, washed with a saline solution, and dried over MgSO₄. MgSO₄ was filtered, an organic layer was concentrated, and the crude product thus obtained was separated by silica gel column chromatography to obtain Intermediate M2 (18.0 g, yield 79%).

By measuring FAB-MS, mass number of m/z = 371 was observed as a molecular ion peak, and Intermediate M2 was confirmed.

### Synthesis of Intermediate M3

Intermediate M3 was synthesized by substantially the same method as the synthetic method of Intermediate M1 except for adding Compound S9 to Intermediate M2. By measuring FAB-MS, mass number of m/z = 403 was observed as a molecular ion peak, and Intermediate M3 was confirmed.

### Synthesis of Intermediate A-29

Intermediate A-29 was synthesized by substantially the same method as the synthetic method of Intermediate A-2 except for utilizing Compound S10 instead of Compound S2.

### Synthesis of Compound A29

Compound A29 was synthesized by substantially the same method as the synthetic method of Compound A2 except for adding Intermediate M3 to Intermediate A-29. By measuring FAB-MS, mass number of m/z = 828 was observed as a molecular ion peak, and Compound A29 was confirmed.

### (4) Synthesis of Compound A73

Amine Compound A73 according to an embodiment may be synthesized, for example, by the steps (acts) of the reactions below.

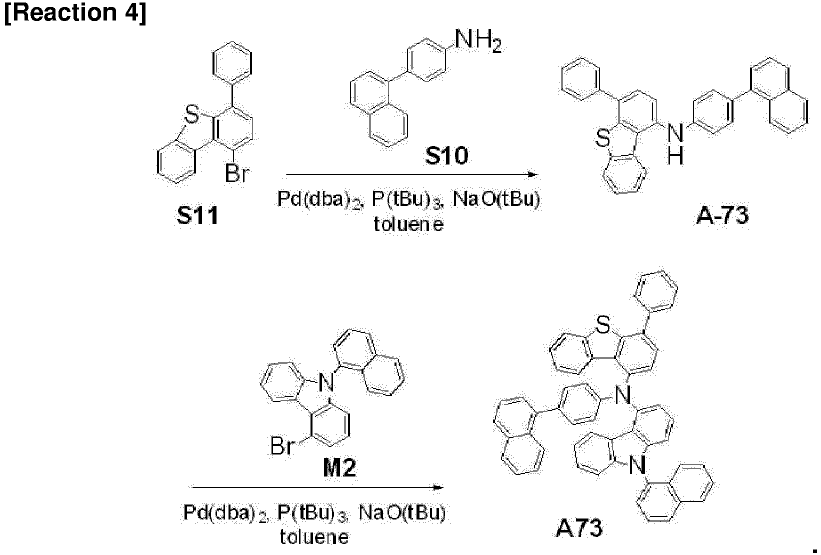

Compound A73 was synthesized by substantially the same method as the synthetic method of Compound A2 except for utilizing Compound S11, Compound S10, and Intermediate M2, as reactants. By measuring FAB-MS, mass number of m/z = 768 was observed as a molecular ion peak, and Compound A73 was confirmed.

### (5) Synthesis of Compound B1

Amine Compound B1 according to an embodiment may be synthesized, for example, by the steps (acts) of the reactions below.

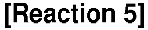

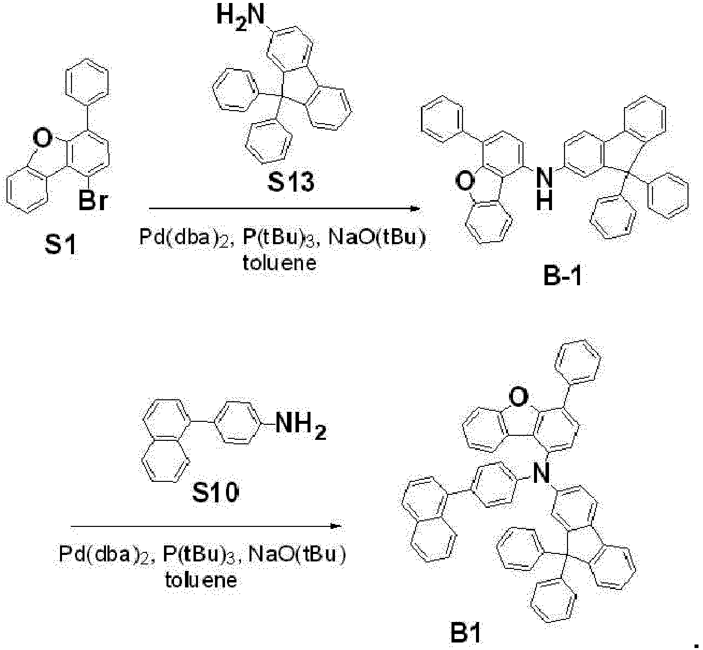

Intermediate B-1 was synthesized by substantially the same method as the synthetic method of Intermediate A-2 except for utilizing Compound S13 instead of Compound S2.

Compound B1 was synthesized by substantially the same method as the synthetic method of Compound A2 by adding Compound S10 to Intermediate B-1. By measuring FAB-MS, mass number of m/z = 777 was observed as a molecular ion peak, and Compound B1 was confirmed.

### (6) Synthesis of Compound B2

Amine Compound B2 according to an embodiment may be synthesized, for example, by the steps (acts) of the reactions below.

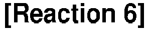

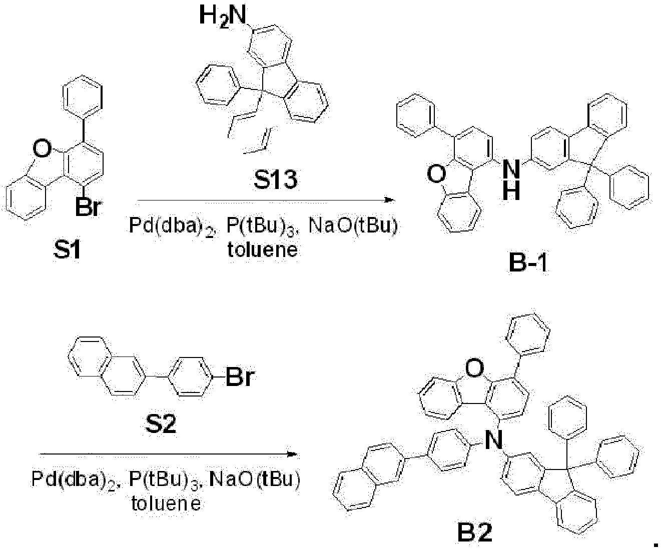

Intermediate B-1 was synthesized by substantially the same method as the synthetic method of Intermediate A-2 except for utilizing Compound S13 instead of Compound S2.

Compound B2 was synthesized by substantially the same method as the synthetic method of Compound B1 by adding Compound S2 to Intermediate B-1. By measuring FAB-MS, mass number of m/z = 777 was observed as a molecular ion peak, and Compound B2 was confirmed.

### (7) Synthesis of Compound C3

Amine Compound C3 according to an embodiment may be synthesized, for example, by the steps (acts) of the reactions below.

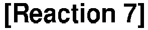

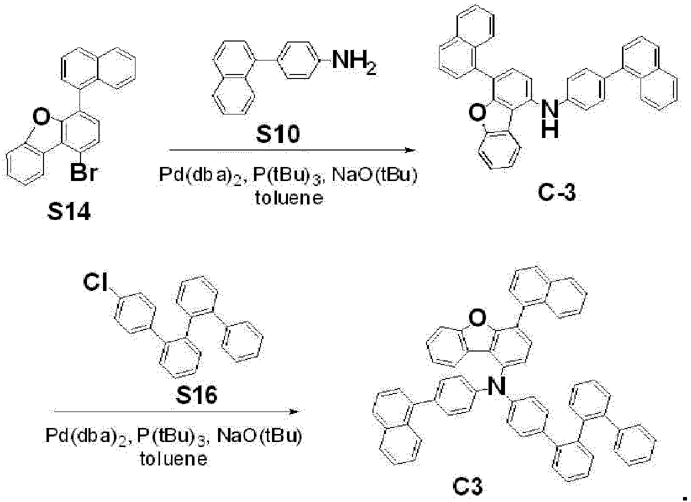

Intermediate C-3 was synthesized by substantially the same method as the synthetic method of Intermediate A-29 except for utilizing Compound S14 instead of Compound S1.

Then, Compound C3 was synthesized by substantially the same method as the synthetic method of Compound A2 by adding Compound S16 to Intermediate C-3. By measuring FAB-MS, mass number of m/z = 815 was observed as a molecular ion peak, and Compound C3 was confirmed.

### 2. Manufacture and evaluation of light emitting elements

The evaluation on the light emitting elements including the Example Compounds and Comparative Compounds in hole transport layers were conducted by a method below. The manufacturing method of the light emitting elements for evaluating the elements is shown below.

### (1) Manufacture of light emitting element

A glass substrate, on which ITO with a thickness of about 150 nm was patterned, was washed with ultrasonic waves utilizing isopropyl alcohol and pure water for about 5 minutes each. After the washing by ultrasonic waves, UV exposure for about 30 minutes and ozone treatment were performed. Then, 2-TNATA was deposited to a thickness of about 60 nm to form a hole injection layer.

After that, a respective one of the Example Compound or Comparative Compound was deposited to a thickness of about 30 nm to form a hole transport layer. Then, an emission layer with a thickness of about 25 nm was formed by concurrently (e.g., simultaneously) depositing ADN and TBP in a weight ratio of about 97:3. Then, Alq₃ was deposited to a thickness of about 25 nm to form an electron transport layer, and LiF was deposited to a thickness of about 1 nm to form an electron injection layer.

After that, a second electrode was formed by depositing Al to a thickness of about 100 nm.

In the Examples and Comparative Examples, the hole injection layer, the hole transport layer, the emission layer, the electron transport layer, the electron injection layer and the second electrode were each formed utilizing a vacuum deposition apparatus.

The Example Compounds and Comparative Compounds utilized for the manufacture of light emitting elements are as follows.

### Example Compounds

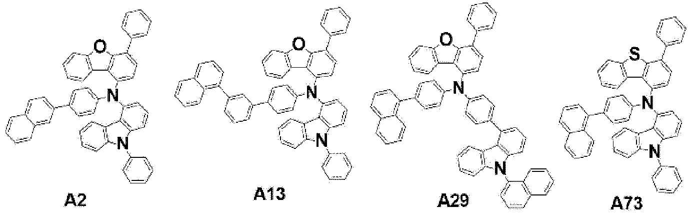

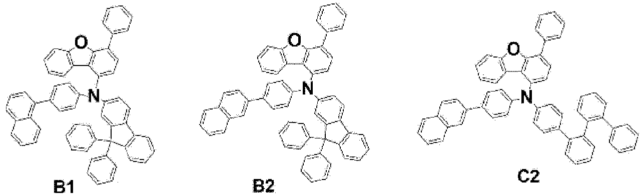

### Comparative Compounds

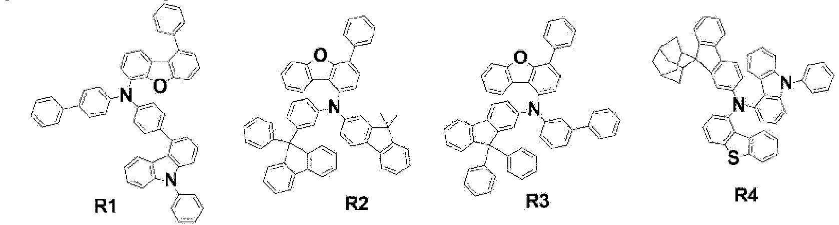

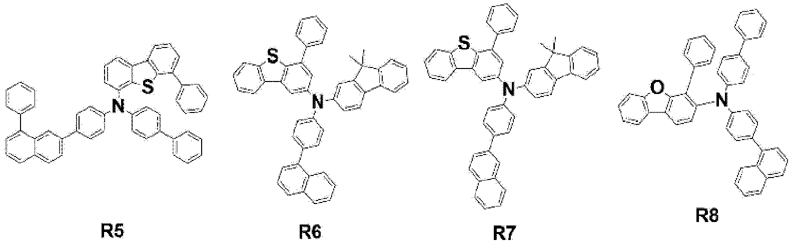

### (2) Evaluation of light emitting elements

Table 1 shows evaluation results on the light emitting elements of Examples 1 to 7, and Comparative Examples 1 to 8. In Table 1, the driving voltage, emission efficiency and half-life of the light emitting elements manufactured are compared and shown. In the evaluation results of the properties of the Examples and Comparative Examples, shown in Tables 1, the emission efficiency shows the current efficiency values of about 10 mA/cm², and the half-life shows luminance half-life from an initial luminance of about 1000 cd/m².

The evaluation on the current density, the driving voltage and the emission efficiency of the elements were performed, utilizing a Source Meter of 2400 Series by Keithley Instruments Co., a luminance and color meter by CS-200 of Konica Minolta Co., Ltd., and a PC Program LabVIEW8.2 for measurement by Japanese National Instruments Co. Ltd., in a dark room.

**[Table 1]**

| Element manufacturing example | Hole transport layer material | Voltage (V) | Emission efficiency (cd/A) | Life LT50 (h) |
|---|---|---|---|---|
| Example 1 | Compound A2 | 5.3 | 8.7 | 2450 |
| Example 2 | Compound A13 | 5.4 | 8.5 | 2400 |
| Example 3 | Compound A29 | 5.5 | 8.4 | 2350 |
| Example 4 | Compound A73 | 5.3 | 8.6 | 2200 |
| Example 5 | Compound B1 | 5.6 | 8.5 | 2400 |
| Example 6 | Compound B2 | 5.2 | 8.6 | 2550 |
| Example 7 | Compound C2 | 5.7 | 8.5 | 2250 |
| Comparative Example 1 | Comparative Compound R1 | 6.0 | 6.2 | 1700 |
| Comparative Example 2 | Comparative Compound R2 | 6.0 | 6.0 | 1500 |
| Comparative Example 3 | Comparative Compound R3 | 5.9 | 7.4 | 1800 |
| Comparative Example 4 | Comparative Compound R4 | 6.1 | 7.5 | 1900 |
| Comparative Example 5 | Comparative Compound R5 | 6.2 | 7.1 | 1950 |
| Comparative Example 6 | Comparative Compound R6 | 5.9 | 7.7 | 1600 |
| Comparative Example 7 | Comparative Compound R7 | 6.0 | 7.3 | 2000 |
| Comparative Example 8 | Comparative Compound R8 | 5.8 | 7.5 | 1650 |

Referring to the results of Table 1, Examples 1 to 7 of the present disclosure showed low voltages, long-life characteristics and high efficiency when compared to Comparative Examples 1 to 8. The light emitting elements of Examples 1 to 4, each including a corresponding one of the Example Compounds essentially including a carbazole group as a substituent showed lower voltages, longer-life characteristics and higher efficiency when compared to Comparative Example 1, including Comparative Compound R1, which is an amine compound including a carbazole group. It is thought that the Example Compounds each further included an aryl group substituted at position 4 of 1-dibenzofuran (or 1-dibenzothiophene), and therefore conjugation was further extended.

When comparing Comparative Compound R2 utilized in Comparative Example 2, the Example Compound utilized in Example 6 is different in having a different bonding position of the nitrogen atom of the amine group with fluorene and not including a naphthyl group. Due to such difference, Example 6 showed element properties of a lower driving voltage, longer lifetime, and higher efficiency.

Meanwhile, Comparative Compound R3 utilized in Comparative Example 3 has the same bonding position of the nitrogen atom of the amine group with fluorene as that of the Example Compounds of the present disclosure, but different from the Example Compounds of the present disclosure in that it includes a m-biphenyl group instead of a naphthyl group as a substituent, and accordingly, Comparative Example 3 showed shorter life-characteristics than the Examples.

The Example Compound utilized in Example 7 includes a Z-type or kind aryl group as a substituent, and accordingly, showed element properties of a lower driving voltage, longer lifetime and higher efficiency.

Meanwhile, the Comparative Compounds utilized in Comparative Example 5 to Comparative Example 8 have a different bonding position of the nitrogen atom of the amine group with respect to a dibenzoheterole group or different bonding position of a substituent substituted at the dibenzoheterole group, when compared with the Example Compounds. Such a difference in bonding type or kind, which is differentiated from the Comparative Examples, is an important feature of the amine compound of the present disclosure, and accordingly, the light emitting elements of embodiments of the present disclosure may show element properties of a lower driving voltage, longer lifetime, and higher efficiency when compared to the Comparative Examples.

For example, from the results of Table 1, when the amine compound of an embodiment of the present disclosure is utilized as the material of a hole transport layer, it could be confirmed that longer lifetime and higher efficiency could be obtained (e.g., shown) when compared to the cases utilizing the Comparative Compounds as the materials of a hole transport layer.

In the amine compound of an embodiment, an aryl group or a heteroaryl group is substituted at position 4 of a dibenzoheterole group, or a naphthalene group is bonded to the nitrogen atom of the amine group via a p-phenylene linker, to increase conjugation, and a fluorene group, a carbazole group, a Z-type or kind aryl, and/or the like is additionally introduced as a substituent, to achieve electrical stability and high charge transport capacity. Accordingly, the amine compound of an embodiment, when utilized as the material of a light emitting element, may contribute to the decrease of a driving voltage, and the increase of lifetime and efficiency of the light emitting element.

The light emitting element of an embodiment includes an amine compound of an embodiment and may show suitable or high efficiency and suitable or long-life characteristics.

The amine compound of an embodiment may be utilized as a material for accomplishing improved properties of a light emitting element of high efficiency and long lifetime.

As used herein, the term "and/or" includes any and all combinations of one or more of the associated listed items. Throughout the disclosure, the expression "at least one of a, b or c", "at least one selected from a, b, and c", "at least one selected from the group consisting of a, b, and c", "at least one from among a, b, and c", etc., indicates only a, only b, only c, both (e.g., simultaneously) a and b, both (e.g., simultaneously) a and c, both (e.g., simultaneously) b and c, all of a, b, and c, or variations thereof.

The use of "may" when describing embodiments of the present disclosure refers to "one or more embodiments of the present disclosure".

As used herein, the terms "substantially", "about", and similar terms are used as terms of approximation and not as terms of degree, and are intended to account for the inherent deviations in measured or calculated values that would be recognized by those of ordinary skill in the art. "About" or "approximately," as used herein, is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" may mean within one or more standard deviations, or within ± 30%, 20%, 10%, 5% of the stated value.

Any numerical range recited herein is intended to include all sub-ranges of the same numerical precision subsumed within the recited range. For example, a range of "1.0 to 10.0" is intended to include all subranges between (and including) the recited minimum value of 1.0 and the recited maximum value of 10.0, that is, having a minimum value equal to or greater than 1.0 and a maximum value equal to or less than 10.0, such as, for example, 2.4 to 7.6. Any maximum numerical limitation recited herein is intended to include all lower numerical limitations subsumed therein and any minimum numerical limitation recited in this specification is intended to include all higher numerical limitations subsumed therein. Accordingly, Applicant reserves the right to amend this specification, including the claims, to expressly recite any sub-range subsumed within the ranges expressly recited herein.

The display device, and/or any other relevant devices or components according to embodiments of the present invention described herein may be implemented utilizing any suitable hardware, firmware (e.g. an application-specific integrated circuit), software, or a combination of software, firmware, and hardware. For example, the various components of the device may be formed on one integrated circuit (IC) chip or on separate IC chips. Further, the various components of the device may be implemented on a flexible printed circuit film, a tape carrier package (TCP), a printed circuit board (PCB), or formed on one substrate. Further, the various components of the device may be a process or thread, running on one or more processors, in one or more computing devices, executing computer program instructions and interacting with other system components for performing the various functionalities described herein. The computer program instructions are stored in a memory which may be implemented in a computing device using a standard memory device, such as, for example, a random access memory (RAM). The computer program instructions may also be stored in other non-transitory computer readable media such as, for example, a CD-ROM, flash drive, or the like. Also, a person of skill in the art should recognize that the functionality of various computing devices may be combined or integrated into a single computing device, or the functionality of a particular computing device may be distributed across one or more other computing devices without departing from the scope of the embodiments of the present disclosure.

Although the embodiments of the present disclosure have been described, it is understood that the present disclosure should not be limited to these embodiments.

## Claims

1. An amine compound represented by Formula 1: 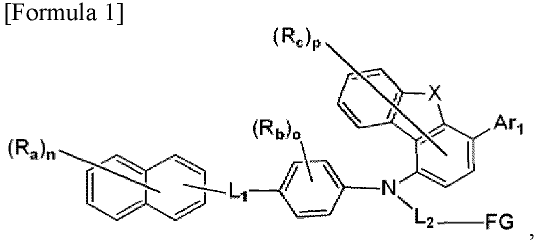
wherein in Formula 1,
X is 0 or S,
L₁ and L₂ are each independently a direct linkage, a substituted or unsubstituted arylene group of 6 to 15 ring-forming carbon atoms, or a substituted or unsubstituted heteroarylene group of 1 to 12 ring-forming carbon atoms,
"n" is an integer of 0 to 7, "o" is an integer of 0 to 4, "p" is an integer of 0 to 6,
Rₐ, R_{b} and R_{c} are each independently a deuterium atom, a halogen atom, a substituted or unsubstituted alkyl group of 1 to 10 carbon atoms, a substituted or unsubstituted aryl group of 6 to 30 ring-forming carbon atoms, or a substituted or unsubstituted heteroaryl group of 1 to 30 ring-forming carbon atoms,
Ar₁ is a substituted or unsubstituted phenyl group, a substituted or unsubstituted naphthyl group, a substituted or unsubstituted phenanthrenyl group, a substituted or unsubstituted dibenzothiophene group, or a substituted or unsubstituted dibenzofuran group, and
FG is represented by any one of Formula 2 to Formula 5: 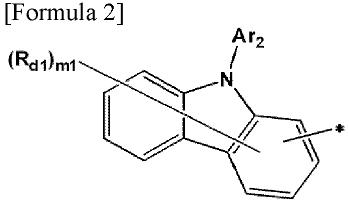 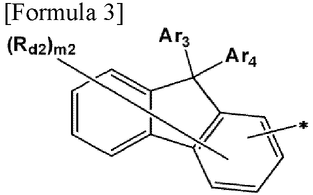 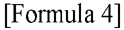 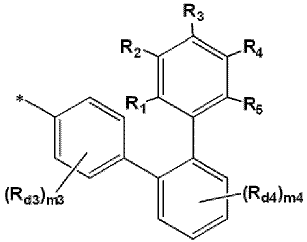 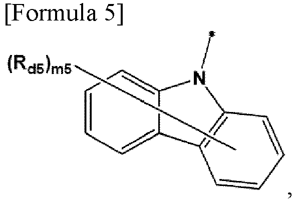
in Formula 2 and Formula 3, Ar₂ to Ar₄ are each independently a substituted or unsubstituted aryl group of 6 to 40 ring-forming carbon atoms, or a substituted or unsubstituted heteroaryl group of 1 to 40 ring-forming carbon atoms,
in Formula 2 to Formula 5, R_{d1} to R_{d5} are each independently a deuterium atom, a halogen atom, a substituted or unsubstituted alkyl group of 1 to 20 carbon atoms, a substituted or unsubstituted alkenyl group of 2 to 20 carbon atoms, a substituted or unsubstituted oxy group, a substituted or unsubstituted thio group, a substituted or unsubstituted amine group, a substituted or unsubstituted aryl group of 6 to 30 ring-forming carbon atoms, a substituted or unsubstituted heteroaryl group of 1 to 30 ring-forming carbon atoms, or combined with an adjacent group to form a hydrocarbon ring or a heterocycle,
m1 and m2 are each independently an integer of 0 to 7,
m3 and m4 are each independently an integer of 0 to 4, and
m5 is an integer of 0 to 8, and
in Formula 4, R₁ to R₅ are each independently a hydrogen atom, a deuterium atom, a halogen atom, a substituted or unsubstituted alkyl group of 1 to 20 carbon atoms, a substituted or unsubstituted alkenyl group of 2 to 20 carbon atoms, a substituted or unsubstituted oxy group, a substituted or unsubstituted thio group, a substituted or unsubstituted amine group, a substituted or unsubstituted aryl group of 6 to 30 ring-forming carbon atoms, a substituted or unsubstituted heteroaryl group of 1 to 30 ring-forming carbon atoms, or combined with an adjacent group to form a hydrocarbon ring or a heterocycle.

2. The amine compound of claim 1, wherein the amine compound is represented by any one of Formula 1-1 to Formula 1-4: 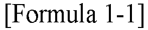 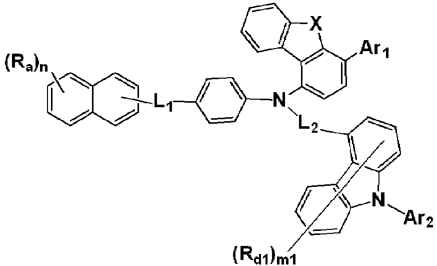 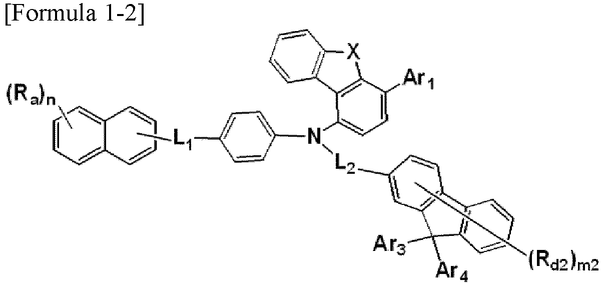 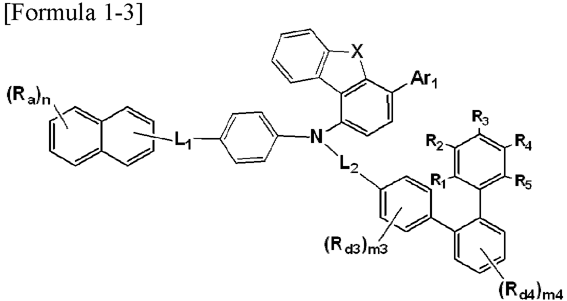 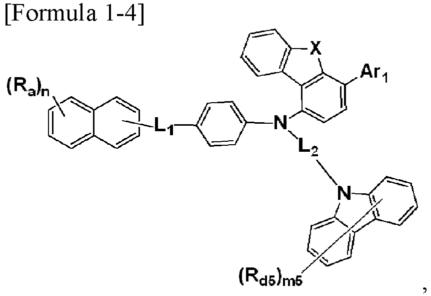
wherein in Formula 1-1 to Formula 1-4,
X, L₁, L₂, "n", Rₐ, and Ar₁ are the same as defined in claim 1,
in Formula 1-1 and Formula 1-2, Ar₂ to Ar₄ are the same as defined in claim 1,
in Formula 1-3, R₁ to R₅ are the same as defined in claim 1, and
in Formula 1-1 to Formula 1-4, R_{d1} to R_{d5}, and m1 to m5 are the same as defined in claim 1.

3. The amine compound of claim 1 or 2, wherein L₁ is a direct linkage, an unsubstituted phenylene group, or an unsubstituted divalent biphenyl group.

4. The amine compound of any one of claims 1 to 3, wherein L₂ is a direct linkage, an unsubstituted phenylene group, an unsubstituted naphthalene group, an unsubstituted divalent dibenzofuran group, or an unsubstituted divalent dibenzothiophene group.

5. The amine compound of any one of claims 1 to 4, wherein Ar₁ is represented by any one of Ar1-1 to Ar1-5:

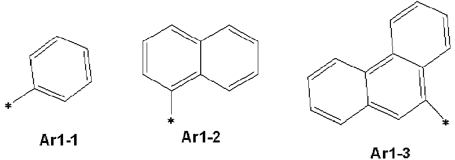

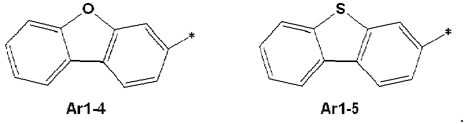

6. The amine compound of any one of claims 1 to 5, wherein, in Formula 4, R₁ to R₅ are combined with the benzene ring at which R₁ to R₅ are substituted, to form a dibenzofuran ring, a dibenzothiophene ring, or a carbazole ring.

7. The amine compound of any one of claims 1 to 6, wherein the amine compound comprises a structure in which at least one hydrogen atom is substituted with a deuterium atom.

8. The amine compound of any one of claims 1 to 7, wherein the amine compound is a monoamine compound.

9. The amine compound of claim 1, wherein the amine compound is any one of the compounds in Compound Group 1: 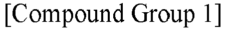 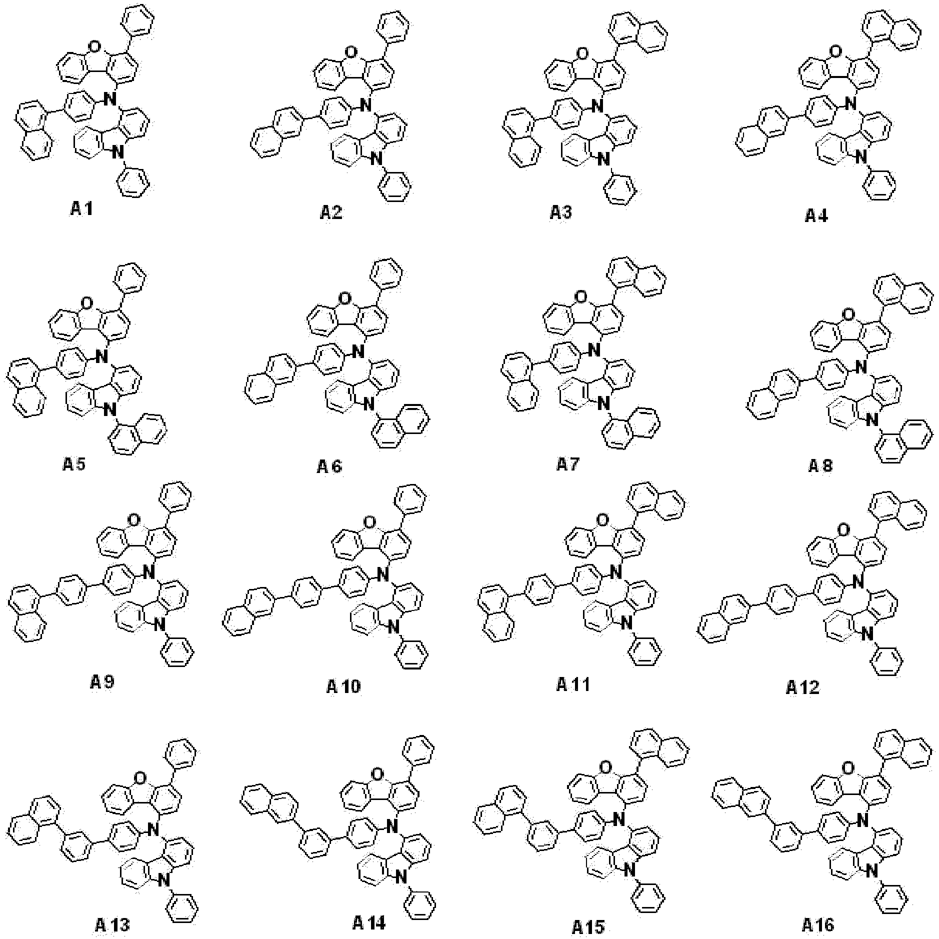 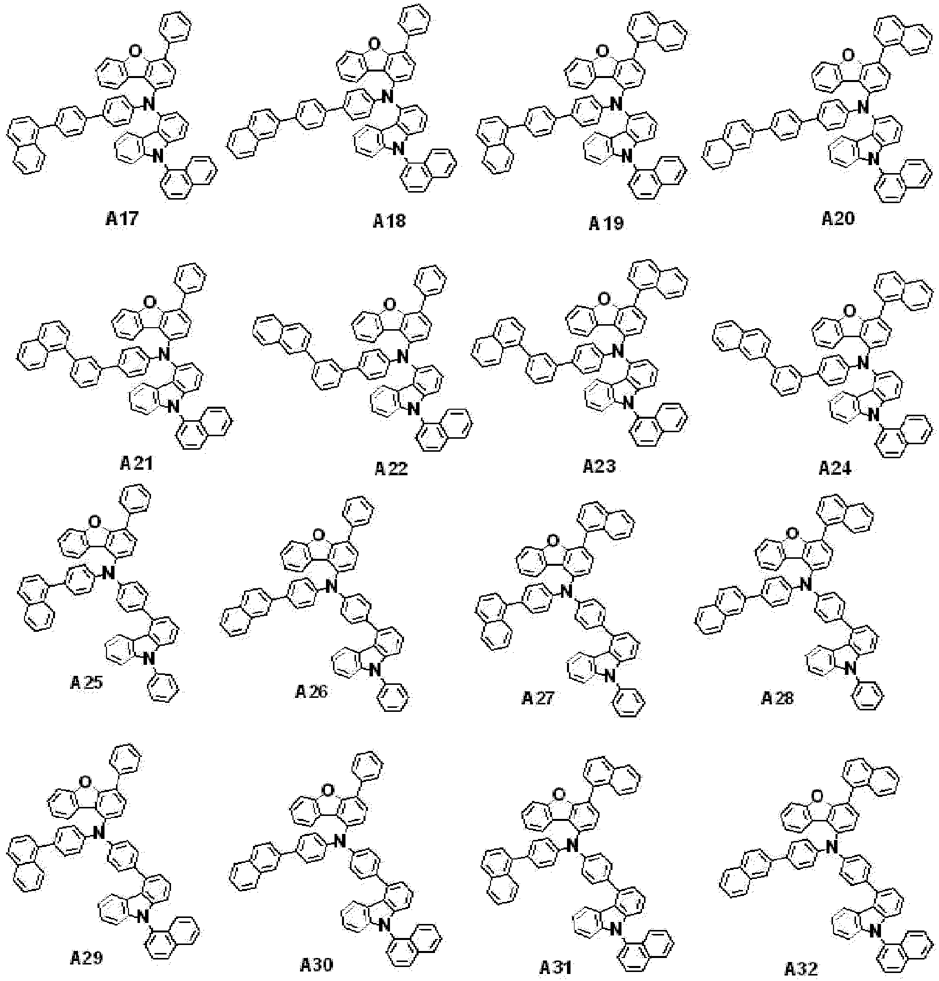 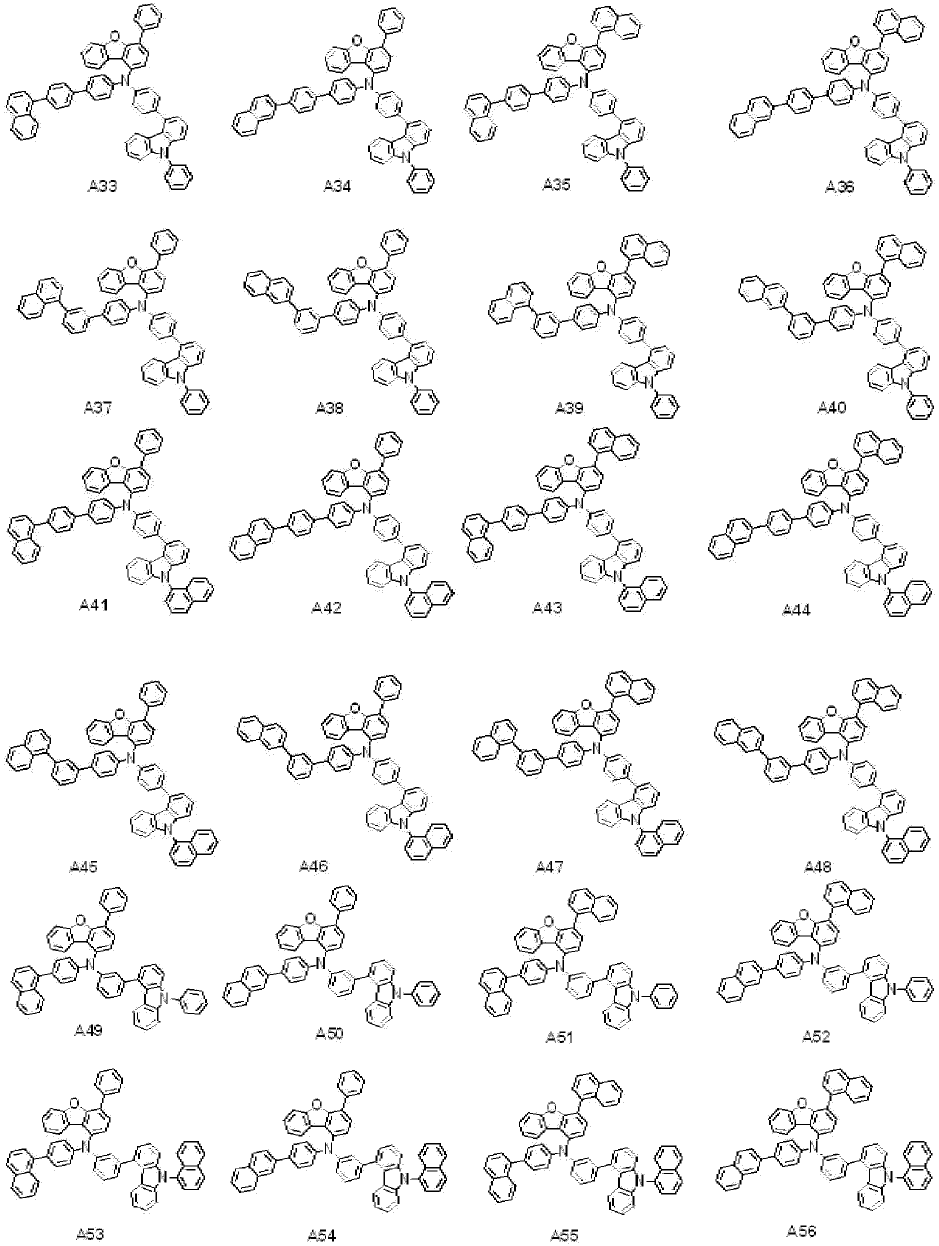 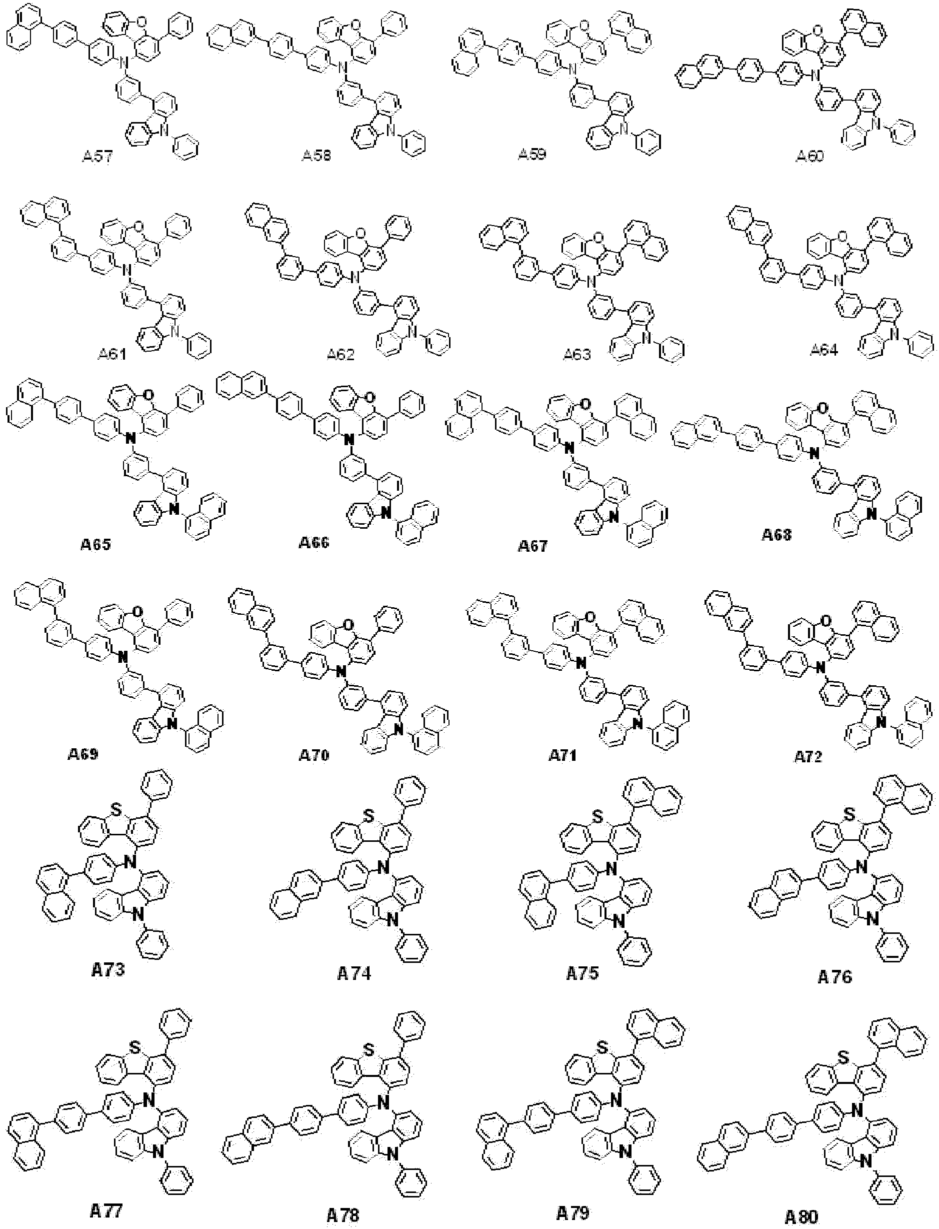 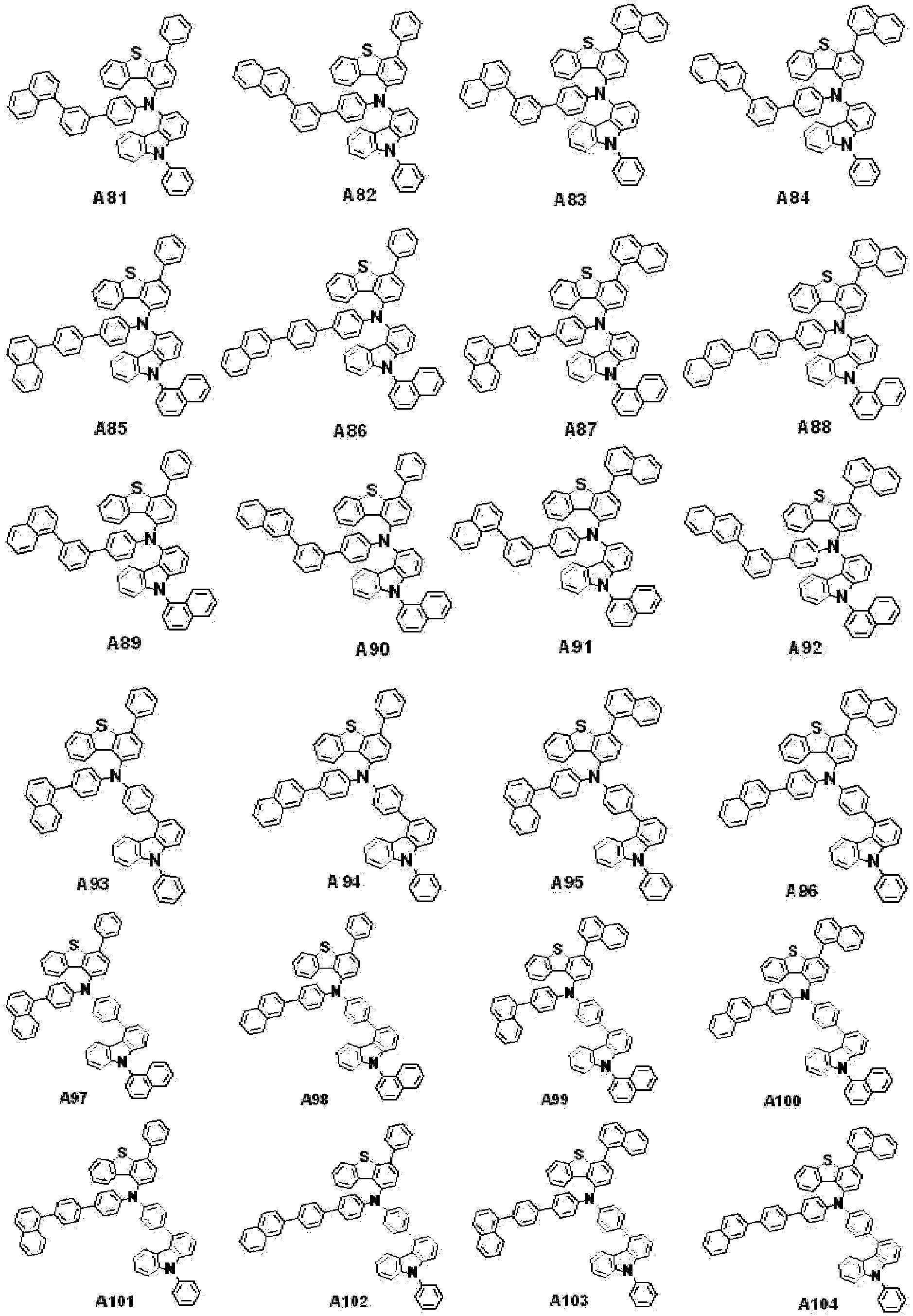 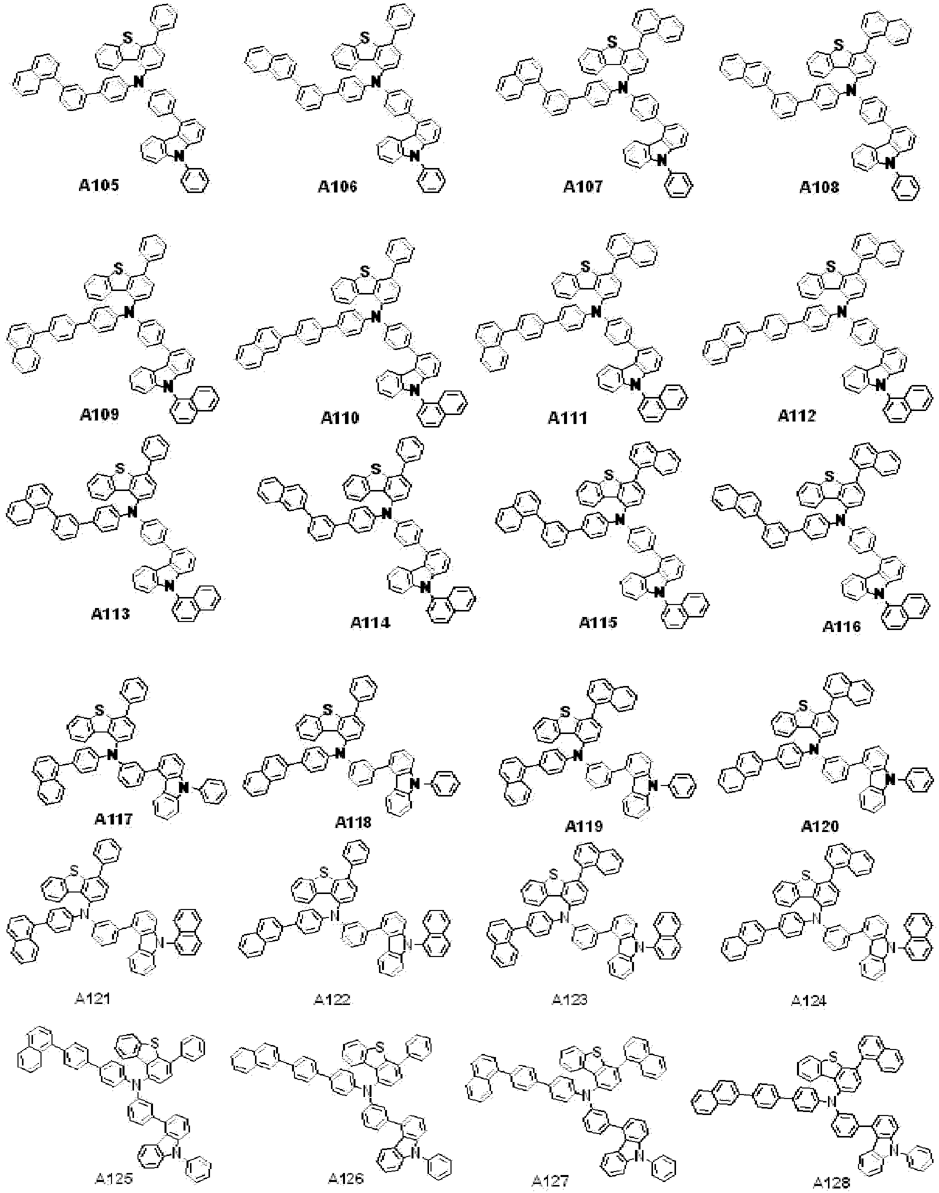 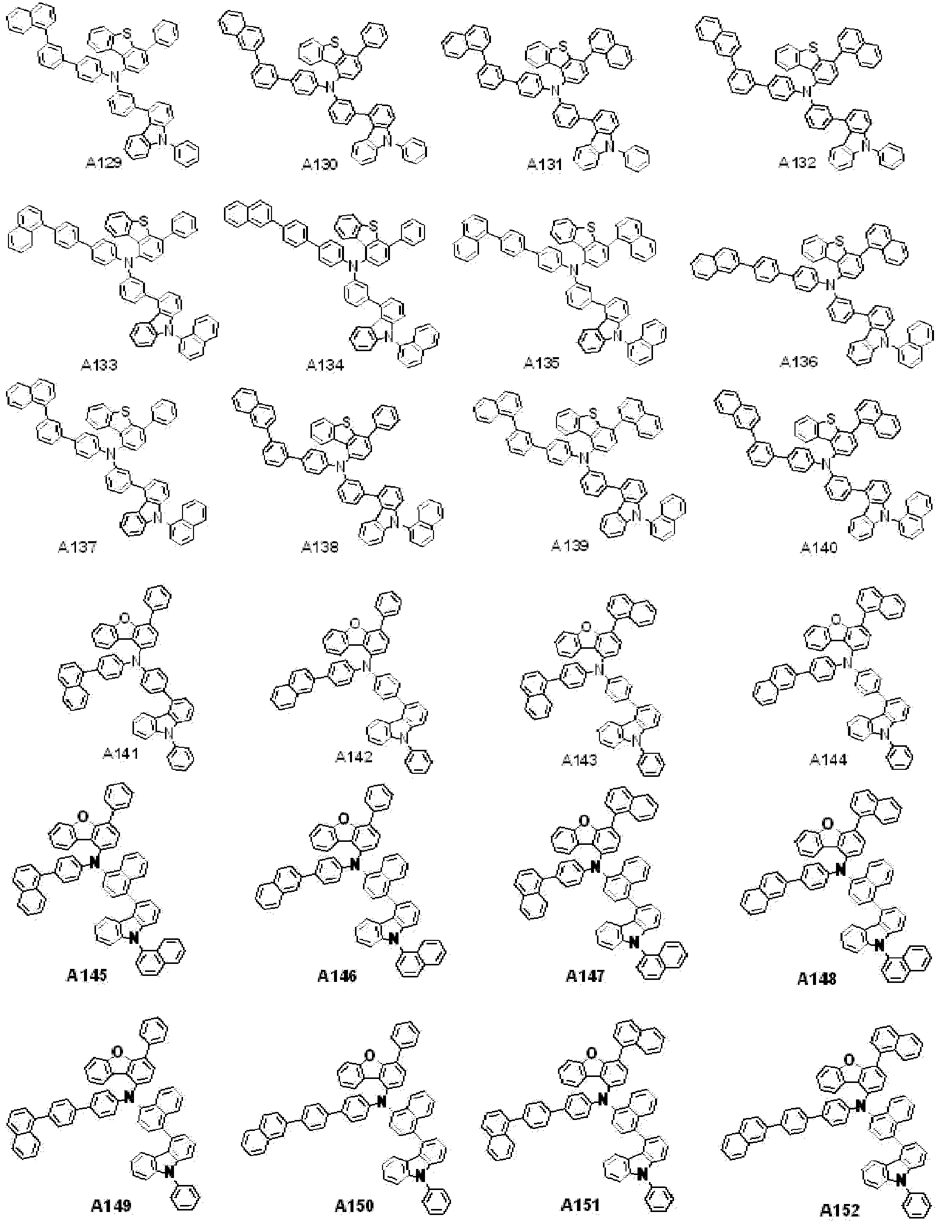 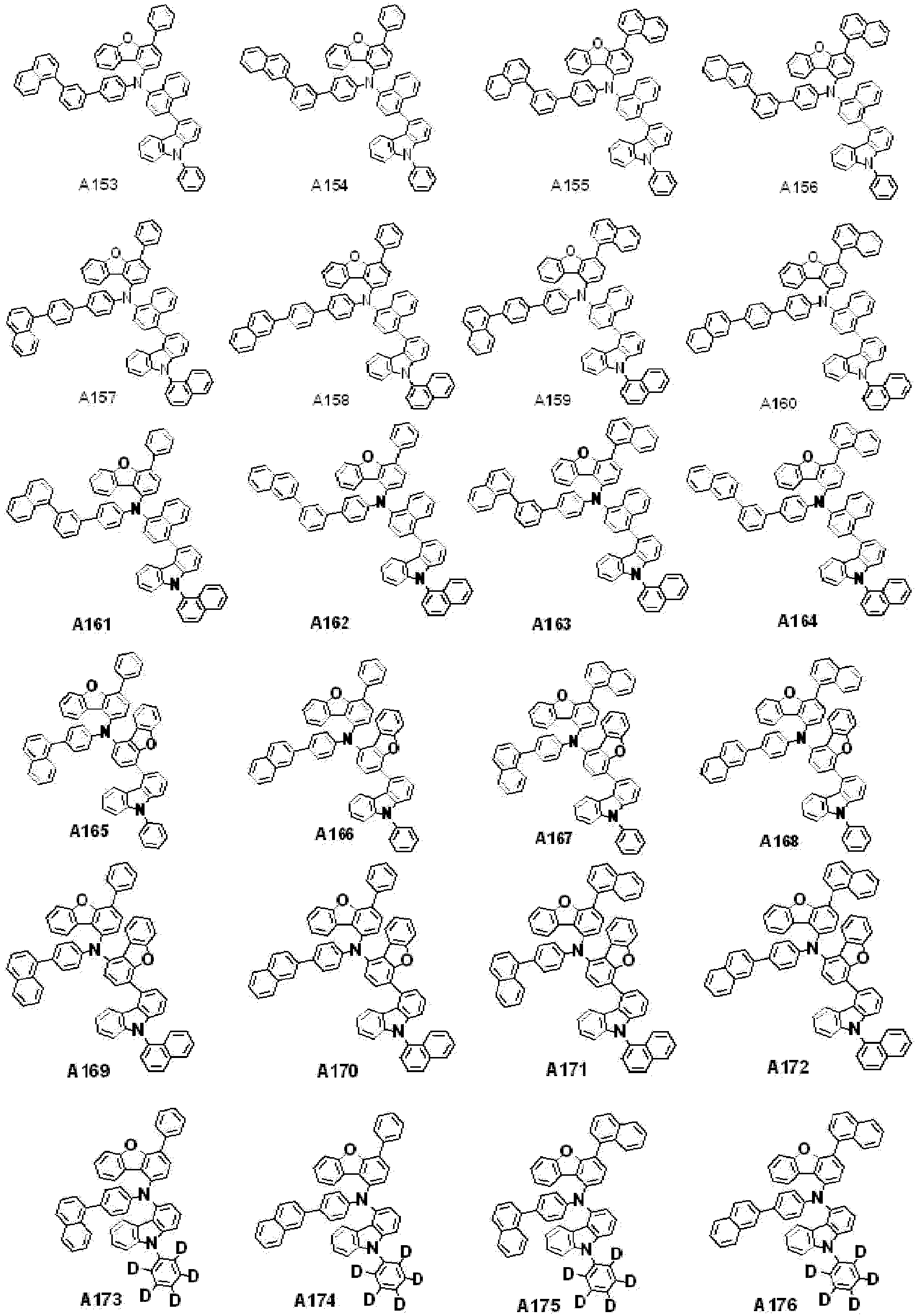 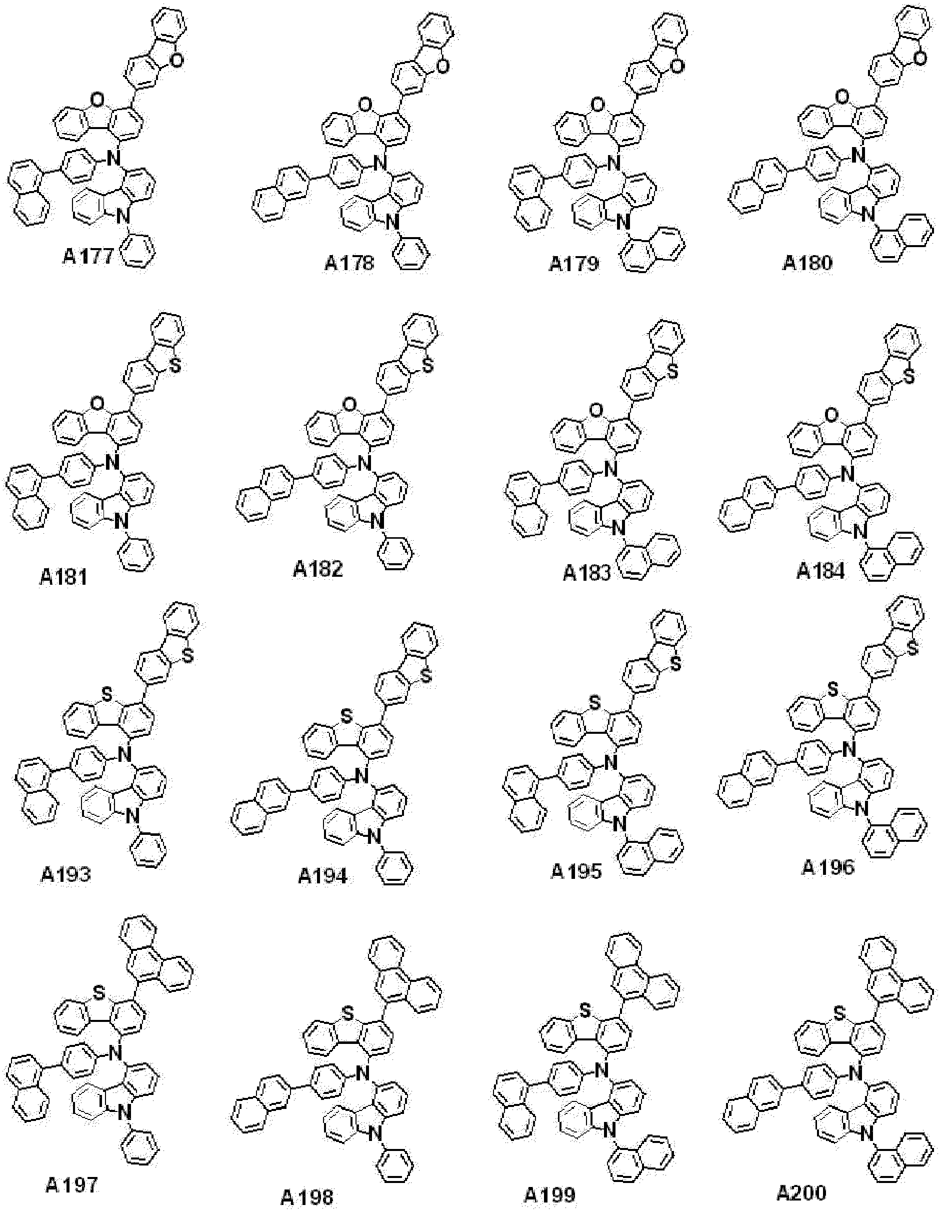 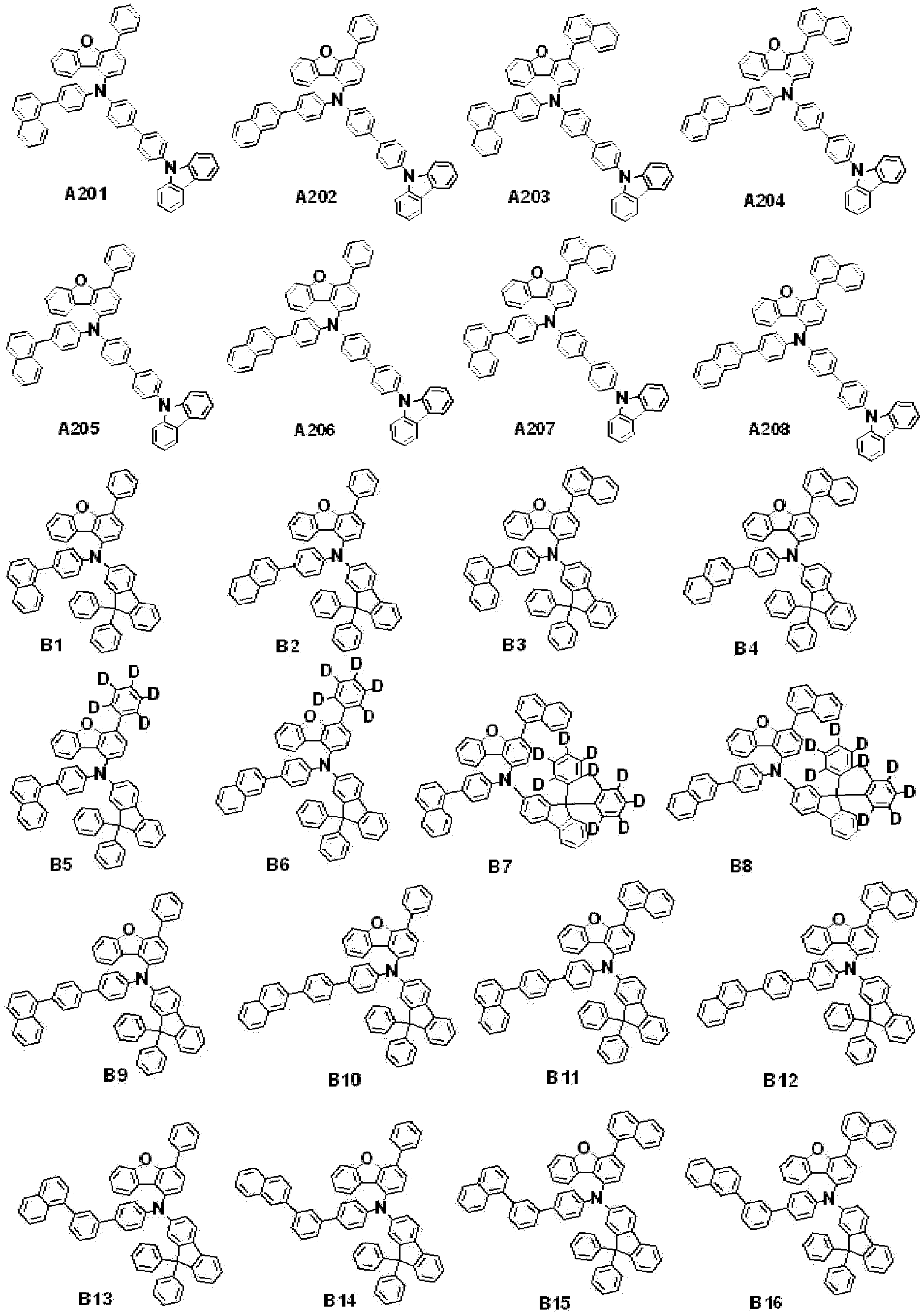 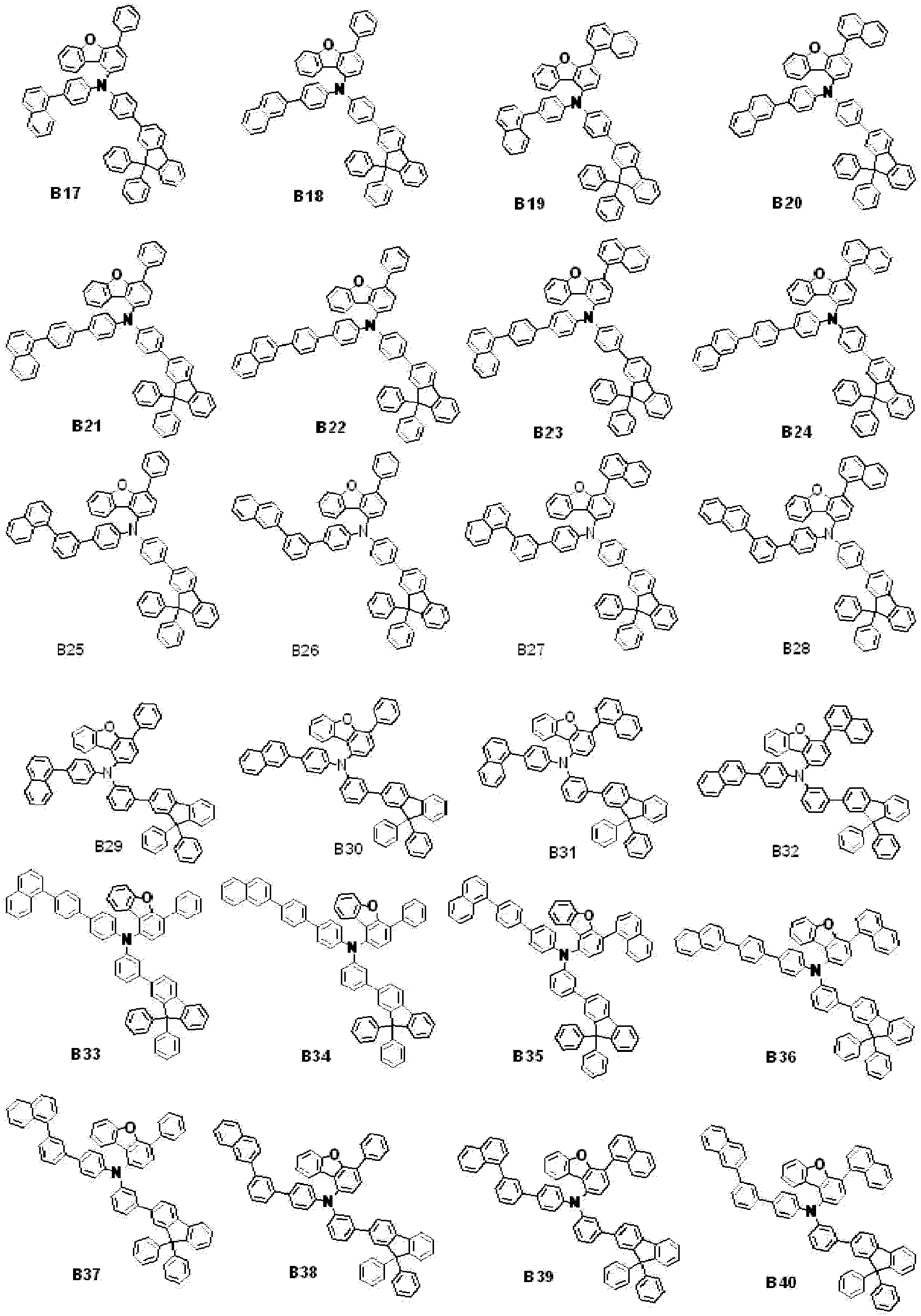 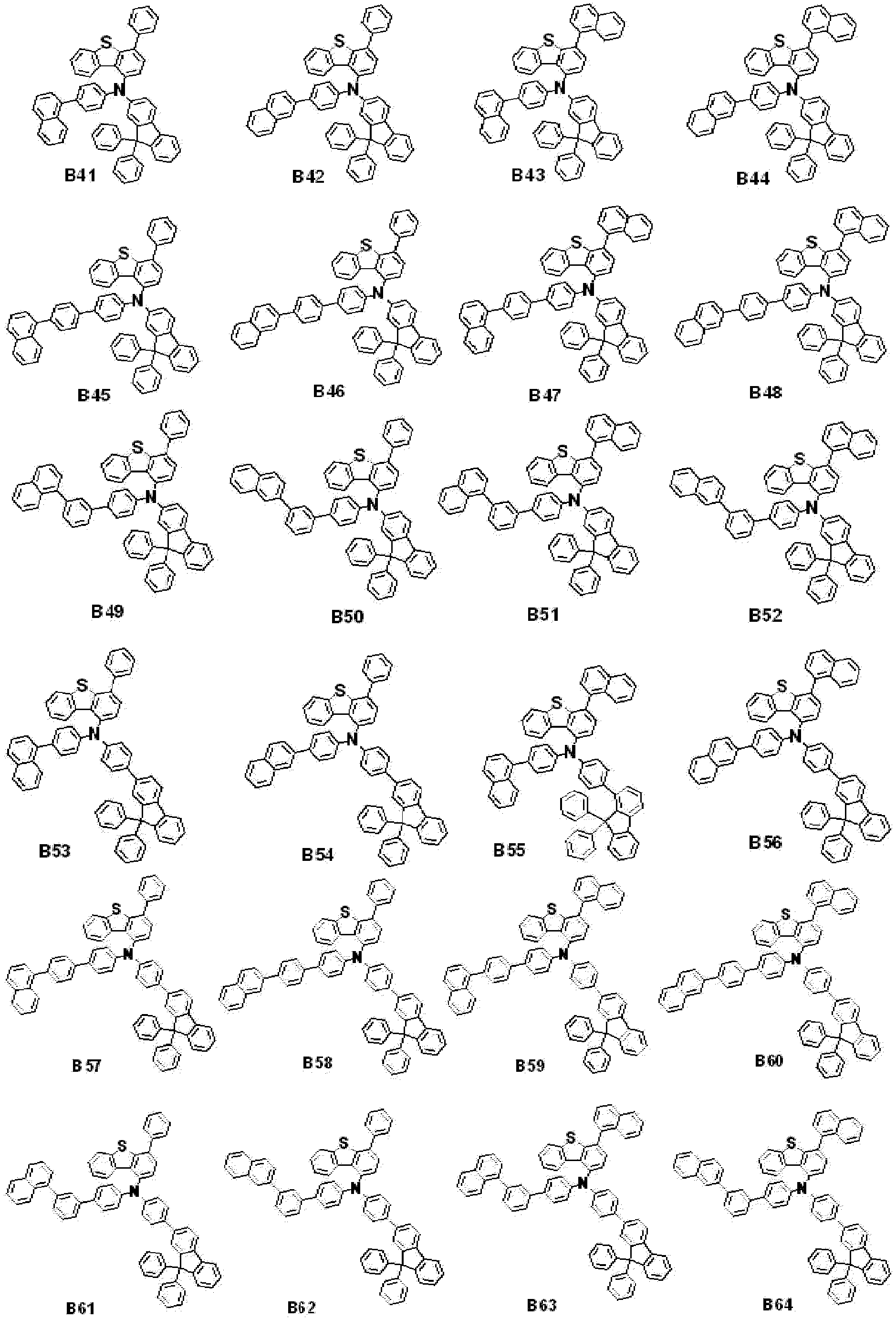 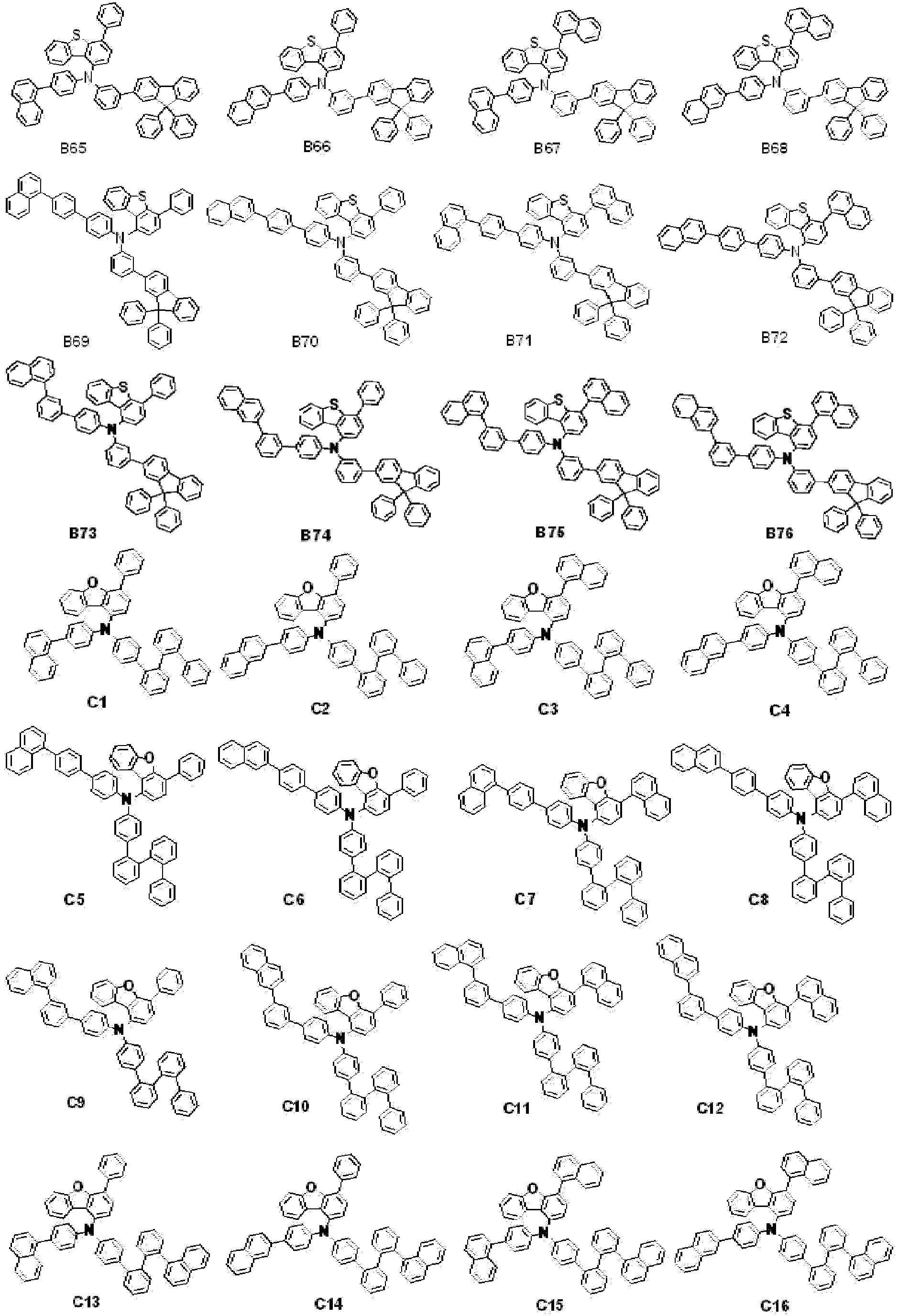 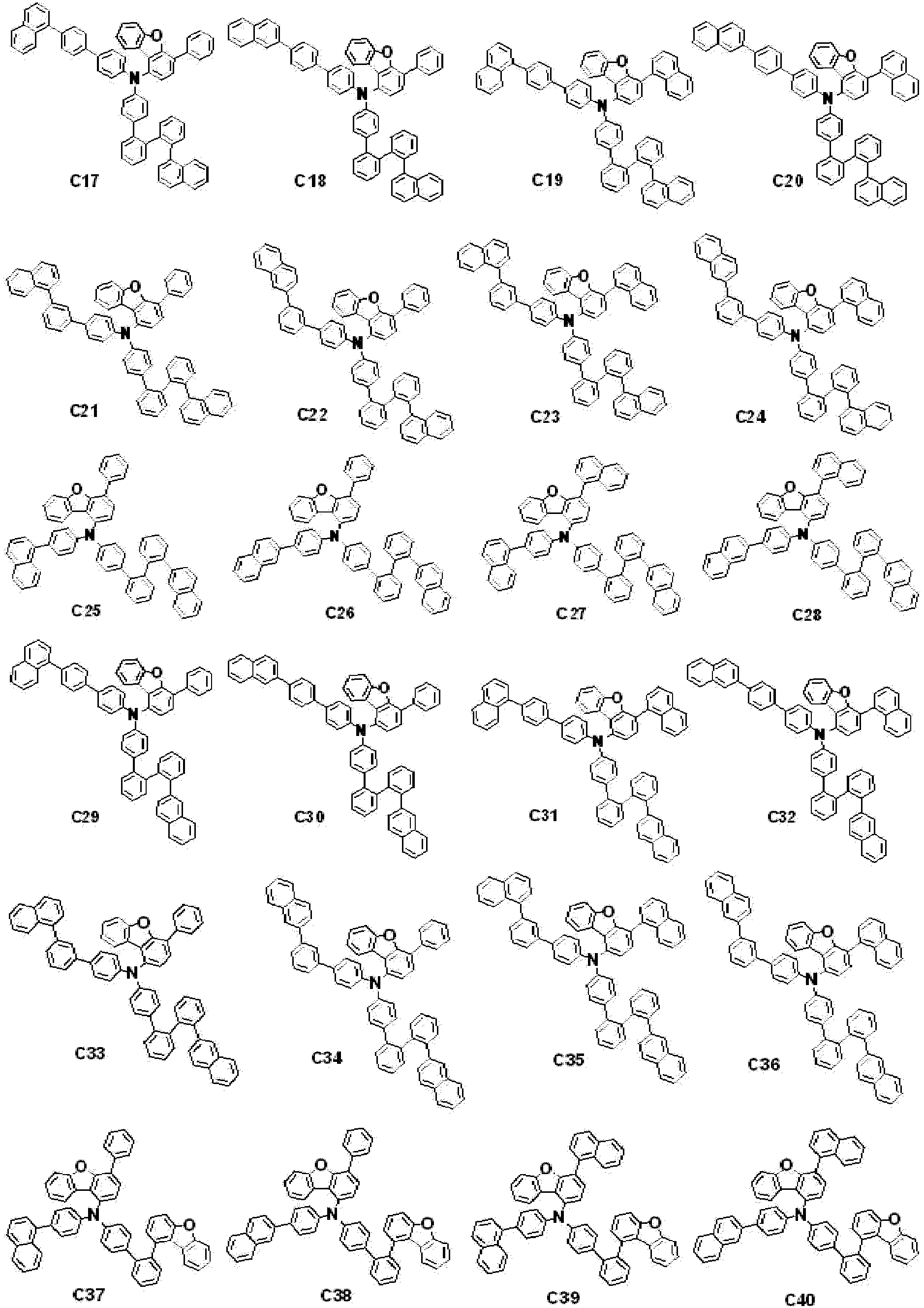 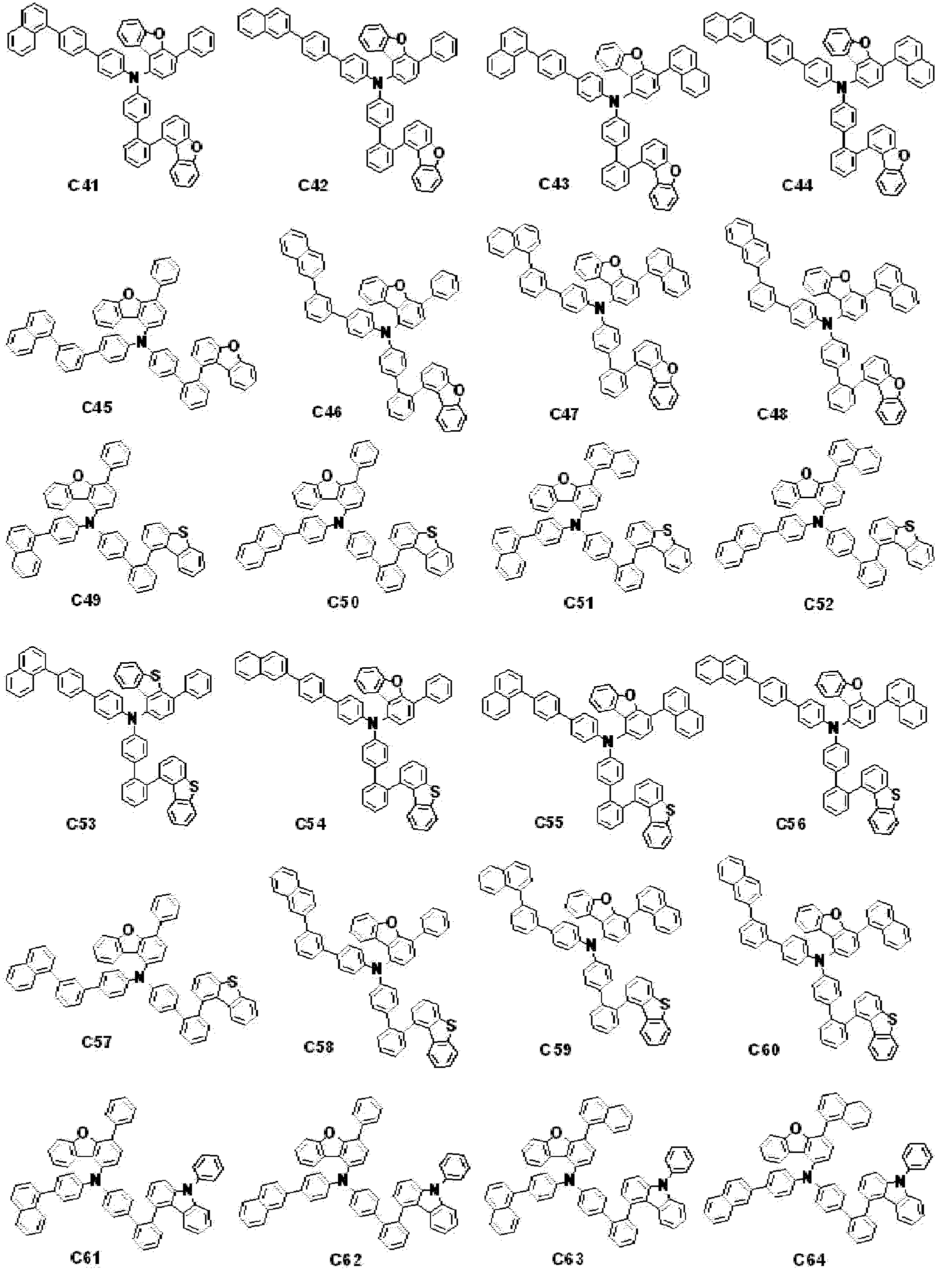 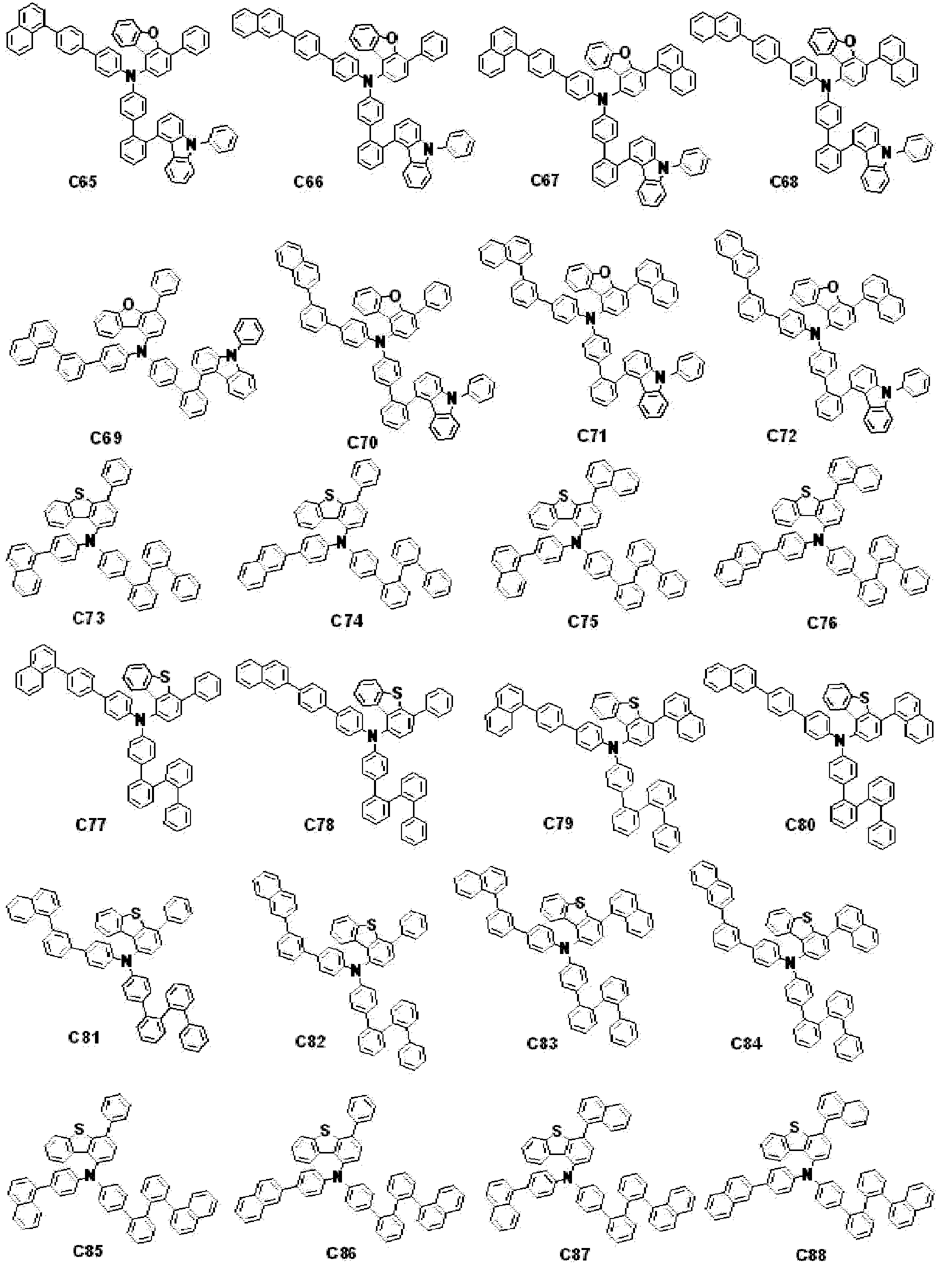 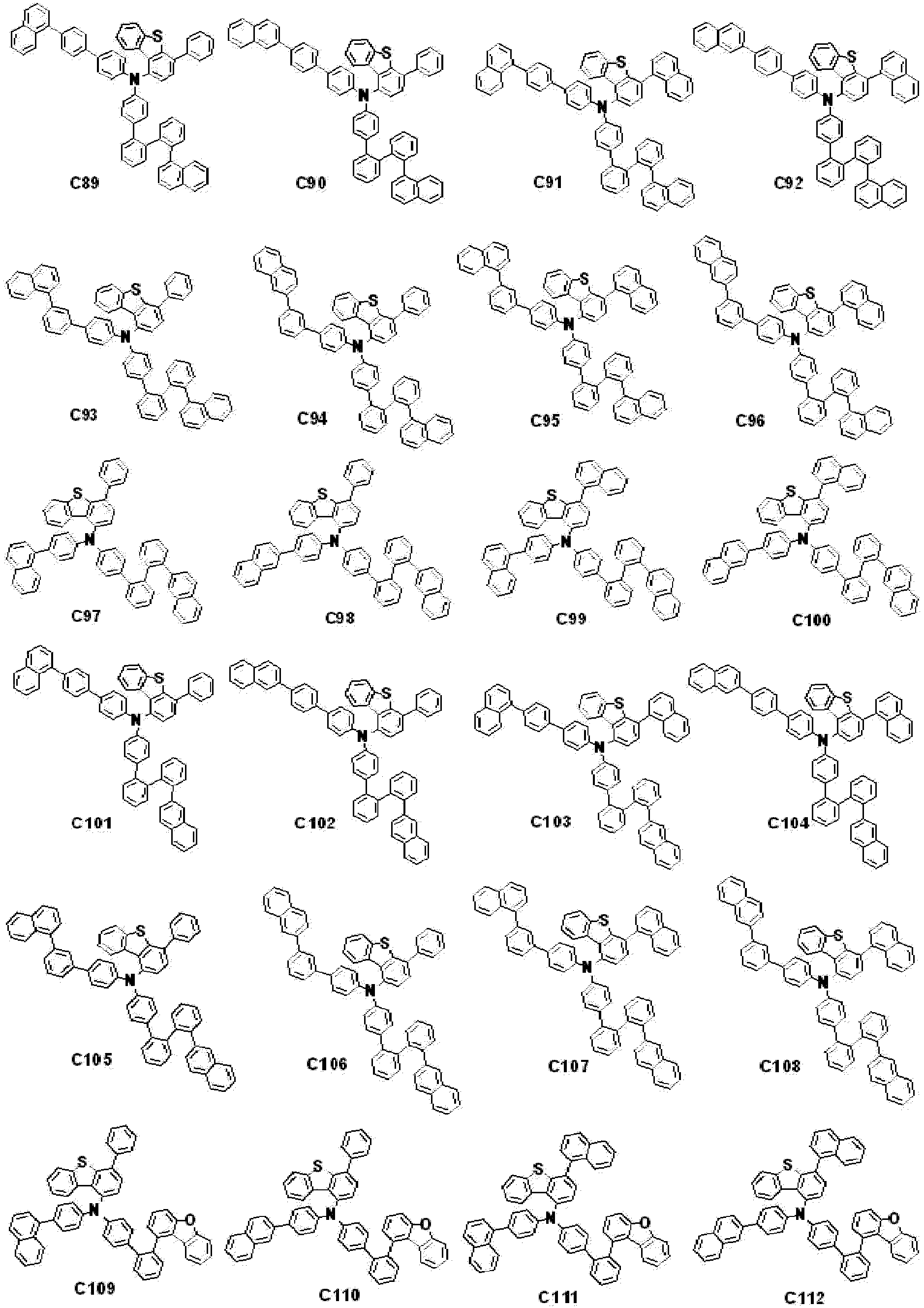 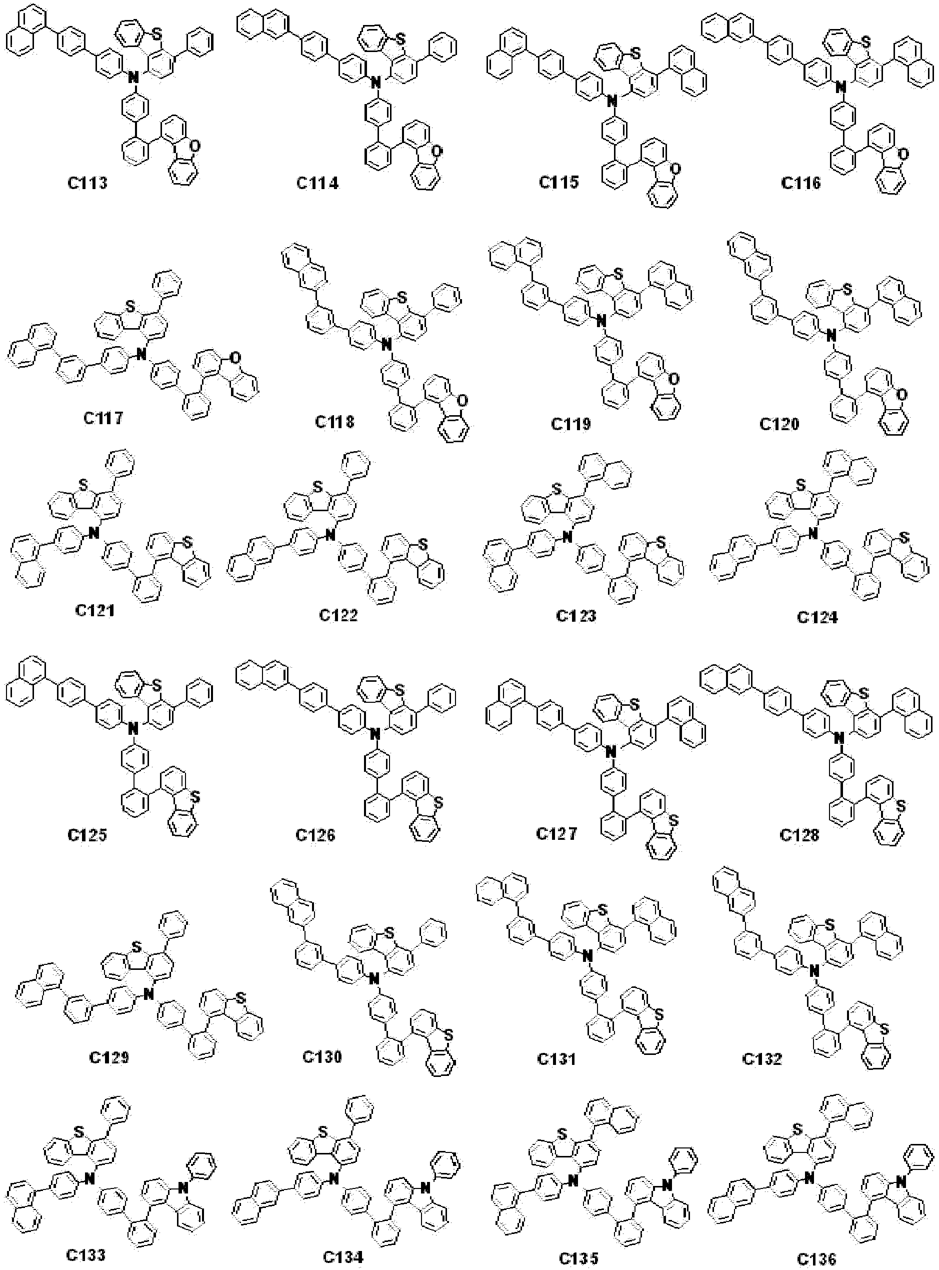 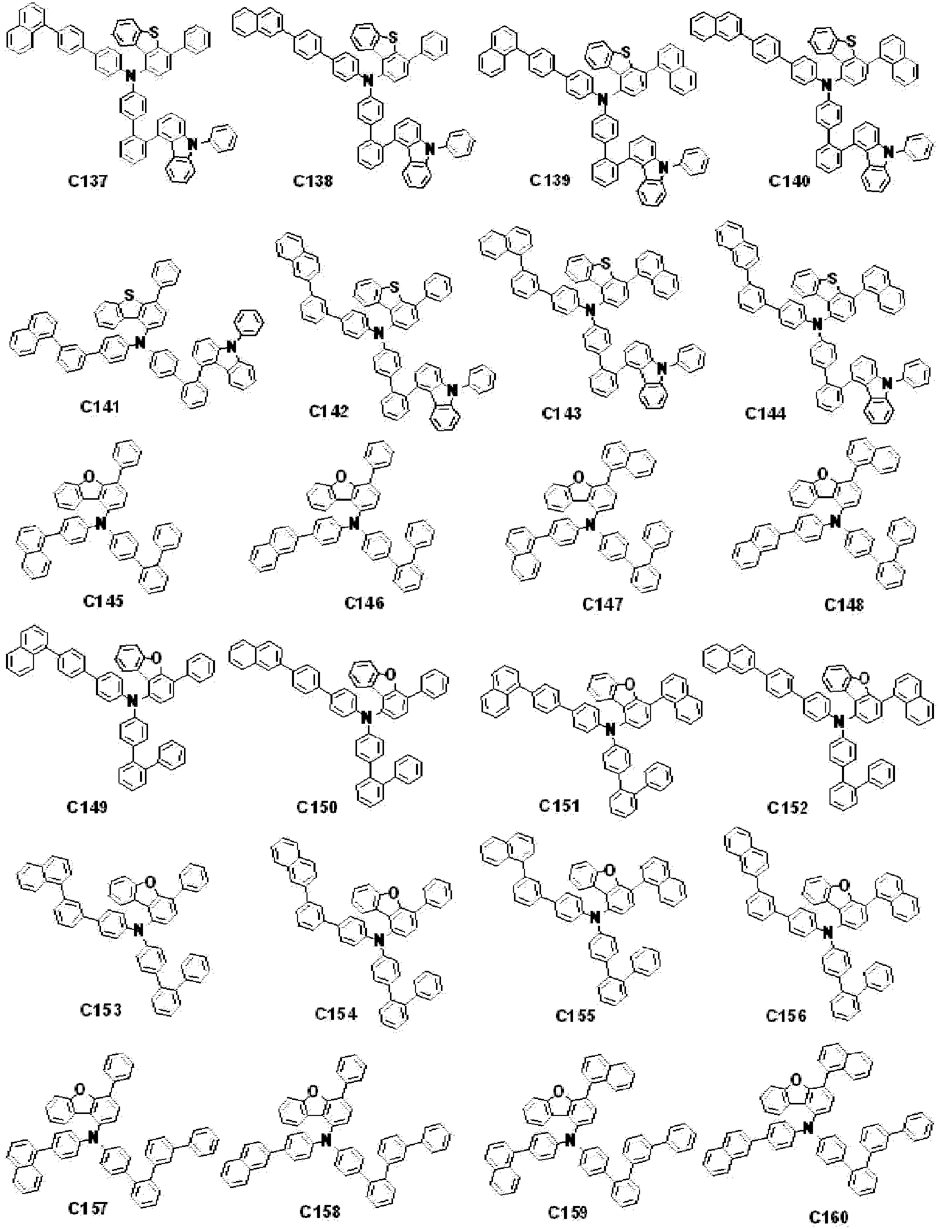 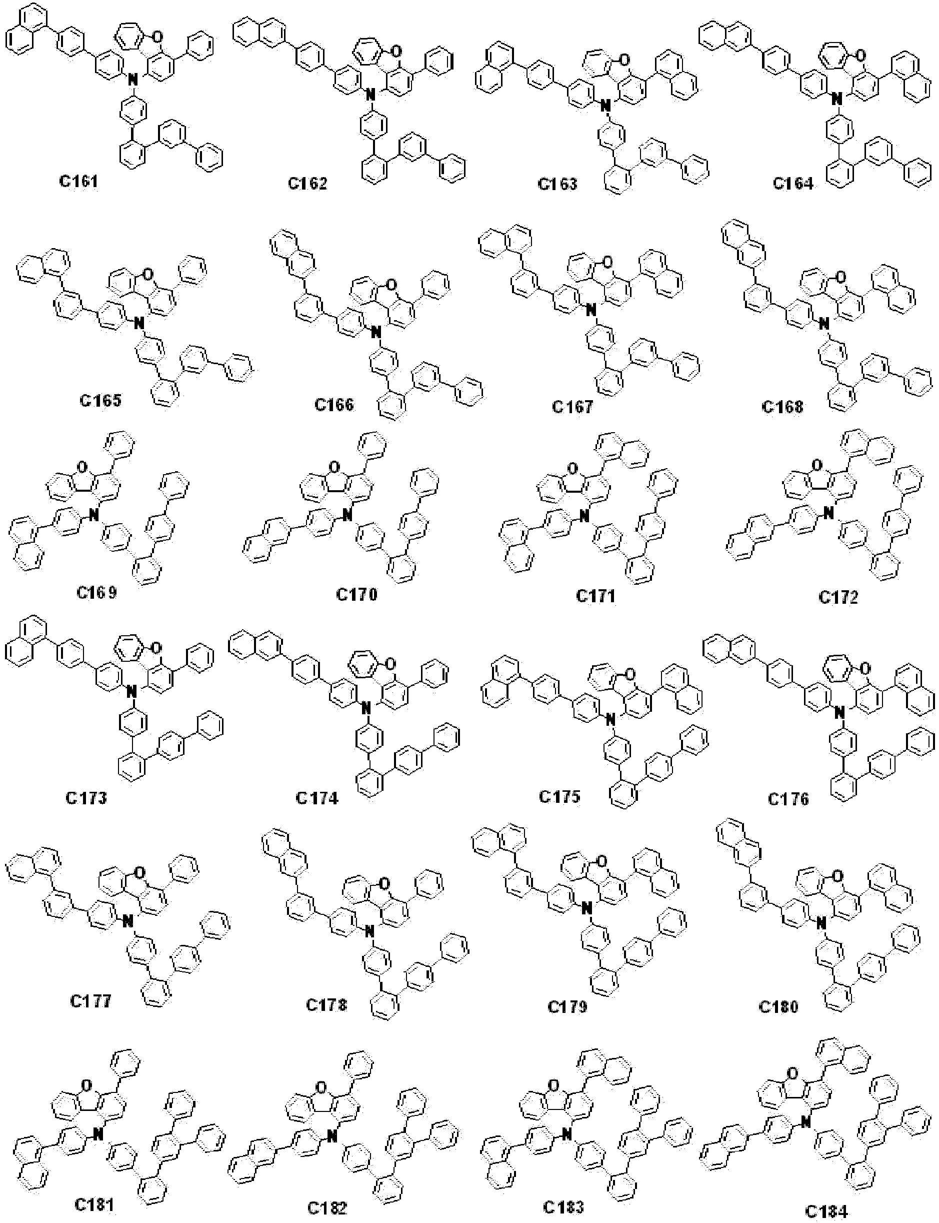 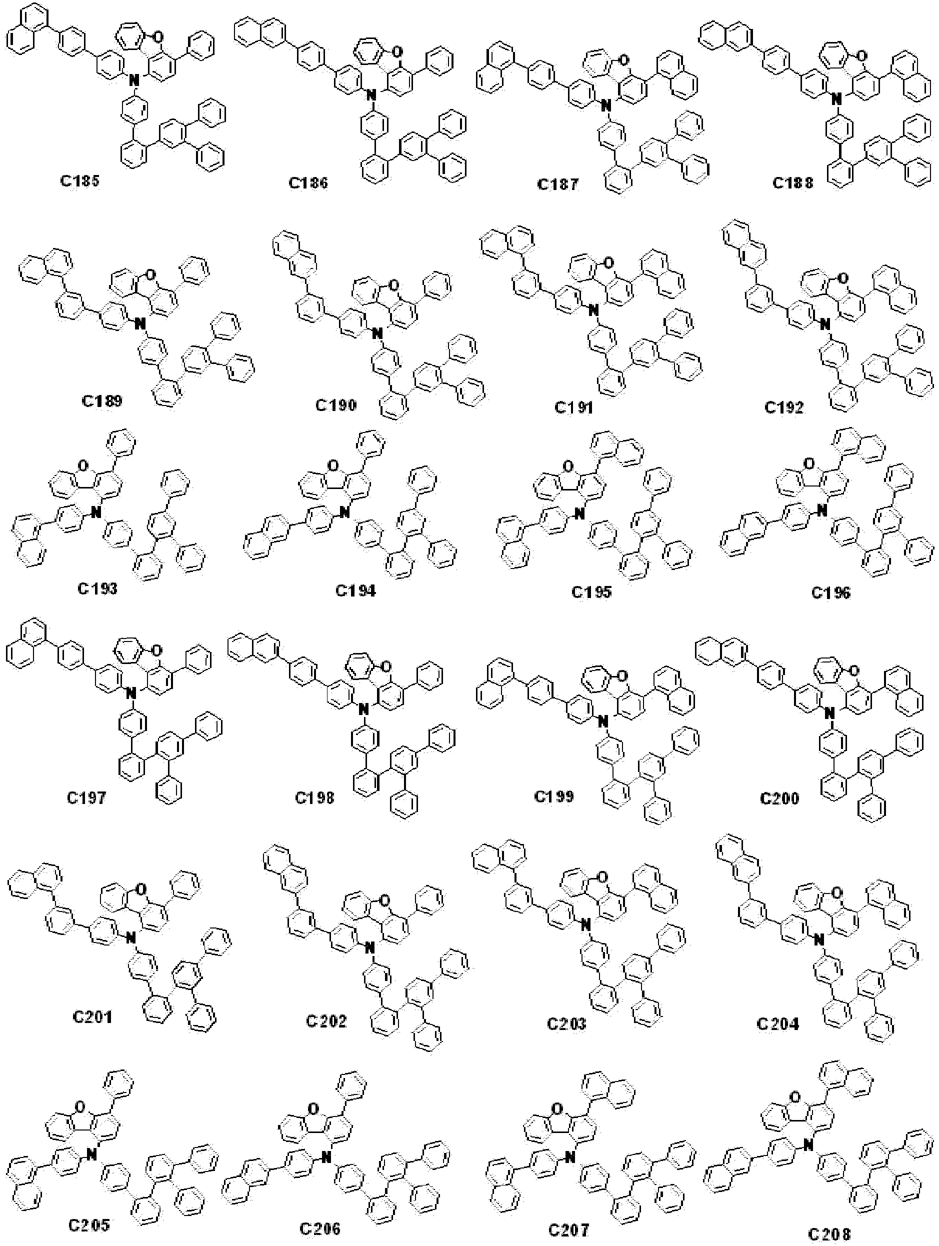 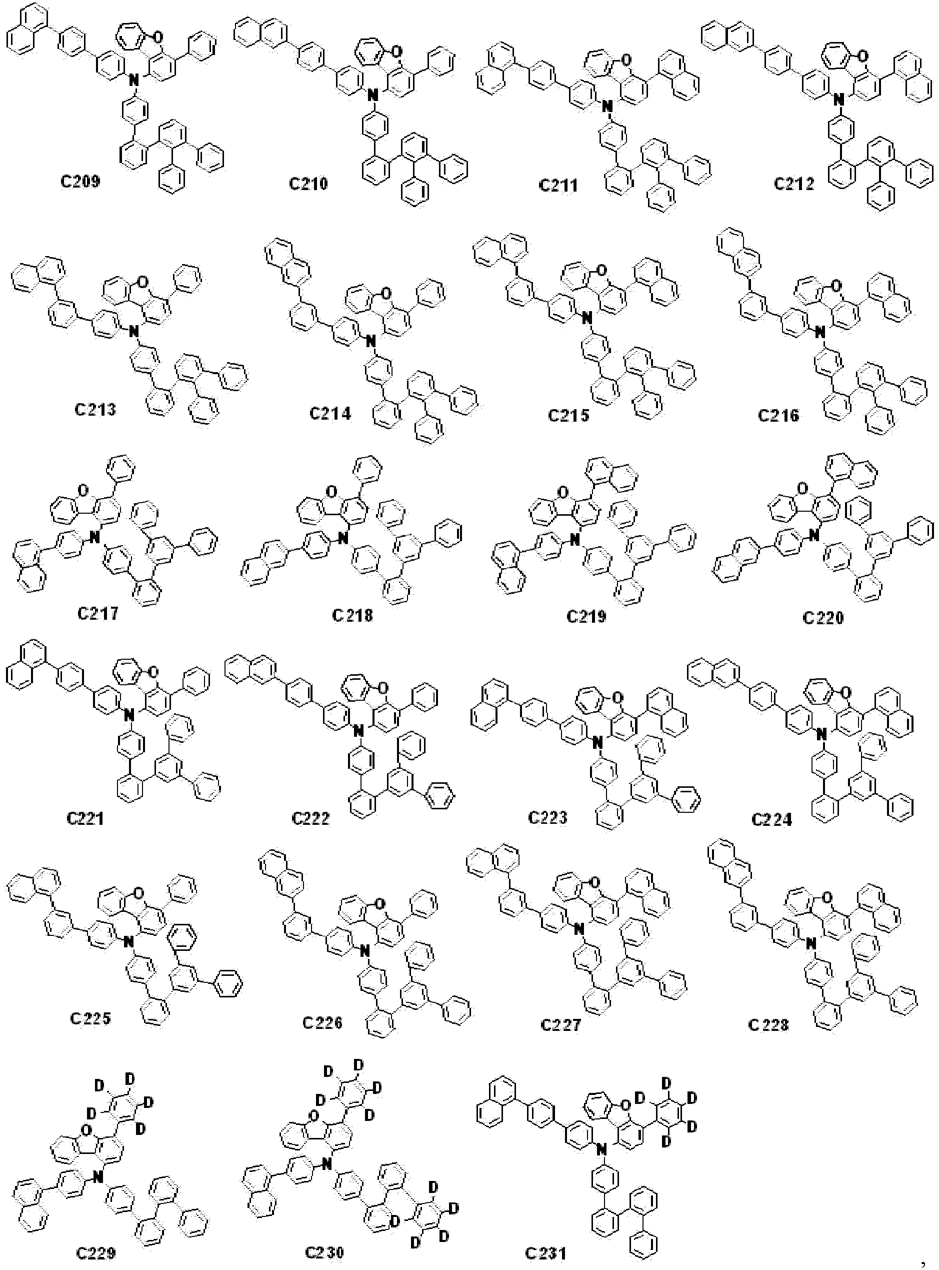 wherein in Compound Group 1, D is a deuterium atom.

10. A light emitting element, comprising:
a first electrode;
a second electrode facing the first electrode; and
at least one functional layer between the first electrode and the second electrode, and comprising an amine compound according to any one of claims 1 to 9.

11. The light emitting element of claim 10, wherein the at least one functional layer comprises an emission layer, a hole transport region between the first electrode and the emission layer, and an electron transport region between the emission layer and the second electrode, and
the hole transport region comprises the amine compound.

12. The light emitting element of claim 11, wherein the hole transport region comprises at least one of a hole injection layer, a hole transport layer, and an electron blocking layer, and
the at least one of the hole injection layer, the hole transport layer, and the electron blocking layer comprises the amine compound.

13. The light emitting element of claim 11, wherein the emission layer comprises a compound represented by Formula E-1: 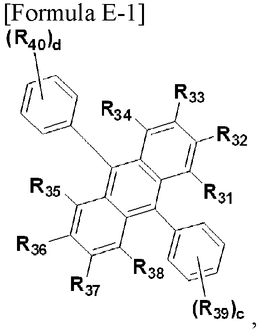
wherein in Formula E-1,
"c" and "d" are each independently an integer of 0 to 5, and
R₃₁ to R₄₀ are each independently a hydrogen atom, a deuterium atom, a halogen atom, a substituted or unsubstituted silyl group, a substituted or unsubstituted thio group, a substituted or unsubstituted oxy group, a substituted or unsubstituted alkyl group of 1 to 10 carbon atoms, a substituted or unsubstituted alkenyl group of 2 to 10 carbon atoms, a substituted or unsubstituted aryl group of 6 to 30 ring-forming carbon atoms, a substituted or unsubstituted heteroaryl group of 2 to 30 ring-forming carbon atoms, or combined with an adjacent group to form a ring.

## Patentansprüche

1. Aminverbindung, dargestellt durch Formel 1: 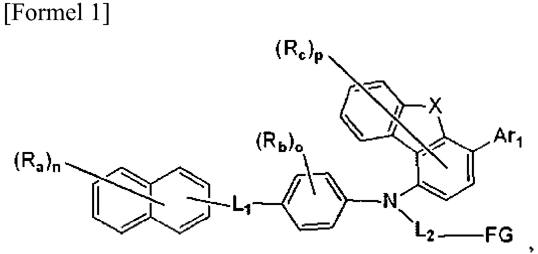
wobei in Formel 1 Folgendes gilt
X ist O oder S,
L₁ und L₂ sind jeweils unabhängig eine direkte Verknüpfung, eine substituierte oder unsubstituierte Arylengruppe mit 6 bis 15 ringbildenden Kohlenstoffatomen oder eine substituierte oder unsubstituierte Heteroarylengruppe mit 1 bis 12 ringbildenden Kohlenstoffatomen,
"n" ist eine ganze Zahl von 0 bis 7, "o" ist eine ganze Zahl von 0 bis 4, "p" ist eine ganze Zahl von 0 bis 6,
Rₐ, R_{b} und R_{c} sind jeweils unabhängig ein Deuteriumatom, ein Halogenatom, eine substituierte oder unsubstituierte Alkylgruppe mit 1 bis 10 Kohlenstoffatomen, eine substituierte oder unsubstituierte Arylgruppe mit 6 bis 30 ringbildenden Kohlenstoffatomen oder eine substituierte oder unsubstituierte Heteroarylgruppe mit 1 bis 30 ringbildenden Kohlenstoffatomen,
Ar₁ ist eine substituierte oder unsubstituierte Phenylgruppe, eine substituierte oder unsubstituierte Naphthylgruppe, eine substituierte oder unsubstituierte Phenanthrenylgruppe, eine substituierte oder unsubstituierte Dibenzothiophengruppe oder eine substituierte oder unsubstituierte Dibenzofurangruppe,
FG durch eine beliebige von Formel 2 bis Formel 5 dargestellt ist: 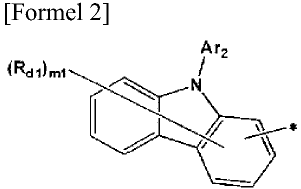 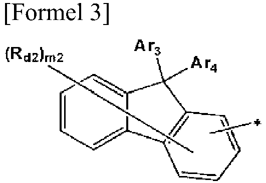 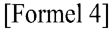 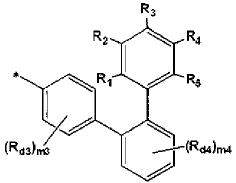 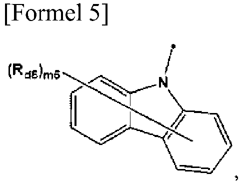
in Formel2 und Formel 3 Ar₂ bis Ar₄ jeweils unabhängig eine substituierte oder unsubstituierte Arylgruppe mit 6 bis 40 ringbildenden Kohlenstoffatomen oder eine substituierte oder unsubstituierte Heteroarylgruppe mit 1 bis 40 ringbildenden Kohlenstoffatomen sind,
in Formel2 bis Formel 5 R_{d1} bis R_{d5} jeweils unabhängig Folgendes sind: ein Deuteriumatom, ein Halogenatom, eine substituierte oder unsubstituierte Alkylgruppe mit 1 bis 20 Kohlenstoffatomen, eine substituierte oder unsubstituierte Alkenylgruppe mit 2 bis 20 Kohlenstoffatomen, eine substituierte oder unsubstituierte Oxygruppe, eine substituierte oder unsubstituierte Thiogruppe, eine substituierte oder unsubstituierte Amingruppe, eine substituierte oder unsubstituierte Arylgruppe mit 6 bis 30 ringbildenden Kohlenstoffatomen, eine substituierte oder unsubstituierte Heteroarylgruppe mit 1 bis 30 ringbildenden Kohlenstoffatomen oder kombiniert mit einer benachbarten Gruppe, um einen Kohlenwasserstoffring oder einen Heterocyclus zu bilden,
m1 und m2 jeweils unabhängig eine ganze Zahl von 0 bis 7 sind,
m3 und m4 jeweils unabhängig eine ganze Zahl von 0 bis 4 sind und
m5 eine ganze Zahl von 0 bis 8 ist, und
in Formel 4 R₁ bis R₅ jeweils unabhängig Folgendes sind: ein Wasserstoffatom, ein Deuteriumatom, ein Halogenatom, eine substituierte oder unsubstituierte Alkylgruppe mit 1 bis 20 Kohlenstoffatomen, eine substituierte oder unsubstituierte Alkenylgruppe mit 2 bis 20 Kohlenstoffatomen, eine substituierte oder unsubstituierte Oxygruppe, eine substituierte oder unsubstituierte Thiogruppe, eine substituierte oder unsubstituierte Amingruppe, eine substituierte oder unsubstituierte Arylgruppe mit 6 bis 30 ringbildenden Kohlenstoffatomen, eine substituierte oder unsubstituierte Heteroarylgruppe mit 1 bis 30 ringbildenden Kohlenstoffatomen oder kombiniert mit einer benachbarten Gruppe, um einen Kohlenwasserstoffring oder einen Heterocyclus zu bilden.

2. Aminverbindung nach Anspruch 1, wobei die Aminverbindung durch eine beliebige der Formeln 1-1 bis Formel 1-4 dargestellt ist: 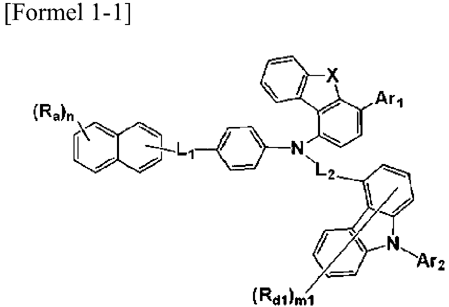 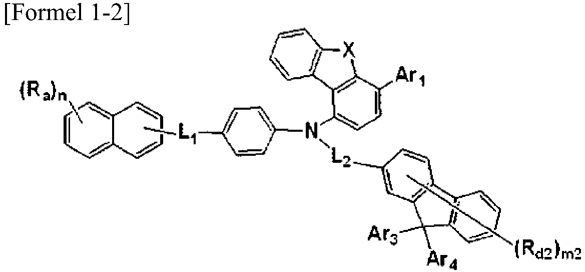 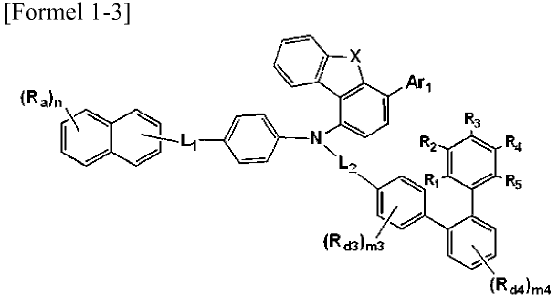 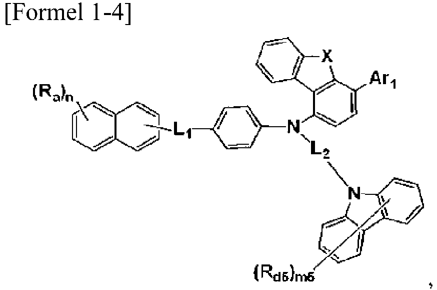 wobei in Formel 1-1 bis Formel 1-4 Folgendes gilt:
X, L₁, L₂, "n", Rₐ und Ar₁ sind die gleichen wie in Anspruch 1 definiert,
in Formel 1-1 und Formel 1-2 Ar₂ bis Ar₄ die gleichen wie in Anspruch 1 definiert sind, in Formel 1-3 R₁ bis R₅ die gleichen wie in Anspruch 1 definiert sind und
in Formel 1-1 bis Formel 1-4 R_{d1} bis R_{d5} und m1 bis m5 die gleichen wie in Anspruch 1 definiert sind.

3. Aminverbindung nach Anspruch 1 oder 2, wobei L₁ eine direkte Verknüpfung, eine unsubstituierte Phenylengruppe oder eine unsubstituierte zweiwertige Biphenylgruppe ist.

4. Aminverbindung nach einem der Ansprüche 1 bis 3, wobei L₂ eine direkte Verknüpfung, eine unsubstituierte Phenylengruppe, eine unsubstituierte Naphthalingruppe, eine unsubstituierte zweiwertige Dibenzofurangruppe oder eine unsubstituierte zweiwertige Dibenzothiophengruppe ist.

5. Aminverbindung nach einem der Ansprüche 1 bis 4, wobei Ar₁ durch ein beliebiges von Arl-1 bis Arl-5 dargestellt ist:

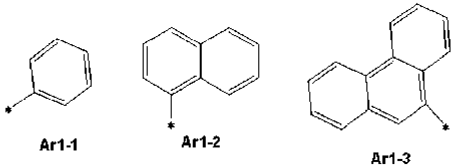

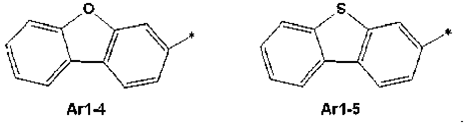

6. Aminverbindung nach einem der Ansprüche 1 bis 5, wobei in Formel 4 R₁ bis R₅ mit dem Benzolring kombiniert sind, an dem R₁ bis R₅ substituiert sind, um einen Dibenzofuranring, einen Dibenzothiophenring oder einen Carbazolring zu bilden.

7. Aminverbindung nach einem der Ansprüche 1 bis 6, wobei die Aminverbindung eine Struktur umfasst, in der mindestens ein Wasserstoffatom mit einem Deuteriumatom substituiert ist.

8. Aminverbindung nach einem der Ansprüche 1 bis 7, wobei die Aminverbindung eine Monoaminverbindung ist.

9. Aminverbindung nach Anspruch 1, wobei die Aminverbindung eine der Verbindungen in Verbindungsgruppe 1 ist: 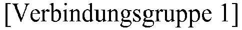 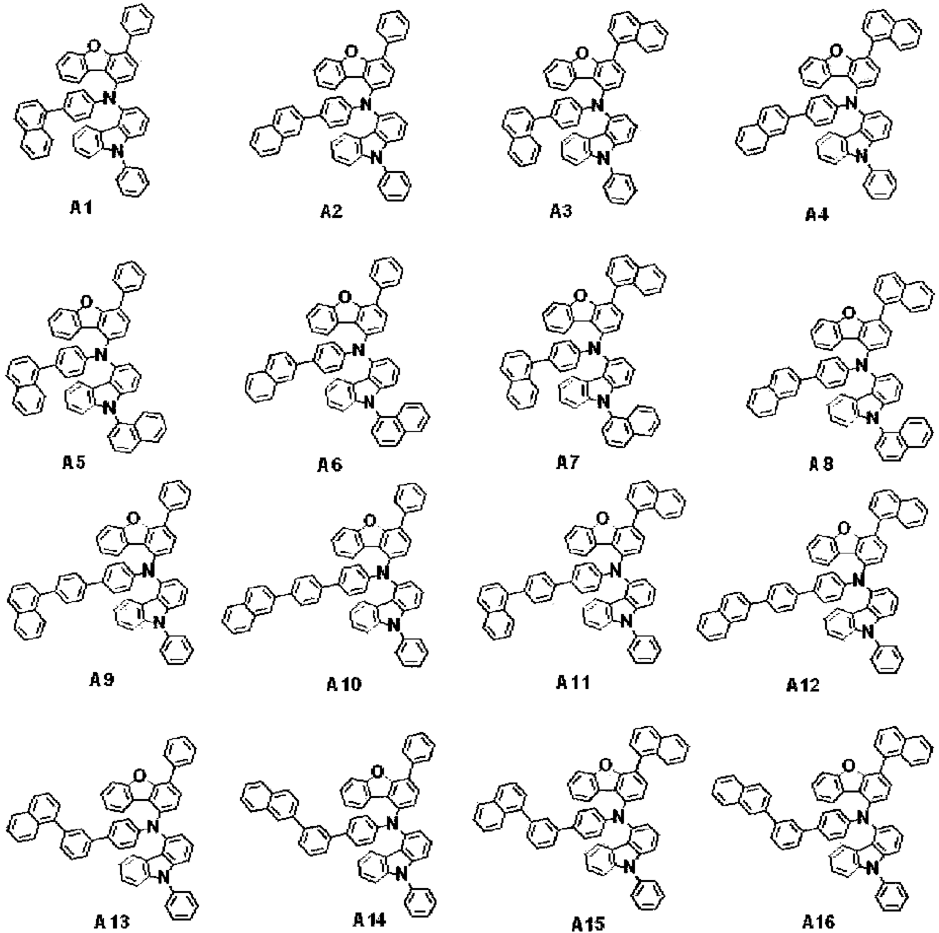 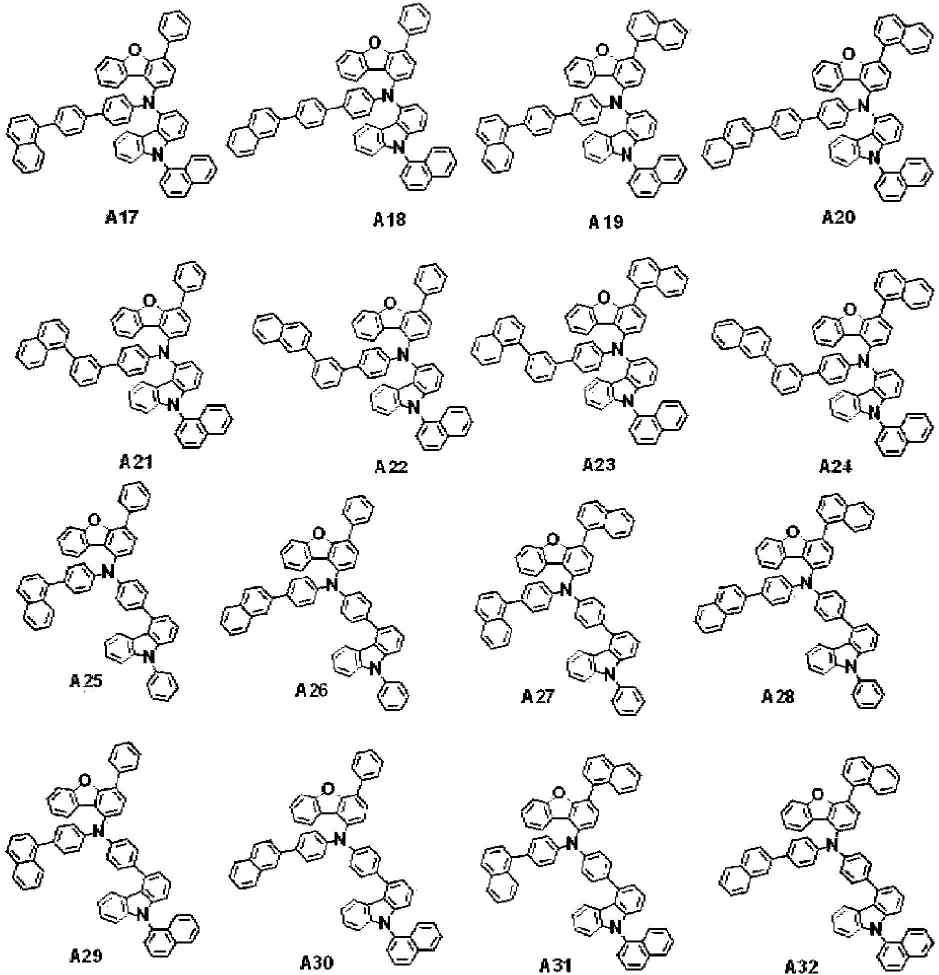 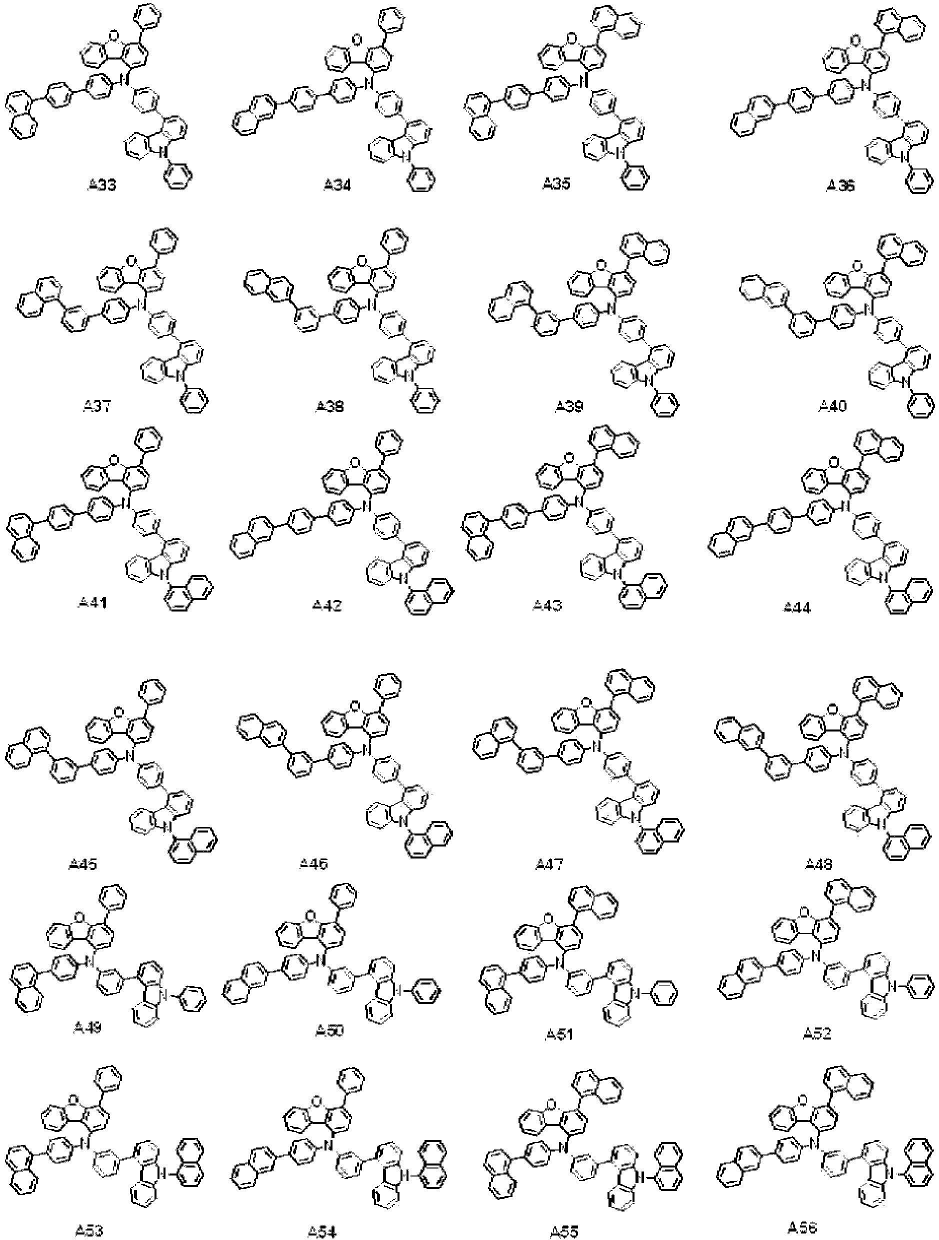 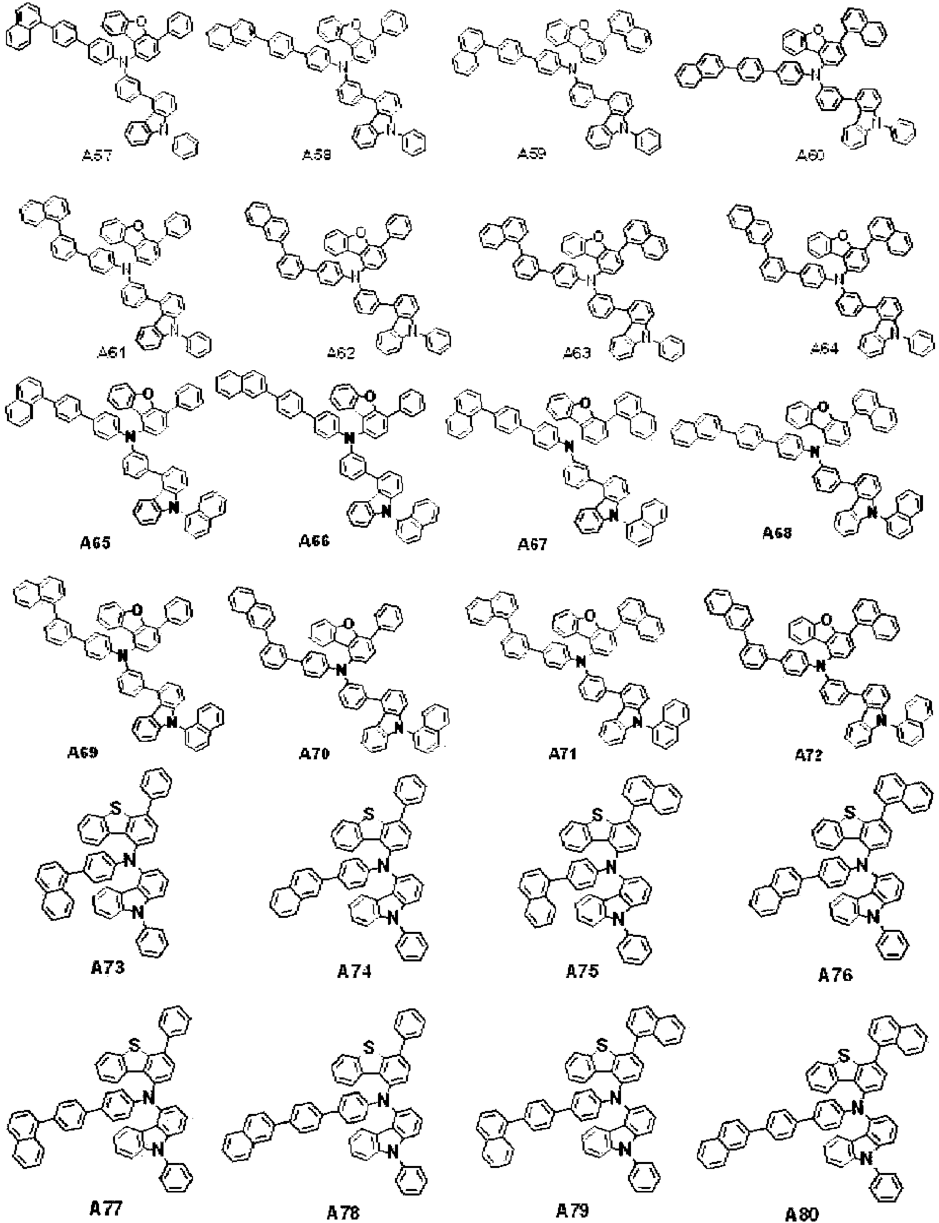 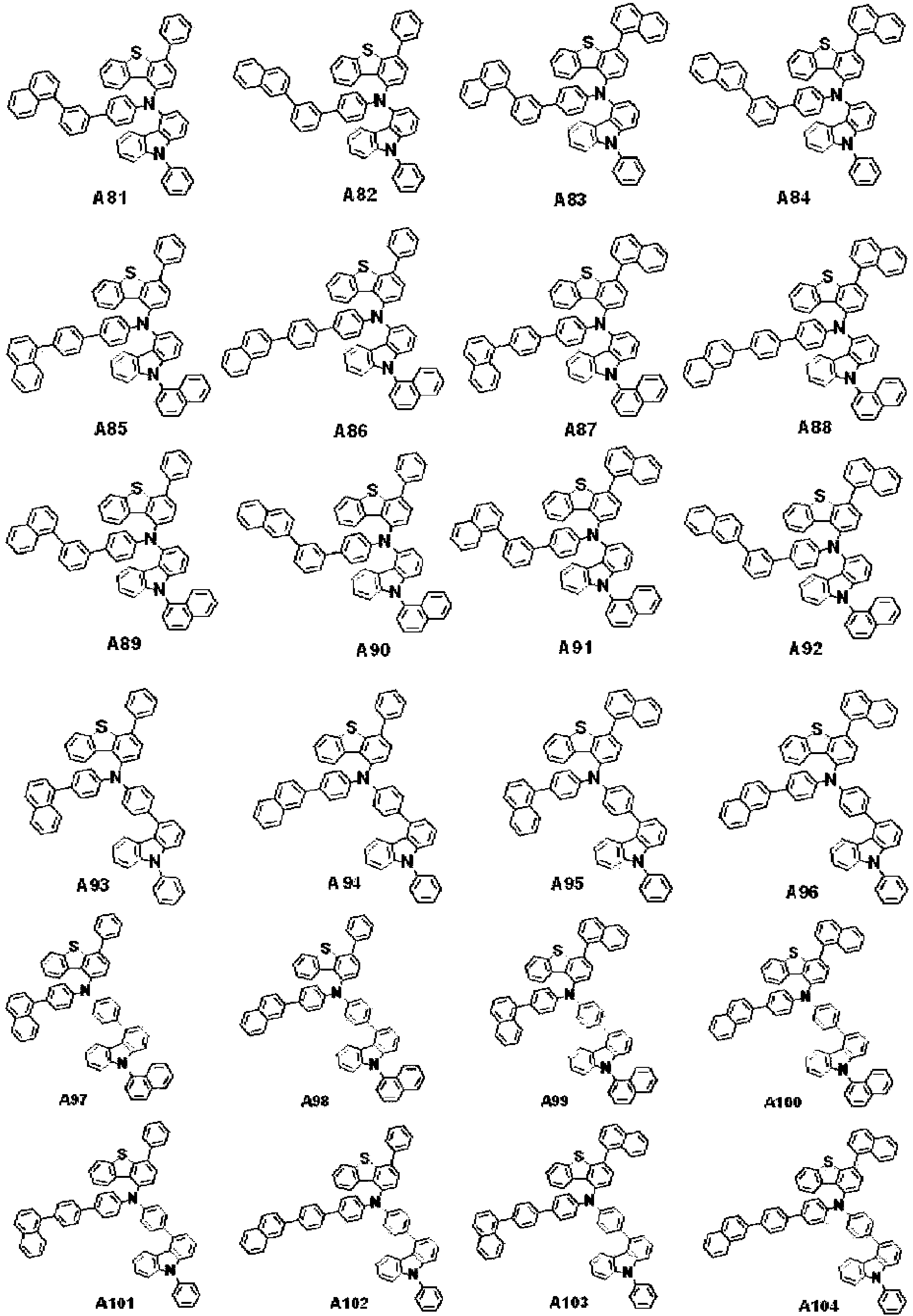 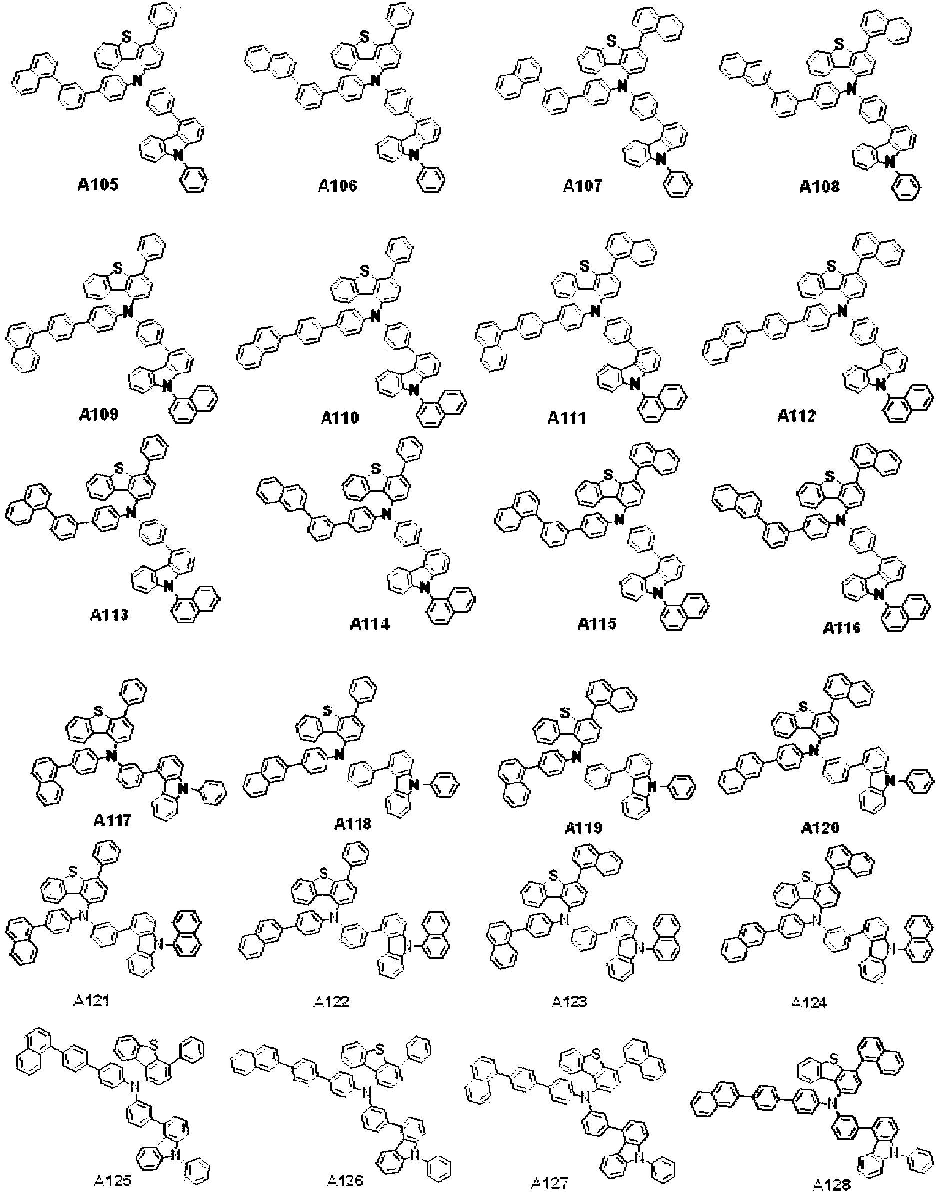 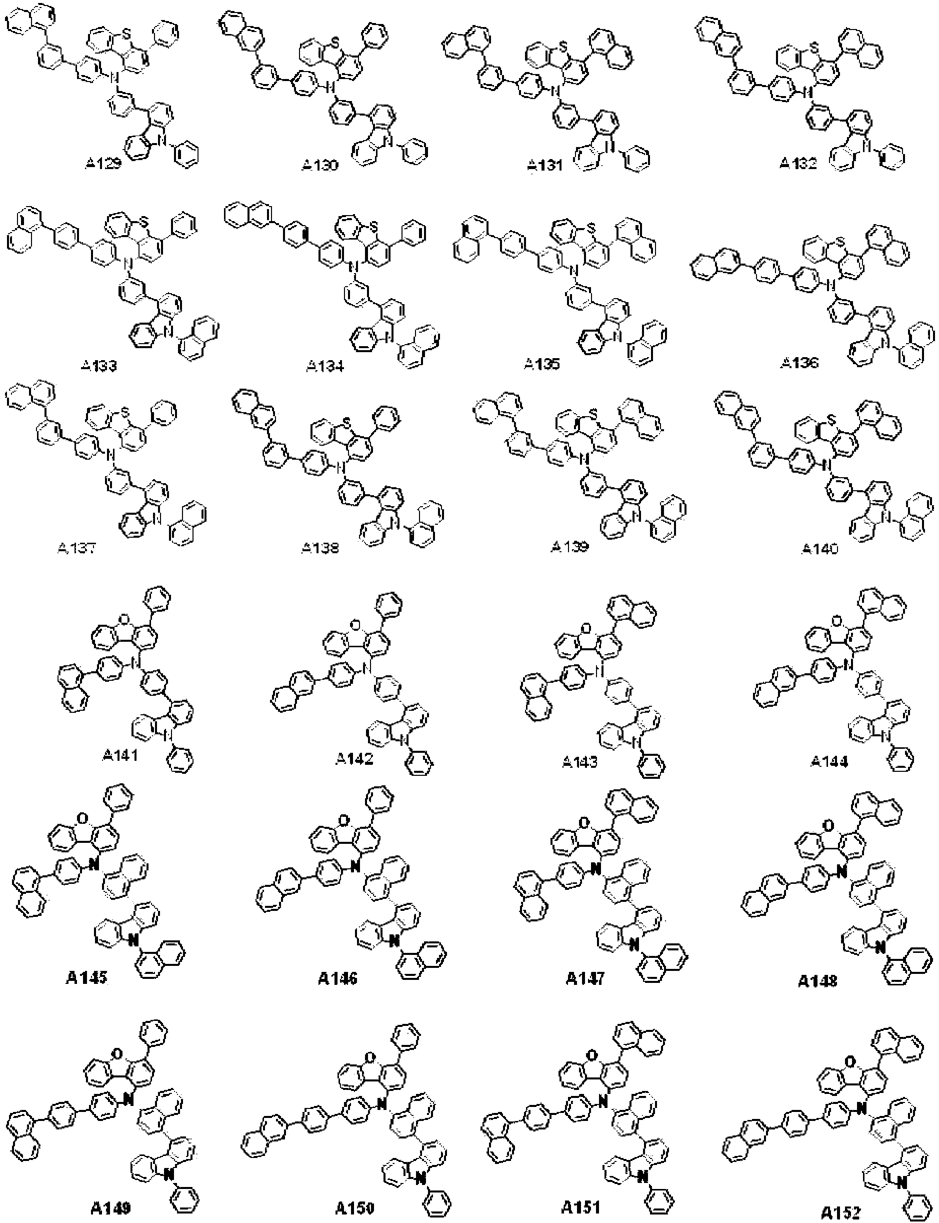 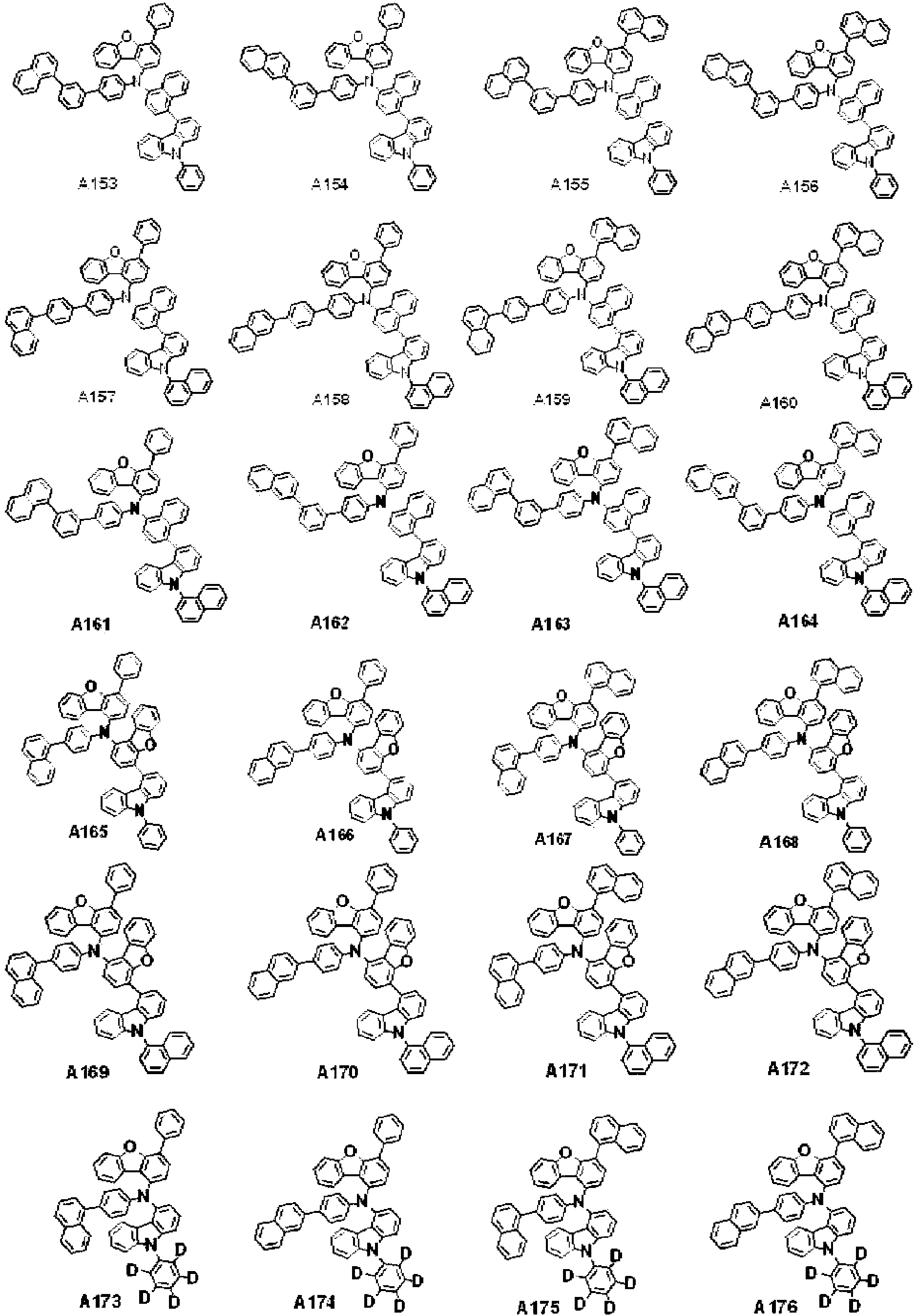 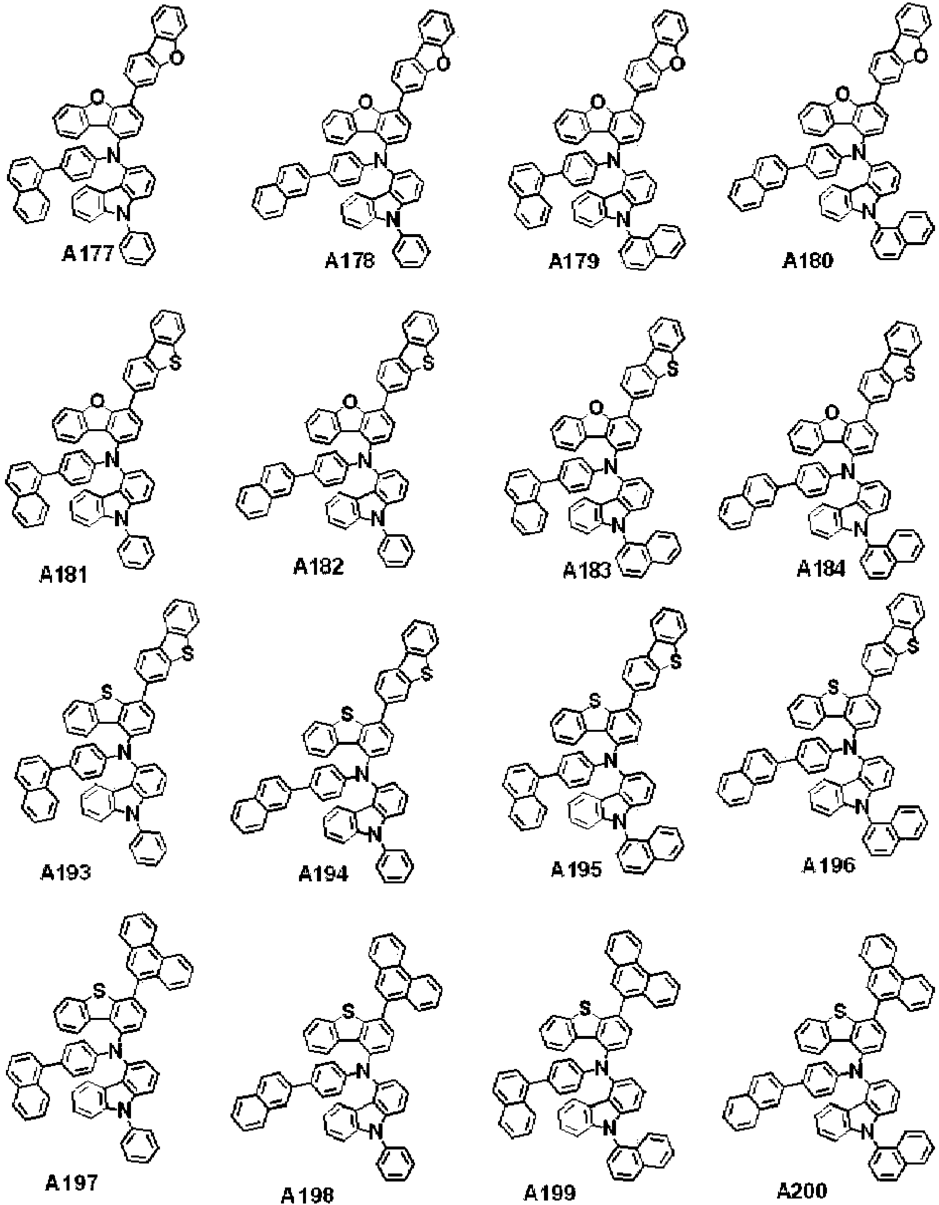 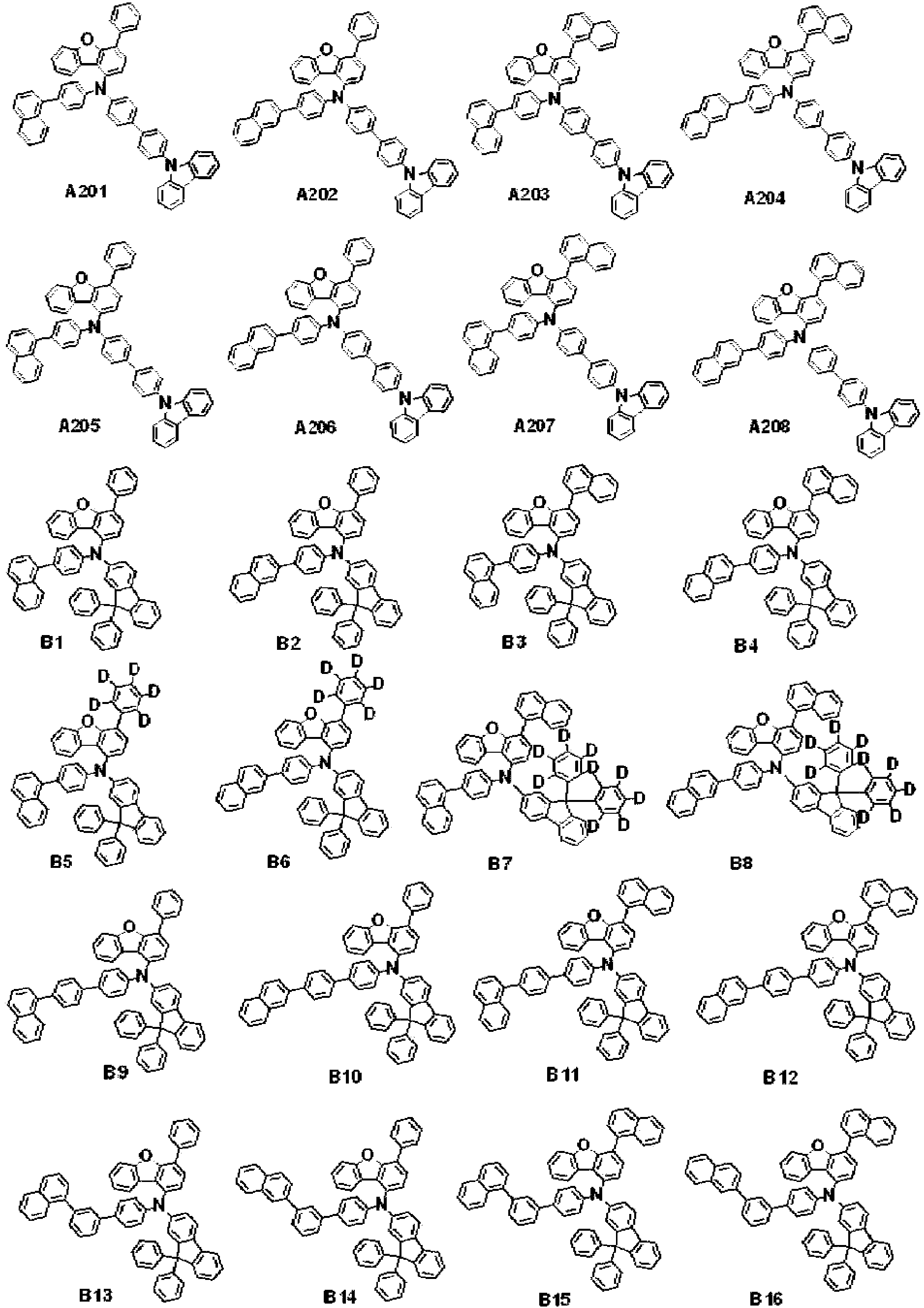 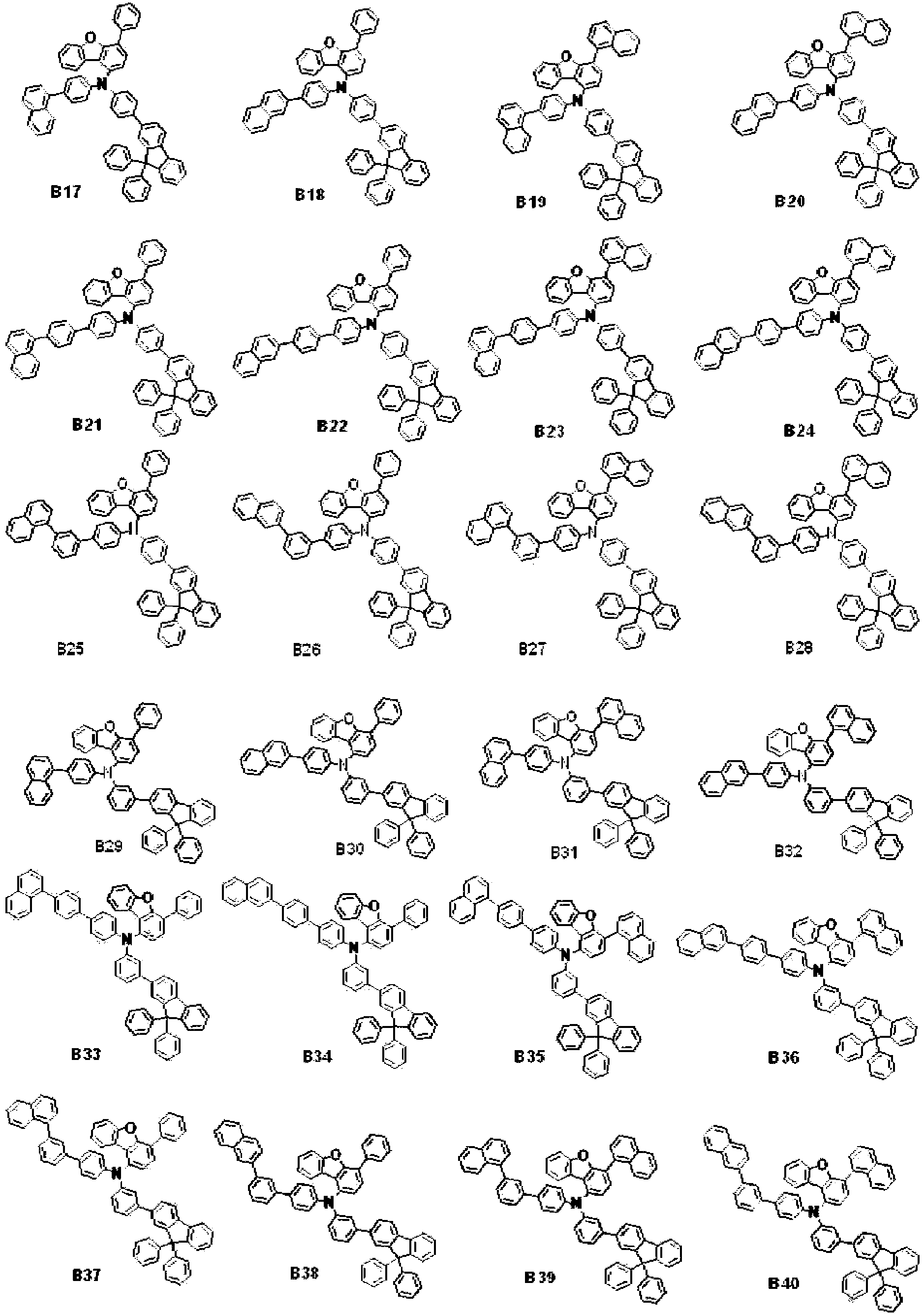 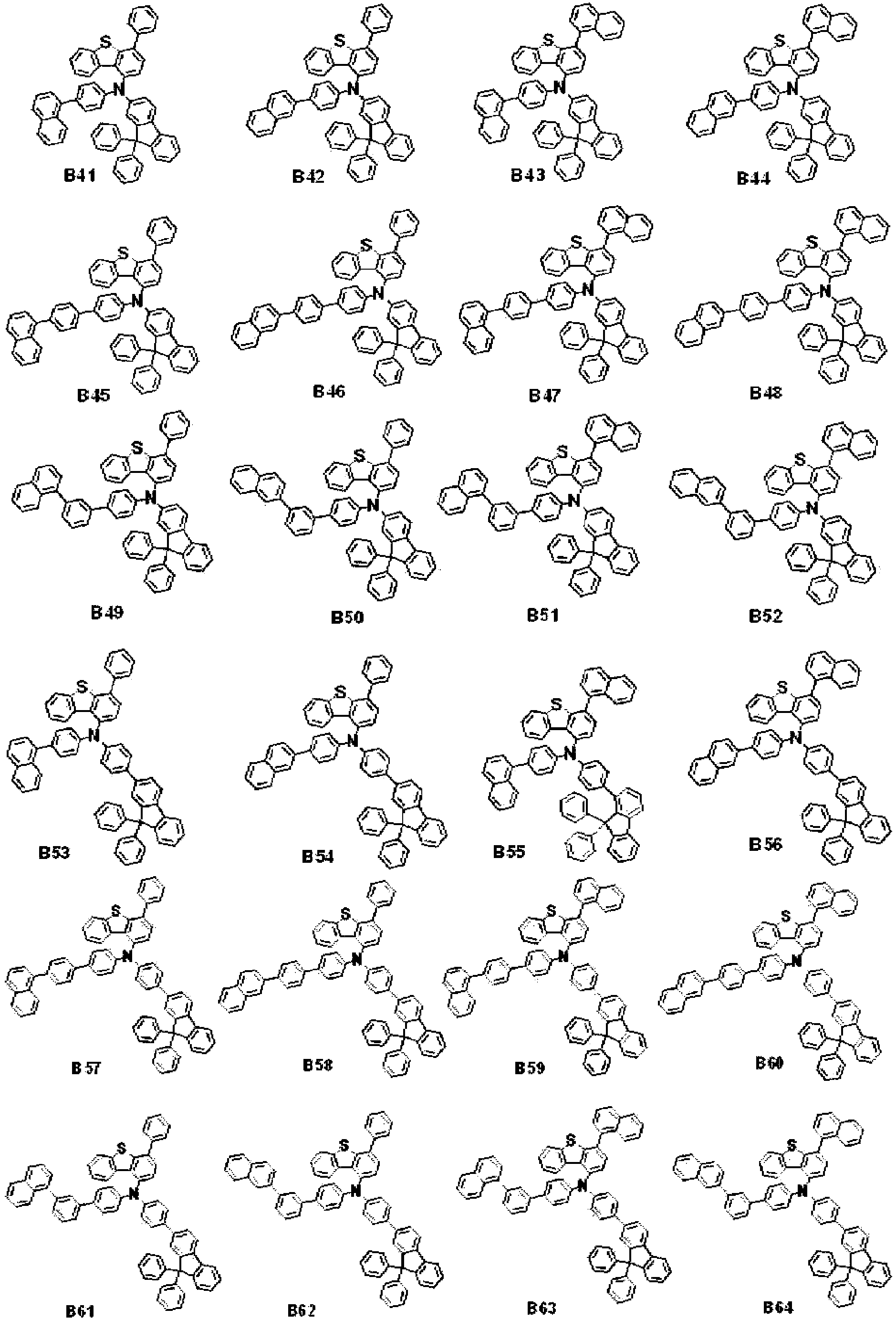 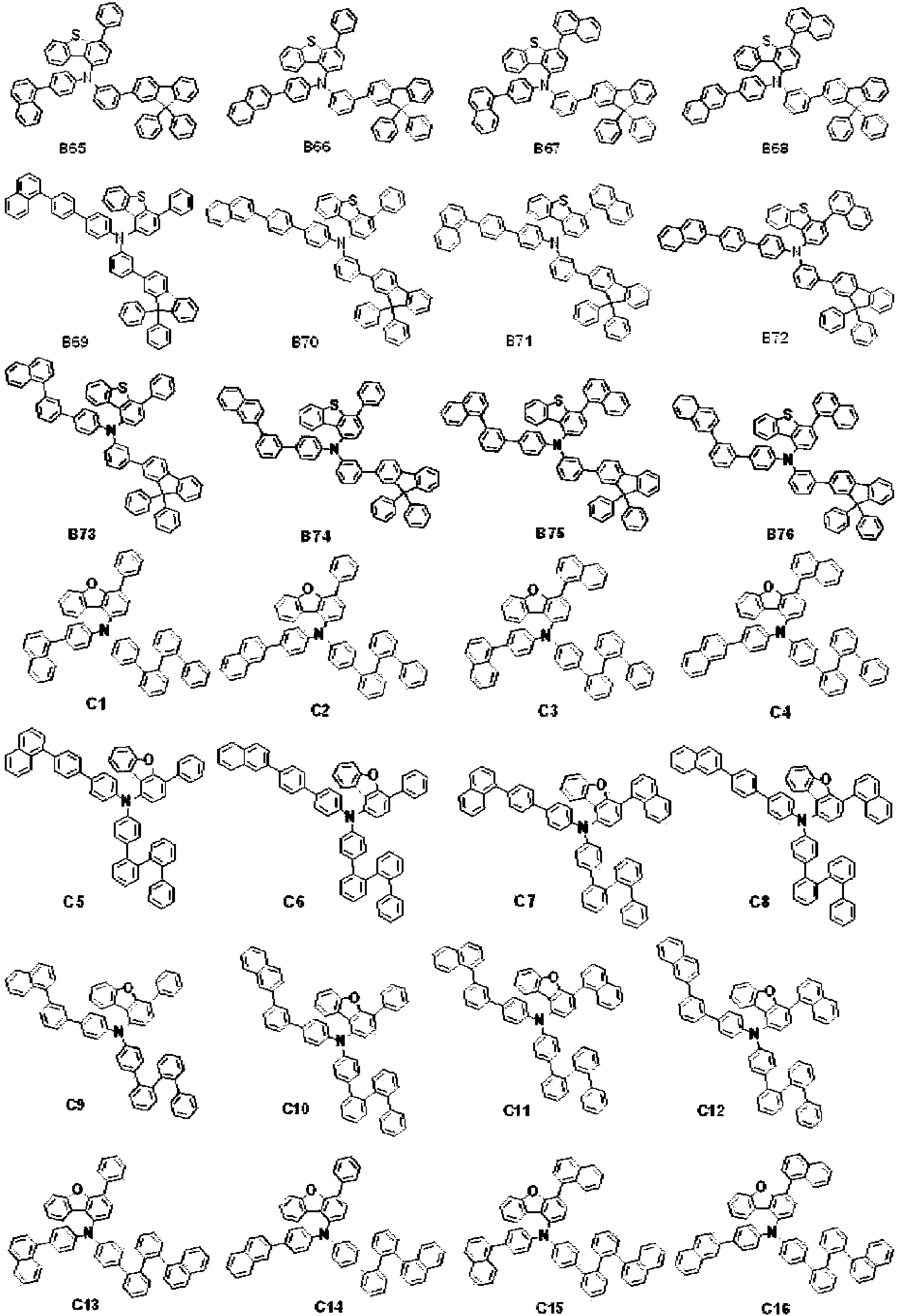 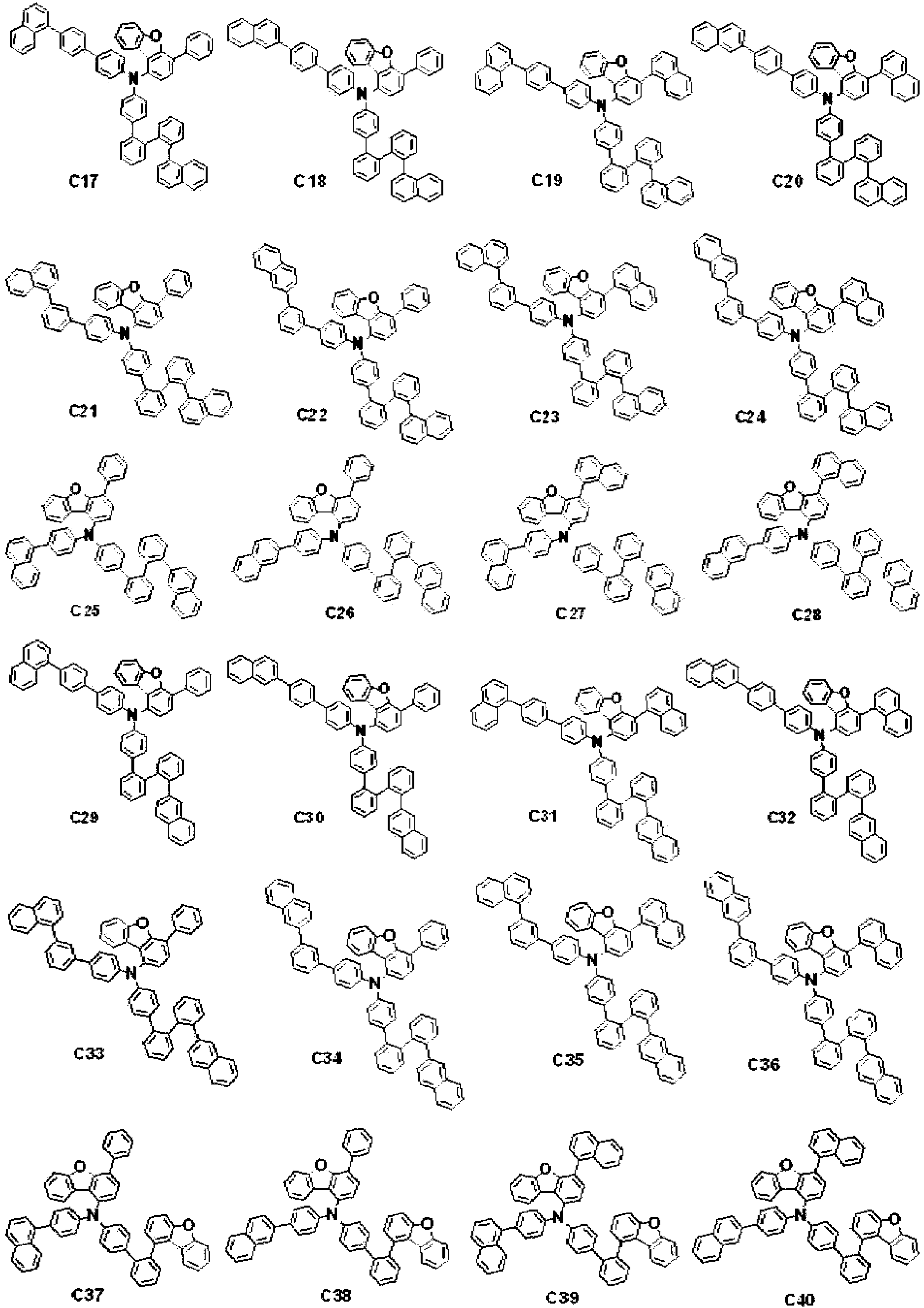 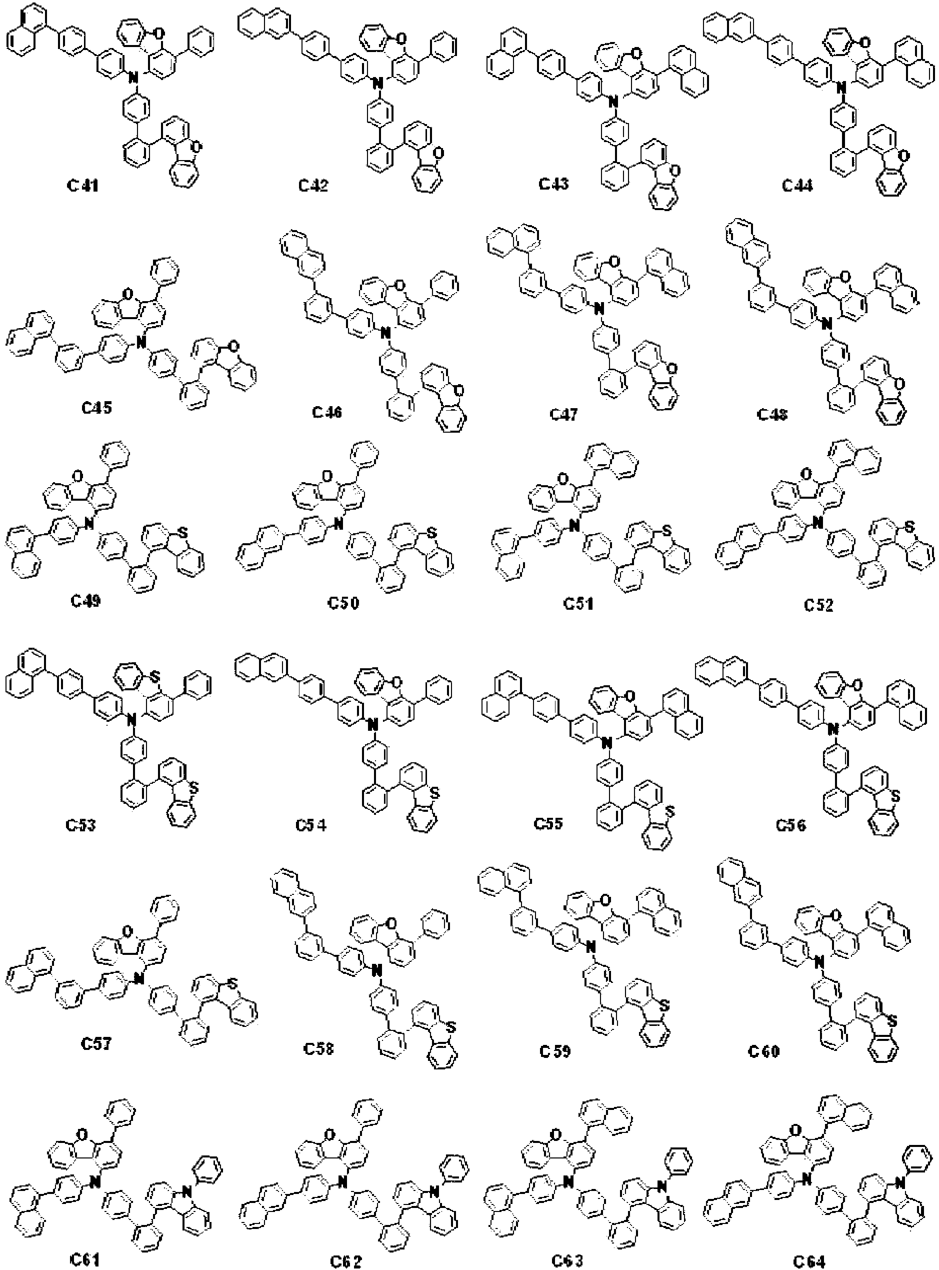 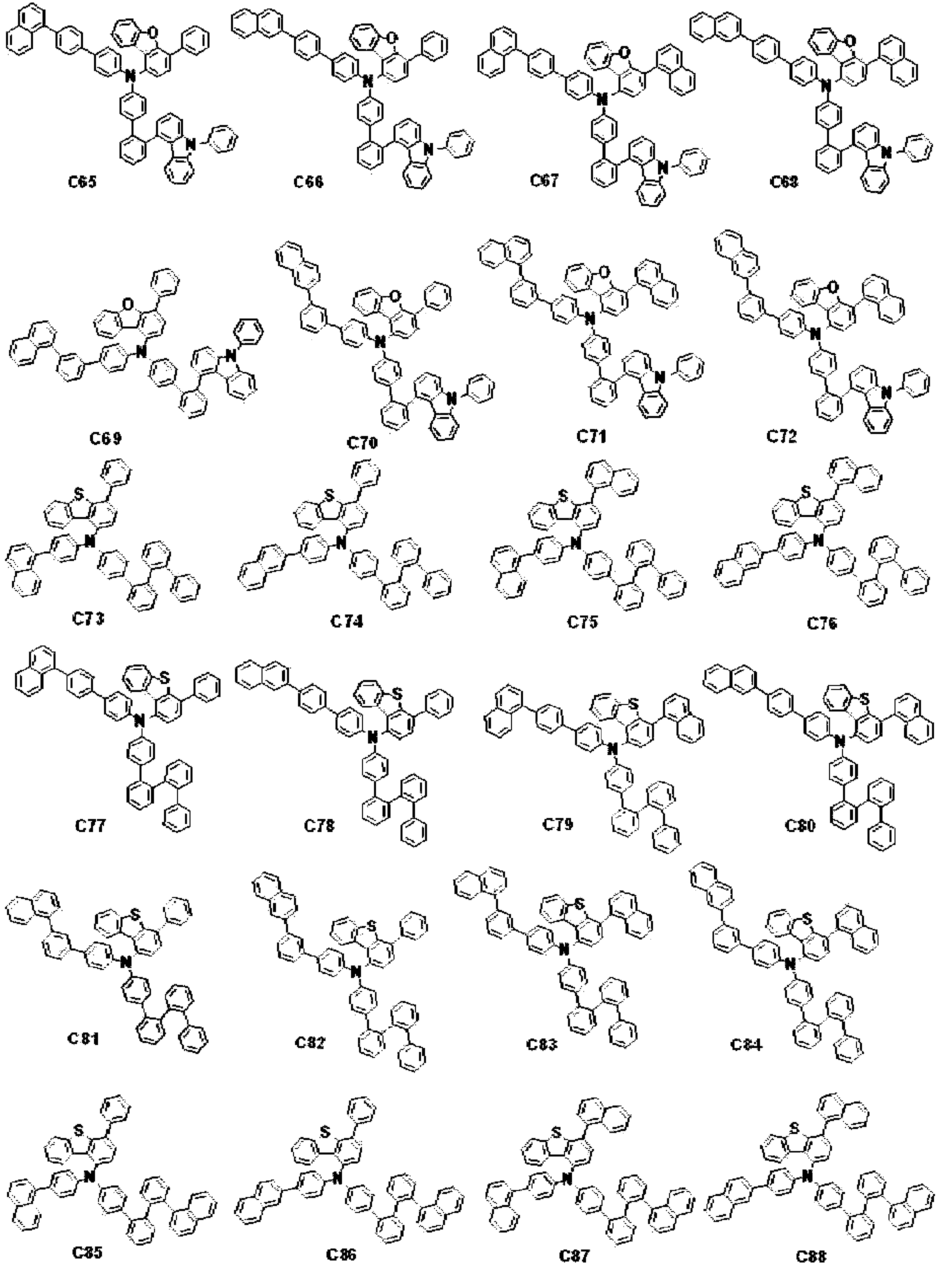 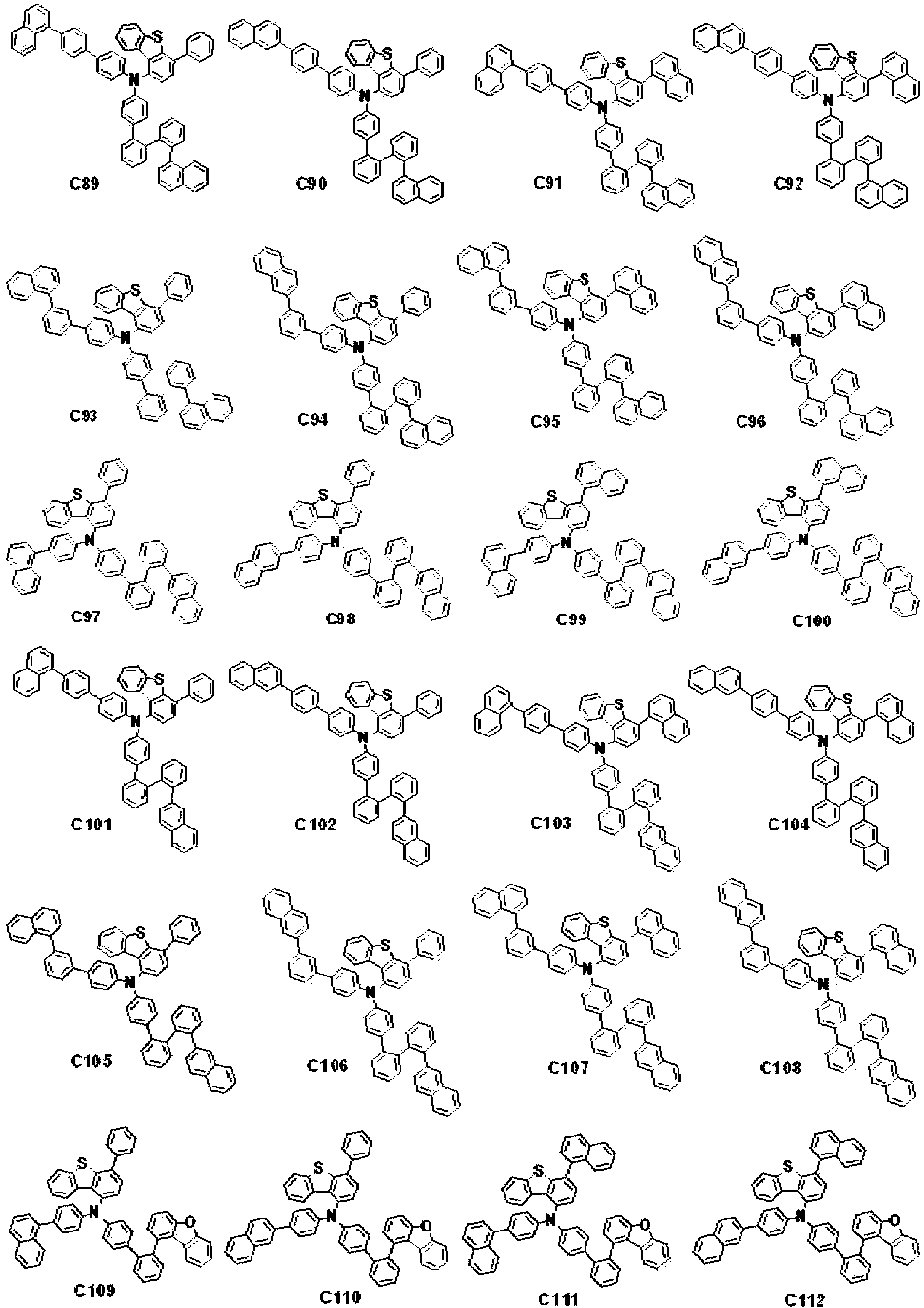 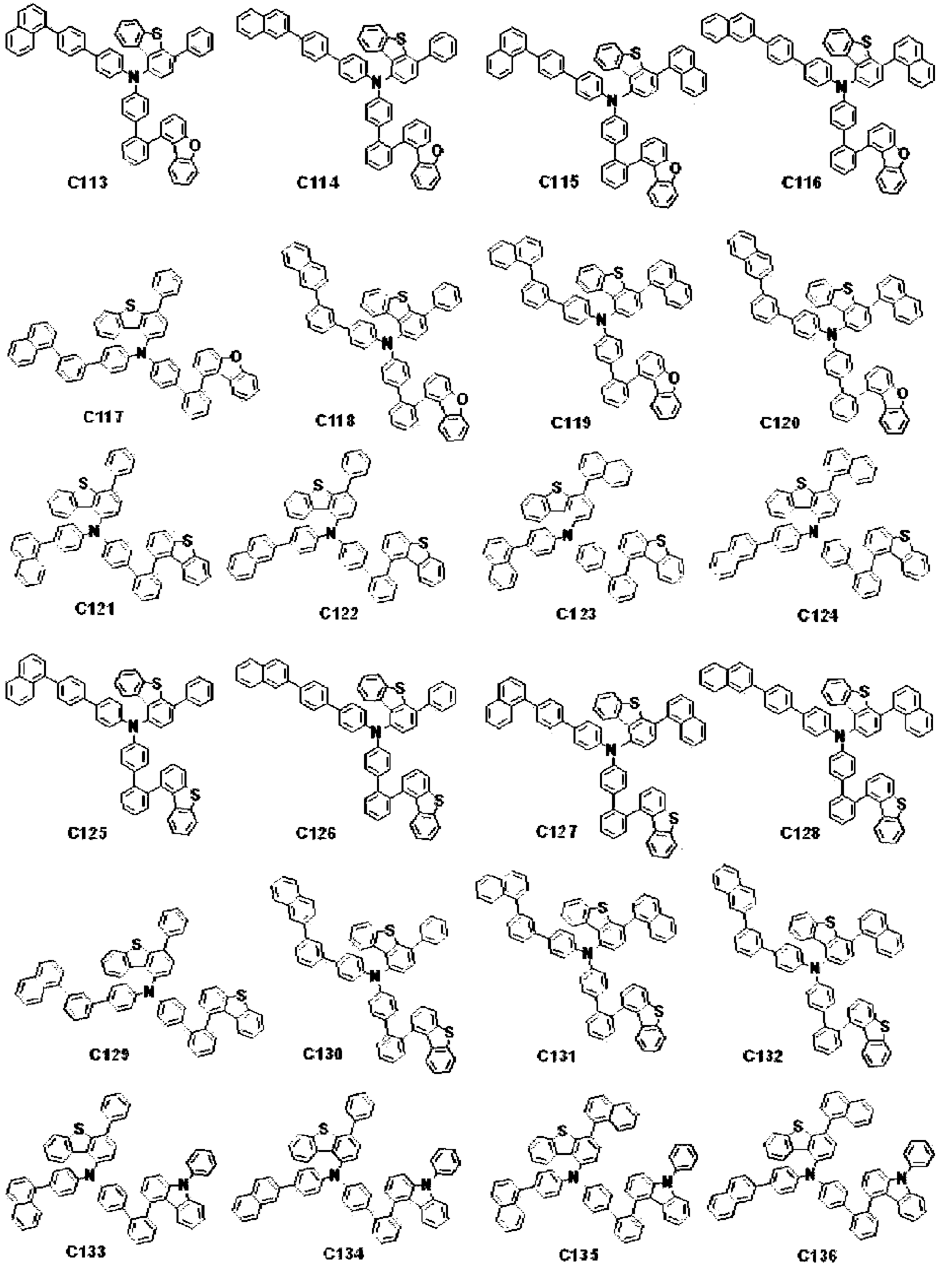 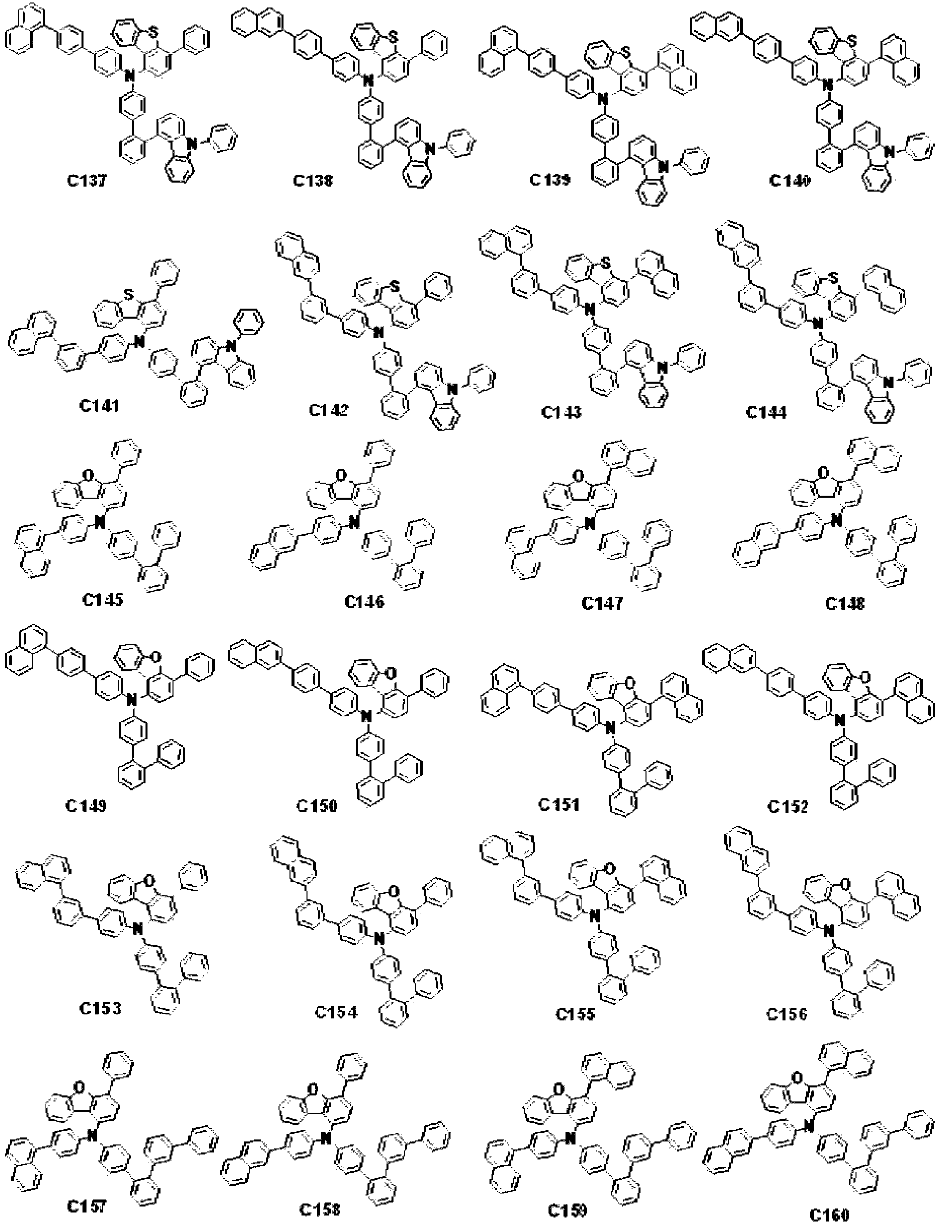 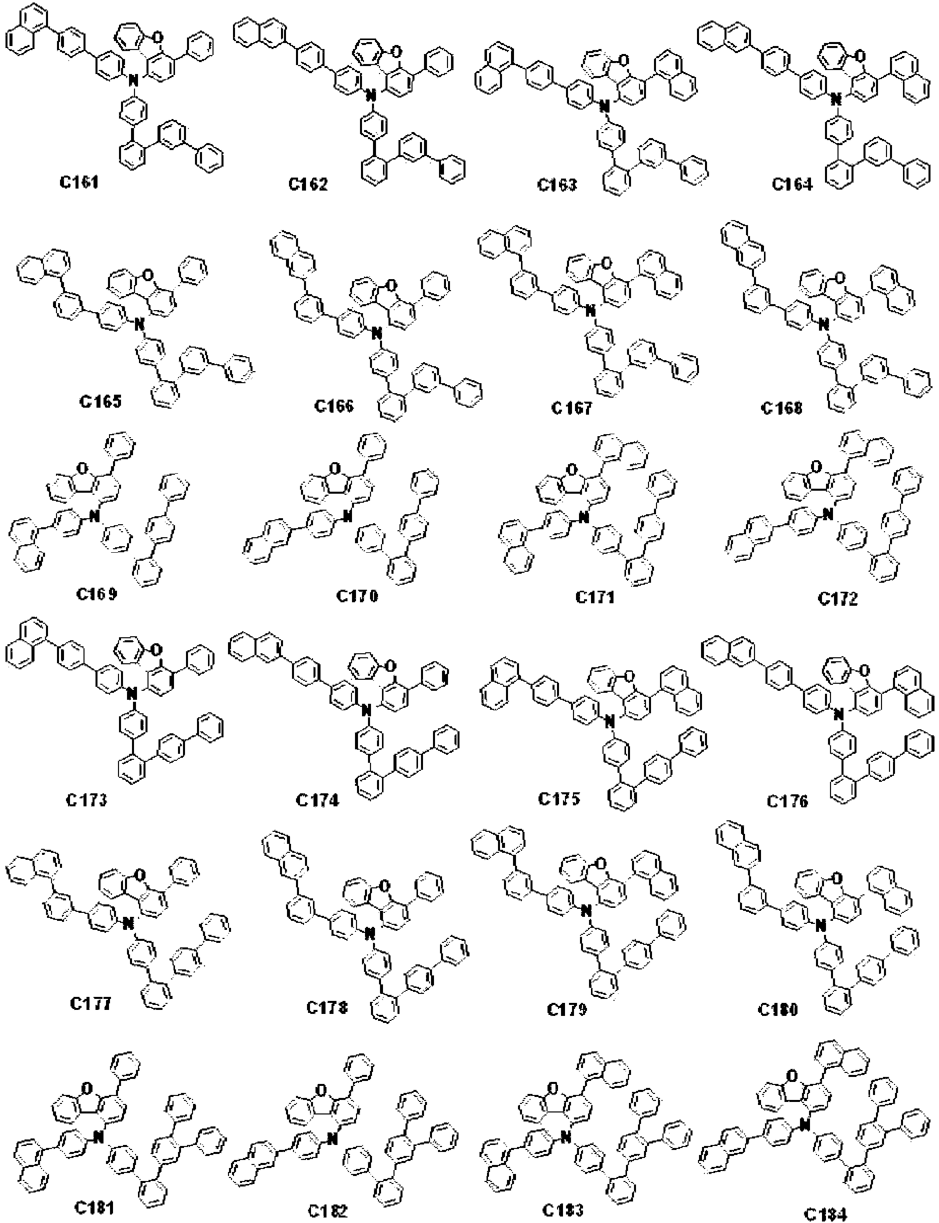 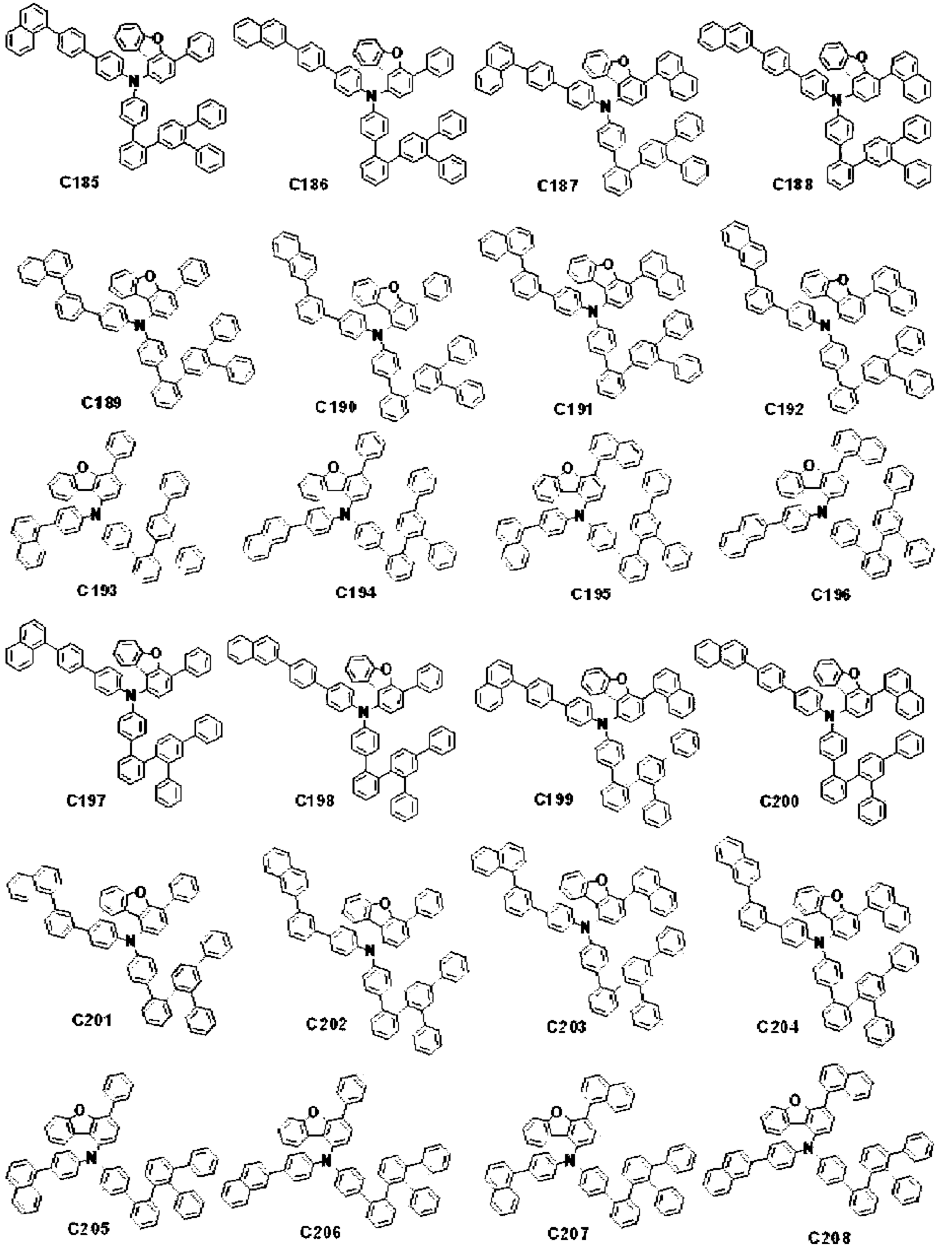 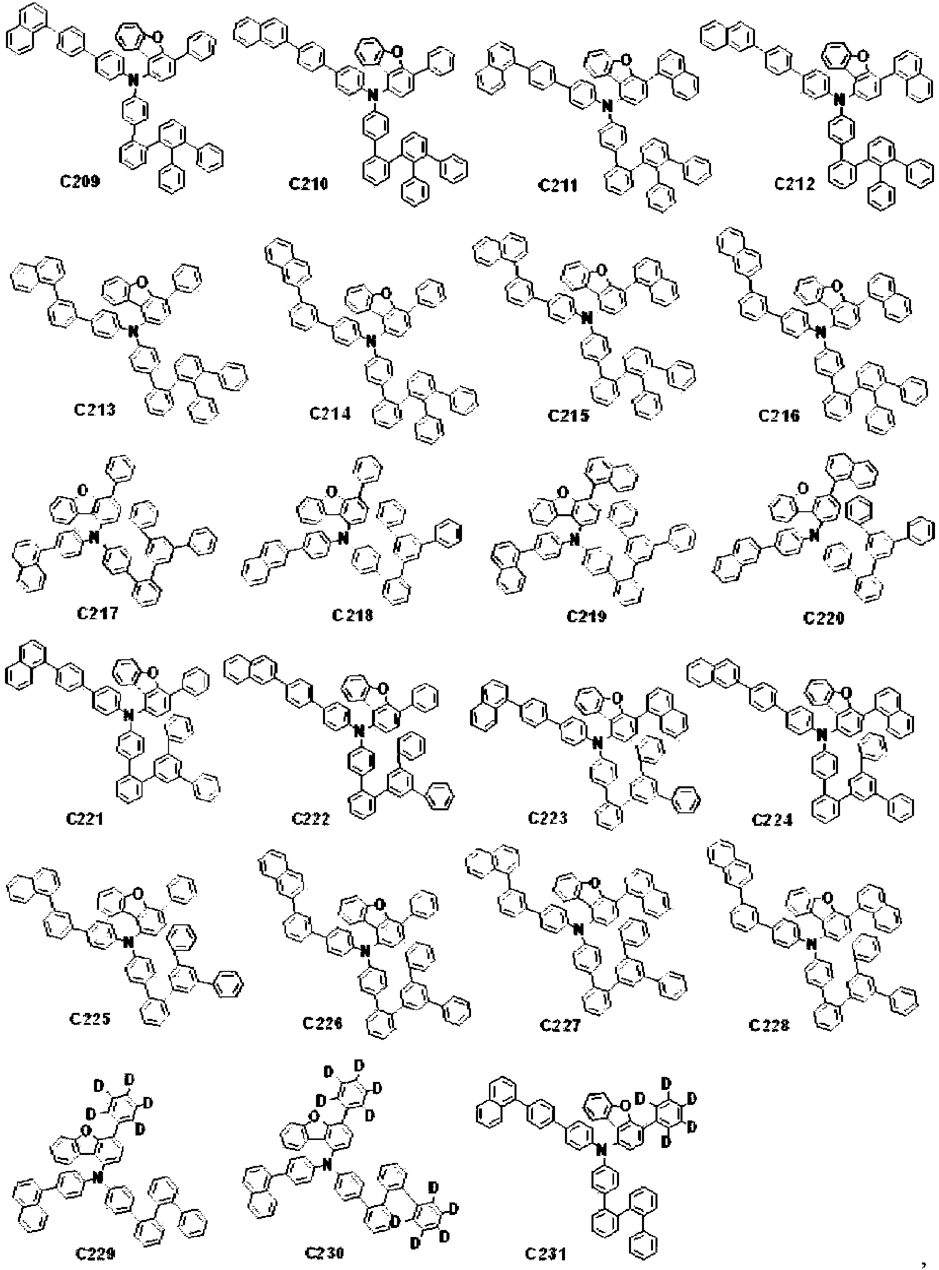 wobei in Verbindungsgruppe 1 D ein Deuteriumatom ist.

10. Lichtemittierendes Element, umfassend:
eine erste Elektrode;
eine zweite Elektrode, die der ersten Elektrode zugewandt ist; und
mindestens eine Funktionsschicht zwischen der ersten Elektrode und der zweiten Elektrode und umfassend eine Aminverbindung nach einem der Ansprüche 1 bis 9.

11. Lichtemittierendes Element nach Anspruch 10, wobei die mindestens eine Funktionsschicht eine Emissionsschicht, eine Lochtransportregion zwischen der ersten Elektrode und der Emissionsschicht und eine Elektronentransportregion zwischen der Emissionsschicht und der zweiten Elektrode umfasst und
die Lochtransportregion die Aminverbindung umfasst.

12. Lichtemittierendes Element nach Anspruch 11, wobei die Lochtransportregion mindestens eine von einer Locheinspritzschicht, eine Lochtransportschicht und/oder eine Elektronenblockierschicht umfasst und
die mindestens eine von der Locheinspritzschicht, der Lochtransportschicht und der Elektronenblockierschicht die Aminverbindung umfasst.

13. Lichtemittierendes Element nach Anspruch 11, wobei die Emissionsschicht eine Verbindung umfasst, die durch Formel E-1 dargestellt ist: 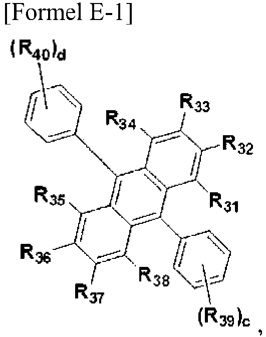 wobei in Formel E-1 Folgendes gilt:
"c" und "d" sind jeweils unabhängig eine ganze Zahl von 0 bis 5, und
R₃₁ bis R₄₀ sind jeweils unabhängig ein Wasserstoffatom, ein Deuteriumatom, ein Halogenatom, eine substituierte oder unsubstituierte Silylgruppe, eine substituierte oder unsubstituierte Thiogruppe, eine substituierte oder unsubstituierte Oxygruppe, eine substituierte oder unsubstituierte Alkylgruppe mit 1 bis 10 Kohlenstoffatomen, eine substituierte oder unsubstituierte Alkenylgruppe mit 2 bis 10 Kohlenstoffatomen, eine substituierte oder unsubstituierte Arylgruppe mit 6 bis 30 ringbildenden Kohlenstoffatomen, eine substituierte oder unsubstituierte Heteroarylgruppe mit 2 bis 30 ringbildenden Kohlenstoffatomen oder kombiniert mit einer benachbarten Gruppe, um einen Ring zu bilden.

## Revendications

1. Composé amine représenté par la Formule 1 : 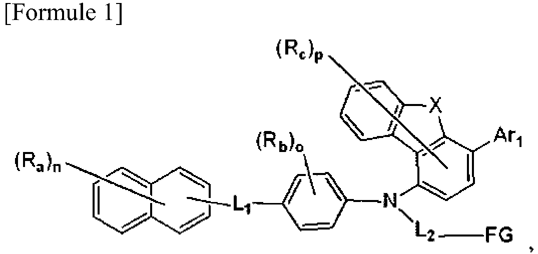
dans lequel, dans la Formule 1,
X est O ou S,
L₁ et L₂ sont chacun indépendamment une liaison directe, un groupe arylène substitué ou non substitué comportant 6 à 15 atomes de carbone formant un cycle, ou un groupe hétéroarylène substitué ou non substitué comportant 1 à 12 atomes de carbone formant un cycle,
« n » est un nombre entier de 0 à 7, « o » est un nombre entier de 0 à 4, « p » est un nombre entier de 0 à 6,
Rₐ, R_{b} et R_{c} sont chacun indépendamment un atome de deutérium, un atome d'halogène, un groupe alkyle substitué ou non substitué comportant 1 à 10 atomes de carbone, un groupe aryle substitué ou non substitué comportant 6 à 30 atomes de carbone formant un cycle ou un groupe hétéroaryle substitué ou non substitué comportant 1 à 30 atomes de carbone formant un cycle,
Ar₁ est un groupe phényle substitué ou non substitué, un groupe naphthyle substitué ou non substitué, un groupe phénanthrényle substitué ou non substitué, un groupe dibenzothiophène substitué ou non substitué, ou un groupe dibenzofurane substitué ou non substitué, et
FG est représenté par l'une quelconque de la Formule 2 ou de la Formule 5 : 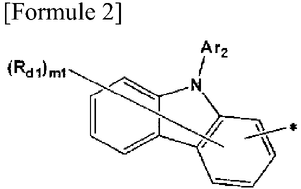 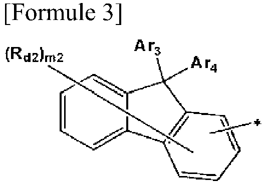 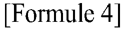 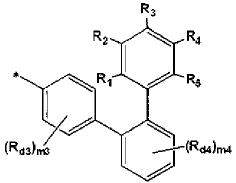 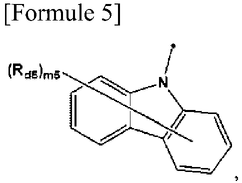
dans la Formule 2 et la Formule 3, Ar₂ à Ar₄ sont chacun indépendamment un groupe aryle substitué ou non substitué comportant 6 à 40 atomes de carbone formant un cycle, ou un groupe hétéroaryle substitué ou non substitué comportant 1 à 40 atomes de carbone formant un cycle,
dans la Formule 2 à la Formule 5, R_{d1} à R_{d5} sont chacun indépendamment un atome de deutérium, un atome d'halogène, un groupe alkyle substitué ou non substitué comportant 1 à 20 atomes de carbone, un groupe alcényle substitué ou non substitué comportant 2 à 20 atomes de carbone, un groupe oxy substitué ou non substitué, un groupe thio substitué ou non substitué, un groupe amine substitué ou non substitué, un groupe aryle substitué ou non substitué comportant 6 à 30 atomes de carbone formant un cycle, un groupe hétéroaryle substitué ou non substitué comportant 1 à 30 atomes de carbone formant un cycle, ou combinés avec un groupe adjacent pour former un cycle hydrocarboné ou un hétérocycle,
m1 et m2 sont chacun indépendamment un nombre entier de 0 à 7,
m3 et m4 sont chacun indépendamment un nombre entier de 0 à 4, et
m5 est un nombre entier de 0 à 8, et
dans la Formule 4, R₁ à R₅ sont chacun indépendamment un atome d'hydrogène, un atome de deutérium, un atome d'halogène, un groupe alkyle substitué ou non substitué comportant 1 à 20 atomes de carbone, un groupe alcényle substitué ou non substitué comportant 2 à 20 atomes de carbone, un groupe oxy substitué ou non substitué, un groupe thio substitué ou non substitué, un groupe amine substitué ou non substitué, un groupe aryle substitué ou non substitué comportant 6 à 30 atomes de carbone formant un cycle, un groupe hétéroaryle substitué ou non substitué comportant 1 à 30 atomes de carbone formant un cycle, ou combinés avec un groupe adjacent pour former un cycle hydrocarboné ou un hétérocycle.

2. Composé amine de la revendication 1, dans lequel le composé amine est représenté par l'une quelconque de la Formule 1-1 à la Formule 1-4 : 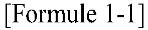 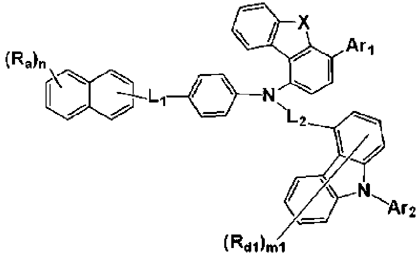 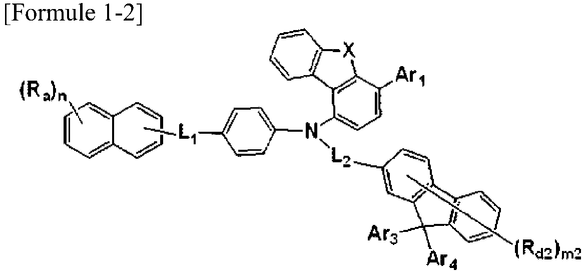 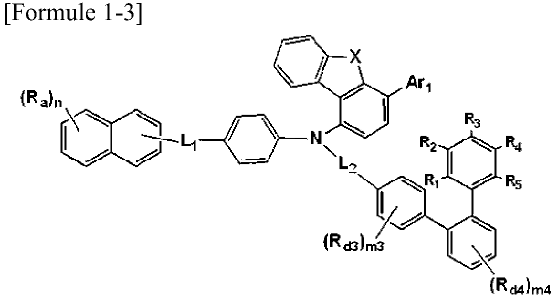 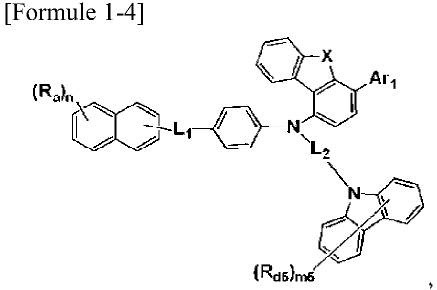
dans lequel, dans la Formule 1-1 à la Formule 1-4,
X, L₁, L₂, « n », Rₐ et Ar₁ sont les mêmes que ceux définis dans la revendication 1,
dans la Formule 1-1 et la Formule 1-2, Ar₂ à Ar₄ sont les mêmes que ceux définis dans la revendication 1,
dans la Formule 1-3, R₁ à R₅ sont les mêmes que ceux définis dans la revendication 1, et
dans la Formule 1-1 à la Formule 1-4, R_{d1} à R_{d5} et m1 à m5 sont les mêmes que ceux définis dans la revendication 1.

3. Composé amine de la revendication 1 ou 2, dans lequel L₁ est une liaison directe, un groupe phénylène non substitué ou un groupe biphényle divalent non substitué.

4. Composé amine de l'une quelconque des revendications 1 à 3, dans lequel L₂ est une liaison directe, un groupe phénylène non substitué, un groupe naphtalène non substitué, un groupe dibenzofurane divalent non substitué, ou un groupe dibenzothiophène divalent non substitué.

5. Composé amine de l'une quelconque des revendications 1 à 4, dans lequel Ar₁ est représenté par l'un quelconque de Ar1-1 à Ar1-5 :

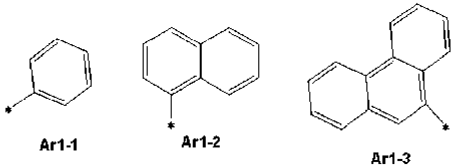

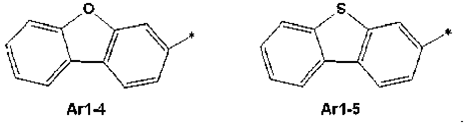

6. Composé amine de l'une quelconque des revendications 1 à 5, dans lequel, dans la Formule 4, R₁ à R₅ sont combinés avec le cycle benzénique auquel R₁ à R₅ sont substitués, pour former un cycle dibenzofuranne, un cycle dibenzothiophène ou un cycle carbazole.

7. Composé amine de l'une quelconque des revendications 1 à 6, dans lequel le composé amine comprend une structure où au moins un atome d'hydrogène est substitué par un atome de deutérium.

8. Composé amine de l'une quelconque des revendications 1 à 7, dans lequel le composé amine est un composé monoamine.

9. Composé amine de la revendication 1, dans lequel le composé amine est l'un quelconque des composés du Groupe de composés 1 : 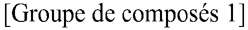 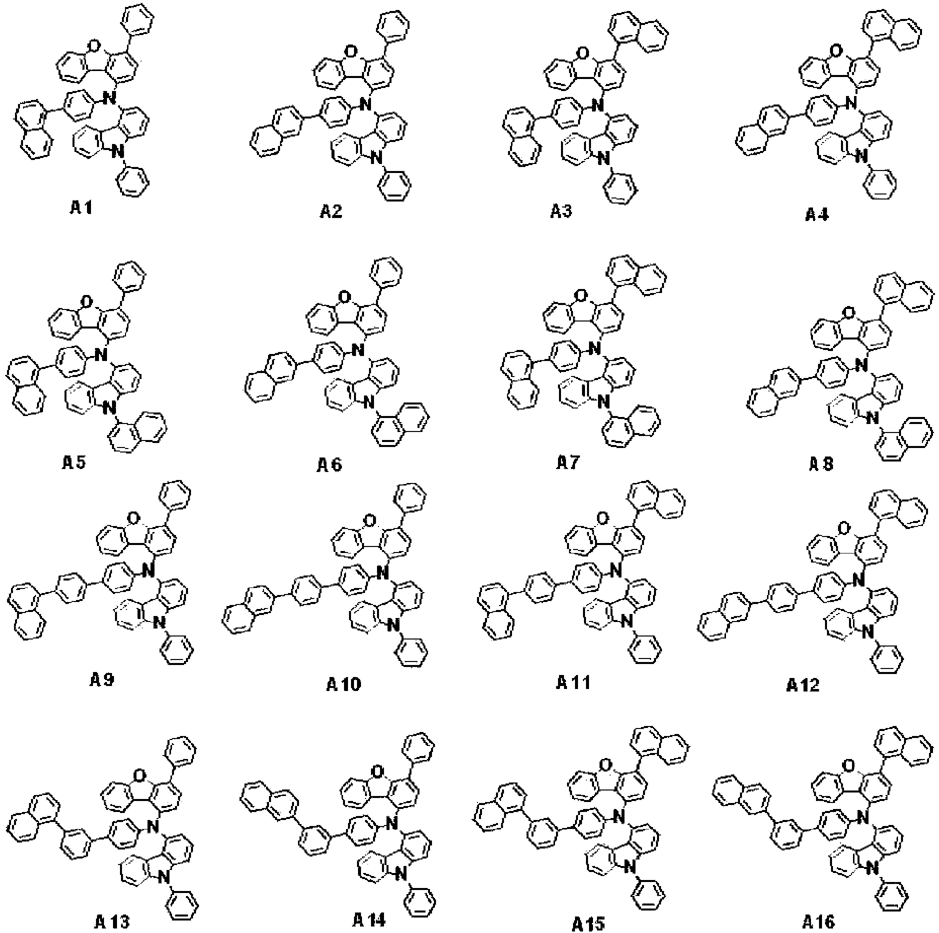 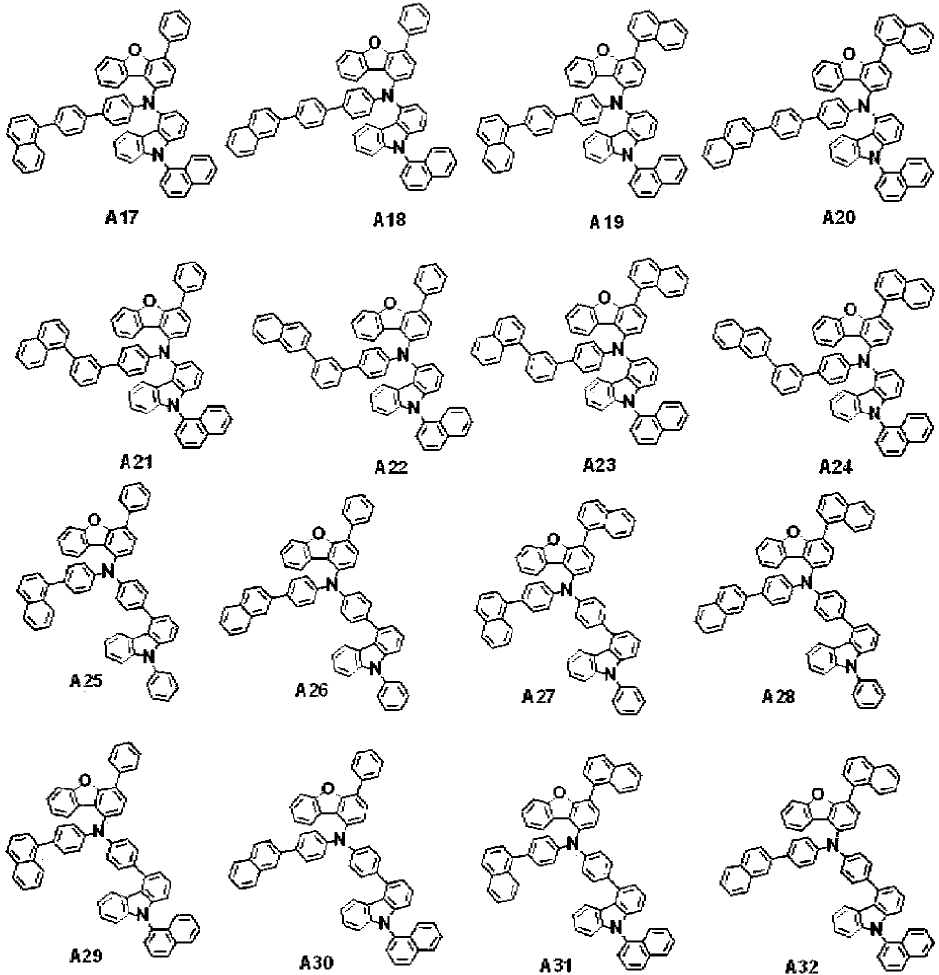 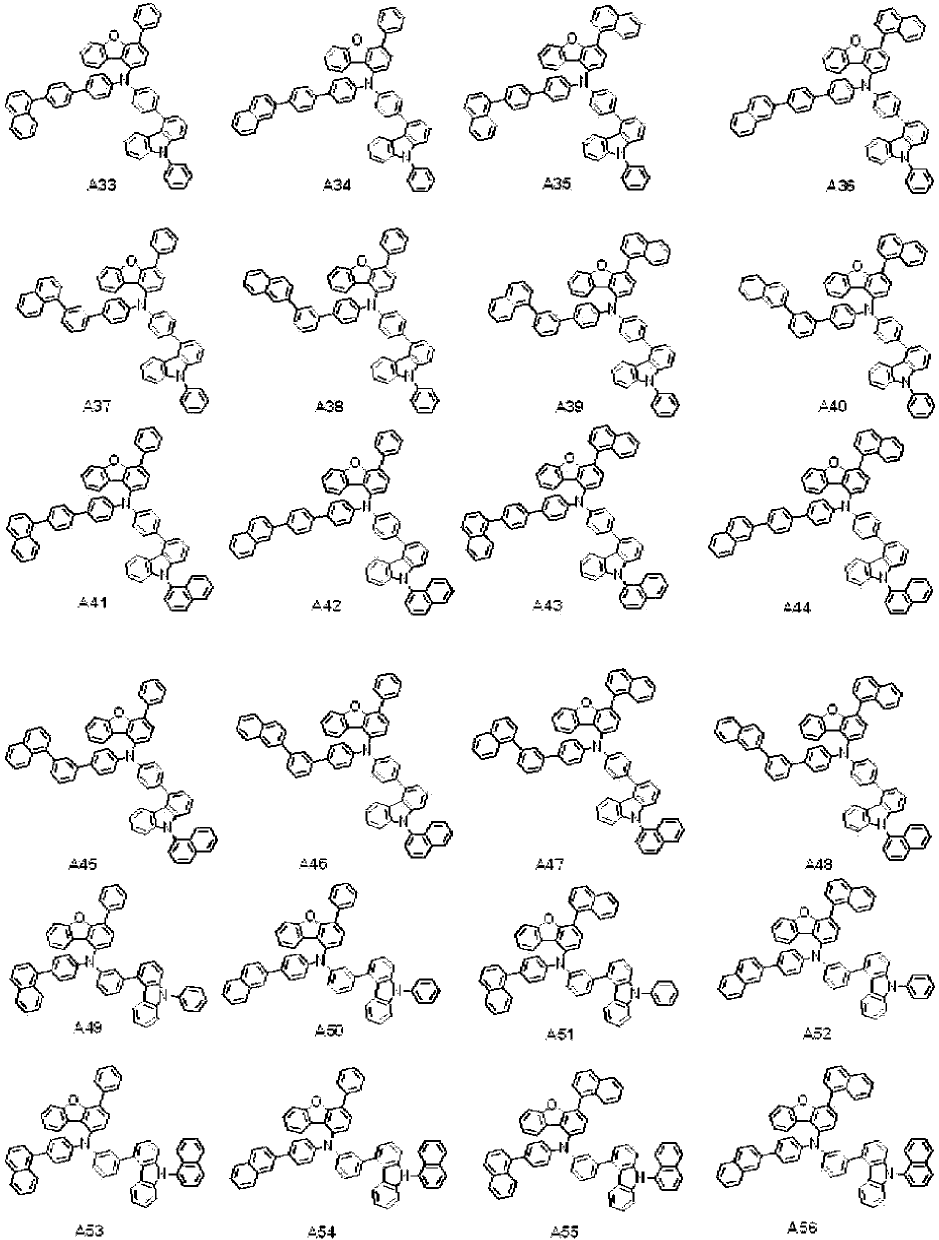 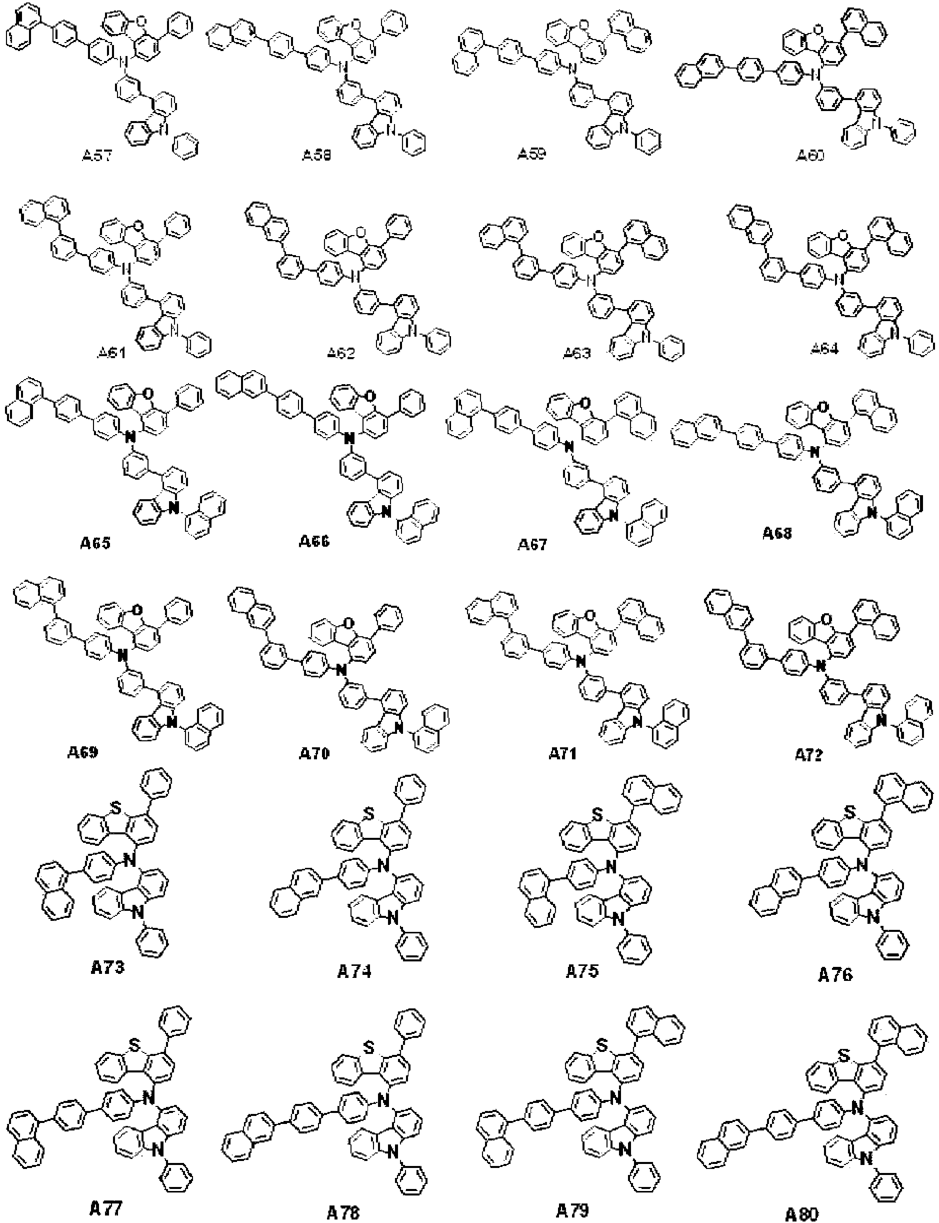 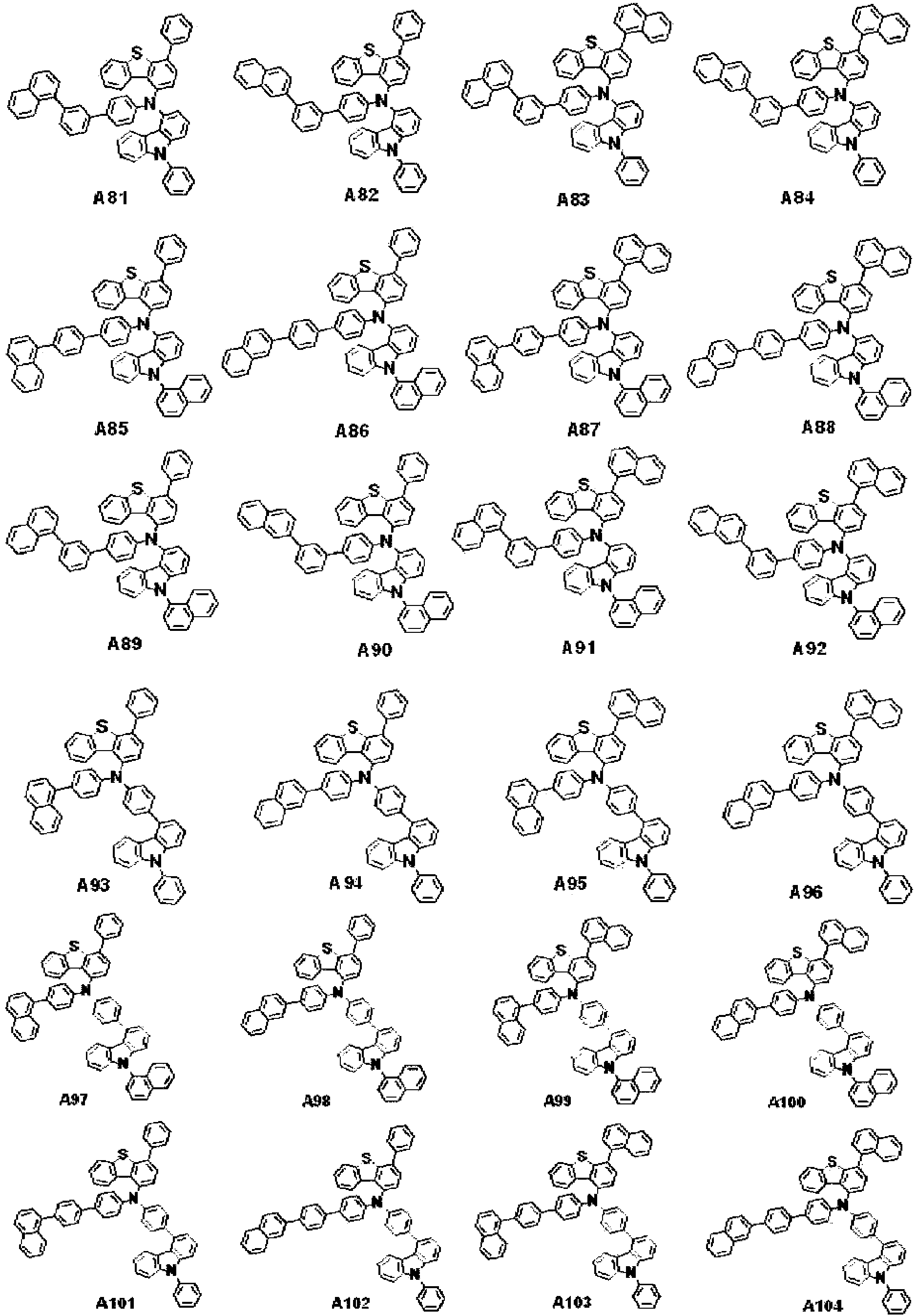 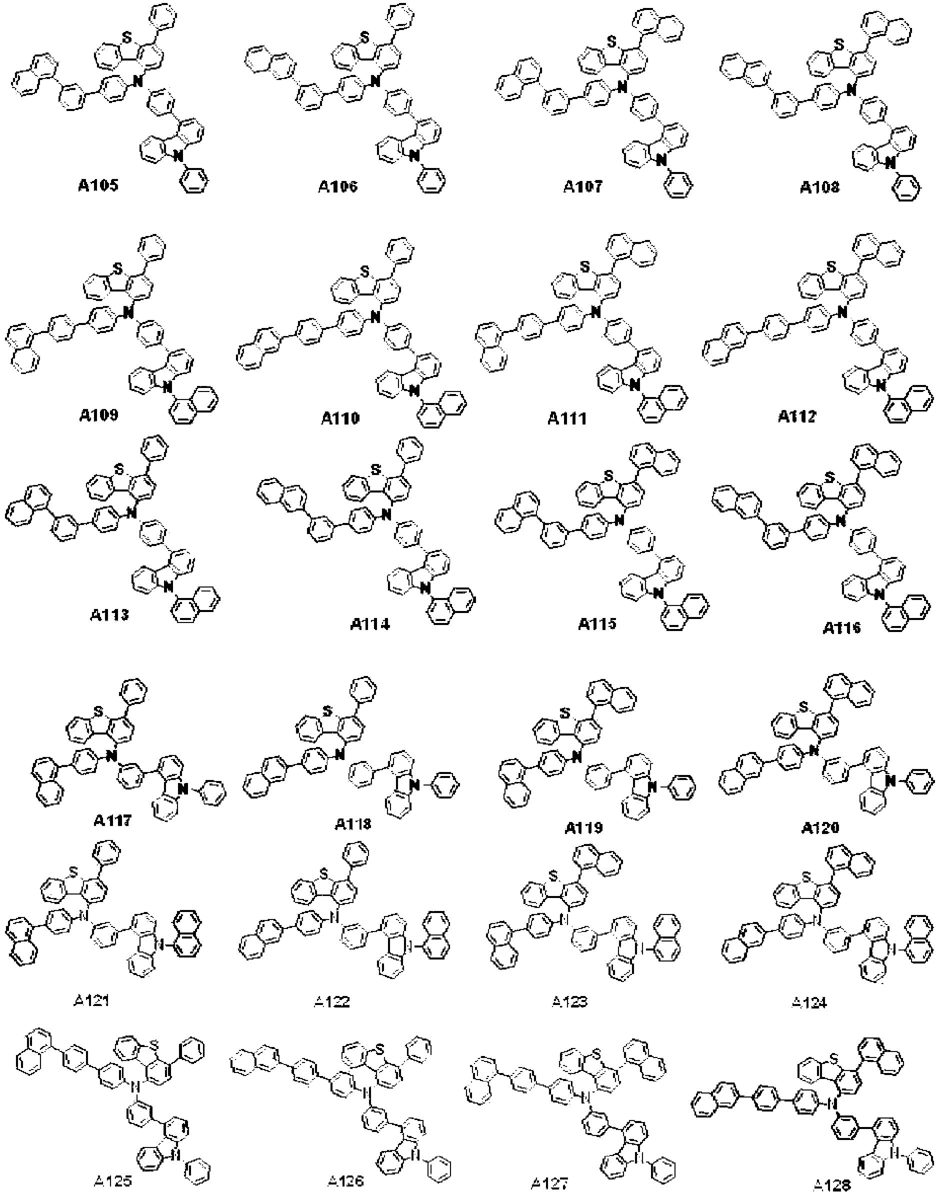 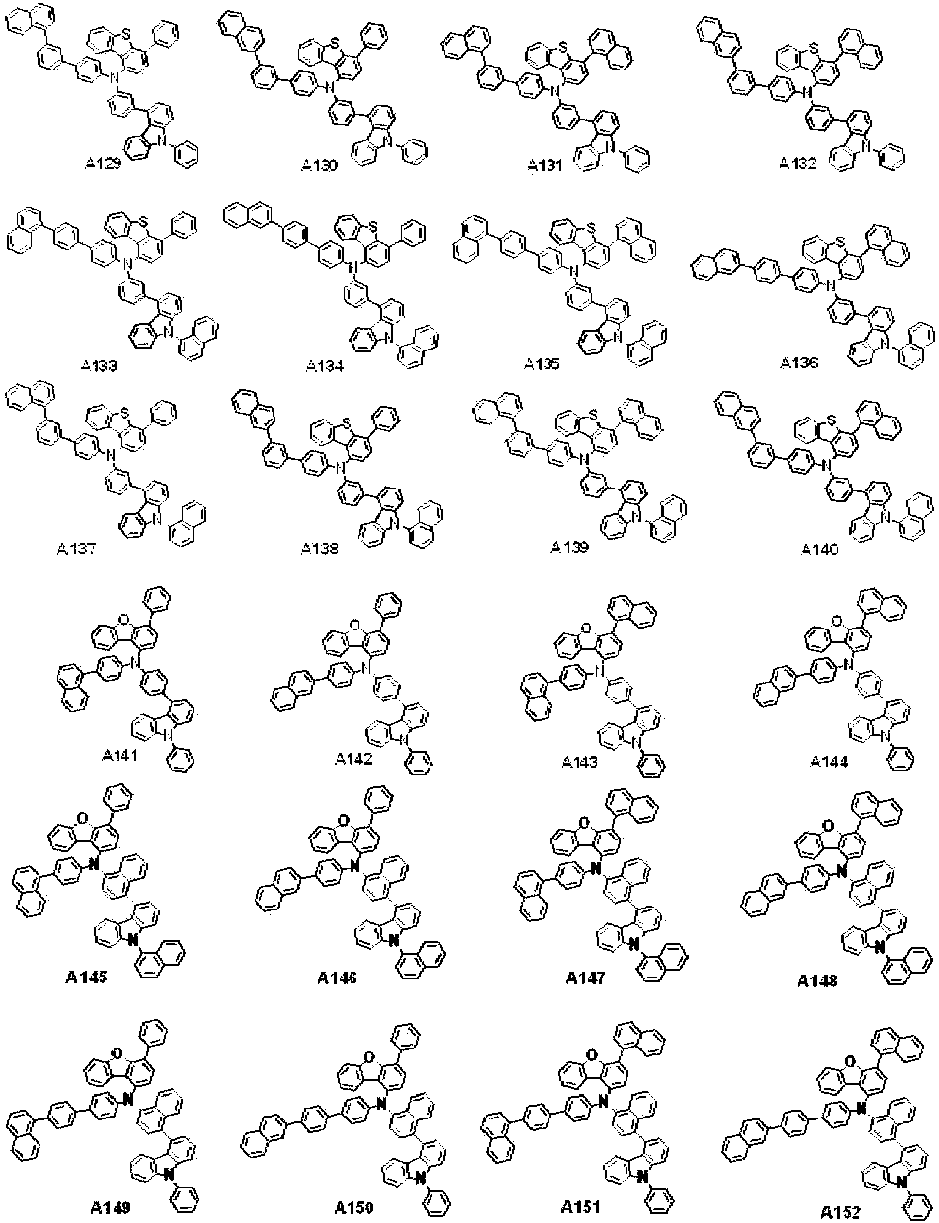 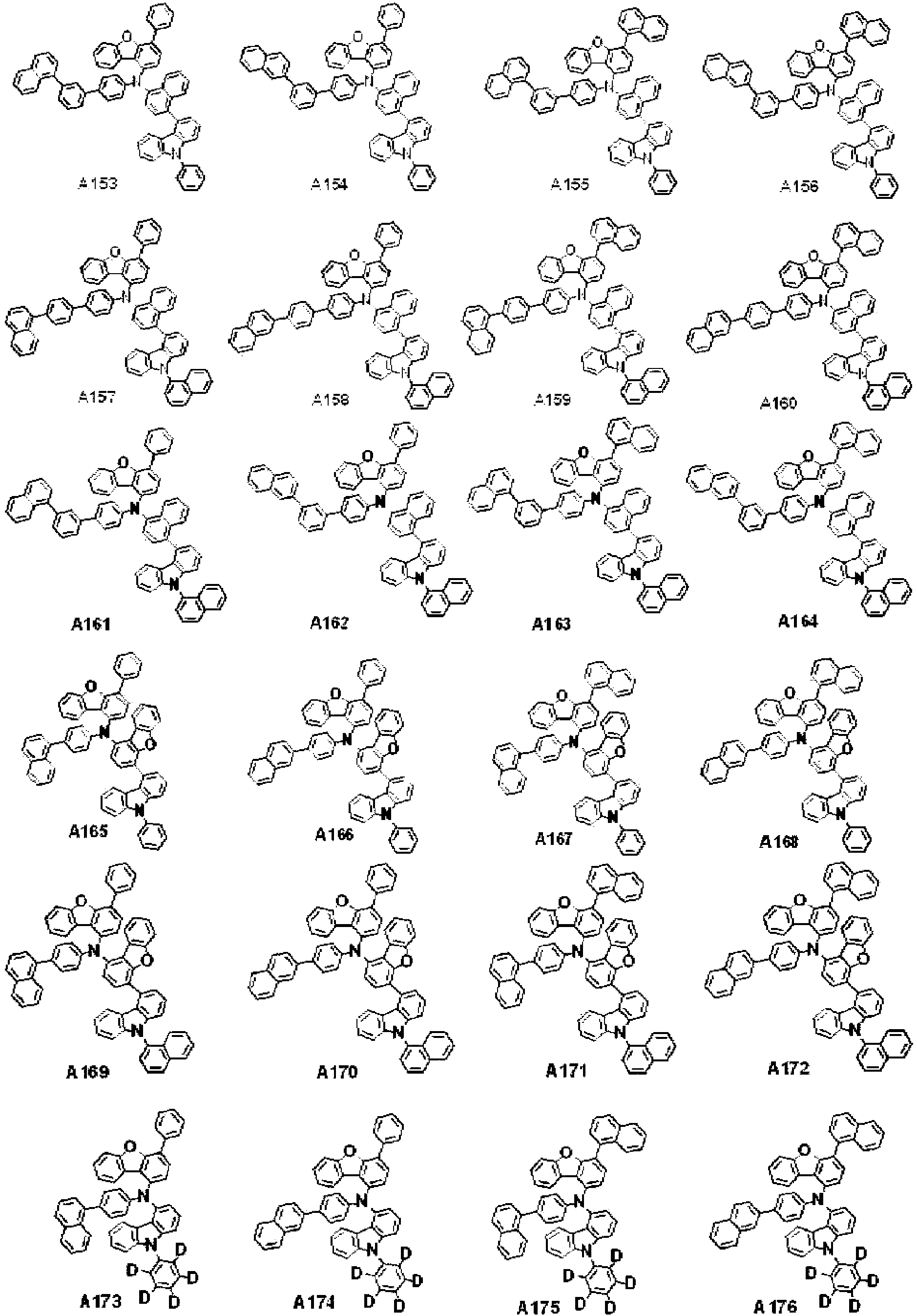 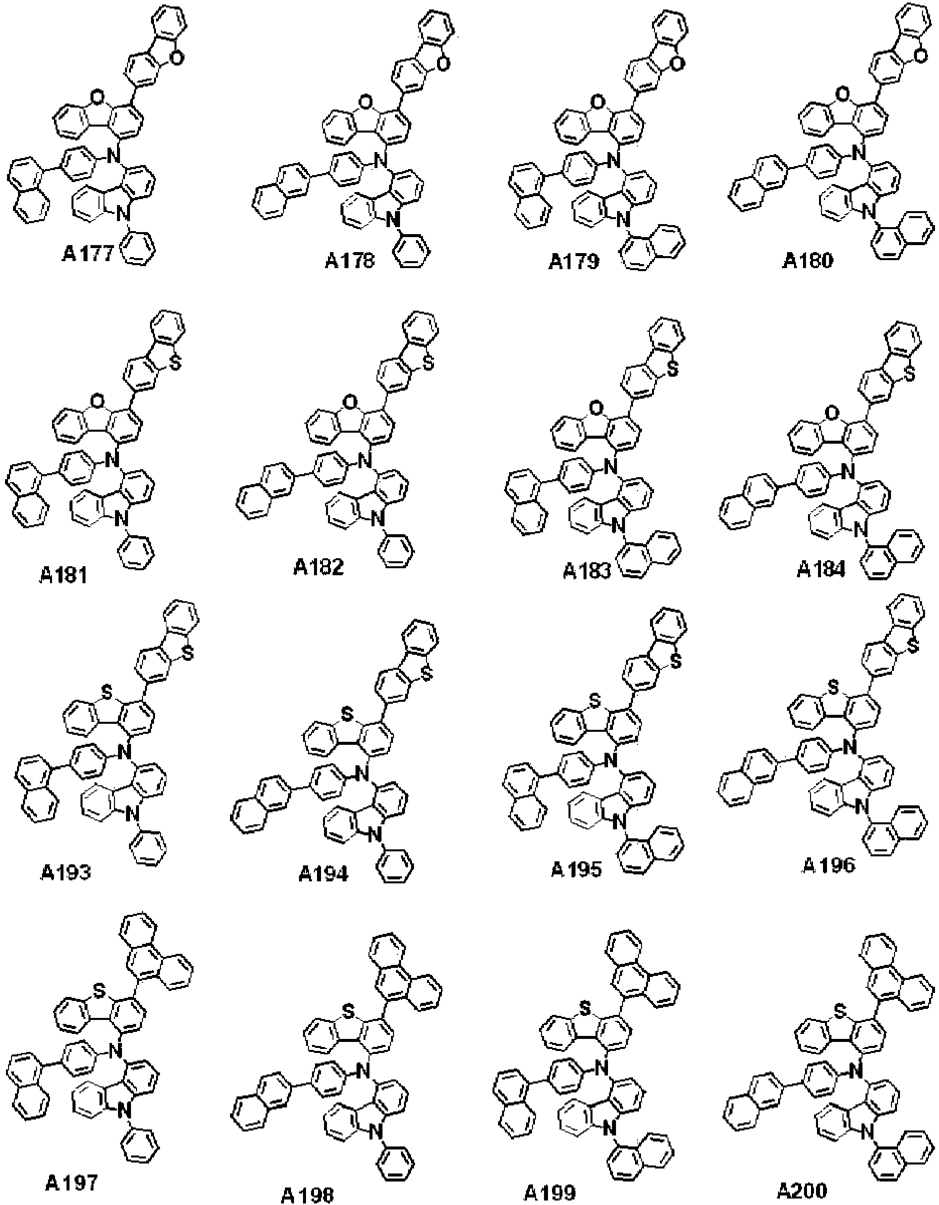 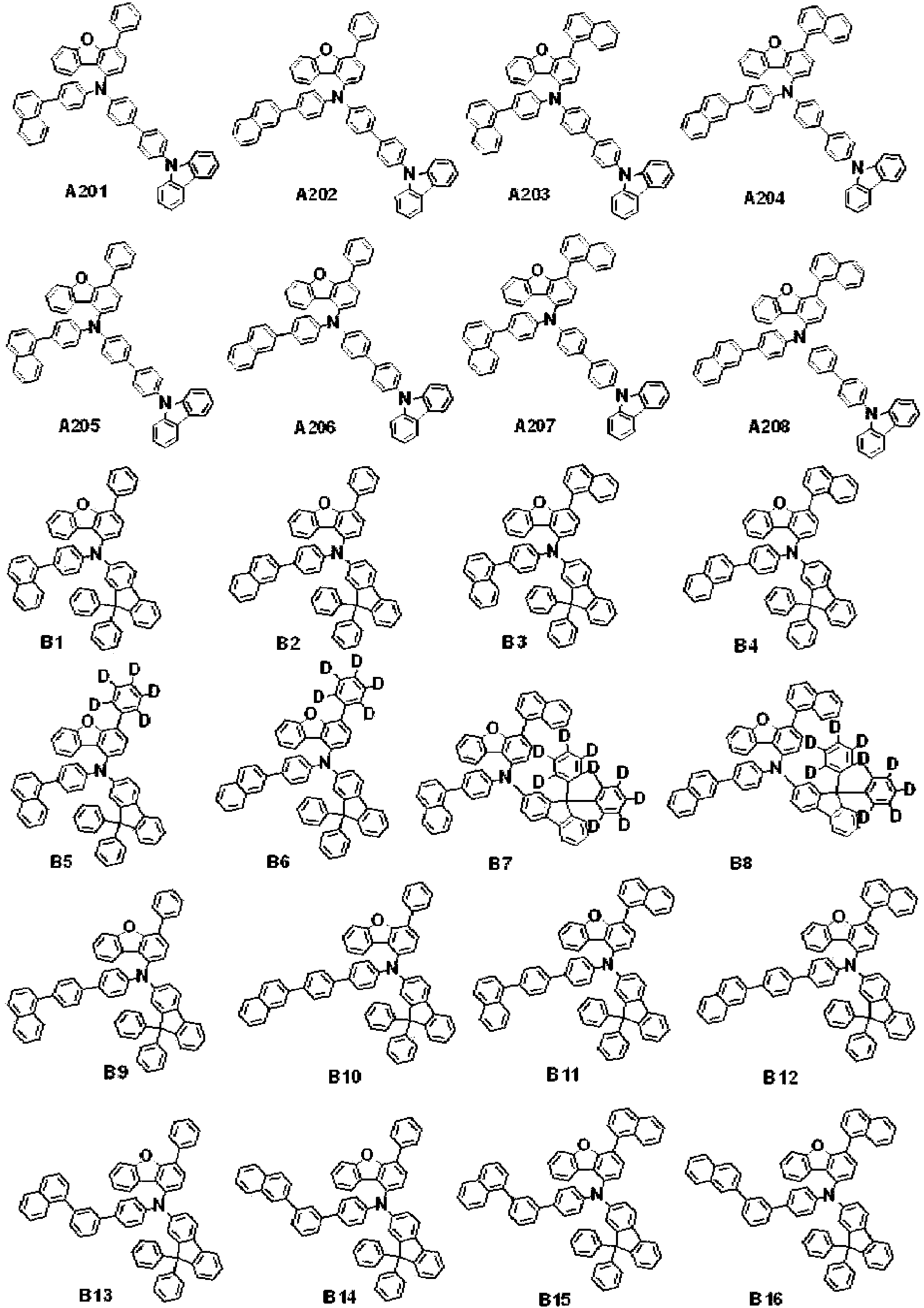 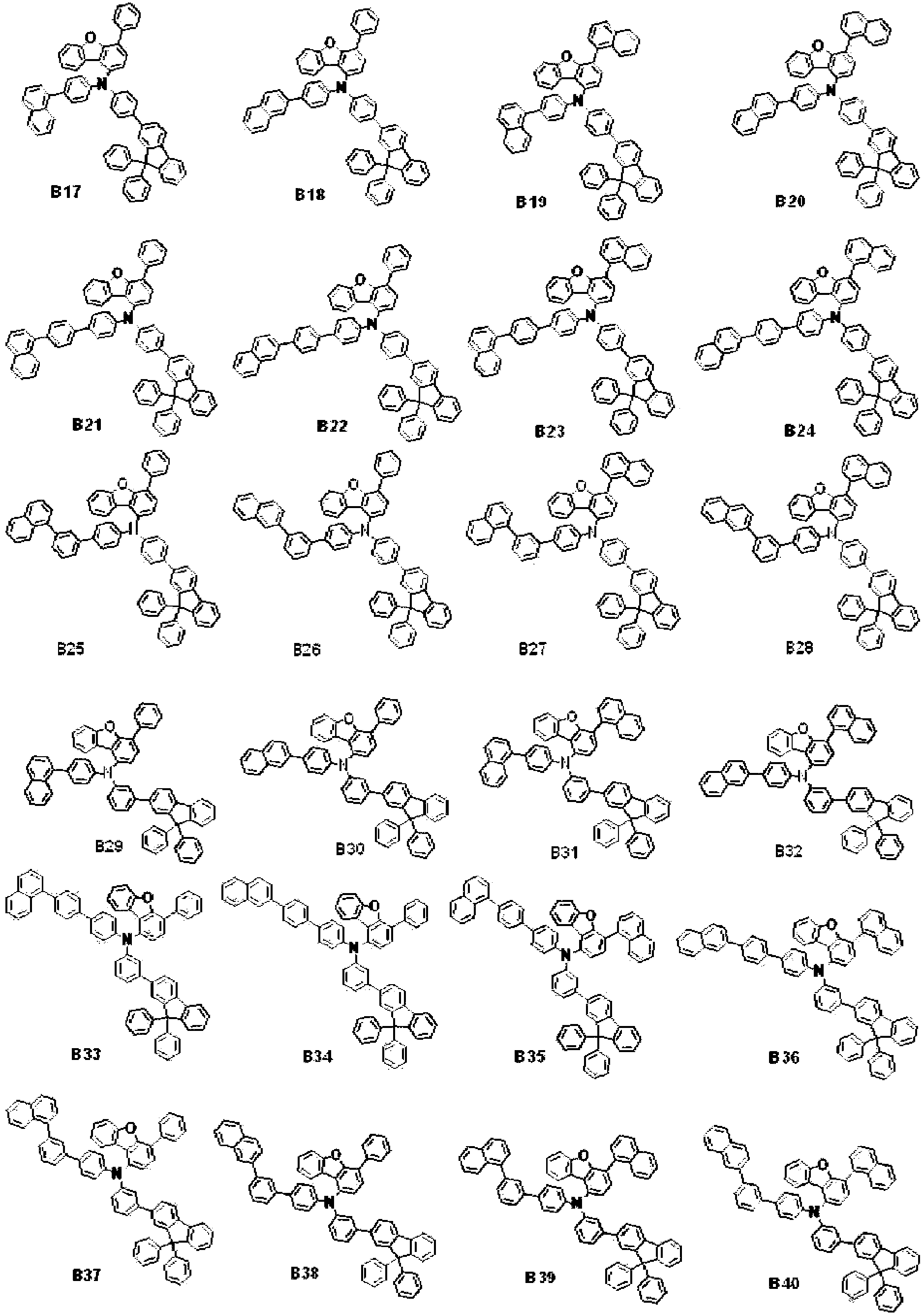 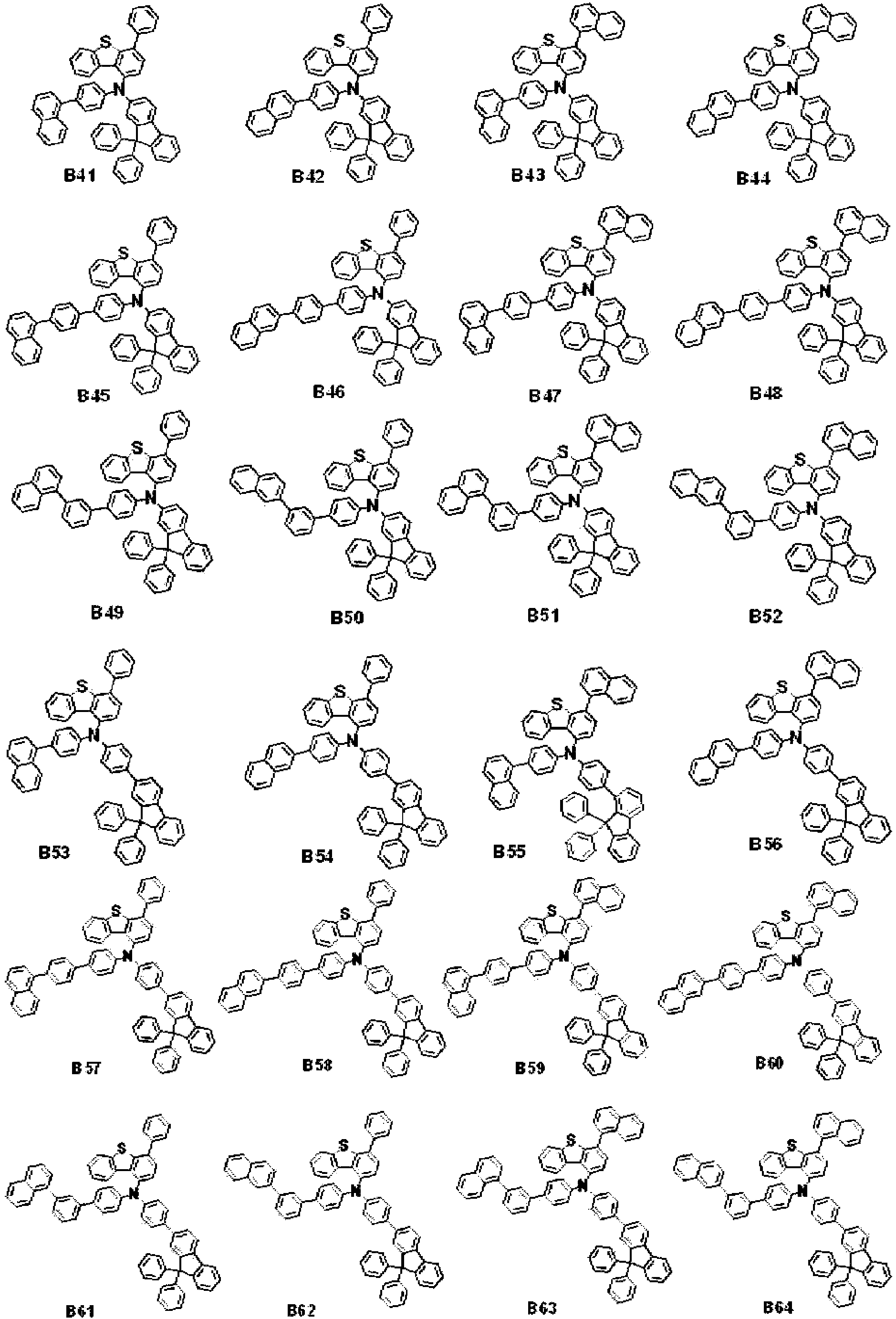 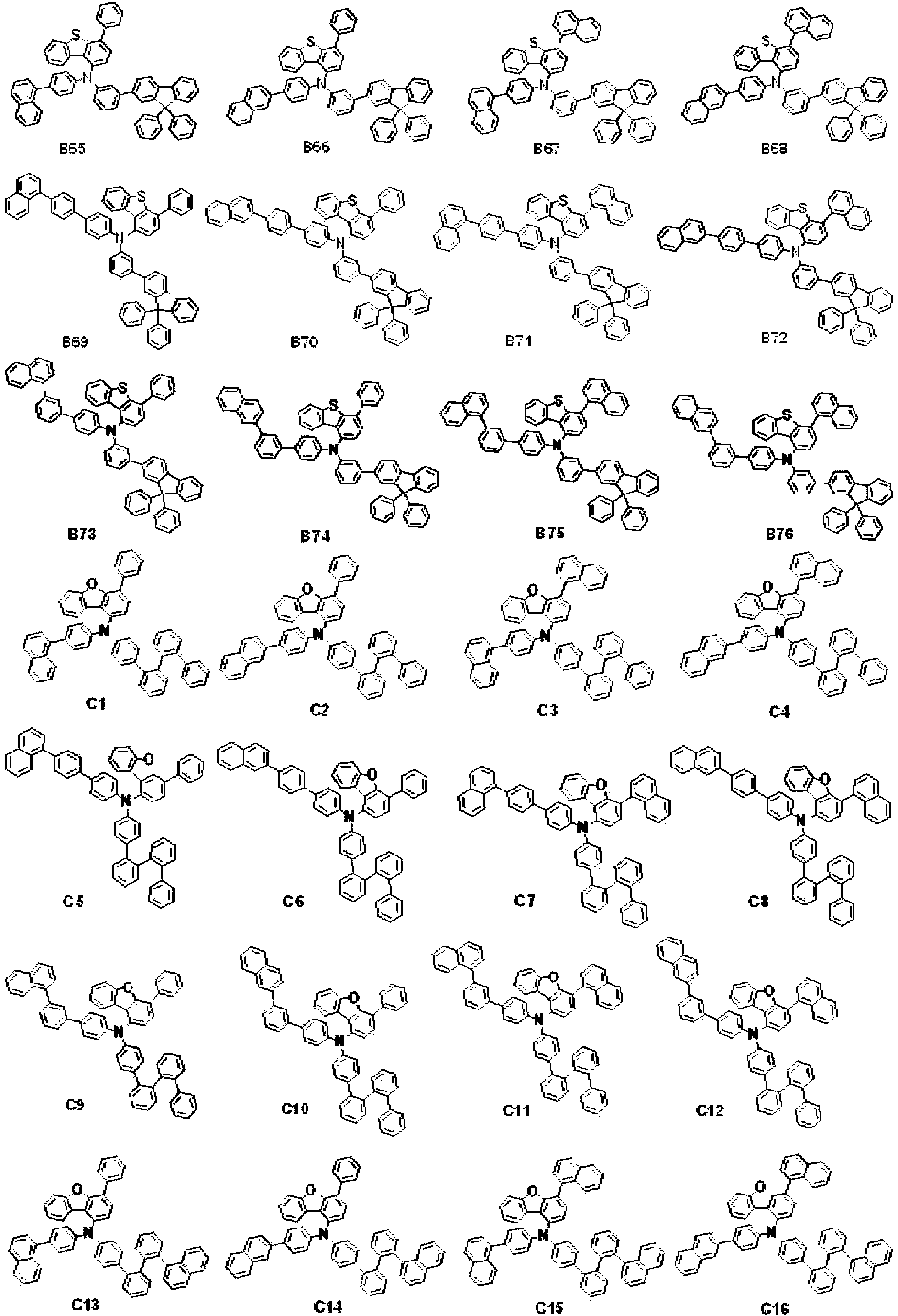 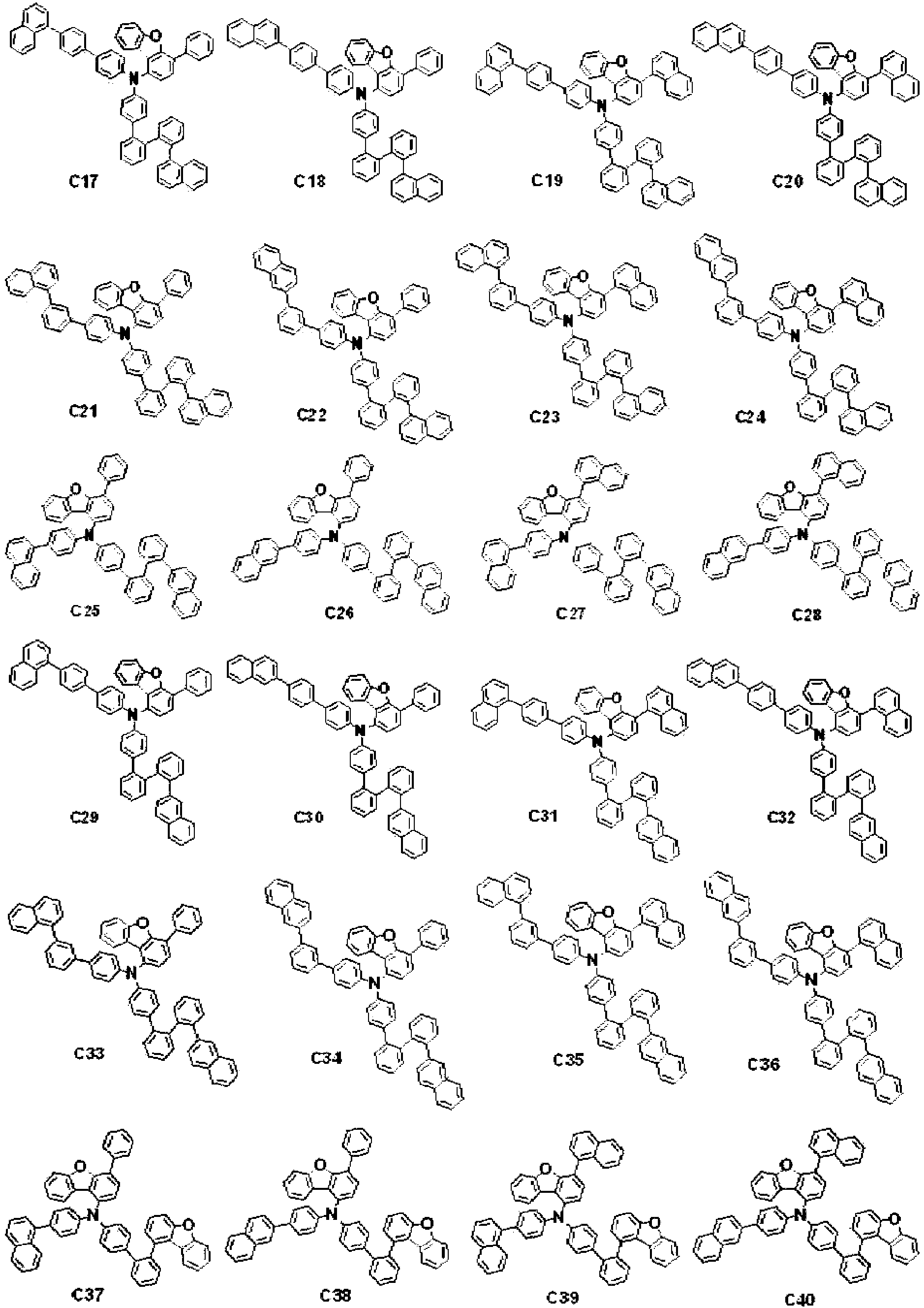 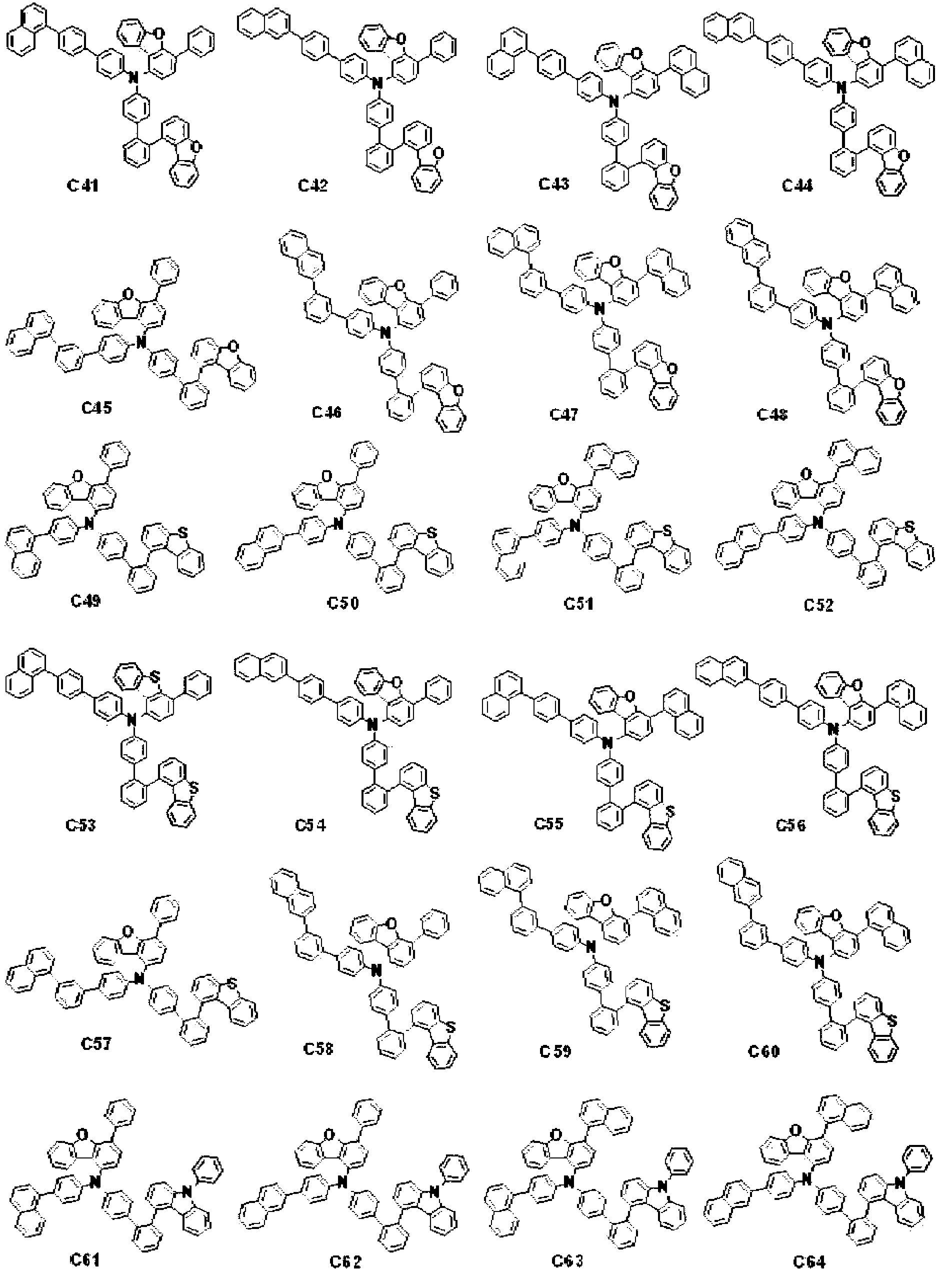 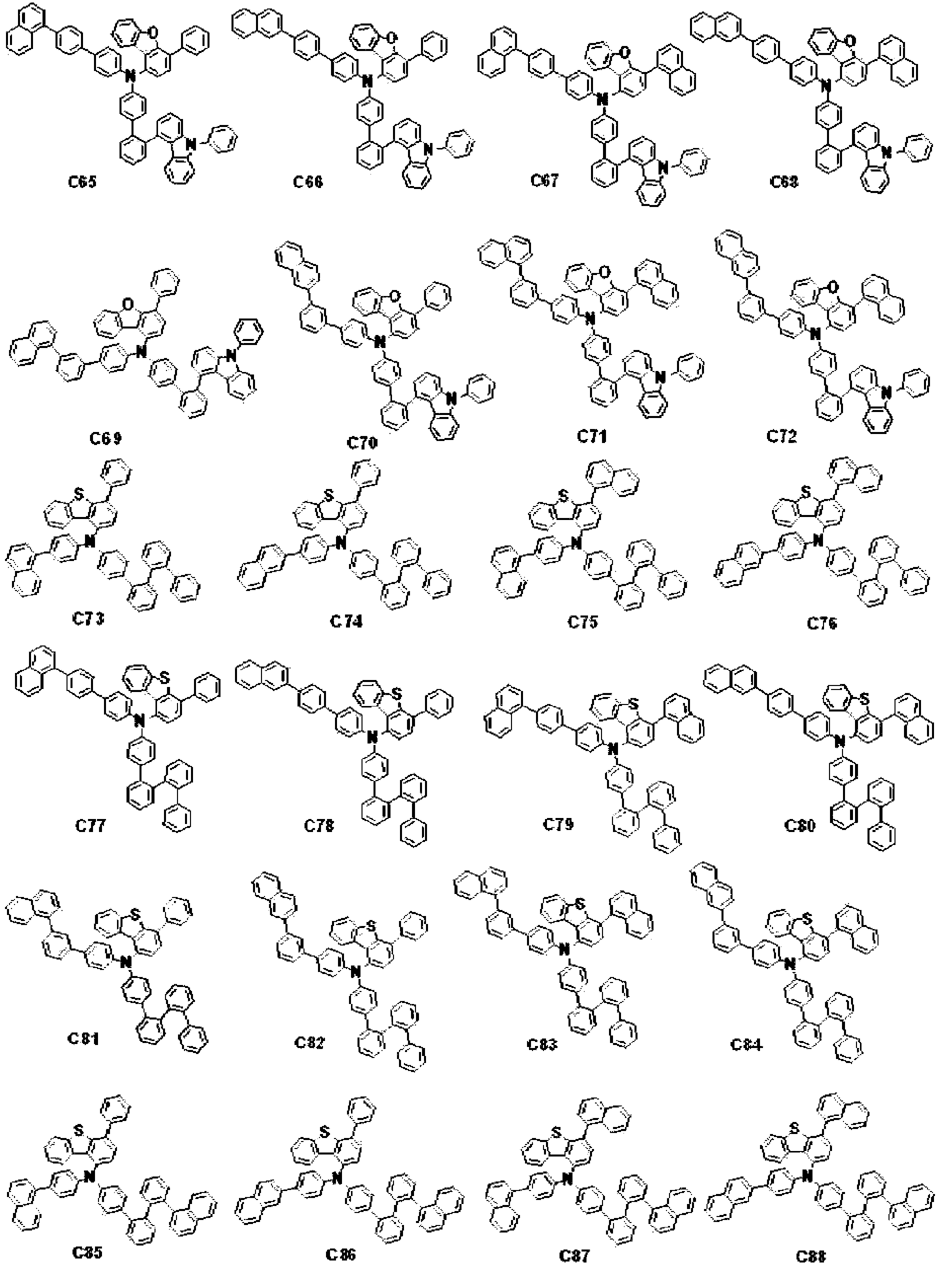 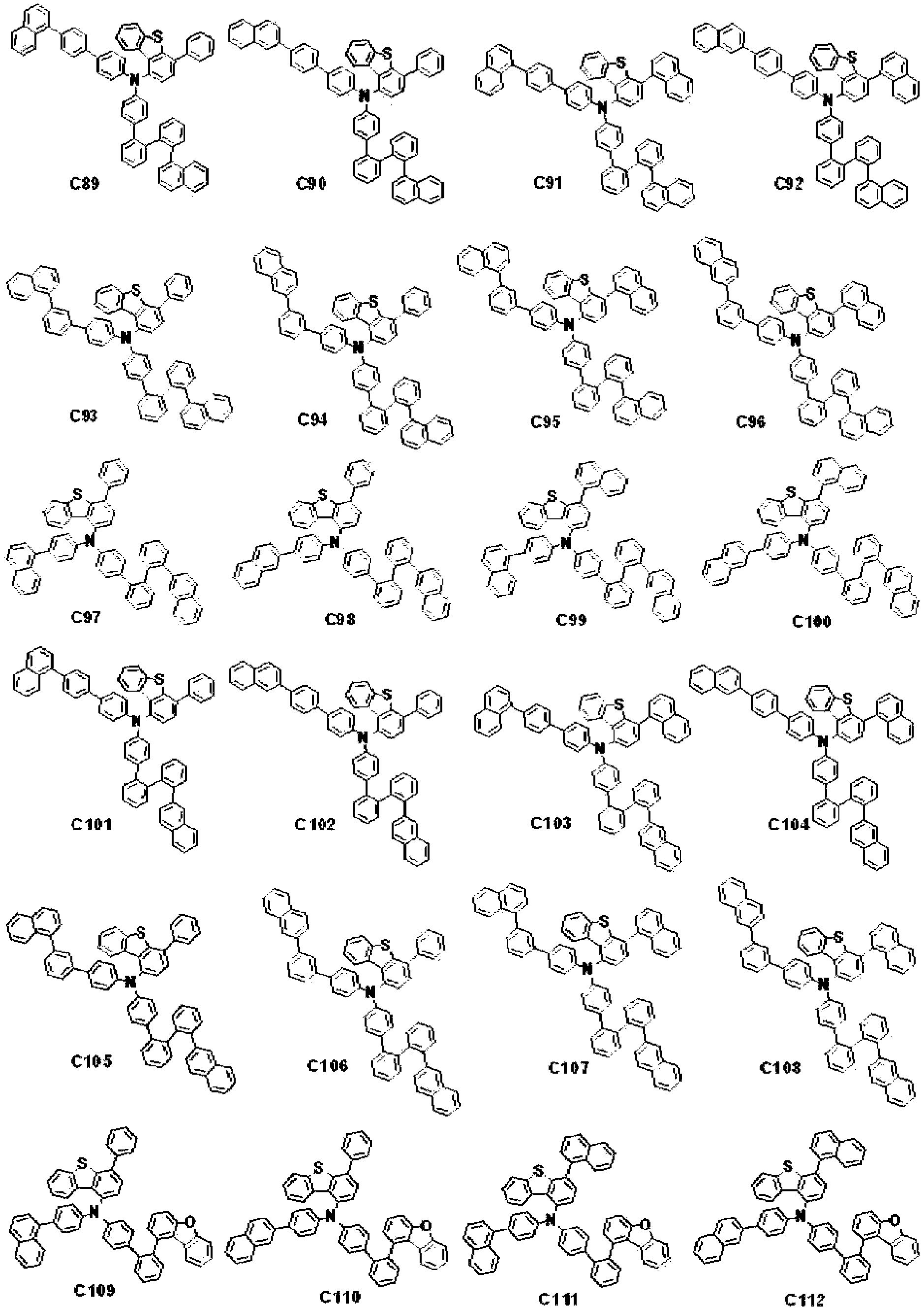 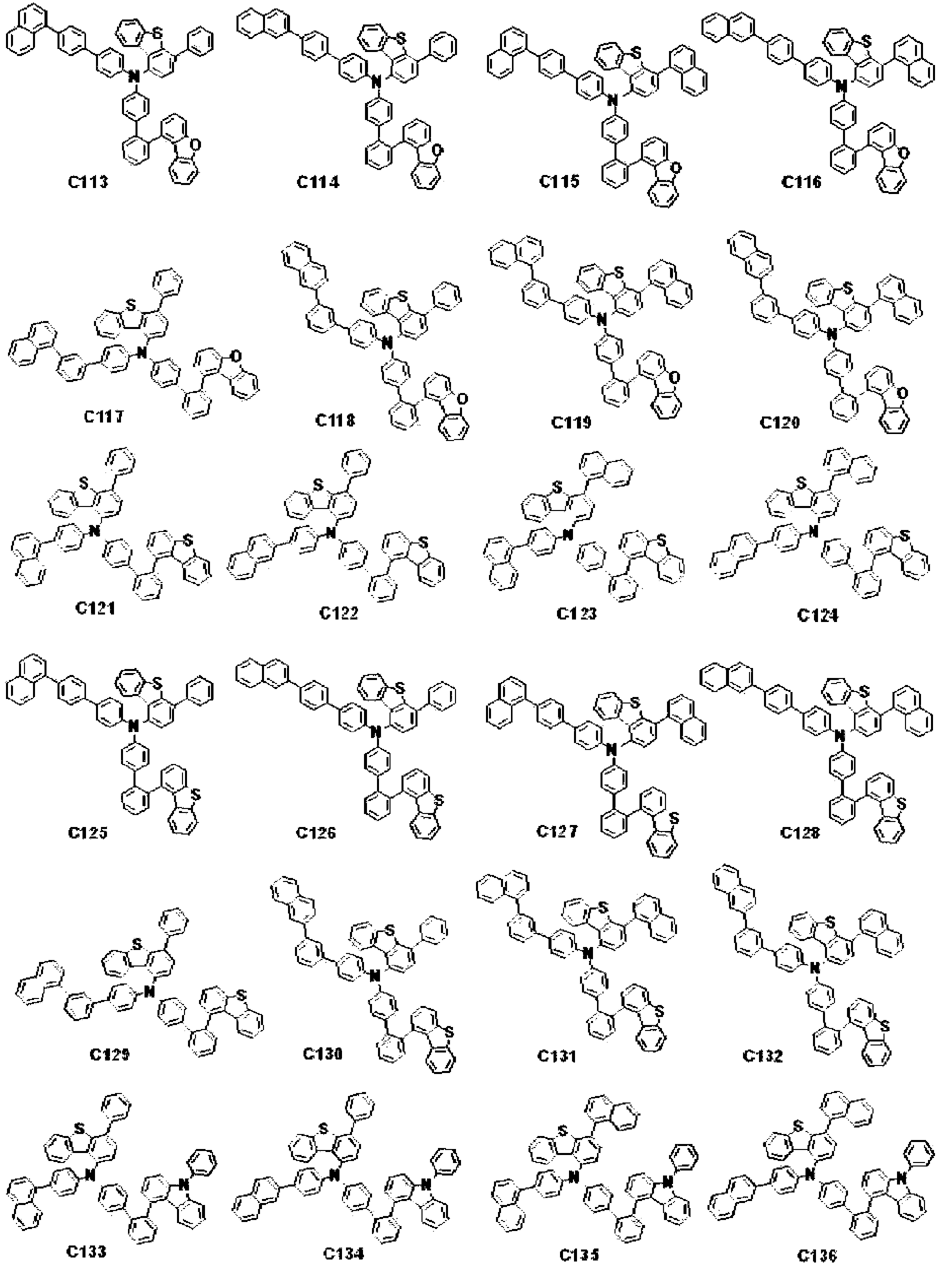 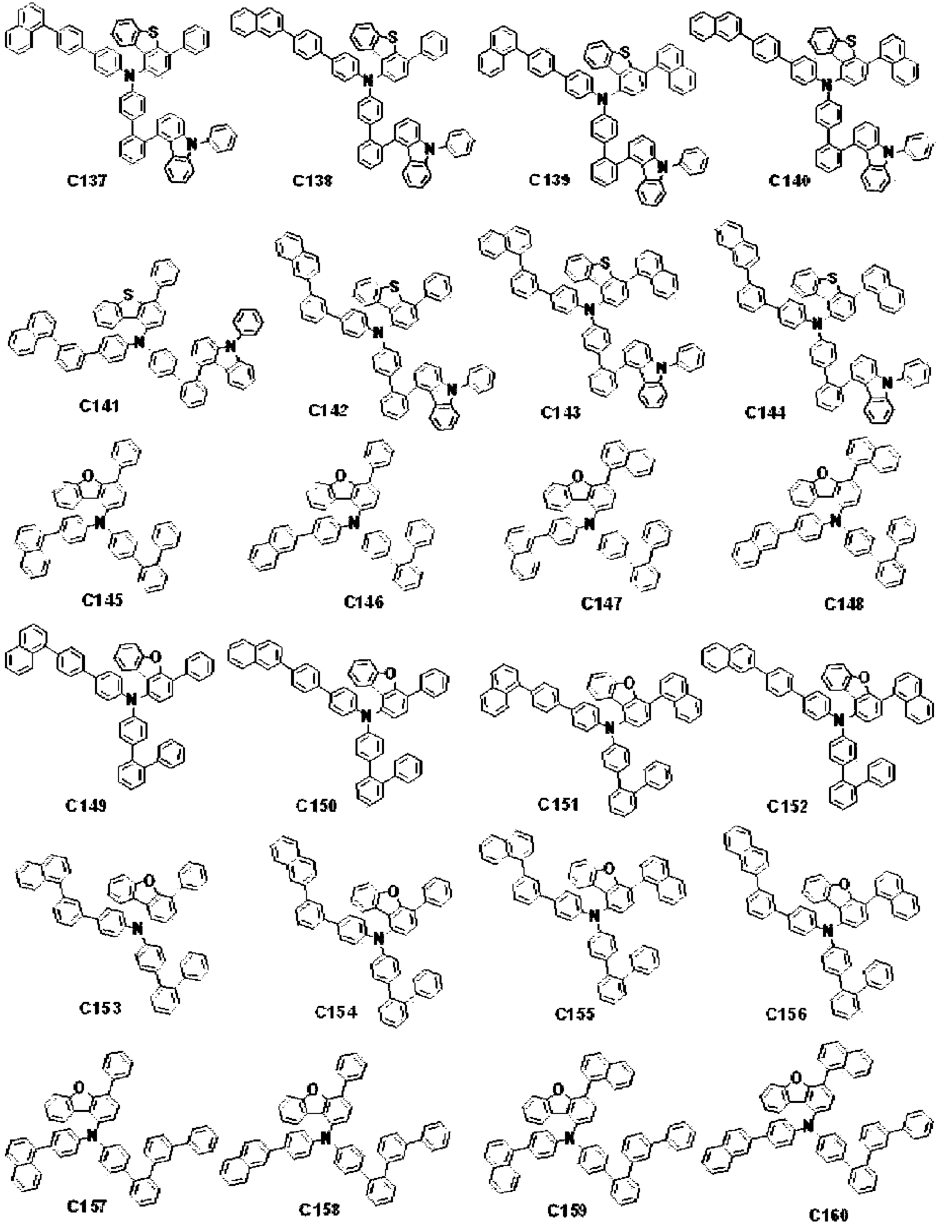 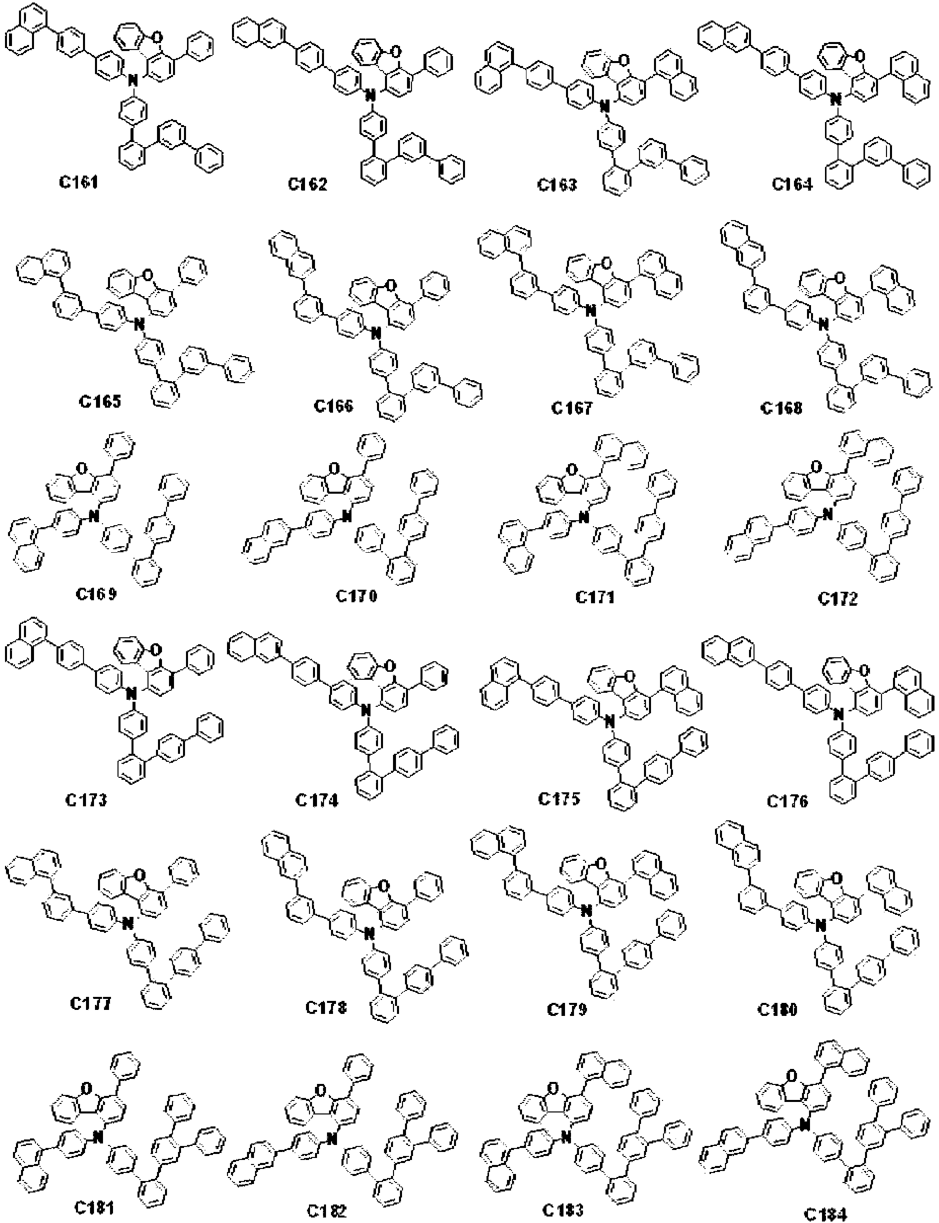 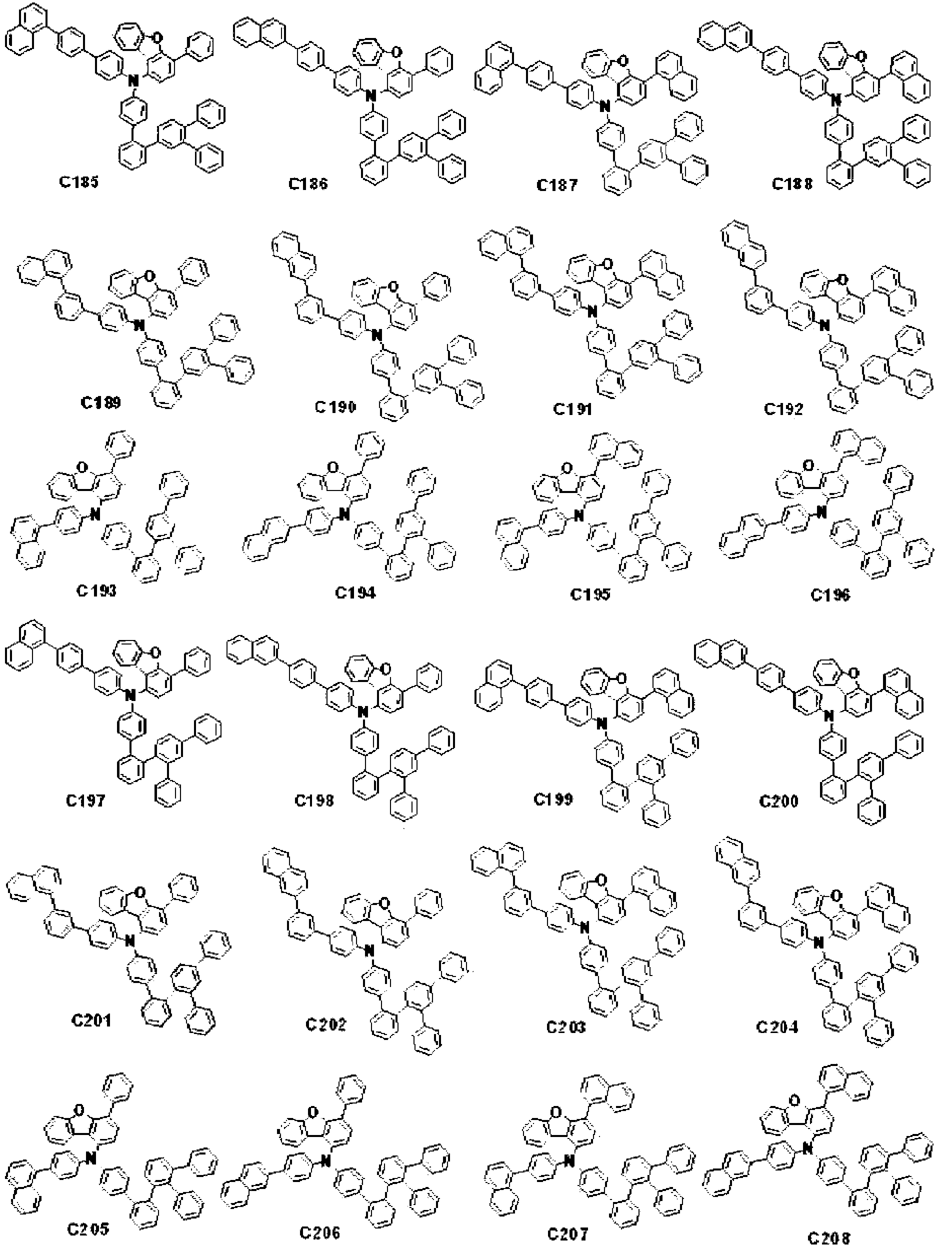 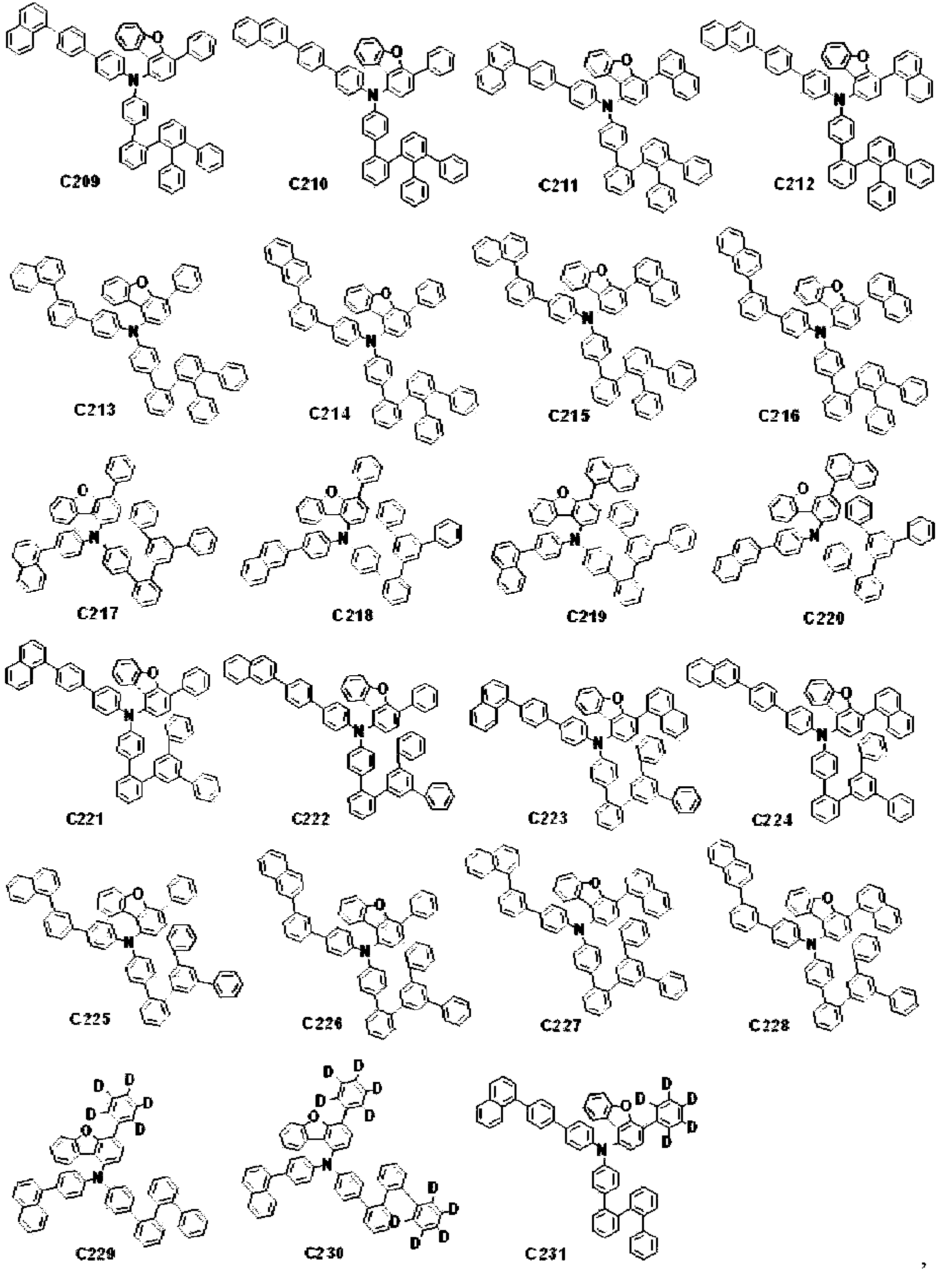 dans lequel, dans le Groupe de composés 1, D est un atome de deutérium.

10. Élément électroluminescent, comprenant :
une première électrode ;
une seconde électrode faisant face à la première électrode ; et
au moins une couche fonctionnelle entre la première électrode et la seconde électrode, et comprenant un composé amine selon l'une quelconque des revendications 1 à 9.

11. Élément électroluminescent de la revendication 10, dans lequel l'au moins une couche fonctionnelle comprend une couche d'émission, une région de transport de trous disposée entre la première électrode et la couche d'émission, et une région de transport d'électrons disposée entre la couche d'émission et la seconde électrode, et
la région de transport de trous comprend le composé amine.

12. Élément électroluminescent de la revendication 11, dans lequel la région de transport de trous comprend au moins l'une d'une couche d'injection de trous, d'une couche de transport de trous et d'une couche de blocage d'électrons, et
l'au moins l'une de la couche d'injection de trous, de la couche de transport de trous et de la couche de blocage d'électrons comprend le composé amine.

13. Élément électroluminescent de la revendication 11, dans lequel la couche d'émission comprend un composé représenté par la Formule E-1 : 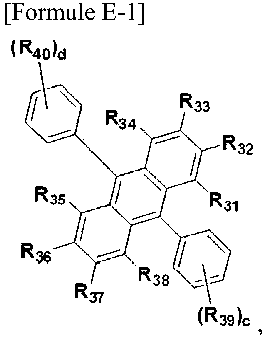
dans lequel, dans la Formule E-1,
« c » et « d » sont chacun indépendamment un nombre entier de 0 à 5, et
R₃₁ à R₄₀ sont chacun indépendamment un atome d'hydrogène, un atome de deutérium, un atome d'halogène, un groupe silyle substitué ou non substitué, un groupe thio substitué ou non substitué, un groupe oxy substitué ou non substitué, un groupe alkyle substitué ou non substitué comportant 1 à 10 atomes de carbone, un groupe alcényle substitué ou non substitué comportant 2 à 10 atomes de carbone, un groupe aryle substitué ou non substitué comportant 6 à 30 atomes de carbone formant un cycle, un groupe hétéroaryle substitué ou non substitué comportant 2 à 30 atomes de carbone formant un cycle, ou combinés avec un groupe adjacent pour former un cycle.
